# EUROPEAN PATENT APPLICATION

(11) **EP 4 467 548 A1**
(43) Date of publication of application: **27.11.2024**
(21) Application number: 23742916.2
(22) Date of filing: 18.01.2023
(51) Int. Cl.: C07D 519/00, C07D 403/14, C07D 403/04, C07D 491/052, A61K 31/517, A61K 31/519, A61P 35/00, A61P 35/02

(54) **BRIDGED RING-SUBSTITUTED HETEROARYL-PYRAN DERIVATIVE, AND USE THEREOF**

(30) Priority: 21.01.2022 CN 202210074707; 24.01.2022 CN 202210082274; 15.03.2022 CN 202210254285; 31.03.2022 CN 202210346571; 07.06.2022 CN 202210642158; 11.07.2022 CN 202210813772; 11.08.2022 CN 202210964127; 09.01.2023 CN 202310029317
(71) Applicant: D3 Bio(Wuxi) Co., Ltd., Wuxi, JiangSu, 214092 (CN)
(72) Inventor: ZHANG, Yang, Shanghai 200131 (CN); WU, Wentao, Shanghai 200131 (CN); LI, Zhixiang, Shanghai 200131 (CN); ZHU, Wenyuan, Shanghai 200131 (CN); YANG, Ping, Shanghai 200131 (CN); LI, Qiu, Shanghai 200131 (CN); LI, Jian, Shanghai 200131 (CN); CHEN, Shuhui, Shanghai 200131 (CN)
(74) Representative: Cabinet Becker et Associés
(86) International application number: PCT/CN2023/072798
(87) International publication number: WO 2023/138601

(57) **Abstract**

The present invention discloses a bridged ring-substituted heteroaryl-pyran derivative and a use thereof, and in particular discloses a compound as represented in formula (VI) or a pharmaceutically acceptable salt thereof.

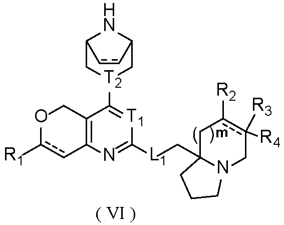

## Description

**This application claims the priority of:**
CN202210074707.1, filed on January 21, 2022;
CN202210082274.4, filed on January 24, 2022;
CN202210254285.6, filed on March 15, 2022;
CN202210346571.5, filed on March 31, 2022;
CN202210642158.3, filed on June 7, 2022;
CN202210813772.1, filed on July 11, 2022;
CN202210964127.X, filed on August 11, 2022;
CN 202310029317.7, filed on January 9, 2023.

### TECHNICAL FIELD

The present disclosure relates to a class of bridged ring-substituted heteroaryl-fused pyran derivatives and an application thereof, specifically to a compound represented by formula (VI) or a pharmaceutically acceptable salt thereof.

### BACKGROUND

RAS oncogene mutations are the most common activating mutations in human cancers, occurring in 30% of human tumors. The RAS gene family includes three subtypes (KRAS, HRAS, and NRAS), and 85% of RAS-driven cancers are caused by mutations in KRAS subtypes. KRAS mutations are commonly found in solid tumors, such as lung adenocarcinoma, pancreatic ductal carcinoma and colorectal cancer, etc. In KRAS mutated tumors, 80% of oncogenic mutations occur at codon 12, and the most common mutations include: p.G12D (41%), p.G12V (28%), and p.G12C (14%).

KRAS is a murine sarcoma virus oncogene and is an important member of the RAS protein. KRAS is like a molecular switch, which can control and regulate the path of cell growth when it is normal. After mutation, KRAS gene can independently transmit signals for growth and proliferation to downstream pathways independent of upstream growth factor receptor signals, causing uncontrolled cell growth and tumor progression. Meanwhile, whether KRAS gene has mutations or not is also an important indicator of tumor prognosis.

Currently, small molecules that directly target KRAS mutations are mainly concentrated in the field of KRAS^{G12C}, wherein Amgen's AMG510 and Mirati Therapeutics' MRTX849 have shown good therapeutic effects on tumor patients with KRAS^{G12C} mutation in clinical studies. But so far, no small molecule that targets KRAS^{G12D} enters a clinical research stage, and tumor patients with KRAS^{G12D} mutation have not yet benefited from precision medicine.

The present application discloses a series of small molecule inhibitors targeting KRAS^{G12D} and a preparation method thereof.

### SUMMARY

The present disclosure provides a compound represented by formula (VI) or a pharmaceutically acceptable salt thereof, 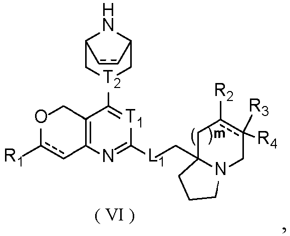 wherein
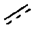 is selected from a single bond and a double bond;
T₁ is selected from CRₐ and N;
T₂ is selected from CH and N;
L₁ is selected from O and S;
R₁ is selected from phenyl, naphthyl, 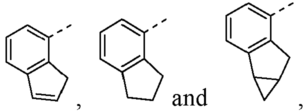 wherein the phenyl, naphthyl, 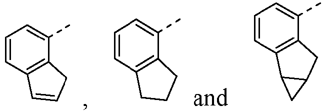 are each independently optionally substituted with 1, 2, 3, 4 or 5 R_{b};
R₂ is H;
R₃ is selected from H, F, CN, CH₃ and OCH₃, wherein the CH₃ and OCH₃ are optionally substituted with 1, 2 or 3 halogens;
alternatively, R₂ and R₃ together with the atoms to which they are attached form a phenyl group or a 5-6 membered heteroaryl group, wherein the phenyl group or the 5-6 membered heteroaryl group is optionally substituted with 1, 2 or 3 halogens;
R₄ is absent or is selected from H, CH₃ and -CH=CH₂, wherein the CH₃ and -CH=CH₂ are optionally substituted with 1, 2 or 3 halogens;
alternatively, R₃ and R₄ together with the atom to which they are attached form 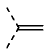 , wherein the 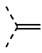 is optionally substituted with 1 or 2 R_{c};
alternatively, R₃ and R₄ together with the atom to which they are attached form a cyclopropyl group, which is optionally substituted with 1, 2 or 3 halogens;
Rₐ is selected from H and CN;
each R_{b} is independently selected from H, F, Cl, Br, I, OH, NH₂, CN, C₁₋₃ alkyl, C₁₋₃ alkoxy, C₂₋₄ alkynyl, C₂₋₄ alkynyl-cyclopropyl, C₂₋₃ alkenyl, -C(=O)C₁₋₃ alkyl and C₃₋₅ cycloalkyl, wherein the C₁₋₃ alkyl, C₁₋₃ alkoxy, C₂₋₄ alkynyl, C₂₋₄ alkynyl-cyclopropyl, C₂₋₃ alkenyl, -C(=O)C₁₋₃ alkyl and C₃₋₅ cycloalkyl are optionally substituted with 1, 2, 3, 4 or 5 halogens;
each R_{c} is independently selected from F, Cl, Br, I, CN, CH₃ and OCH₃;
m is selected from 0 and 1.

The present disclosure also provides a compound represented by formula (P-1) or a pharmaceutically acceptable salt thereof, 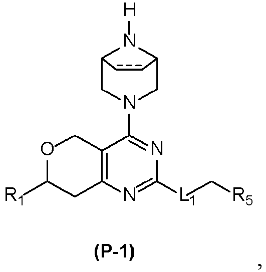 wherein
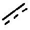 is selected from a single bond and a double bond;
L₁ is selected from O and S;
R₁ is selected from phenyl, naphthyl, 5-10 membered heteroaryl, 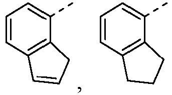 and 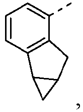 wherein the phenyl, naphthyl, 5-10 membered heteroaryl, 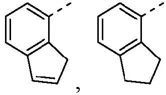 and 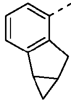 are each independently optionally substituted with 1, 2, 3, 4 or 5 R_{b}, and R₅ is selected from 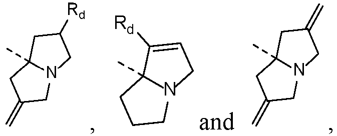 wherein the 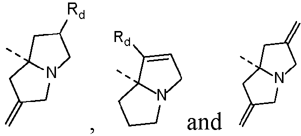 are each independently optionally substituted with 1, 2, 3, 4 or 5 R_{d};
alternatively, R₁ is 5-10 membered heteroaryl, wherein the 5-10 membered heteroaryl is optionally substituted with 1, 2, 3, 4 or 5 R_{b}, and R₅ is 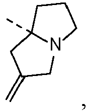 wherein the 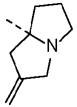 is optionally substituted with 1, 2, 3, 4 or 5 R_{d};
each R_{b} is independently selected from H, F, Cl, Br, I, OH, NH₂, CN, C₁₋₃ alkyl, C₁₋₃ alkoxy, C₂₋₄ alkynyl, C₂₋₄ alkynyl-cyclopropyl, C₂₋₃ alkenyl, -C(=O)C₁₋₃ alkyl and C₃₋₅ cycloalkyl, wherein the C₁₋₃ alkyl, C₁₋₃ alkoxy, C₂₋₄ alkynyl, C₂₋₄ alkynyl-cyclopropyl, C₂₋₃ alkenyl, -C(=O)C₁₋₃ alkyl and C₃₋₅ cycloalkyl are optionally substituted with 1, 2, 3, 4 or 5 R;
each R_{d} is independently selected from F, Cl, Br, I, CN, CH₃ and OCH₃,
each R is independently selected from halogen and D.

The present disclosure also provides a compound represented by formula (P-2) or a pharmaceutically acceptable salt thereof, 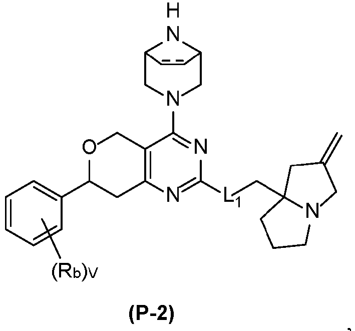 wherein
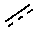 is selected from a single bond and a double bond;
L₁ is selected from O and S;
each R_{b} is independently selected from F, Cl, Br, I, OH, NH₂, CN, C₁₋₃ alkyl, C₁₋₃ alkoxy, C₂₋₄ alkynyl, C₂₋₄ alkynyl-cyclopropyl, C₂₋₃ alkenyl, -C(=O)C₁₋₃ alkyl and C₃₋₅ cycloalkyl, wherein the C₁₋₃ alkyl, C₁₋₃ alkoxy, C₂₋₄ alkynyl, C₂₋₄ alkynyl-cyclopropyl, C₂₋₃ alkenyl, -C(=O)C₁₋₃ alkyl and C₃₋₅ cycloalkyl are optionally substituted with 1, 2, 3, 4 or 5 R;
each R is independently selected from halogen and D;
v is selected from 1, 2, 3, 4 and 5;
provided that at least one R_{b} is substituted with 1, 2, 3, 4 or 5 D.

In some embodiments of the present disclosure, each R_{b} is independently selected from H, F, Cl, Br, I, OH, NH₂, CN, CH₃, CH₂CH₃, OCH₃, OCH₂CH₃, -CH=CH₂, C≡CH, -C≡CCH₃, -C≡CCH₂CH₃, 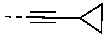 and cyclopropyl, wherein the CH₃, CH₂CH₃, OCH₃, OCH₂CH₃, -CH=CH₂, -C≡CH, -C≡CCH₃, -C≡CCH₂CH₃, 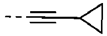 and cyclopropyl are optionally substituted with 1, 2, 3, 4 or 5 halogens, and other variables are as defined herein.

In some embodiments of the present disclosure, each R_{b} is independently selected from H, F, Cl, Br, I, OH, NH₂, CN, CH₃, CH₂F, CF₂H, CF₃, CH₂CH₃, CF₂CF₃, -CH=CH₂, -C≡CH, -C≡CF, -C≡CBr, -C≡CCH₃, -C≡CCF₃, -C≡CCH₂CF₃, 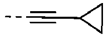 and cyclopropyl, and other variables are as defined herein.

In some embodiments of the present disclosure, each R_{b} is independently selected from H, F, Cl, Br, I, OH, NH₂, CN, CH₃, CH₂CH₃, OCH₃, OCH₂CH₃, -CH=CH₂, -C≡CH, -C≡CCH₃, -C≡CCH₂CH₃, 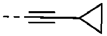 and cyclopropyl, wherein the CH₃, CH₂CH₃, OCH₃, OCH₂CH₃, -CH=CH₂, -C≡CH, -C≡CCH₃, -C≡CCH₂CH₃, 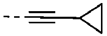 and cyclopropyl are optionally substituted with 1, 2, 3, 4 or 5 R, and other variables are as defined herein.

In some embodiments of the present disclosure, each R_{b} is independently selected from H, F, Cl, Br, I, OH, NH₂, CN, CH₃, CD₃, CH₂F, CF₂H, CF₃, CH₂CH₃, CF₂CF₃, -CH=CH₂, -C≡CH, -C≡CF, -C≡CBr, -C≡CCH₃, -C≡CCH₂CH₃, 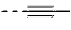 CD₃, -C≡CCF₃, -C≡CCH₂CH₃, 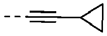 and cyclopropyl, and other variables are as defined herein.

In some embodiments of the present disclosure, the R₁ is selected from 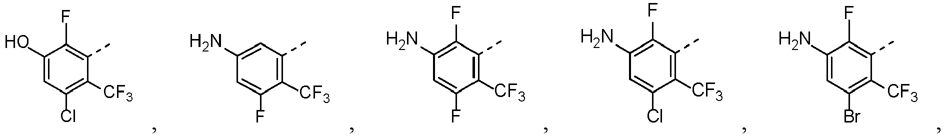 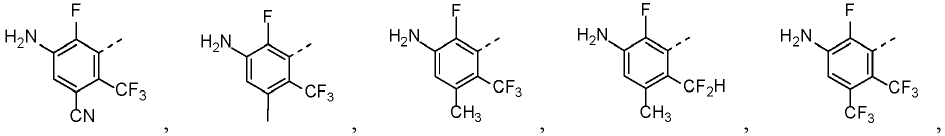 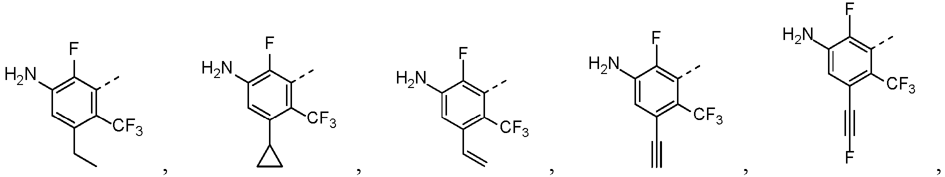 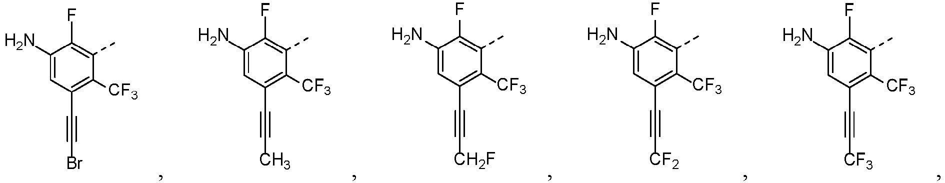 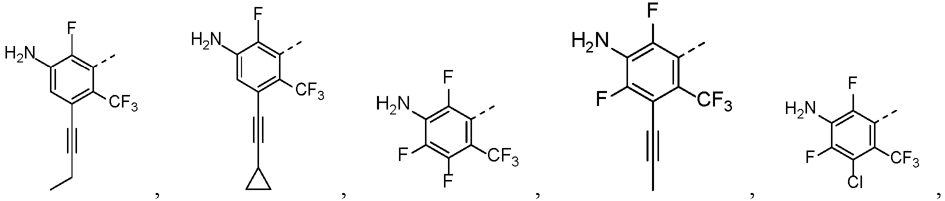 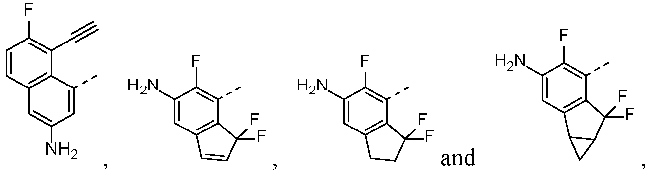 and other variables are as defined herein.

In some embodiments of the present disclosure, the R₁ is selected from 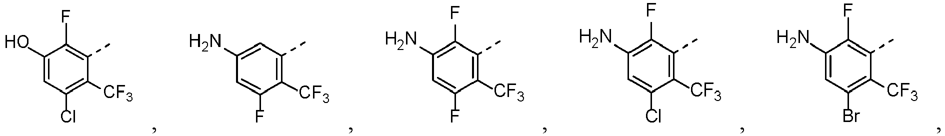 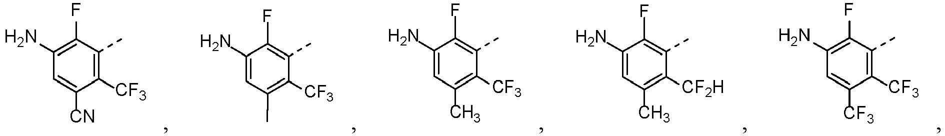 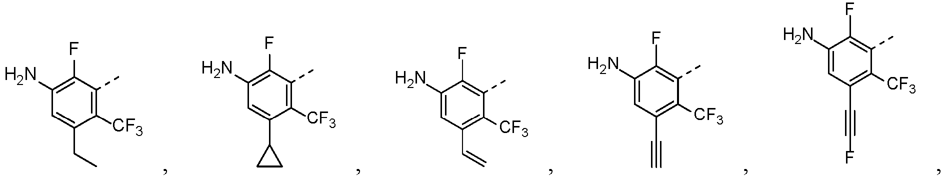 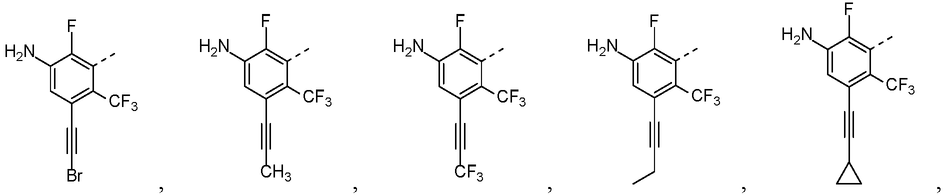 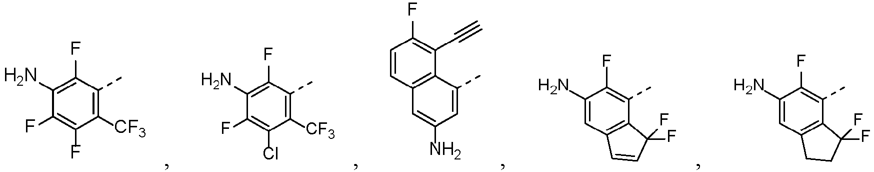 and 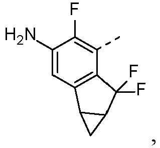 and other variables are as defined herein.

In some embodiments of the present disclosure, the R₁ is selected from 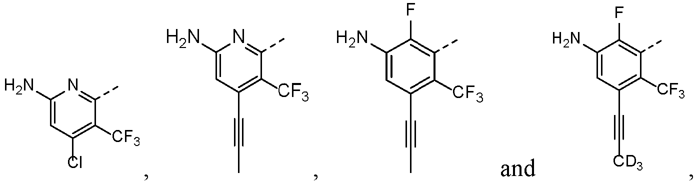 and other variables are as defined herein.

In some embodiments of the present disclosure, the structural moiety 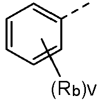 is 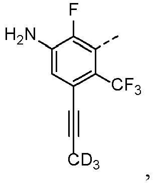 and other variables are as defined herein.

In some embodiments of the present disclosure, the R₃ is selected from H, F, CN, CH₃ and OCH₃, and other variables are as defined herein.

In some embodiments of the present disclosure, the R₂ and R₃ together with the atoms to which they are attached form phenyl, furanyl and pyridinyl, wherein the phenyl, furanyl and pyridinyl are optionally substituted with one F, and other variables are as defined herein.

In some embodiments of the present disclosure, the R₄ is absent or is selected from H, CH₂F and -CH=CH₂, and other variables are as defined herein.

In some embodiments of the present disclosure, the R₃ and R₄ together with the atom to which they are attached form 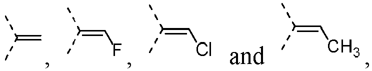 and other variables are as defined herein.

In some embodiments of the present disclosure, the R₃ and R₄ together with the atom to which they are attached form 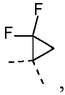 and other variables are as defined herein.

In some embodiments of the present disclosure, the R₅ is selected from 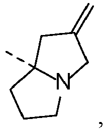 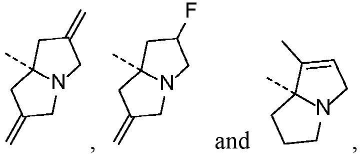 and other variables are as defined herein.

In some embodiments of the present disclosure, the structural moiety 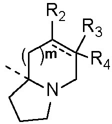 is selected from 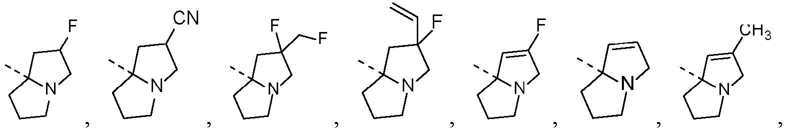 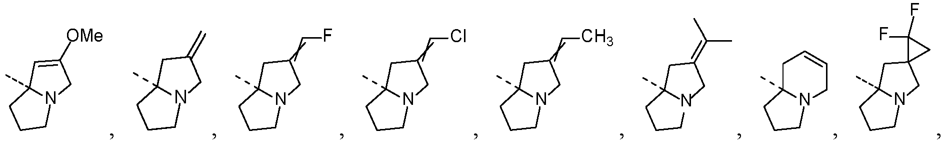 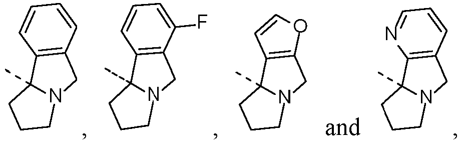 and other variables are as defined herein.

In some embodiments of the present disclosure, the structural moiety 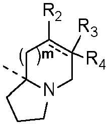 is selected from 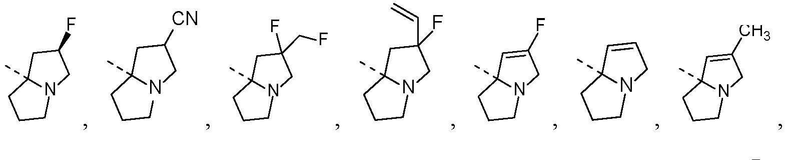 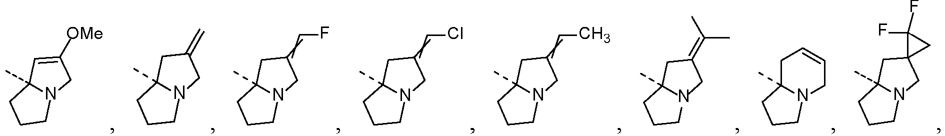 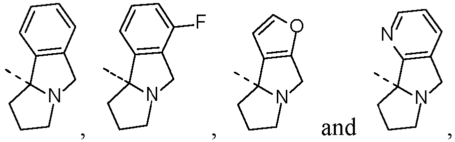 and other variables are as defined herein.

In some embodiments of the present disclosure, the structural moiety 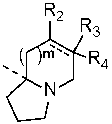 is selected from 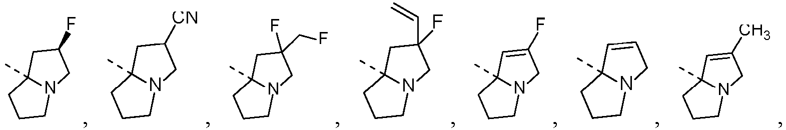 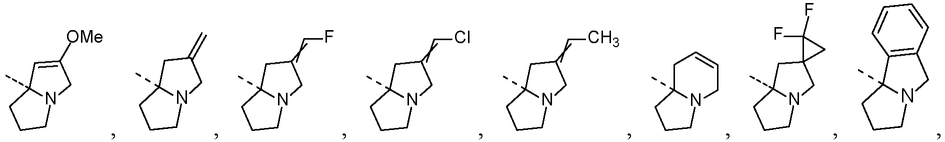 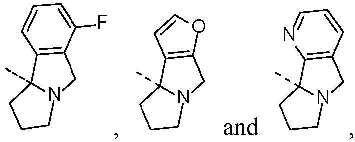 and other variables are as defined herein.

In some embodiments of the present disclosure, the structural moiety 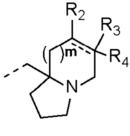 is selected from 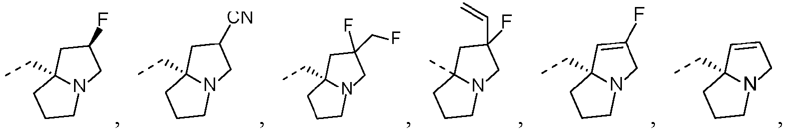 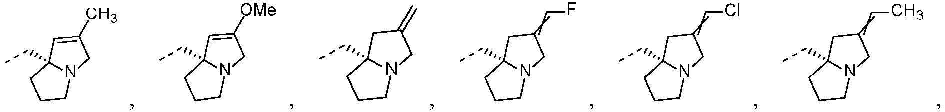 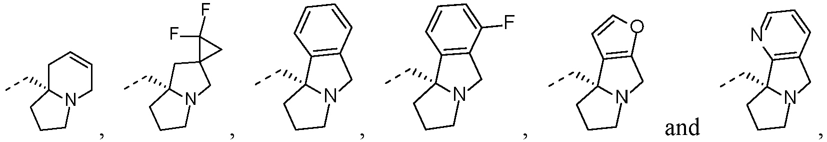 and other variables are as defined herein.

In some embodiments of the present disclosure, the structural moiety 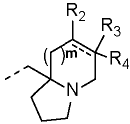 is selected from 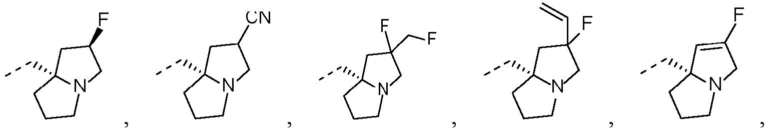 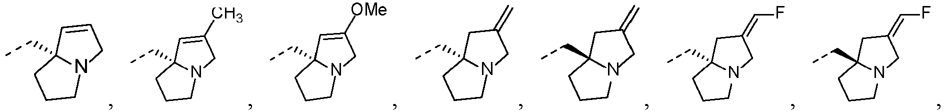 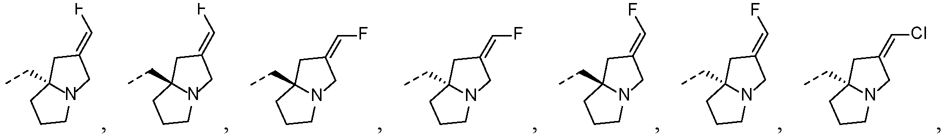 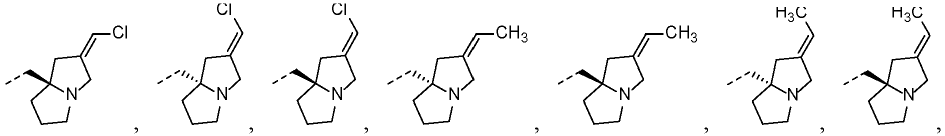 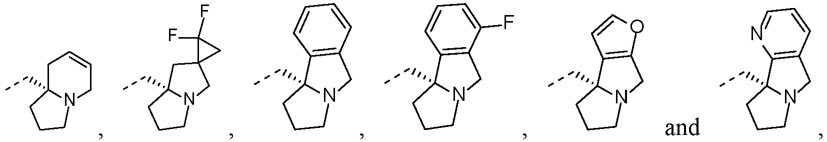 and other variables are as defined herein.

In some embodiments of the present disclosure, the structural moiety 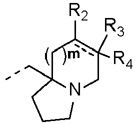 is selected from 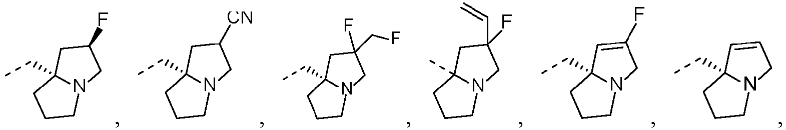 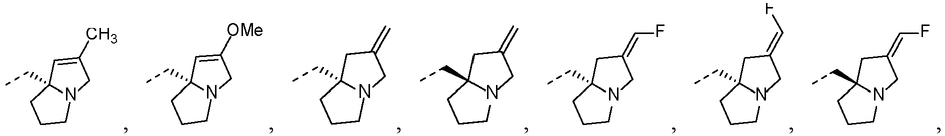 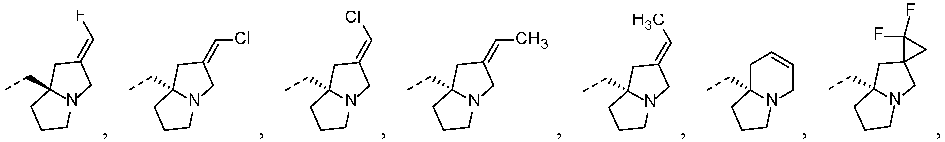 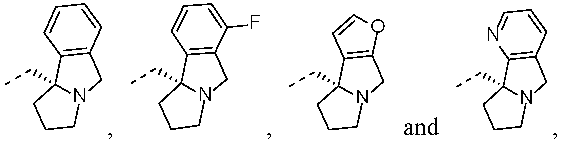 and other variables are as defined herein.

In some embodiments of the present disclosure, the structural moiety 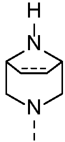 is 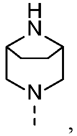 and other variables are as defined herein.

In some embodiments of the present disclosure, provided is a compound or a pharmaceutically acceptable salt thereof, wherein the compound is 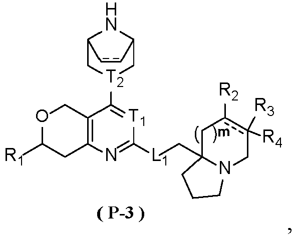 wherein 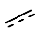
is selected from a single bond and a double bond;
T₁, T₂, R₁, R₂, R₃, R₄, L₁ and m are as defined herein.

In some embodiments of the present disclosure, provided is a compound or a pharmaceutically acceptable salt thereof, wherein the compound is 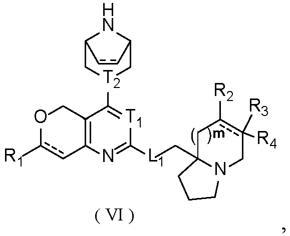 wherein 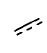
is selected from a single bond and a double bond;
T₁, T₂, R₁, R₂, R₃, R₄, L₁ and m are as defined herein;
provided that, when the structural moiety 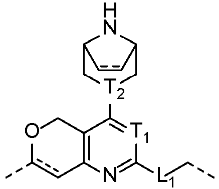 is 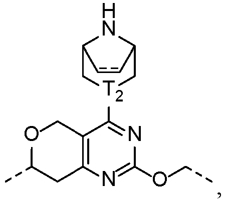 the structural moiety 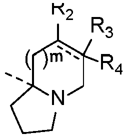 is not 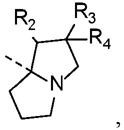 R₂ is H; R₃ is selected from H, F, CN, CH₃ and OCH₃, wherein the CH₃ and OCH₃ are optionally substituted with 1, 2 or 3 halogens; R₄ is selected from H and CH₃.

In some embodiments of the present disclosure, provided is a compound or a pharmaceutically acceptable salt thereof, wherein the compound is 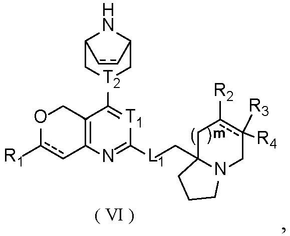 wherein 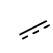
is selected from a single bond and a double bond;
the structural moiety 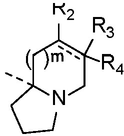 is selected from 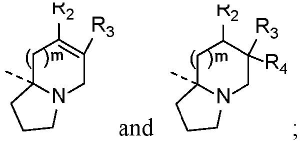
R₂ is H;
R₃ is selected from H, F, CN, CH₃ and OCH₃, wherein the CH₃ and OCH₃ are optionally substituted with 1, 2 or 3 halogens;
alternatively, R₂ and R₃ together with the atoms to which they are attached form a phenyl group or a 5-6 membered heteroaryl group, wherein the phenyl group or the 5-6 membered heteroaryl group is optionally substituted with 1, 2 or 3 halogens;
R₄ is -CH=CH₂, wherein the -CH=CH₂ is optionally substituted with 1, 2 or 3 halogens;
alternatively, R₃ and R₄ together with the atom to which they are attached form 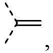 wherein the 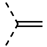 is optionally substituted with 1 or 2 R_{c};
alternatively, R₃ and R₄ together with the atom to which they are attached form a cyclopropyl group, which is optionally substituted with 1, 2 or 3 halogens;
T₁, T₂, R₁, each R_{c}, L₁ and m are as defined herein.

In some embodiments of the present disclosure, the structural moiety 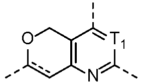 is 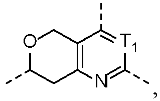 and other variables are as defined herein.

In some embodiments of the present disclosure, T₁ is N, and other variables are as defined herein.

In some embodiments of the present disclosure, the structural moiety 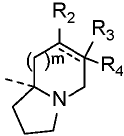 is selected from 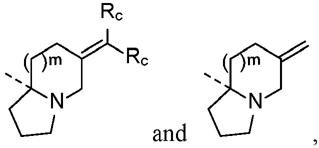 and other variables are as defined herein.

In some embodiments of the present disclosure, the structural moiety 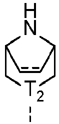 is 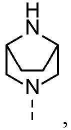 and other variables are as defined herein.

In some embodiments of the present disclosure, provided is a compound or a pharmaceutically acceptable salt thereof, wherein the compound is selected from 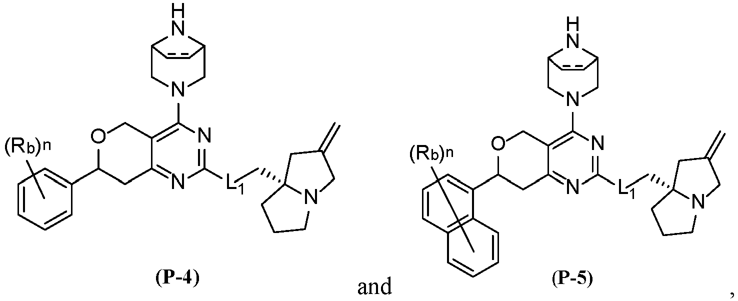 wherein
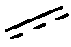 is selected from a single bond and a double bond;
each R_{b} and L₁ are as defined herein;
n is selected from 0, 1, 2, 3, 4 and 5.

In some embodiments of the present disclosure, the structural moiety 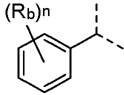 is selected from 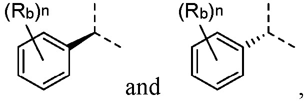 and other variables are as defined herein.

In some embodiments of the present disclosure, the structural moiety 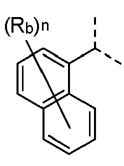 is selected from 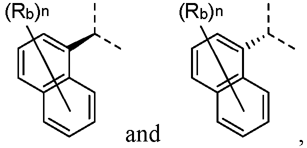 and other variables are as defined herein.

In some embodiments of the present disclosure, provided is a compound or a pharmaceutically acceptable salt thereof, wherein the compound is selected from 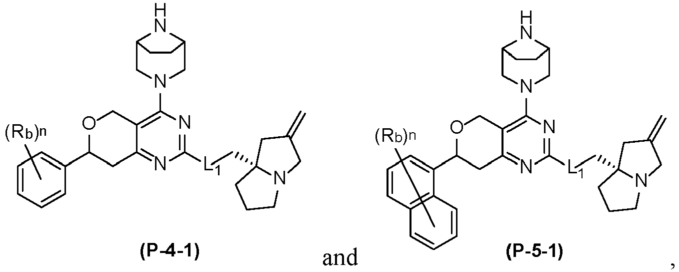 wherein
each R_{b} and L₁ are as defined herein;
n is selected from 0, 1, 2, 3, 4 and 5.

In some embodiments of the present disclosure, provided is a compound or a pharmaceutically acceptable salt thereof, wherein the compound is selected from 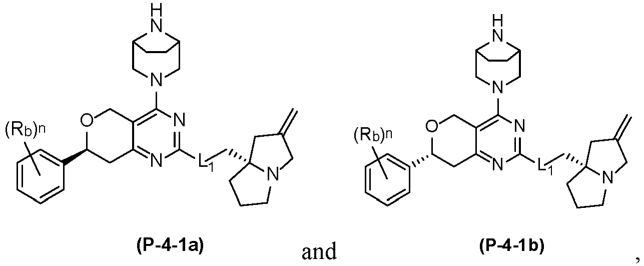 wherein
each R_{b} and L₁ are as defined herein;
n is selected from 0, 1, 2, 3, 4 and 5.

In some embodiments of the present disclosure, provided is a compound or a pharmaceutically acceptable salt thereof, wherein the compound is selected from 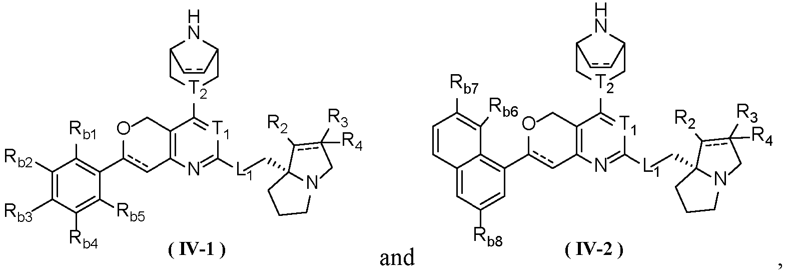 wherein 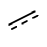
is selected from a single bond and a double bond;
T₁, T₂, R₂, R₃, R₄ and L₁ are as defined herein;
R_{b1}, R_{b2}, R_{b3}, R_{b4}, R_{b5}, R_{b6} and R_{b7} are independently selected from H, F, Cl, Br, I, OH, NH₂, CN, C₁₋₃ alkyl, C₁₋₃ alkoxy, C₂₋₄ alkynyl, C₂₋₄ alkynyl-cyclopropyl, C₂₋₃ alkenyl, -C(=O)C₁₋₃ alkyl and C₃₋₅ cycloalkyl, wherein the C₁₋₃ alkyl, C₁₋₃ alkoxy, C₂₋₄ alkynyl, C₂₋₄ alkynyl-cyclopropyl, C₂₋₃ alkenyl, -C(=O)C₁₋₃ alkyl and C₃₋₅ cycloalkyl are optionally substituted with 1, 2, 3, 4 or 5 halogens.

In some embodiments of the present disclosure, R_{b1}, R_{b2}, R_{b3}, R_{b4}, R_{b5}, R_{b6}, R_{b7} and R_{b8} are independently selected from H, F, Cl, Br, I, OH, NH₂, CN, CH₃, CH₂CH₃, OCH₃, OCH₂CH₃, -CH=CH₂, -C≡CH, -C≡CCH₃, -C≡CCH₂CH₃, 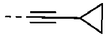 and cyclopropyl, wherein the CH₃, CH₂CH₃, OCH₃, OCH₂CH₃, -CH=CH₂, -C≡CH, -C≡CCH₃, -C≡CCH₂CH₃, 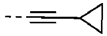 and cyclopropyl are optionally substituted with 1, 2, 3, 4 or 5 R, and other variables are as defined herein.

In some embodiments of the present disclosure, R_{b1}, R_{b2}, R_{b3}, R_{b4}, R_{b5}, R_{b6}, R_{b7} and R_{b8} are independently selected from H, F, Cl, Br, I, OH, NH₂, CN, CH₃, CF₃, CH₂CH₃, CF₂CF₃, -CH=CH₂, -C≡CH, -C≡CF, -C≡CBr, -C≡CCH₃, -C≡CCF₃, -C≡CCH₂CH₃, 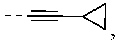 and cyclopropyl, and other variables are as defined herein.

The present disclosure also provides a compound represented by formula (IV) or a pharmaceutically acceptable salt thereof, 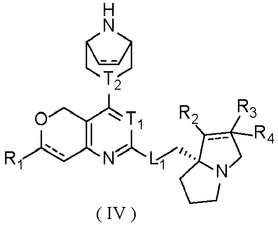 wherein 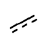
is selected from a single bond and a double bond;
T₁ is selected from CRₐ and N;
T₂ is selected from CH and N;
L₁ is selected from O and S;
R₁ is selected from phenyl, naphthyl, 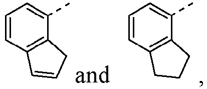 wherein the phenyl, naphthyl, 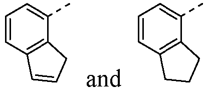 are each independently optionally substituted with 1, 2, 3, 4 or 5 R_{b};
R₂ is H;
R₃ is selected from H, F, CN and OCH₃, wherein the OCH₃ is optionally substituted with 1, 2 or 3 halogens;
alternatively, R₂ and R₃ together with the atoms to which they are attached form a phenyl group or a 5-6 membered heteroaryl group, wherein the phenyl group and the 5-6 membered heteroaryl group are optionally substituted with 1, 2 or 3 halogens;
R₄ is absent or is selected from H, CH₃ and -CH=CH₂, wherein the CH₃ and -CH=CH₂ are optionally substituted with 1, 2 or 3 halogens;
alternatively, R₃ and R₄ together with the atom to which they are attached form 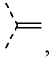 wherein the 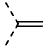 is optionally substituted with 1 or 2 halogens;
alternatively, R₃ and R₄ together with the atom to which they are attached form a cyclopropyl group, which is optionally substituted with 1, 2 or 3 halogens;
Rₐ is selected from Hand CN;
each R_{b} is independently selected from H, F, Cl, Br, I, OH, NH₂, CN, C₁₋₃ alkyl, C₁₋₃ alkoxy, C₂₋₄ alkynyl, C₂₋₄ alkynyl-cyclopropyl, C₂₋₃ alkenyl, -C(=O)C₁₋₃ alkyl and C₃₋₅ cycloalkyl, wherein the C₁₋₃ alkyl, C₁₋₃ alkoxy, C₂₋₄ alkynyl, C₂₋₄ alkynyl-cyclopropyl, C₂₋₃ alkenyl, -C(=O)C₁₋₃ alkyl and C₃₋₅ cycloalkyl are optionally substituted with 1, 2, 3, 4 or 5 halogens.

In some embodiments of the present disclosure, each R_{b} is independently selected from H, F, Cl, Br, I, OH, NH₂, CN, CH₃, CH₂CH₃, OCH₃, OCH₂CH₃, -CH=CH₂, -C≡CH, -C≡CCH₃, -C≡CCH₂CH₃, 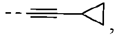 and cyclopropyl, wherein the CH₃, CH₂CH₃, OCH₃, OCH₂CH₃, -CH=CH₂, -C≡CH, -C≡CCH₃, -C≡CCH₂CH₃, 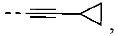 and cyclopropyl are optionally substituted with 1, 2, 3, 4 or 5 halogens, and other variables are as defined herein.

In some embodiments of the present disclosure, each R_{b} is independently selected from H, F, Cl, Br, I, OH, NH₂, CN, CH₃, CH₂F, CF₂H, CF₃, CH₂CH₃, CF₂CF₃, -CH=CH₂, -C≡CH, -C≡CF, -C≡CBr, -C≡CCH₃, -C≡CCF₃, -C≡CCH₂CF₃, 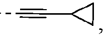 and cyclopropyl, and other variables are as defined herein.

In some embodiments of the present disclosure, R₁ is selected from 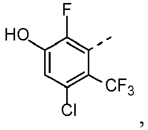 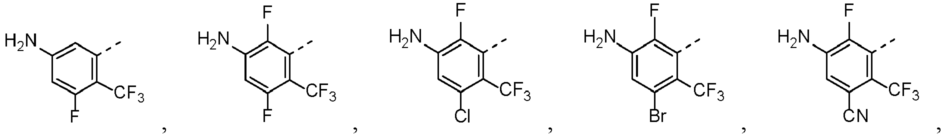 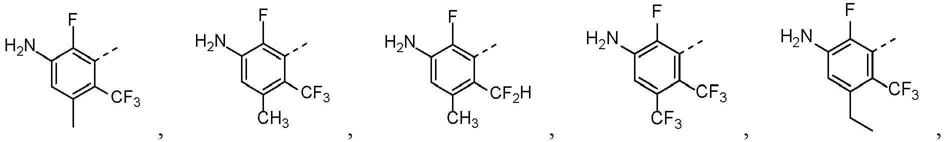 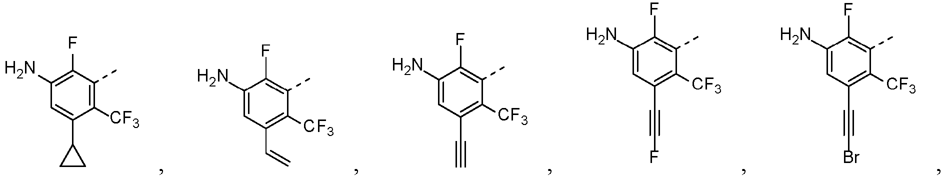 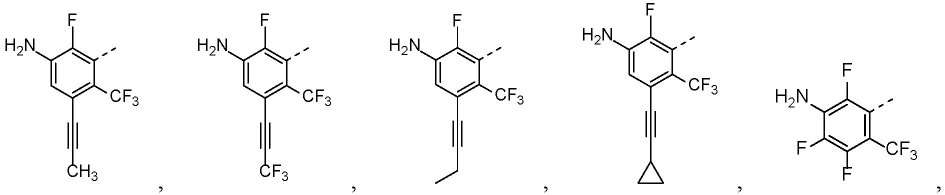 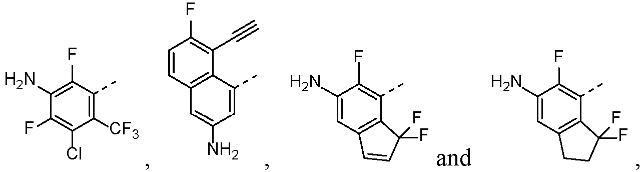 and other variables are as defined herein.

In some embodiments of the present disclosure, R₃ is selected from H, F, CN and OCH₃, and other variables are as defined herein.

In some embodiments of the present disclosure, R₂ and R₃ together with the atoms to which they are attached form phenyl, furanyl or pyridinyl, wherein the phenyl, furanyl and pyridinyl are optionally substituted with one F, and other variables are as defined herein.

In some embodiments of the present disclosure, R₄ is absent or is selected from H, CH₂F and -CH=CH₂, and other variables are as defined herein.

In some embodiments of the present disclosure, R₃ and R₄ together with the atom to which they are attached form 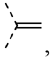 and other variables are as defined herein.

In some embodiments of the present disclosure, R₃ and R₄ together with the atom to which they are attached form 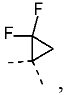 and other variables are as defined herein.

In some embodiments of the present disclosure, the structural moiety 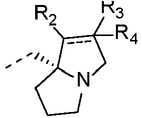 is selected from 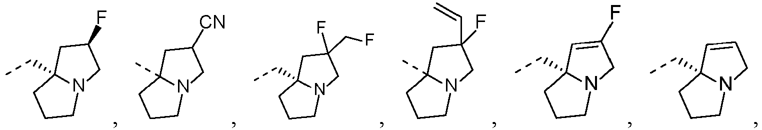 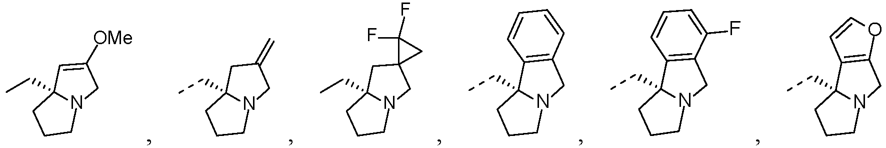 and 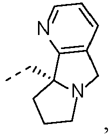 and other variables are as defined herein.

In some embodiments of the present disclosure, provided is a compound or a pharmaceutically acceptable salt thereof, wherein the compound is selected from 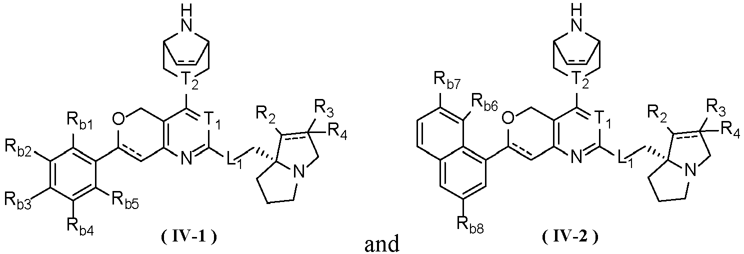 wherein
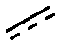 is selected from a single bond and a double bond;
T₁, T₂, R₂, R₃, R₄ and L₁ are as defined herein;
R_{b1}, R_{b2}, R_{b3}, R_{b4}, R_{b5}, R_{b6} and R_{b7} are each independently selected from H, F, Cl, Br, I, OH, NH₂, CN, C₁₋₃ alkyl, C₁₋₃ alkoxy, C₂₋₄ alkynyl, C₂₋₄ alkynyl-cyclopropyl, C₂₋₃ alkenyl, -C(=O)C₁₋₃ alkyl and C₃₋₅ cycloalkyl, wherein the C₁₋₃ alkyl, C₁₋₃ alkoxy, C₂₋₄ alkynyl, C₂₋₄ alkynyl-cyclopropyl, C₂₋₃ alkenyl, -C(=O)C₁₋₃ alkyl and C₃₋₅ cycloalkyl are optionally substituted with 1, 2, 3, 4 or 5 halogens.

In some embodiments of the present disclosure, R_{b1}, R_{b2}, R_{b3}, R_{b4}, R_{b5}, R_{b6}, R_{b7} and R_{b8} are each independently selected from H, F, Cl, Br, I, OH, NH₂, CN, CH₃, CH₂CH₃, OCH₃, OCH₂CH₃, -CH=CH₂, -C≡CH, -C≡CCH₃, -C≡CCH₂CH₃, 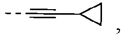 and cyclopropyl, wherein the CH₃, CH₂CH₃, OCH₃, OCH₂CH₃, -CH=CH₂, -C≡CH, -C≡CCH₃, -C≡CCH₂CH₃, 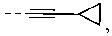 and cyclopropyl are optionally substituted with 1, 2, 3, 4 or 5 R, and other variables are as defined herein.

In some embodiments of the present disclosure, R_{b1}, R_{b2}, R_{b3}, R_{b4}, R_{b5}, R_{b6}, R_{b7} and R_{b8} are each independently selected from H, F, Cl, Br, I, OH, NH₂, CN, CH₃, CF₃, CH₂CH₃, CF2CF3, -CH=CH₂, -C≡CH, -C≡CF, -C≡CBr, -C≡CCH₃, -C≡CCF₃, -C≡CCH₂CH₃, 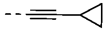 and cyclopropyl, and other variables are as defined herein.

The present disclosure also provides a compound represented by formula (IV) or a pharmaceutically acceptable salt thereof, 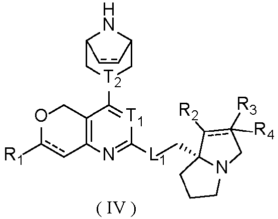 wherein
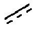 is selected from a single bond and a double bond;
T₁ is selected from CRₐ and N;
T₂ is selected from CH and N;
L₁ is selected from O and S;
R₁ is selected from phenyl and naphthyl, wherein the phenyl and naphthyl are each independently optionally substituted with 1, 2, 3, 4 or 5 R_{b};
R₂ is H;
R₃ is selected from H, F and CN;
alternatively, R₂ and R₃ together with the atoms to which they are attached form a phenyl group;
R₄ is absent or is selected from H and -CH=CH₂,
Rₐ is selected from Hand CN;
each R_{b} is independently selected from H, F, Cl, Br, I, OH, NH₂, CN, C₁₋₃ alkyl, C₁₋₃ alkoxy, C₂₋₃ alkynyl, C₂₋₃ alkenyl, -C(=O)C₁₋₃ alkyl and C₃₋₅ cycloalkyl, wherein the C₁₋₃ alkyl, C₁₋₃ alkoxy, C₂₋₃ alkynyl, C₂₋₃ alkenyl, -C(=O)C₁₋₃ alkyl and C₃₋₅ cycloalkyl are optionally substituted with 1, 2, 3, 4 or 5 halogens.

In some embodiments of the present disclosure, each R_{b} is independently selected from H, F, Cl, Br, I, OH, NH₂, CN, CH₃, CH₂CH₃, OCH₃, OCH₂CH₃, -CH=CH₂, -C≡CH, and cyclopropyl, wherein the CH₃, CH₂CH₃, OCH₃, OCH₂CH₃, -CH=CH₂, -C≡CH, and cyclopropyl are optionally substituted with 1, 2, 3, 4 or 5 halogens; and other variables are as defined herein.

In some embodiments of the present disclosure, each R_{b} is independently selected from H, F, Cl, Br, I, OH, NH₂, CN, CH₃, CH₂F, CF₂H, CF₃, CH₂CH₃, CF₂CF₃, -CH=CH₂, -C≡CH and cyclopropyl, and other variables are as defined herein.

In some embodiments of the present disclosure, R₁ is selected from 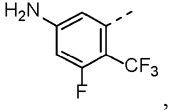 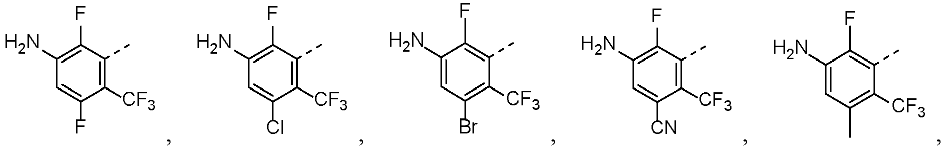 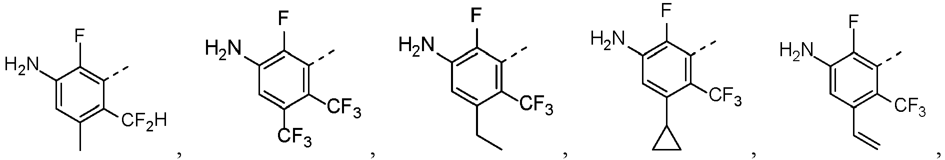 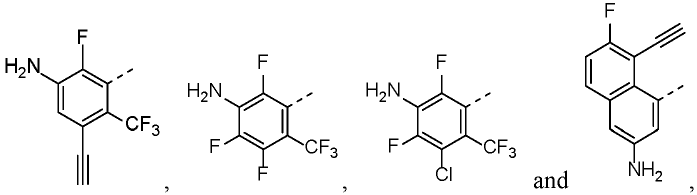 and other variables are as defined herein.

In some embodiments of the present disclosure, the structural moiety 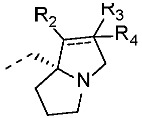 is selected from 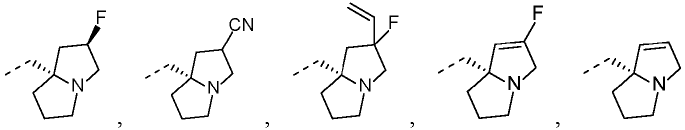 and 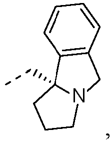 and other variables are as defined herein.

In some embodiments of the present disclosure, provided is a compound or a pharmaceutically acceptable salt thereof, wherein the compound is selected from 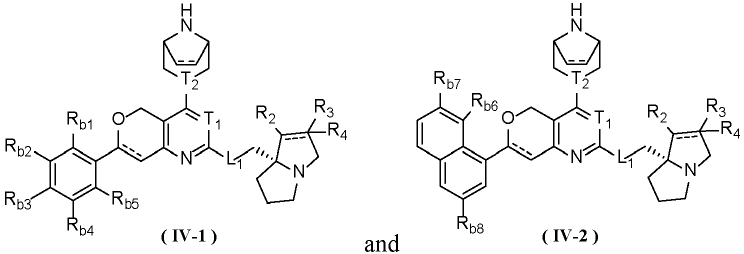 wherein 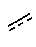
is selected from a single bond and a double bond;
T₁, T₂, R₂, R₃, R₄ and L₁ are as defined herein;
R_{b1}, R_{b2}, R_{b3}, R_{b4}, R_{b5}, R_{b6} and R_{b7} are each independently selected from H, F, Cl, Br, I, OH, NH₂, CN, C₁₋₃ alkyl, C₁₋₃ alkoxy, C₂₋₃ alkynyl, C₂₋₃ alkenyl, -C(=O)C₁₋₃ alkyl and C₃₋₅ cycloalkyl, wherein the C₁₋₃ alkyl, C₁₋₃ alkoxy, C₂₋₃ alkynyl, C₂₋₃ alkenyl, -C(=O)C₁₋₃ alkyl and C₃₋₅ cycloalkyl are optionally substituted with 1, 2, 3, 4 or 5 halogens.

In some embodiments of the present disclosure, R_{b1}, R_{b2}, R_{b3}, R_{b4}, R_{b5}, R_{b6}, R_{b7} and R_{b8} are each independently selected from H, F, Cl, Br, I, OH, NH₂, CN, CH₃, CH₂CH₃, OCH₃, OCH₂CH₃, -CH=CH₂, -C≡CH, and cyclopropyl, wherein the CH₃, CH₂CH₃, OCH₃, OCH₂CH₃, -CH=CH₂, -C≡CH and cyclopropyl are optionally substituted with 1, 2, 3, 4 or 5 R, and other variables are as defined herein.

In some embodiments of the present disclosure, R_{b1}, R_{b2}, R_{b3}, R_{b4}, R_{b5}, R_{b6}, R_{b7} and R_{b8} are each independently selected from H, F, Cl, Br, I, OH, NH₂, CN, CH₃, CF₃, CH₂CH₃, CF₂CF₃, -CH=CH₂, -C≡CH and cyclopropyl, and other variables are as defined herein.

The present disclosure also provides a compound represented by formula (III) or a pharmaceutically acceptable salt thereof, 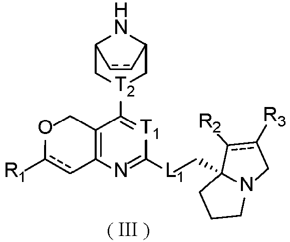 wherein 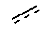
is selected from a single bond and a double bond;
T₁ is selected from CRₐ and N;
T₂ is selected from CH and N;
L₁ is selected from O and S;
R₁ is selected from phenyl and naphthyl, wherein the phenyl and naphthyl are each independently optionally substituted with 1, 2, 3, 4 or 5 R_{b};
R₂ is H;
R₃ is selected from Hand F;
alternatively, R₂ and R₃ together with the atoms to which they are attached form a phenyl group;
Rₐ is selected from Hand CN;
each R_{b} is independently selected from H, F, Cl, Br, I, OH, NH₂, CN, C₁₋₃ alkyl, C₁₋₃ alkoxy, C₂₋₃ alkynyl, C₂₋₃ alkenyl, -C(=O)C₁₋₃ alkyl and C₃₋₅ cycloalkyl, wherein the C₁₋₃ alkyl, C₁₋₃ alkoxy, C₂₋₃ alkynyl, C₂₋₃ alkenyl, -C(=O)C₁₋₃ alkyl and C₃₋₅ cycloalkyl are optionally substituted with 1, 2, 3, 4 or 5 halogens.

In some embodiments of the present disclosure, each R_{b} is independently selected from H, F, Cl, Br, I, OH, NH₂, CN, CH₃, CH₂CH₃, OCH₃, OCH₂CH₃, -CH=CH₂, -C≡CH, and cyclopropyl, wherein the CH₃, CH₂CH₃, OCH₃, OCH₂CH₃, -CH=CH₂, -C≡CH and cyclopropyl are optionally substituted with 1, 2, 3, 4 or 5 halogens; and other variables are as defined herein.

In some embodiments of the present disclosure, each R_{b} is independently selected from H, F, Cl, Br, I, OH, NH₂, CN, CH₃, CH₂F, CF₂H, CF₃, CH₂CH₃, CF₂CF₃, -CH=CH₂, -C≡CH and cyclopropyl, and other variables are as defined herein.

In some embodiments of the present disclosure, R₁ is selected from 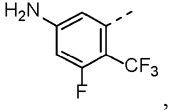 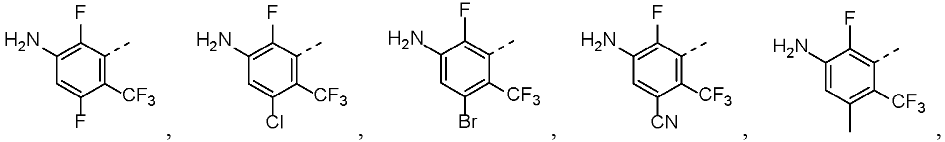 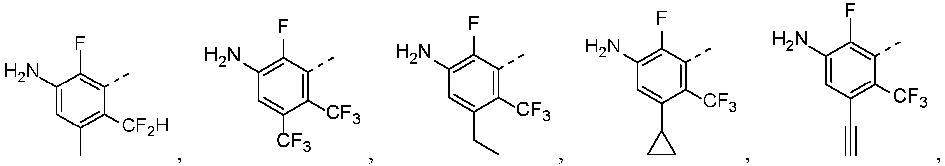 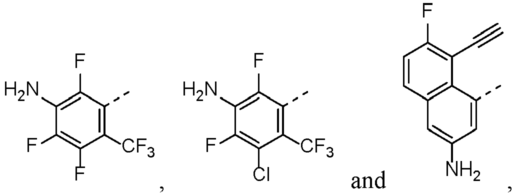 and other variables are as defined herein.

In some embodiments of the present disclosure, provided is a compound or a pharmaceutically acceptable salt thereof, wherein the compound is selected from 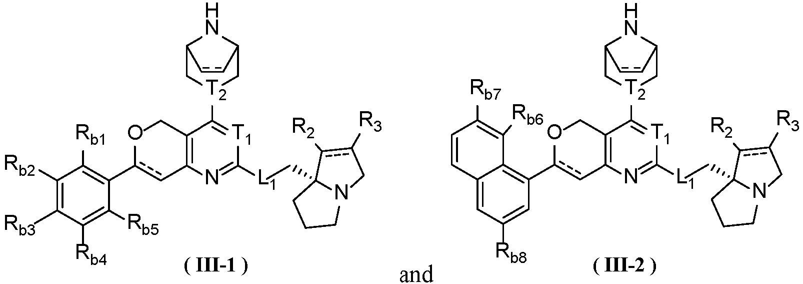 wherein 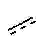
is selected from a single bond and a double bond;
T₁, T₂, R₂, R₃ and L₁ are as defined herein;
R_{b1}, R_{b2}, R_{b3}, R_{b4}, R_{b5}, R_{b6} and R_{b7} are each independently selected from H, F, Cl, Br, I, OH, NH₂, CN, C₁₋₃ alkyl, C₁₋₃ alkoxy, C₂₋₃ alkynyl, C₂₋₃ alkenyl, -C(=O)C₁₋₃ alkyl and C₃₋₅ cycloalkyl, wherein the C₁₋₃ alkyl, C₁₋₃ alkoxy, C₂₋₃ alkynyl, C₂₋₃ alkenyl, -C(=O)C₁₋₃ alkyl and C₃₋₅ cycloalkyl are optionally substituted with 1, 2, 3, 4 or 5 halogens.

In some embodiments of the present disclosure, R_{b1}, R_{b2}, R_{b3}, R_{b4}, R_{b5}, R_{b6}, R_{b7} and R_{b8} are each independently selected from H, F, Cl, Br, I, OH, NH₂, CN, CH₃, CH₂CH₃, OCH₃, OCH₂CH₃, -CH=CH₂, -C≡CH, and cyclopropyl, wherein the CH₃, CH₂CH₃, OCH₃, OCH₂CH₃, -CH=CH₂, -C≡CH, and cyclopropyl are optionally substituted with 1, 2, 3, 4 or 5 R, and other variables are as defined herein.

In some embodiments of the present disclosure, R_{b1}, R_{b2}, R_{b3}, R_{b4}, R_{b5}, R_{b6}, R_{b7} and R_{b8} are each independently selected from H, F, Cl, Br, I, OH, NH₂, CN, CH₃, CF₃, CH₂CH₃, CF₂CF₃, -CH=CH₂, -C≡CH and cyclopropyl, and other variables are as defined herein.

The present disclosure also provides a compound represented by formula (I) or a pharmaceutically acceptable salt thereof, 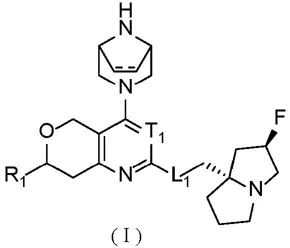 wherein 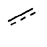
is selected from a single bond and a double bond;
T₁ is selected from CRₐ and N;
L₁ is selected from O and S;
R₁ is selected from phenyl and naphthyl, wherein the phenyl and naphthyl are each independently optionally substituted with 1, 2, 3, 4 or 5 R_{b};
Rₐ is selected from Hand CN;
each R_{b} is independently selected from H, F, Cl, Br, I, OH, NH₂, CN, C₁₋₃ alkyl, C₁₋₃ alkoxy, C₂₋₃ alkynyl, C₂₋₃ alkenyl, -C(=O)C₁₋₃ alkyl and C₃₋₅ cycloalkyl, wherein the C₁₋₃ alkyl, C₁₋₃ alkoxy, C₂₋₃ alkynyl, C₂₋₃ alkenyl, -C(=O)C₁₋₃ alkyl and C₃₋₅ cycloalkyl are optionally substituted with 1, 2, 3, 4 or 5 halogens.

In some embodiments of the present disclosure, each R_{b} is independently selected from H, F, Cl, Br, I, OH, NH₂, CN, CH₃, CH₂CH₃, OCH₃, OCH₂CH₃, -CH=CH₂, -C≡CH, and cyclopropyl, wherein the CH₃, CH₂CH₃, OCH₃, OCH₂CH₃, -CH=CH₂, -C≡CH, and cyclopropyl are optionally substituted with 1, 2, 3, 4 or 5 halogens; and other variables are as defined herein.

In some embodiments of the present disclosure, each R_{b} is independently selected from H, F, Cl, Br, I, OH, NH₂, CN, CH₃, CF₃, CH₂CH₃, CF₂CF₃, -CH=CH₂, -C≡CH and cyclopropyl, and other variables are as defined herein.

In some embodiments of the present disclosure, provided is a compound or a pharmaceutically acceptable salt thereof, wherein the compound is selected from 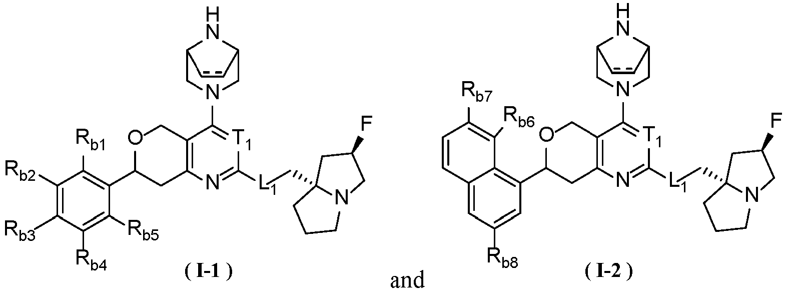 wherein 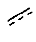
is selected from a single bond and a double bond;
T₁ and L₁ are as defined herein;
R_{b1}, R_{b2}, R_{b3}, R_{b4}, R_{b5}, R_{b6} and R_{b7} are each independently selected from H, F, Cl, Br, I, OH, NH₂, CN, C₁₋₃ alkyl, C₁₋₃ alkoxy, C₂₋₃ alkynyl, C₂₋₃ alkenyl, -C(=O)C₁₋₃ alkyl and C₃₋₅ cycloalkyl, wherein the C₁₋₃ alkyl, C₁₋₃ alkoxy, C₂₋₃ alkynyl, C₂₋₃ alkenyl, -C(=O)C₁₋₃ alkyl and C₃₋₅ cycloalkyl are optionally substituted with 1, 2, 3, 4 or 5 halogens.

In some embodiments of the present disclosure, R_{b1}, R_{b2}, R_{b3}, R_{b4}, R_{b5}, R_{b6}, R_{b7} and R_{b8} are each independently selected from H, F, Cl, Br, I, OH, NH₂, CN, CH₃, CH₂CH₃, OCH₃, OCH₂CH₃, -CH=CH₂, -C≡CH, and cyclopropyl, wherein the CH₃, CH₂CH₃, OCH₃, OCH₂CH₃, -CH=CH₂, -C≡CH and cyclopropyl are optionally substituted with 1, 2, 3, 4 or 5 R, and other variables are as defined herein.

In some embodiments of the present disclosure, R_{b1}, R_{b2}, R_{b3}, R_{b4}, R_{b5}, R_{b6}, R_{b7} and R_{b8} are each independently selected from H, F, Cl, Br, I, OH, NH₂, CN, CH₃, CF₃, CH₂CH₃, CF₂CF₃, -CH=CH₂, -C≡CH and cyclopropyl, and other variables are as defined herein.

In Example 8 of the present disclosure, the hydrochloride of compound 8 was prepared from compound 8-3 via steps 6 and 7, wherein the compound 8-3 was prepared from compound 8-2 and compound 1-12B via step 5, and compound 8-2 and compound 1-12B were prepared from compound 8-1A and compound 1-11B, respectively.

In Example 8 of the present disclosure, the hydrochloride of compound 8 was prepared from compound 8-1A, wherein ccompound 8-1A was analyzed by SFC (chromatographic column: Chiralpak IH-3, 100×4.6mm ID, 3µm; mobile phase: A (supercritical CO₂) and B (ethanol, containing 0.1% isopropylamine); gradient: B%=10-50%, 4 min; flow rate: 3.4 mL/min; wavelength: 220nm; pressure: 2000psi) and showed a retention time of 1.489 min.

In Example 8 of the present disclosure, the hydrochloride of compound 8 was prepared from compound 1-11B, wherein compound 1-11B was analyzed by SFC (chromatographic column: (S,S)Whelk-O1 100×4.6mm ID, 5.0µm; mobile phase: A: supercritical CO₂, B: [75% isopropanol/25% acetonitrile/0.05% diethylamine]; B%: 50% - 50 %, flow rate: 2.5 mL/min) and showed a retention time of 3.317 min, and the retention time of its enantiomer was 2.536 min.

In Example 8 of the present disclosure, compound 8 was prepared from compound 1-11B, wherein the compound 1-11B was analyzed by SFC (chromatographic column: (S,S)Whelk-O1 100×4.6mm ID, 5.0µm; mobile phase: A: supercritical CO₂, B: [75% isopropanol/25% acetonitrile/0.05% diethylamine]; B%: 50% - 50 %, flow rate: 2.5 mL/min) and showed a retention time of 3.317 min, and the retention time of its enantiomer was 2.536 min.

In Example 8 of the present disclosure, compound 1-11B was analyzed by SFC (chromatographic column: (S,S)Whelk-O1 100×4.6mm ID, 5.0µm; mobile phase: A: supercritical CO₂, B: [75% isopropanol/25% acetonitrile/0.05% diethylamine]; B%: 50% - 50 %, flow rate: 2.5 mL/min) and showed a retention time of 3.317 min, and the retention time of its enantiomer was 2.536 min. The hydrochloride of compound 8 was synthesized from 1-11B via the steps described in Example 8.

Some other solutions of the present disclosure are obtained by arbitrarily combining the above variables.

The present disclosure provides a compound represented by the following formula or a pharmaceutically acceptable salt thereof:

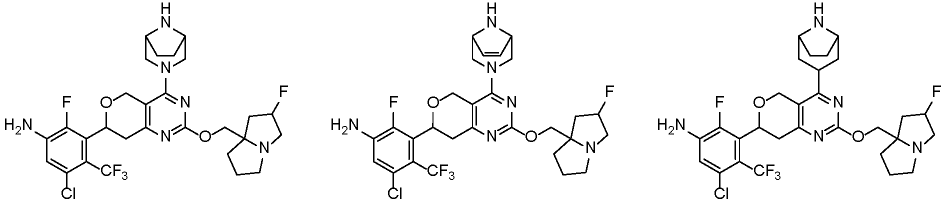

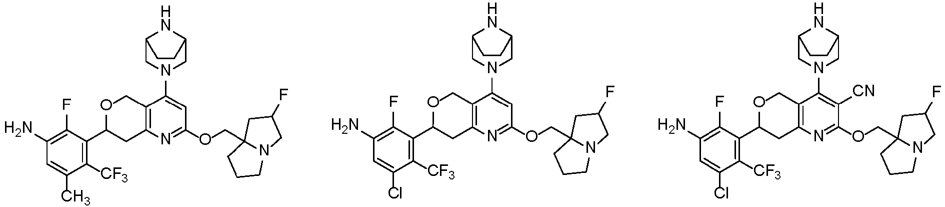

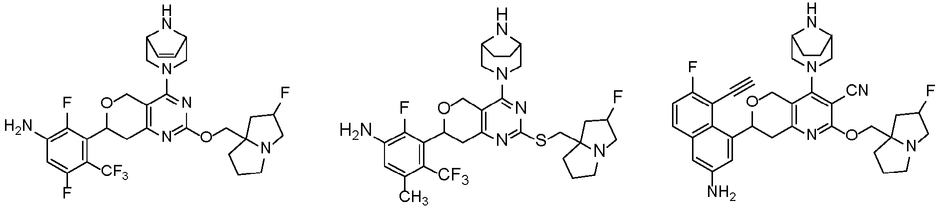

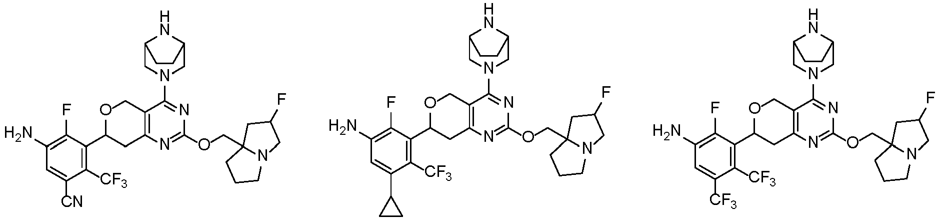

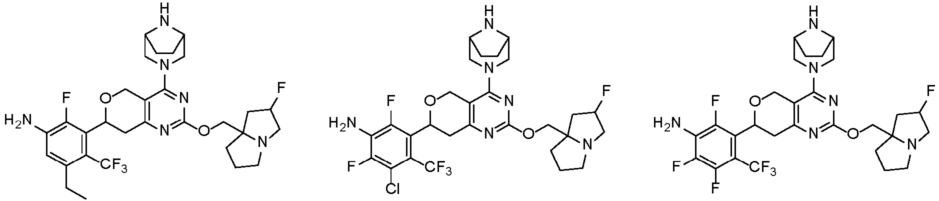

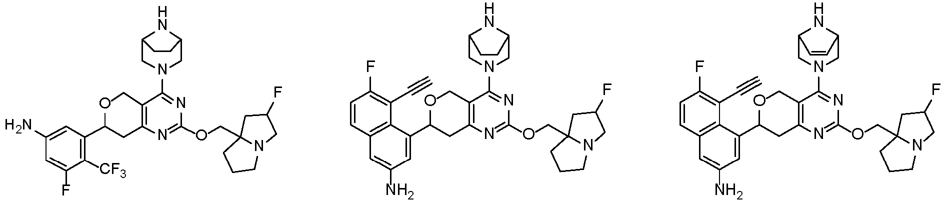

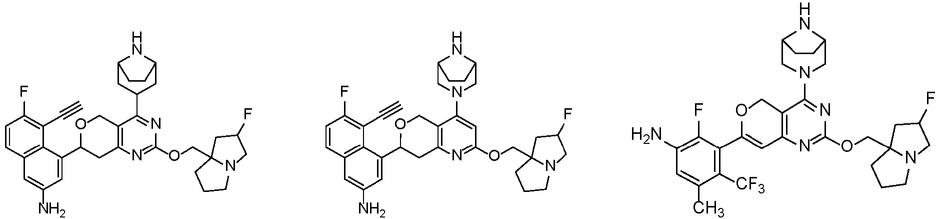

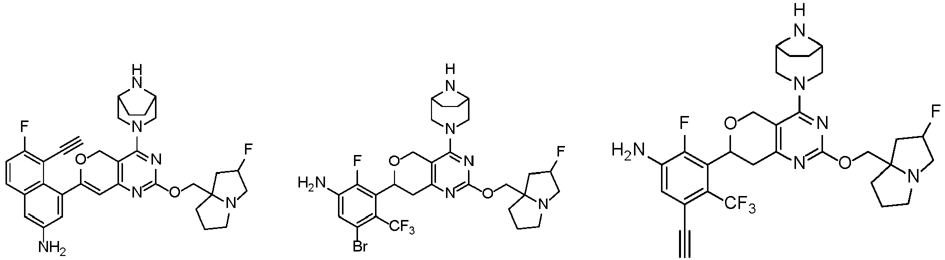

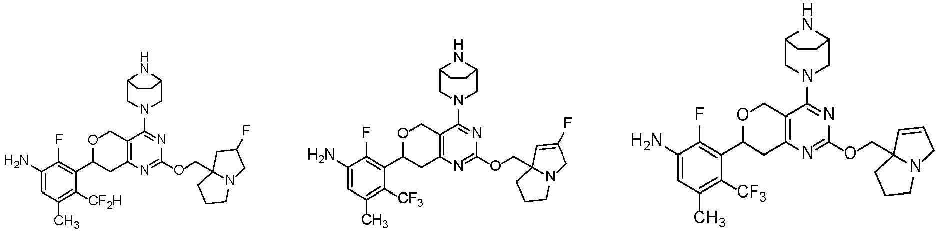

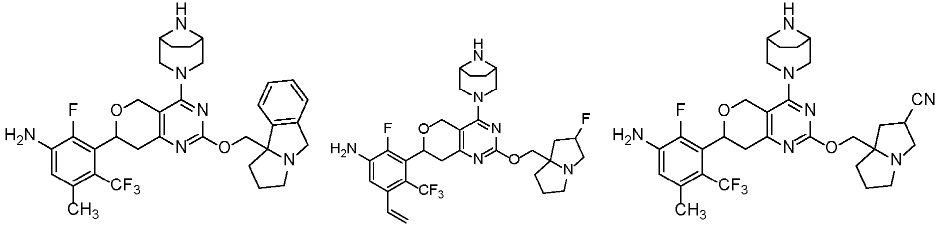

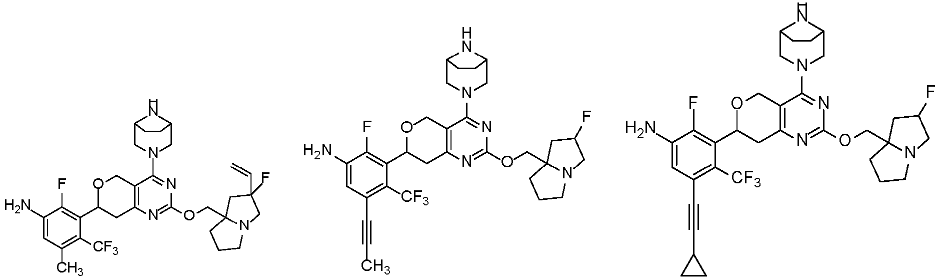

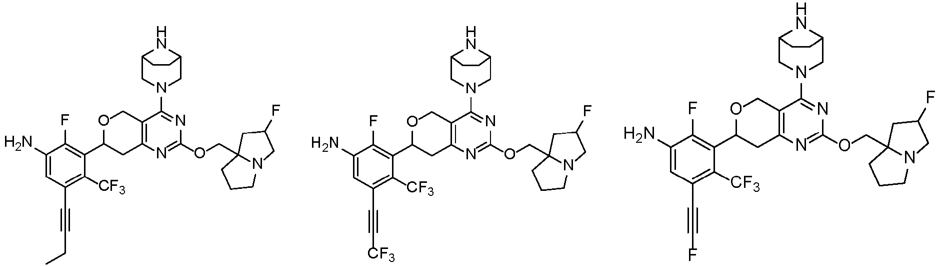

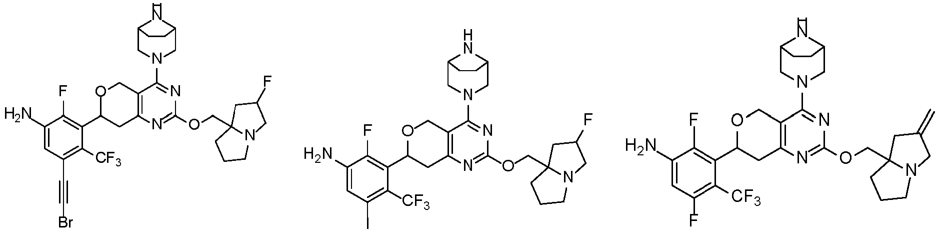

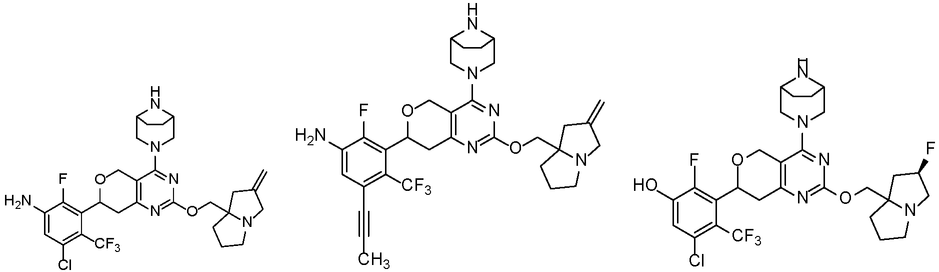

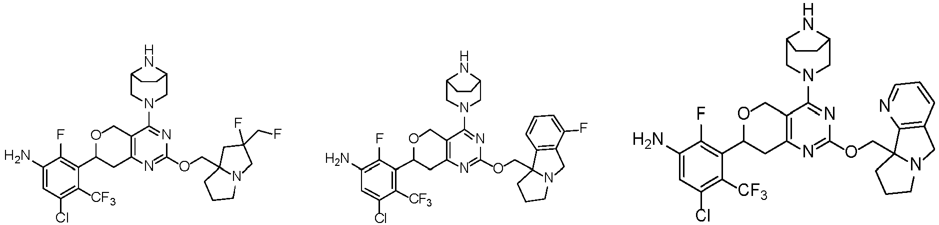

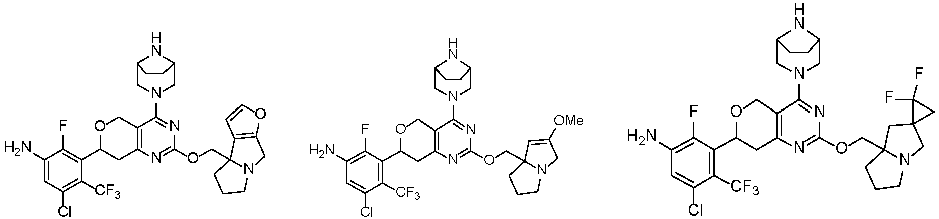

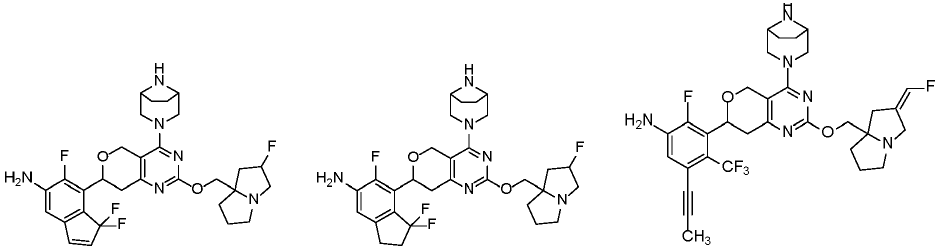

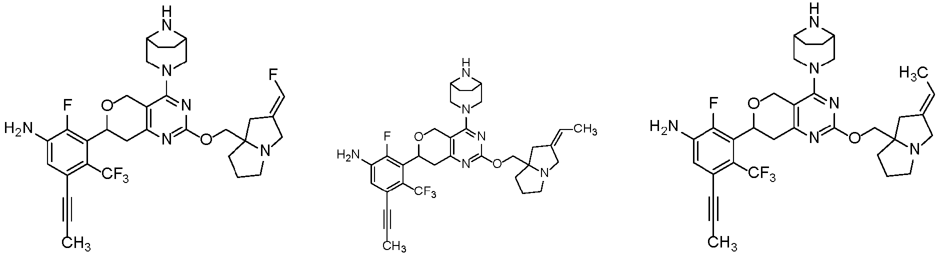

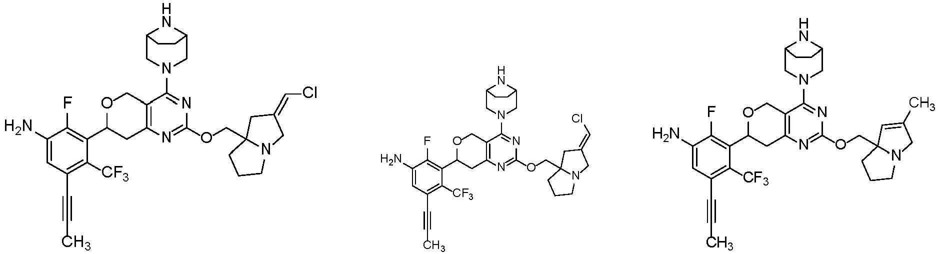

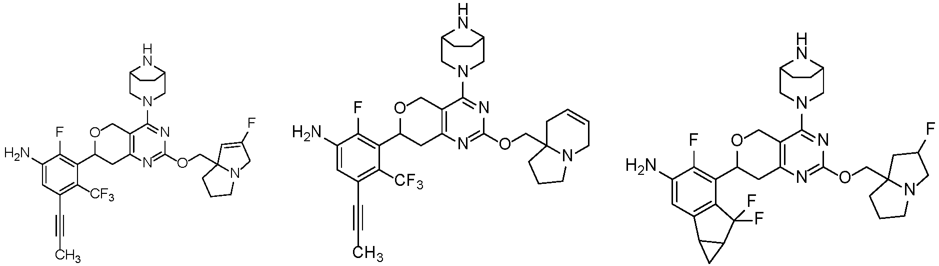

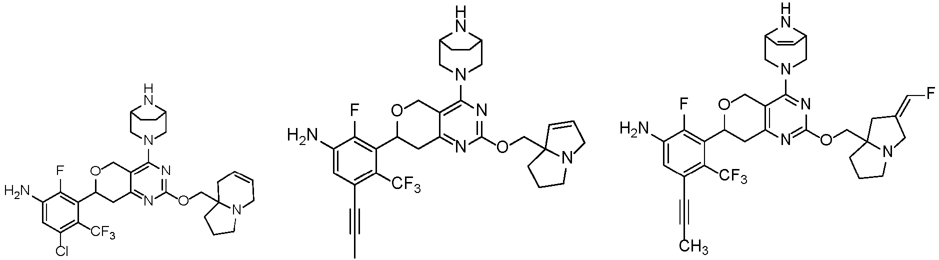

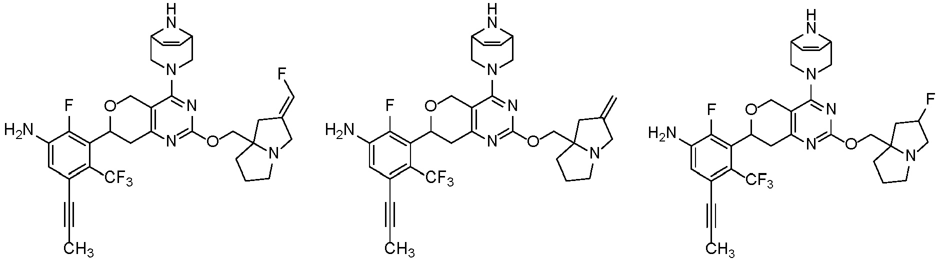

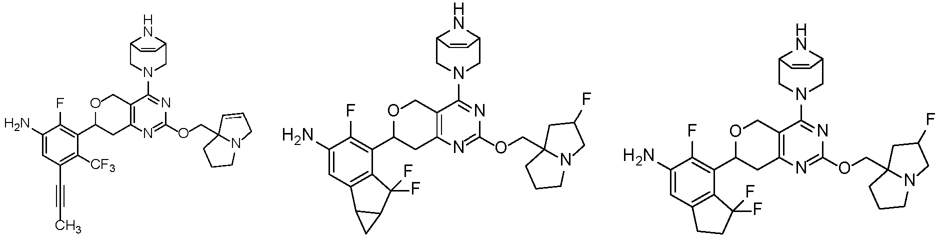

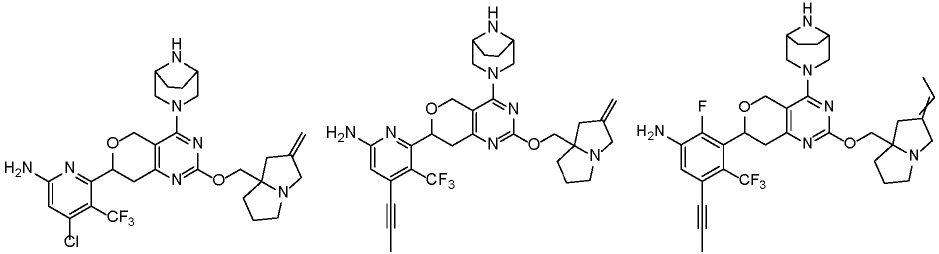

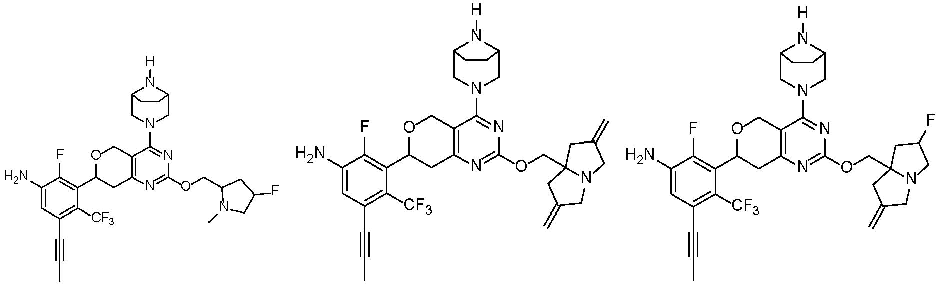

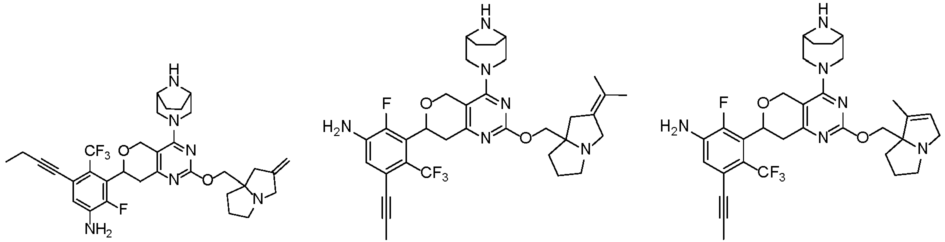

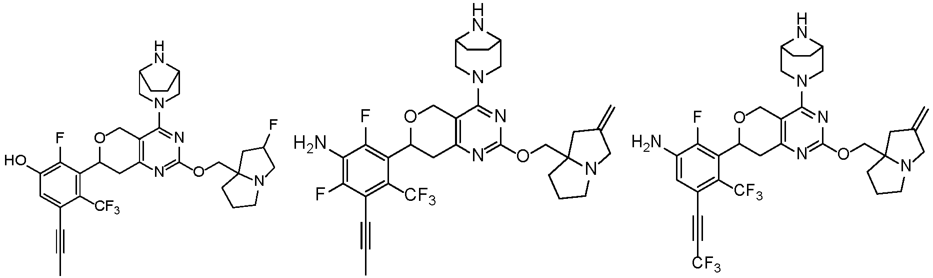

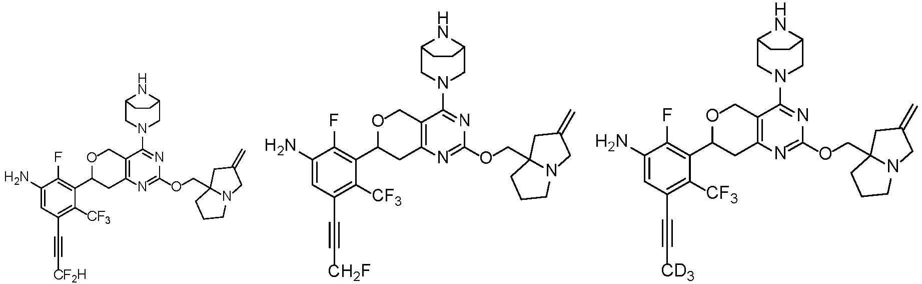

The present disclosure provides a compound represented by the following formula or a pharmaceutically acceptable salt thereof:

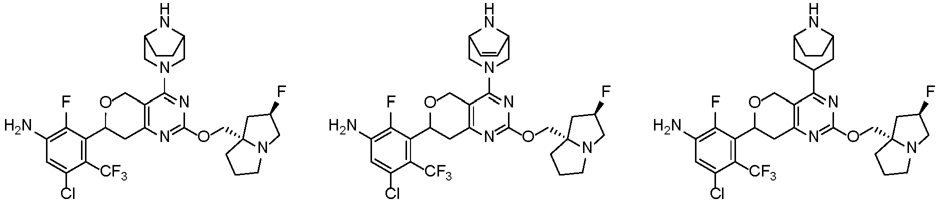

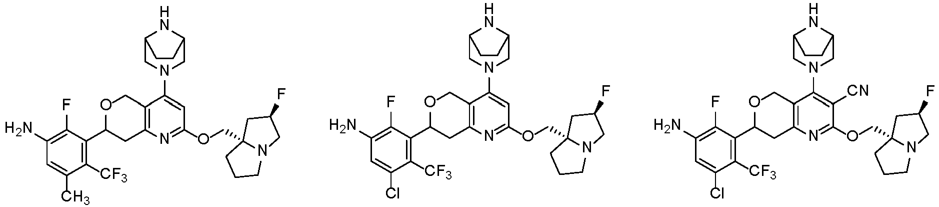

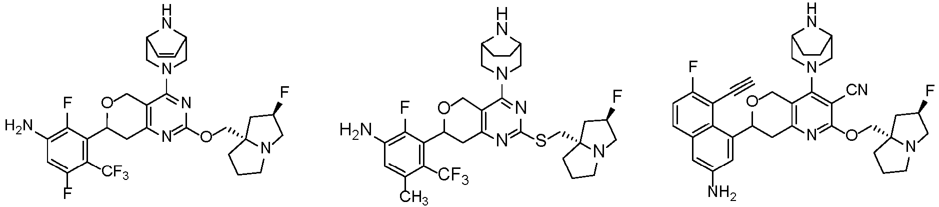

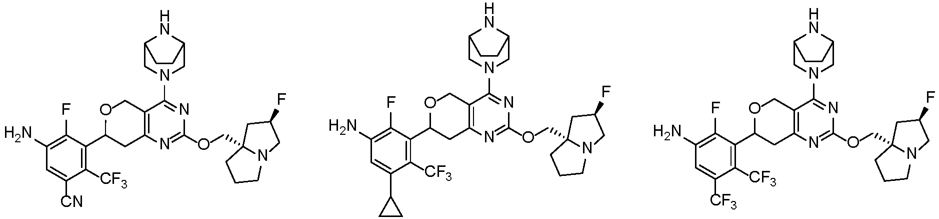

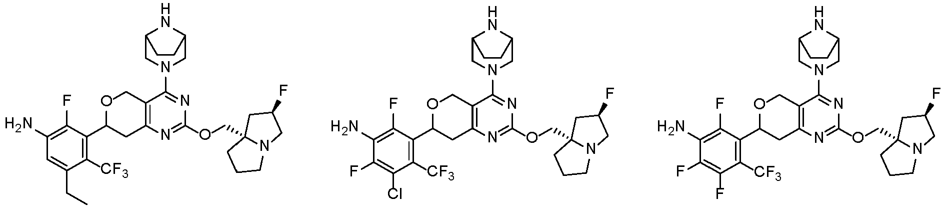

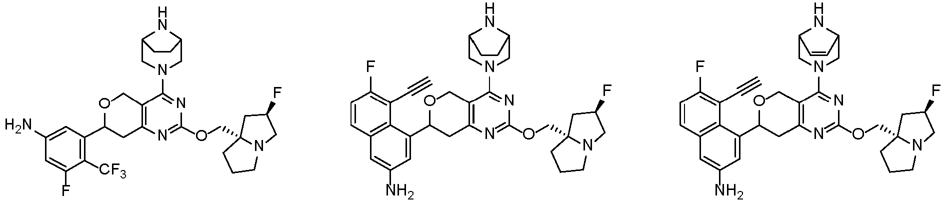

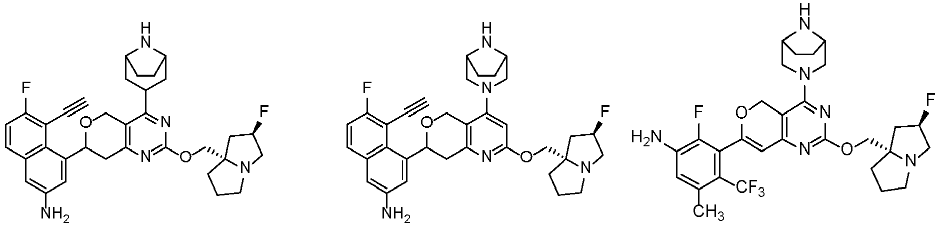

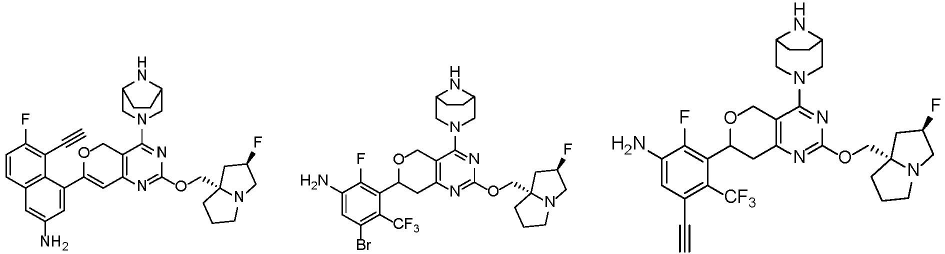

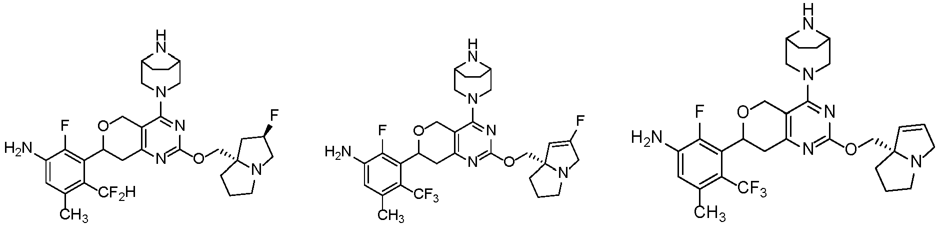

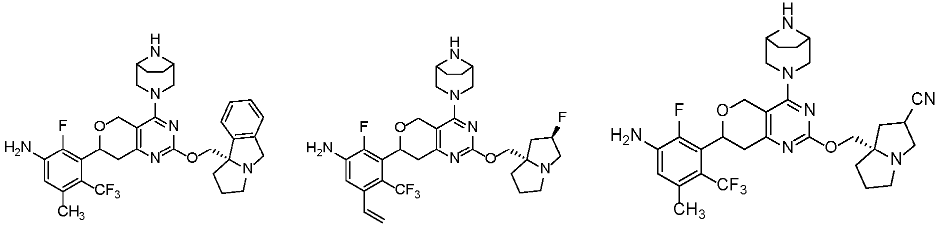

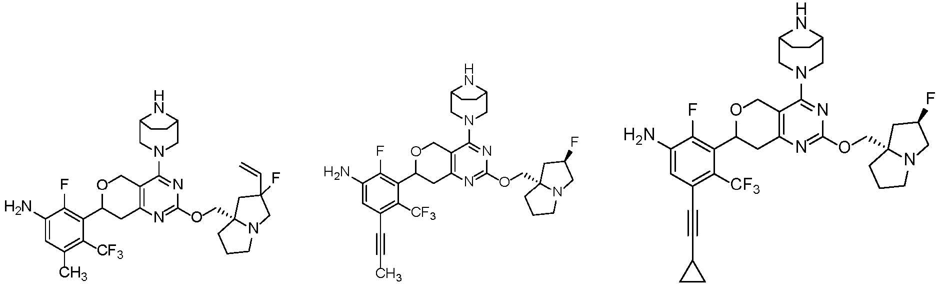

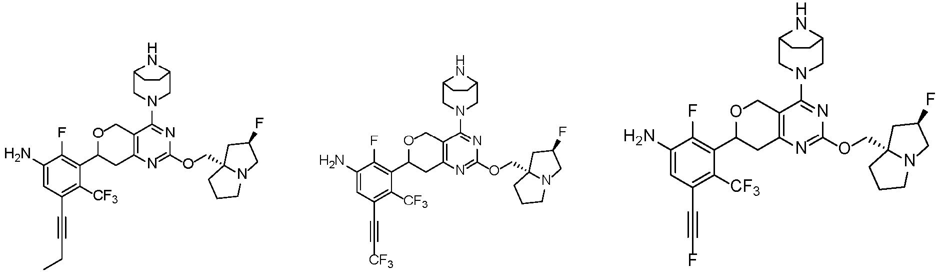

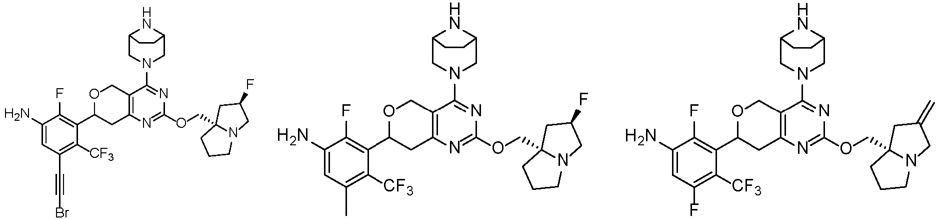

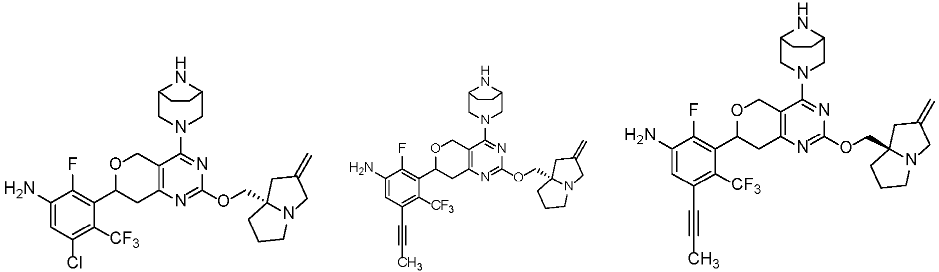

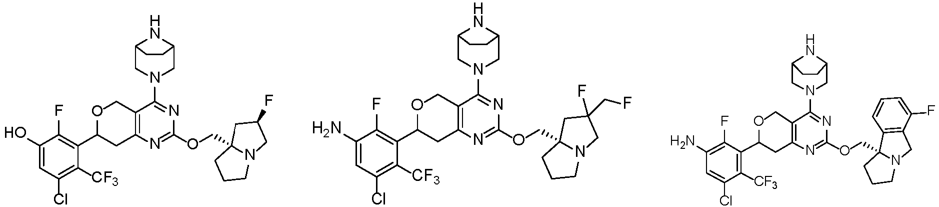

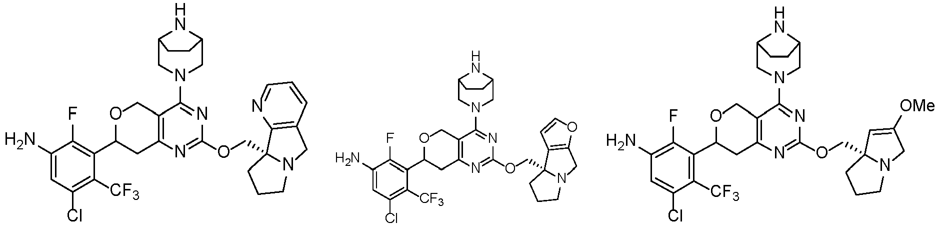

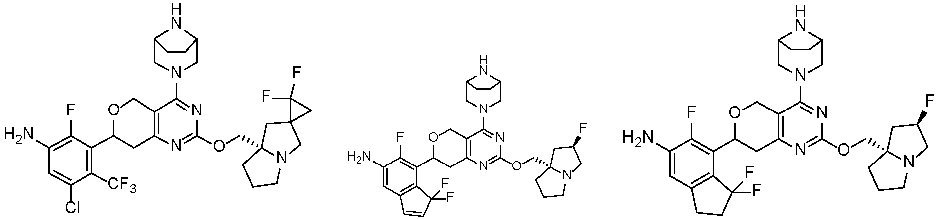

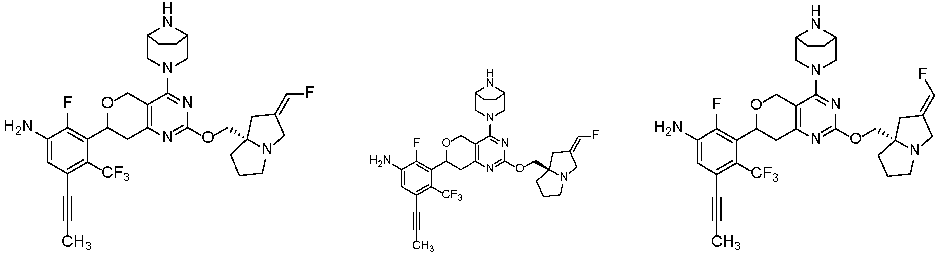

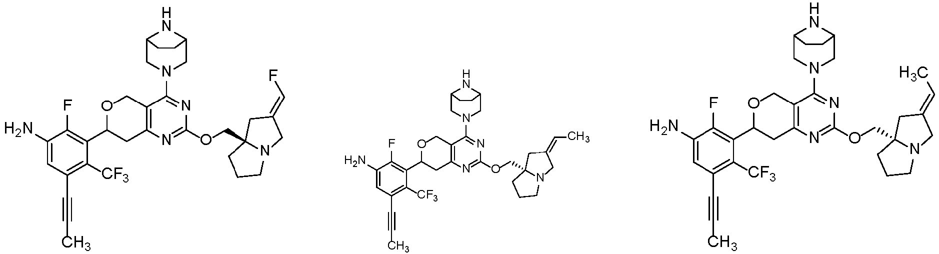

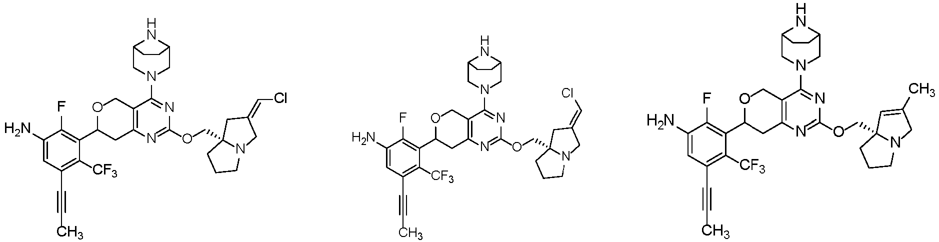

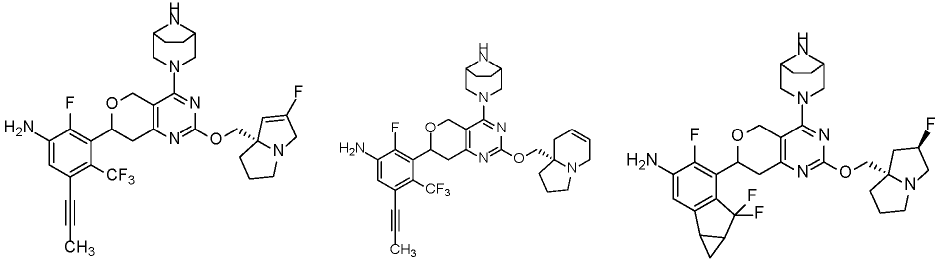

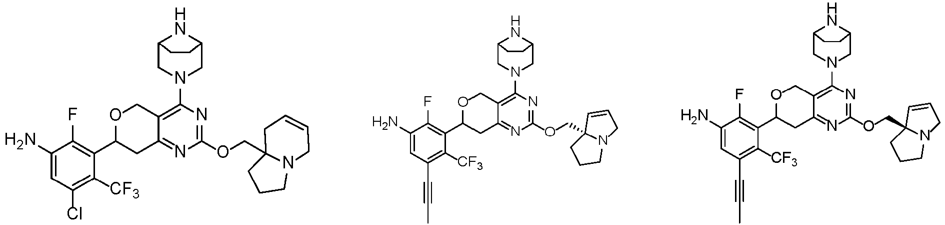

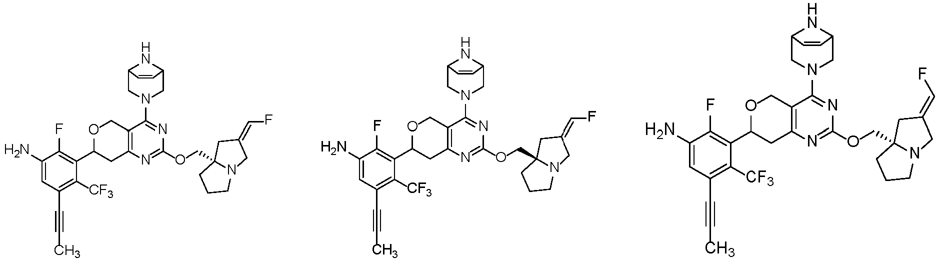

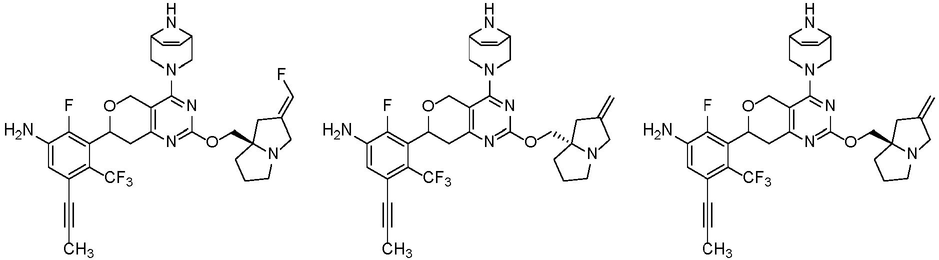

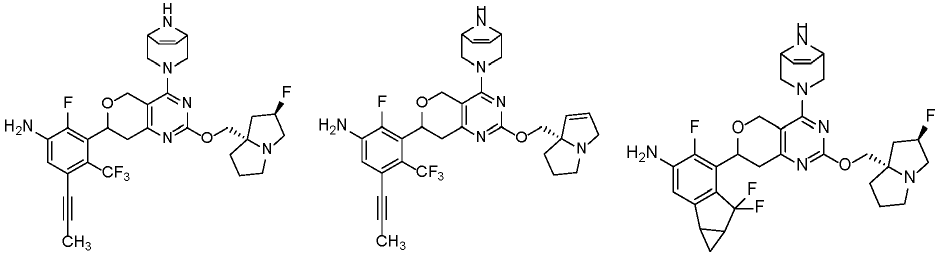

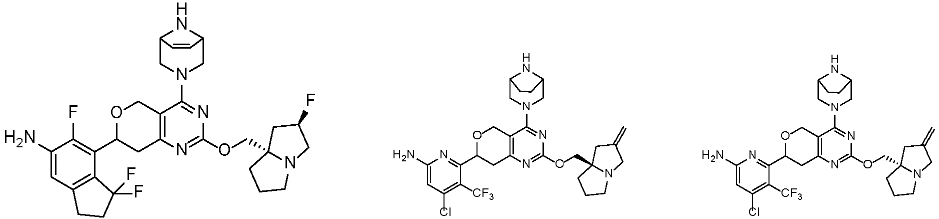

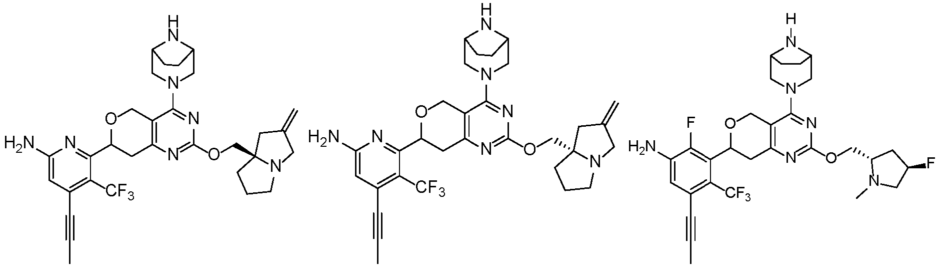

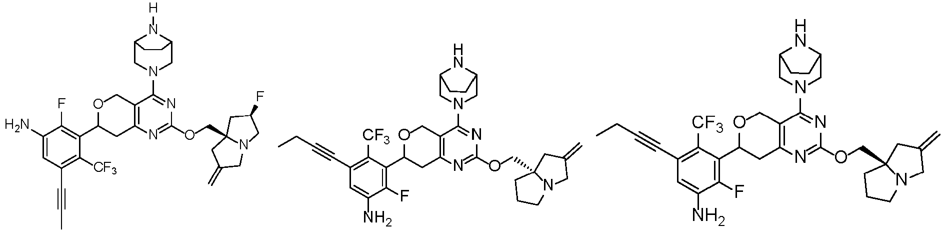

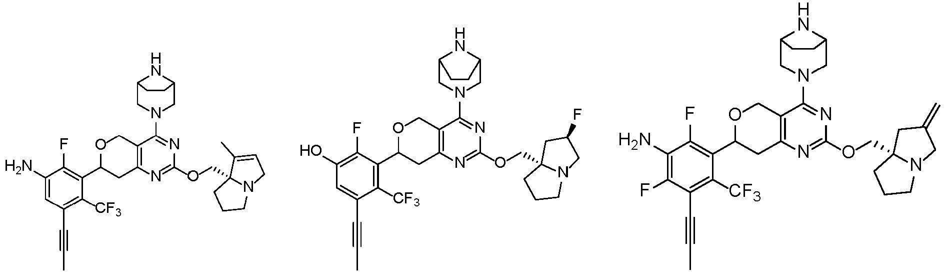

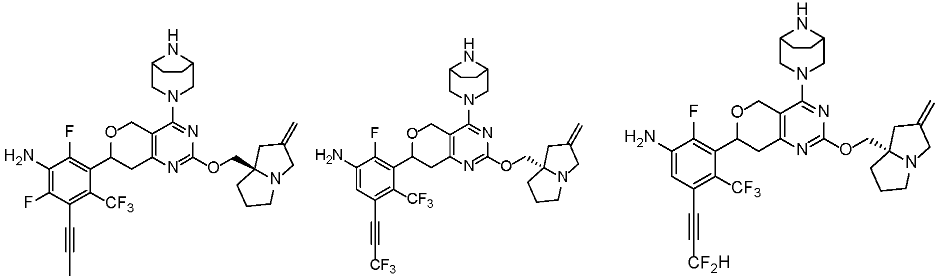

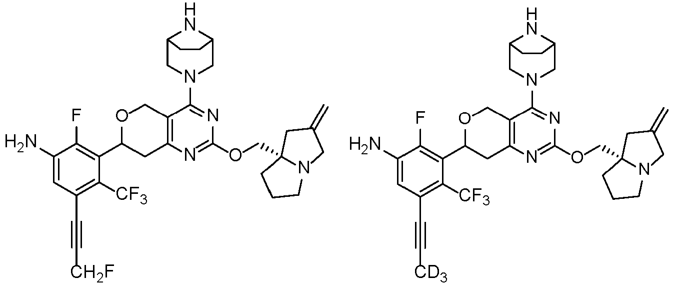

The present disclosure provides a compound represented by the following formula or a pharmaceutically acceptable salt thereof:

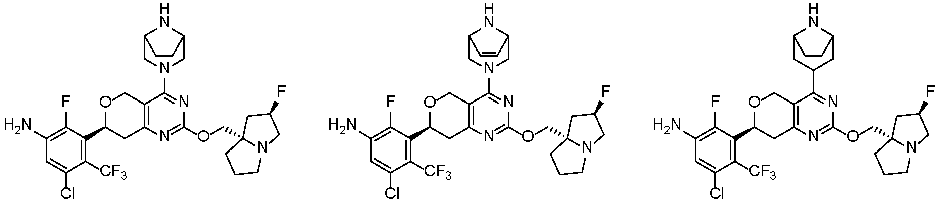

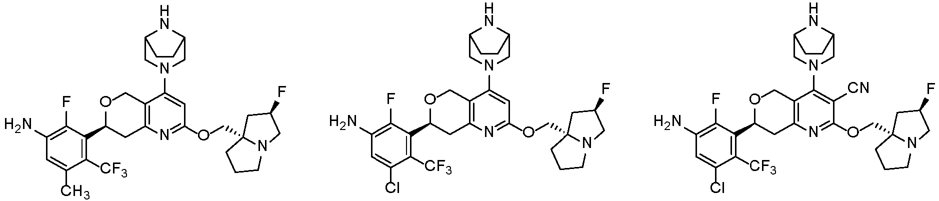

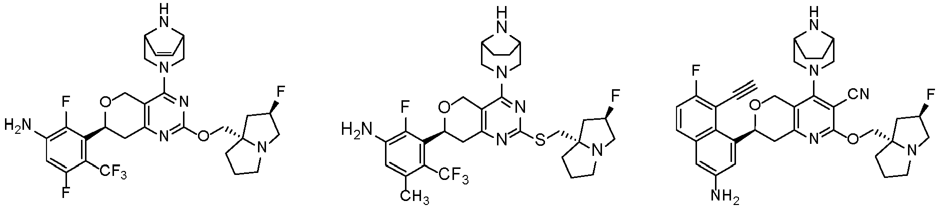

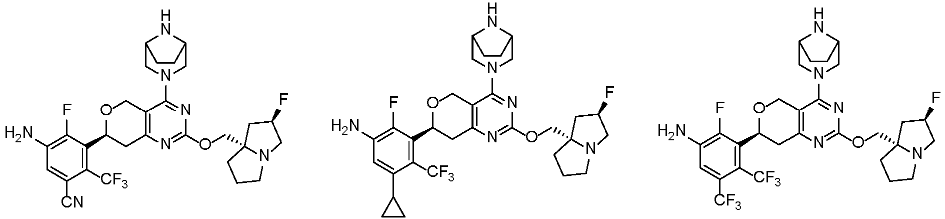

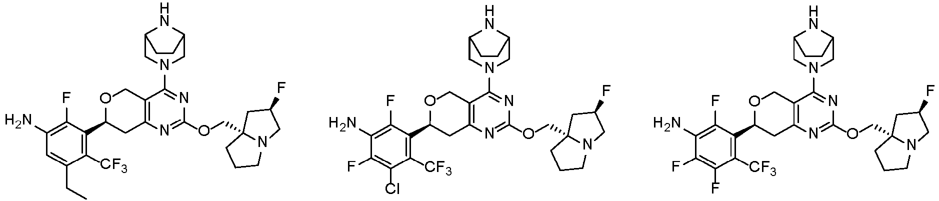

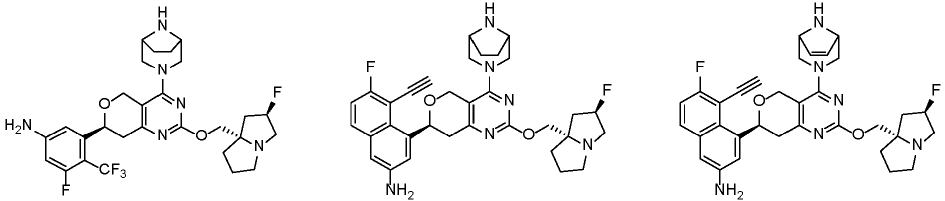

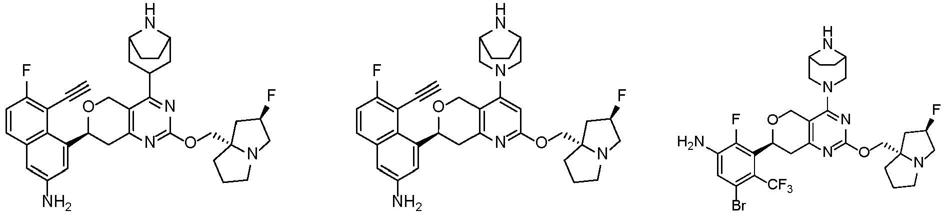

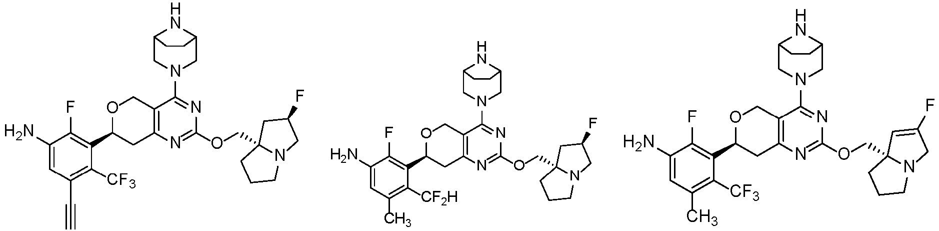

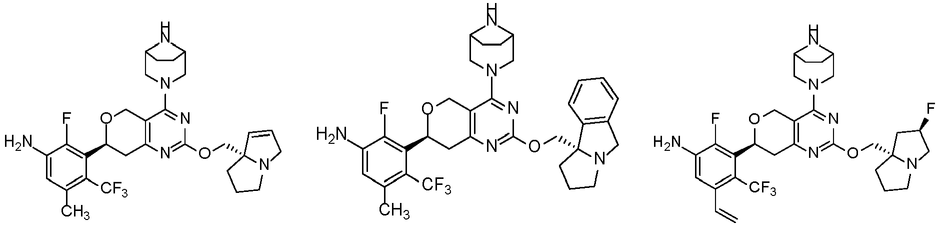

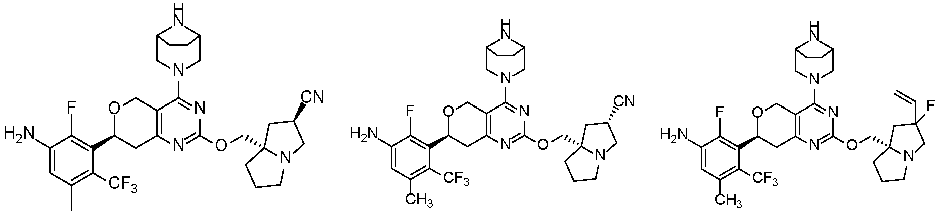

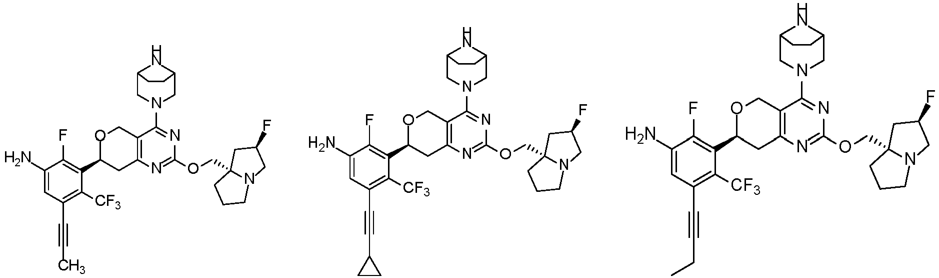

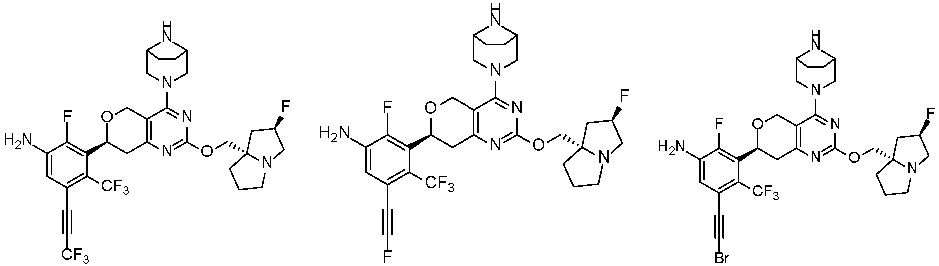

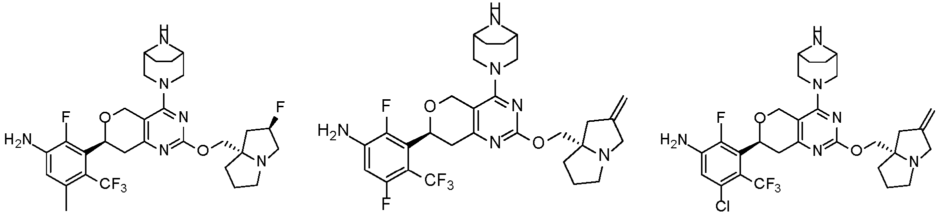

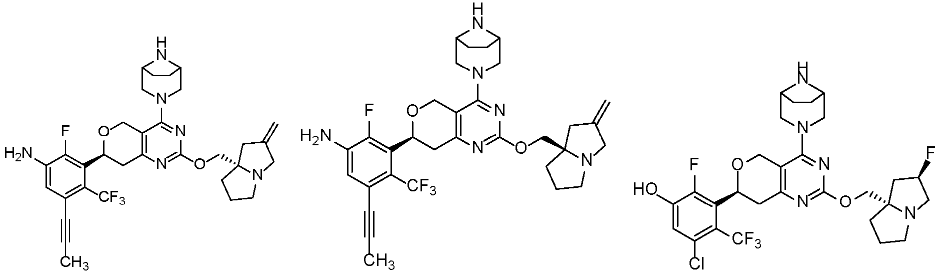

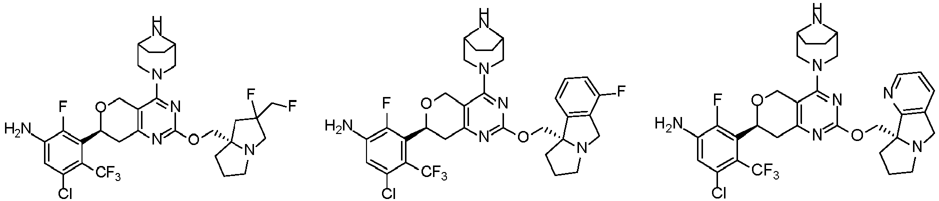

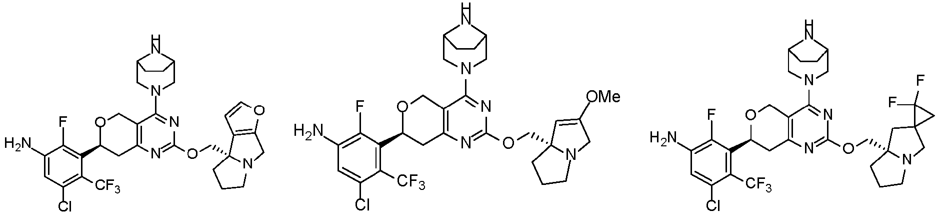

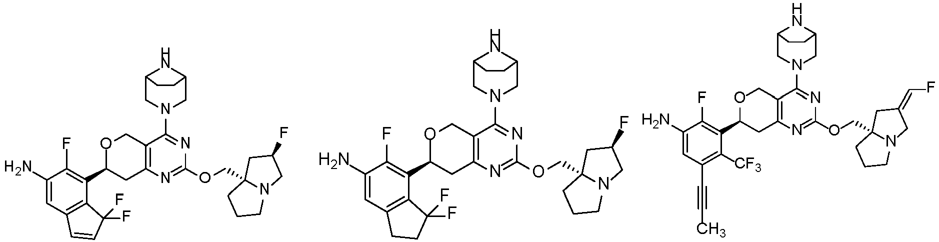

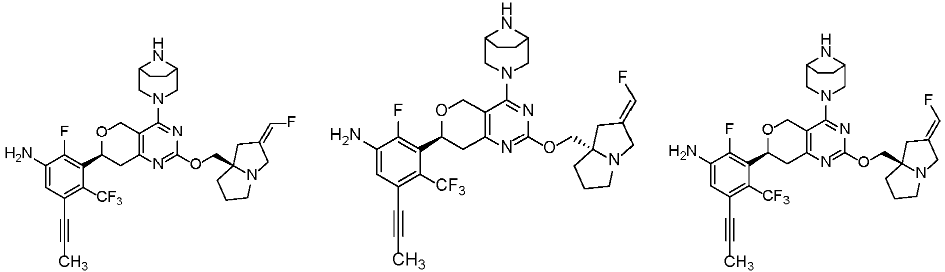

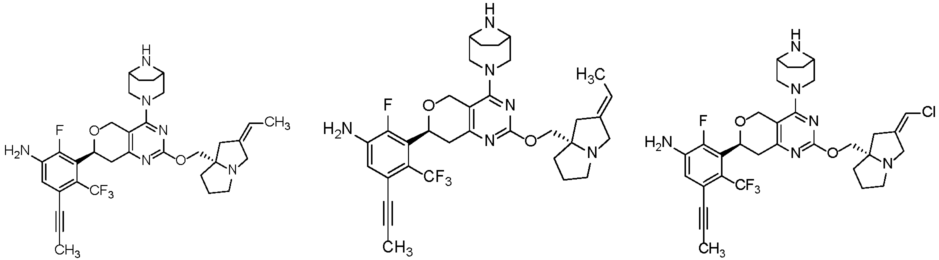

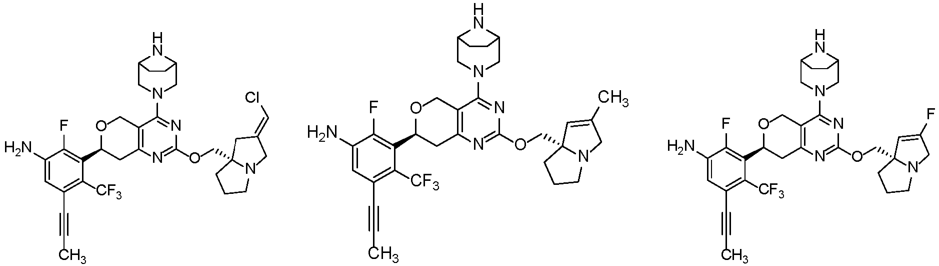

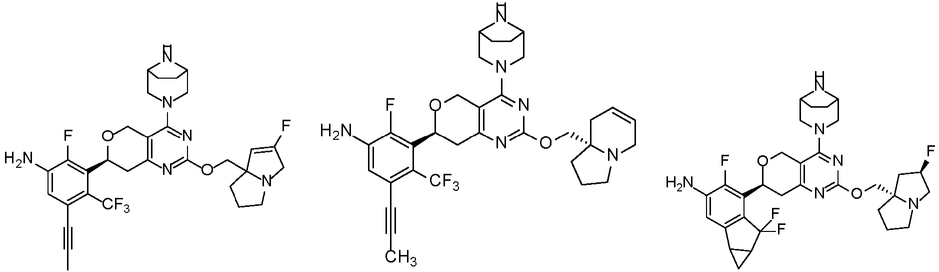

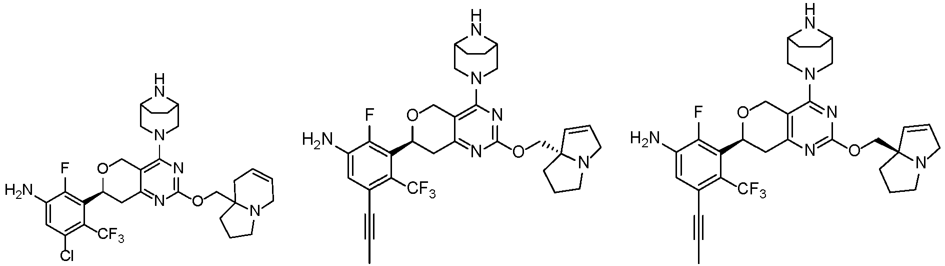

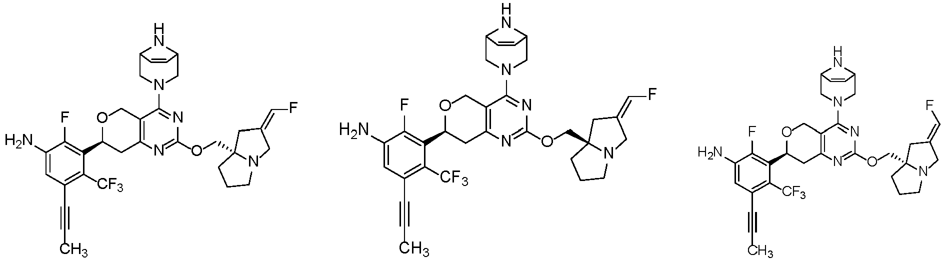

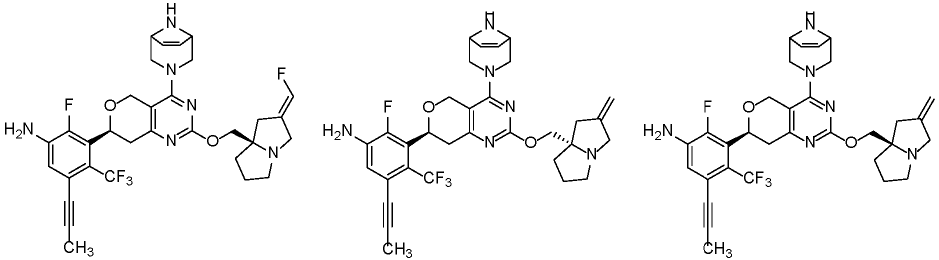

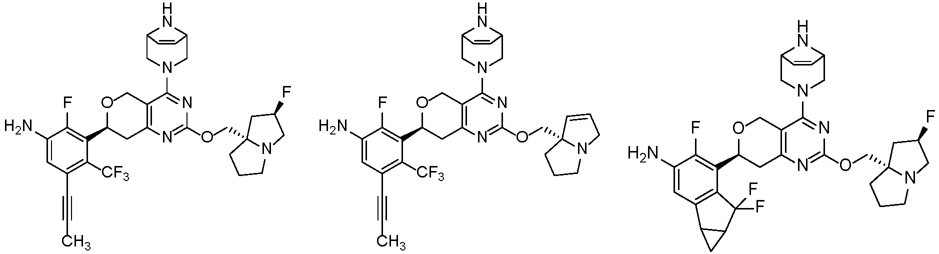

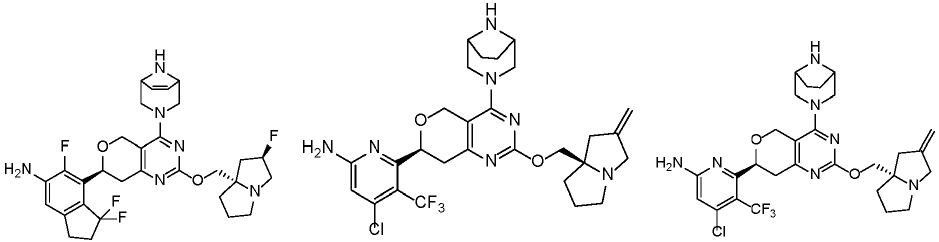

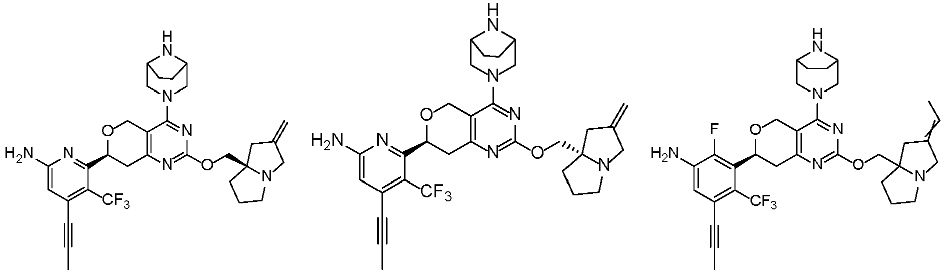

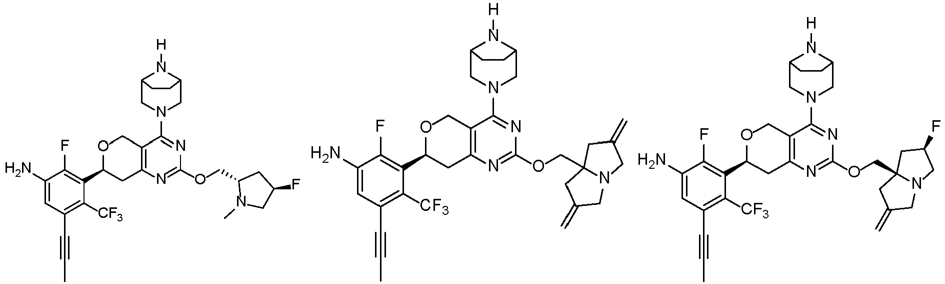

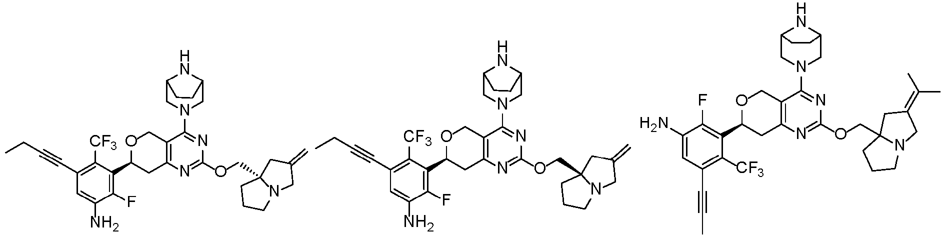

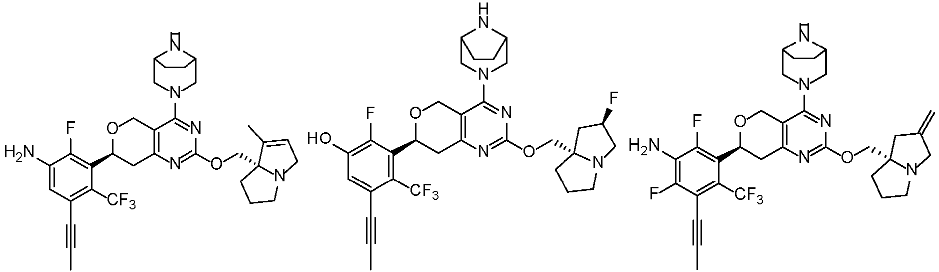

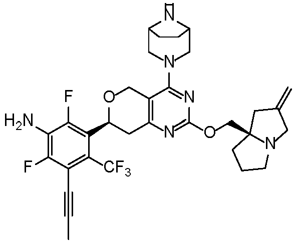

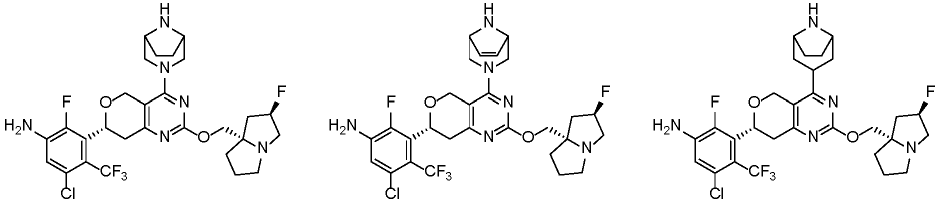

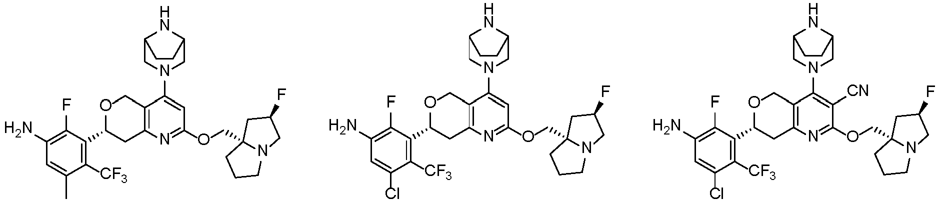

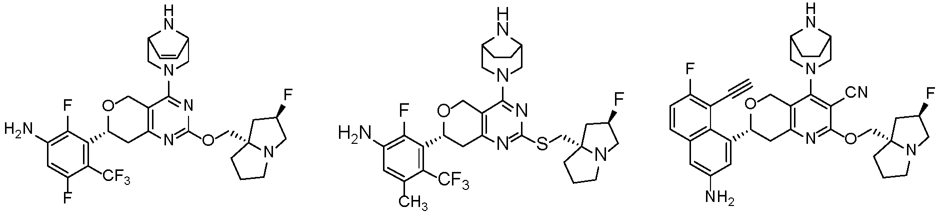

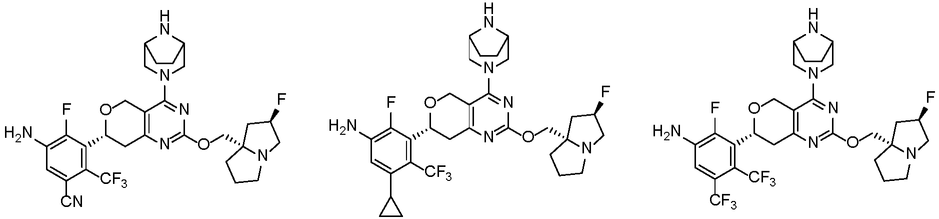

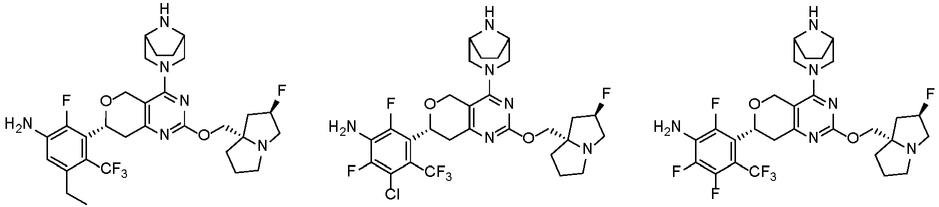

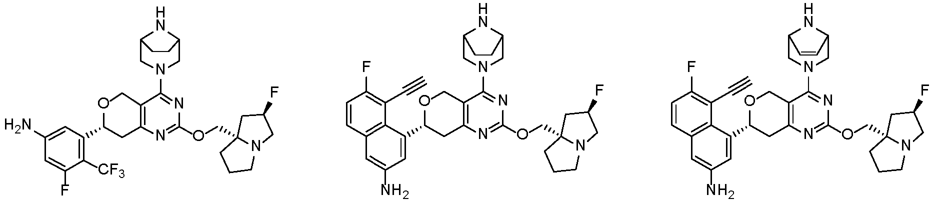

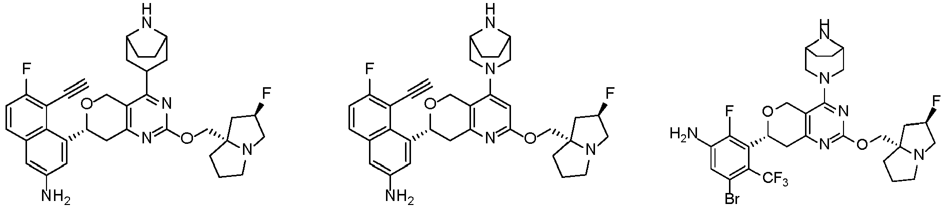

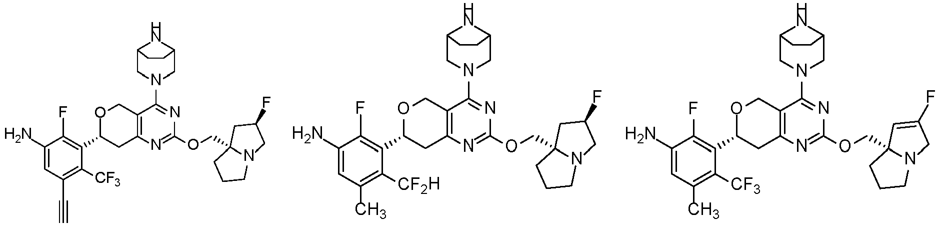

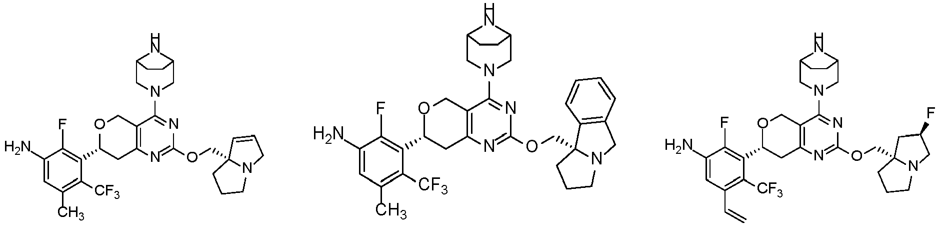

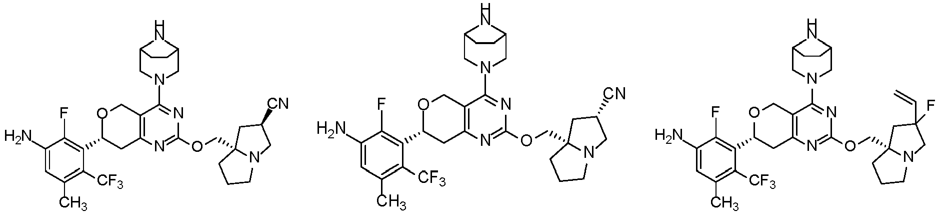

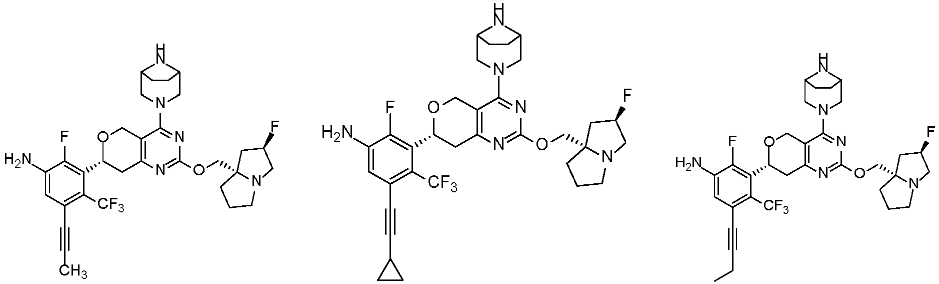

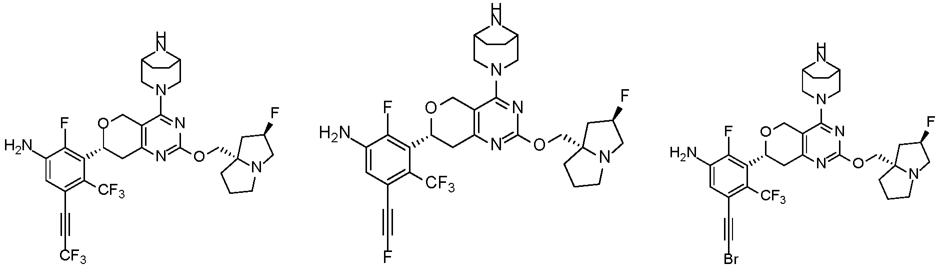

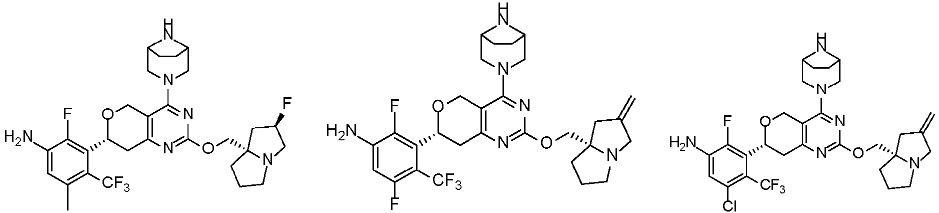

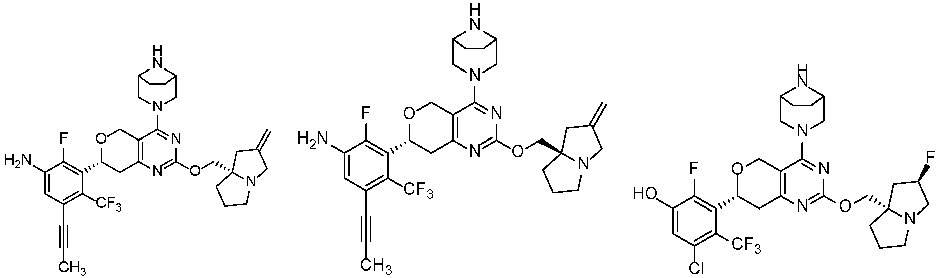

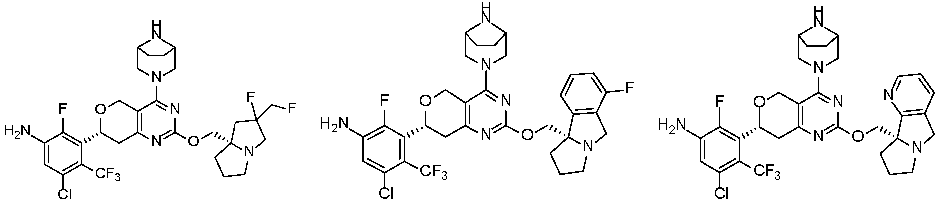

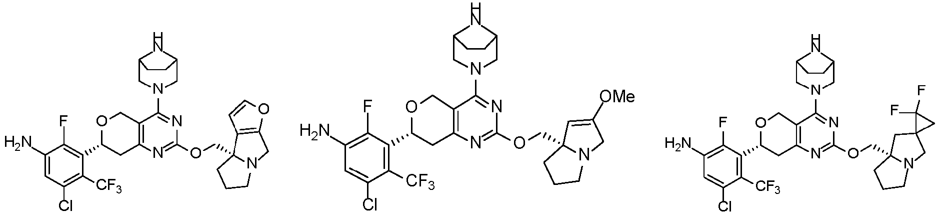

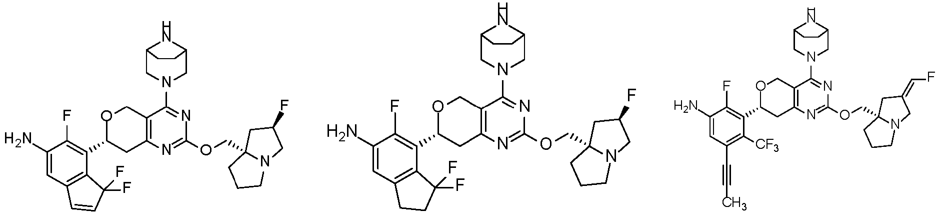

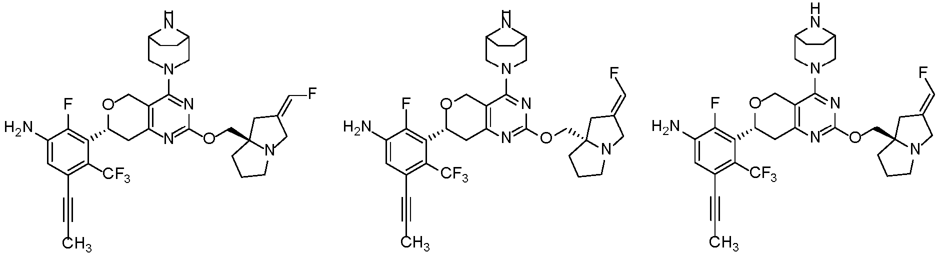

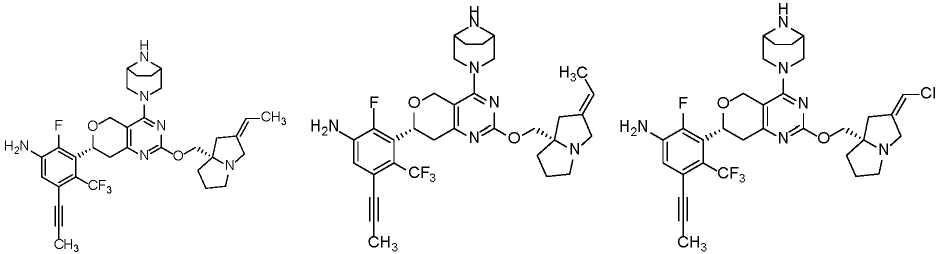

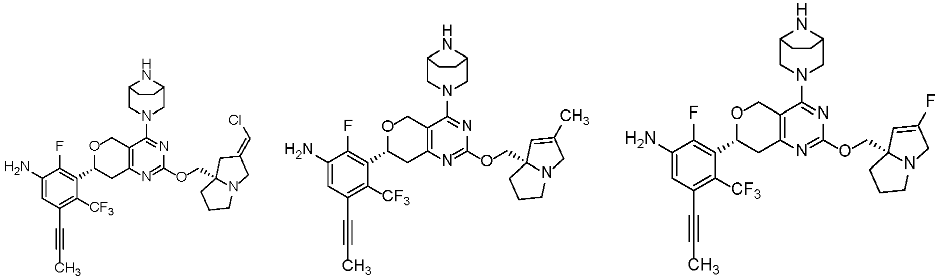

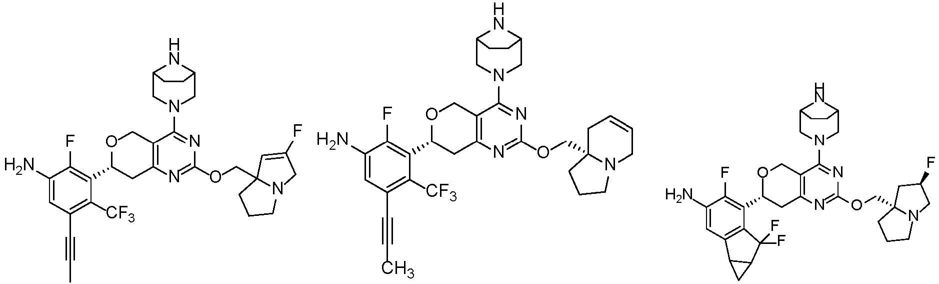

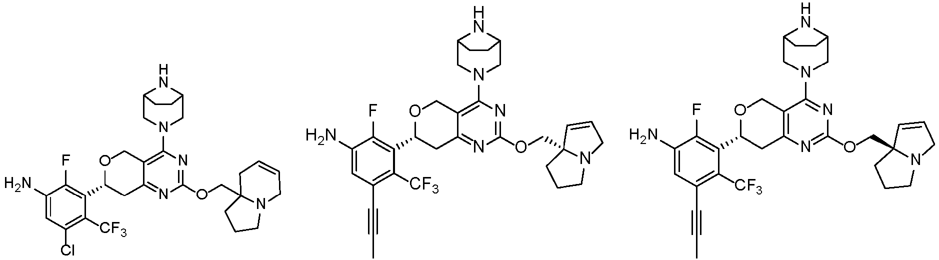

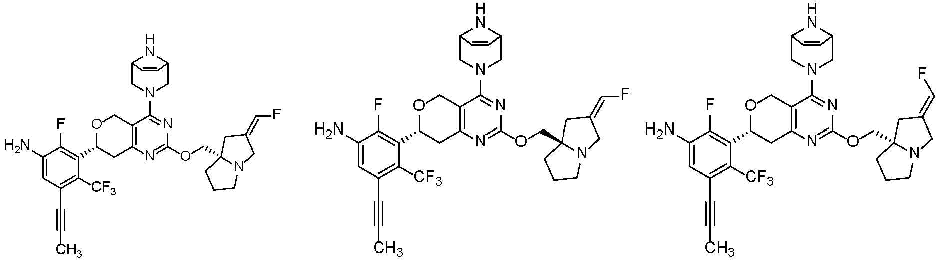

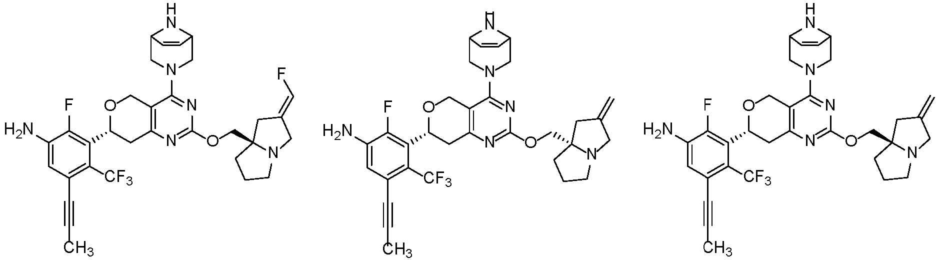

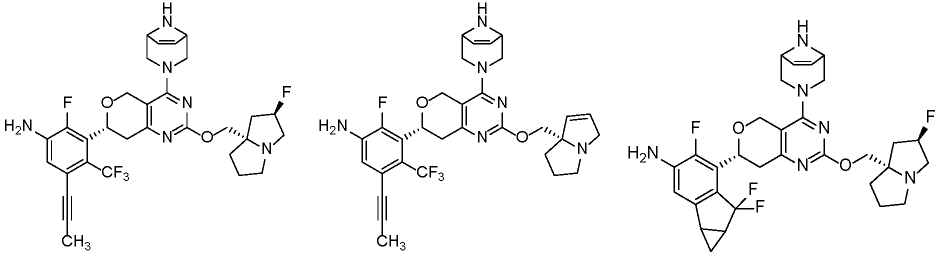

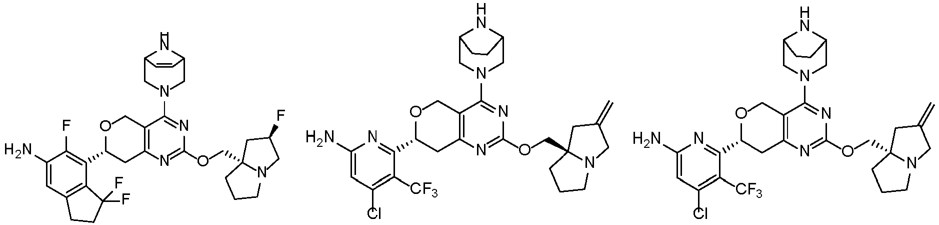

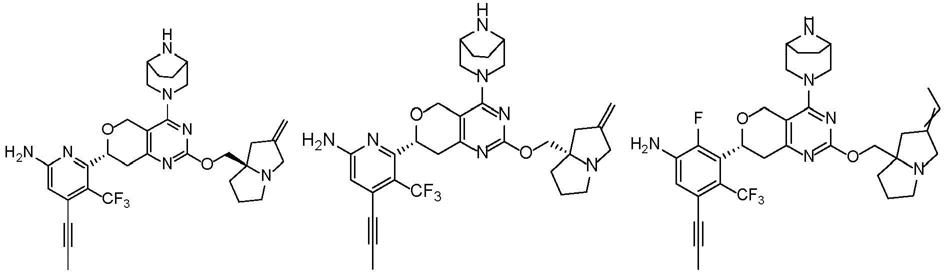

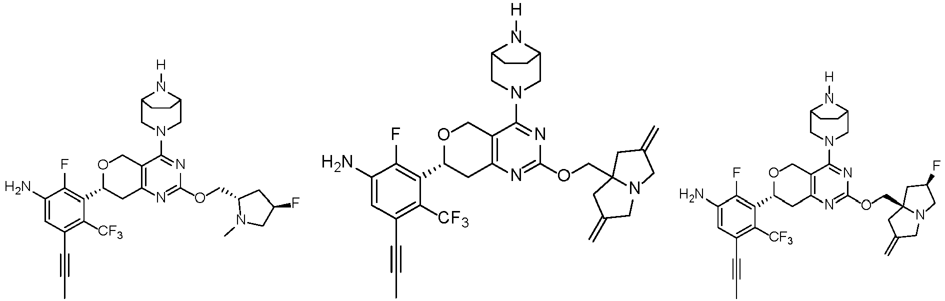

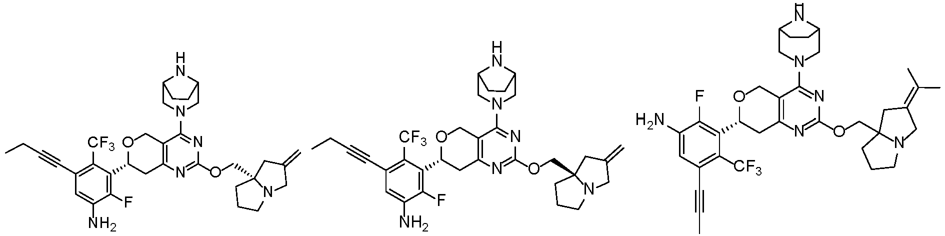

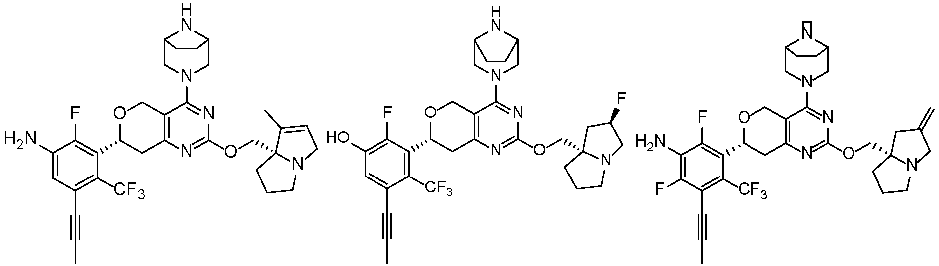

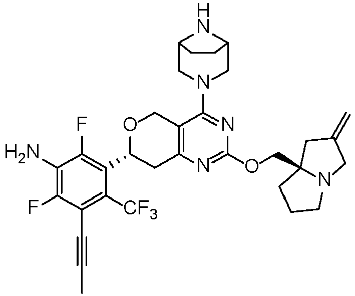

The present disclosure also provides use of the above compound or a pharmaceutically acceptable salt thereof in the manufacture of a medicament for treating a disease associated with KRAS^{G12D} mutation.

The present disclosure also provides use of the above compound or a pharmaceutically acceptable salt thereof in the manufacture of a medicament for treating a disease associated with a tumor.

The present disclosure also provides the following synthesis methods:

### Synthesis method 1:

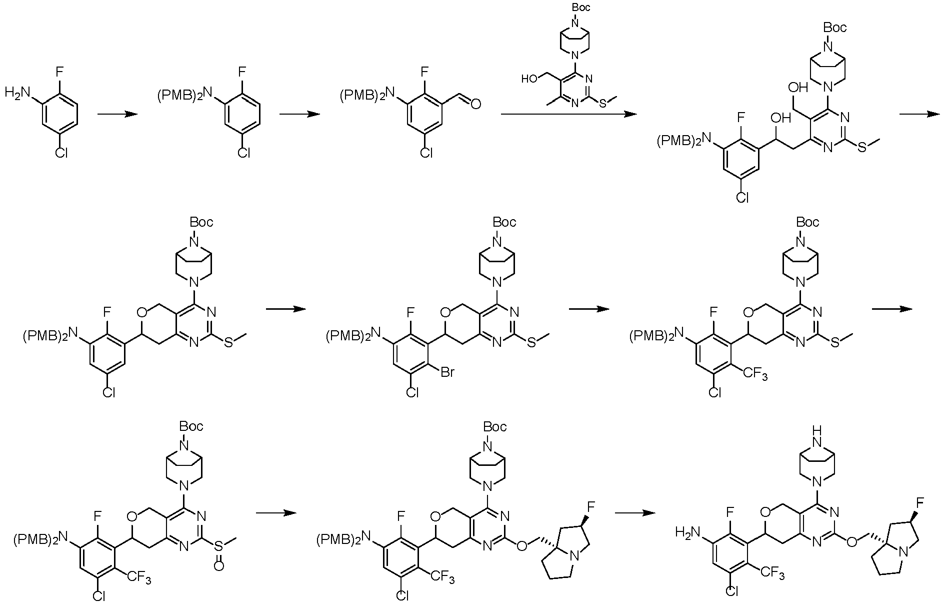

### Synthesis method 2:

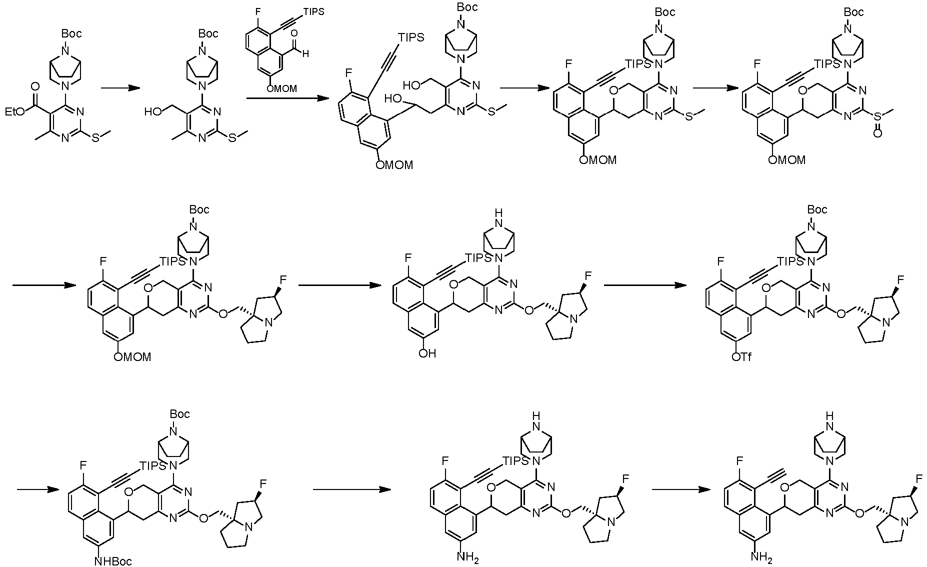

The present disclosure also provides the following assay methods:

### Assay method 1. Assay of activity of inhibiting KRAS^{G12D}

### 1. Purpose

Compounds that can effectively inhibit the binding of KRAS to GTP were screened out by a TR-FRET method.

### 2. Consumable and instrument

**Table 1. Consumable and instrument**

| Name | Vendor | Item No. |
|---|---|---|
| HEPES (4-(2-hydroxyethyl)-1-piperazineethanesulfonic acid) pH 7.3 | Thermo Fisher | BP299-500 |
| Sodium chloride | Promega | V4221 |
| EDTA (ethylenediaminetetraacetic acid) | EMD Millipore | 324506 |
| Tween 20 | Bole | 1706531 |
| Magnesium chloride | MP Biomedicine | 191421 |
| Bodipy GDP (guanosine 5'-diphosphate, BODIPY^{™} FL 2'-(or -3')-O-(N-(2-aminoethyl)urethane), bis(triethylammonium) salt) | Yingjie | G22360 |
| GTP (guanine-5'-triphosphate) | Sigma | G8877 |
| Tb-SA (Terbium-labeled streptavidin) | Yingjie | PV3576 |
| SOS (son of sevenless) protein | | |
| KRAS^{G12D} (Kirsten rat sarcoma viral oncogene) protein | | |
| Compound plate | Labcyte | LP-0200 |
| Assay plate | Perkin Elmer | 6008269 |
| 15-milliliter centrifuge tube | Corning | 430791 |
| 1.5-milliliter centrifuge tube | Axygen | MCT-150-C |
| Dragonfly automatic sampler | TTP | |
| Bravo | Agilent | |
| Echo 550 | Labcyte | |
| Envision | Perkin Elmer | |

### 3. Reagent Preparation

### a. Stock reagents:

### 1) KRAS nucleotide exchange buffer

20 mL of 1000mM HEPES, 20 mL of 500mM EDTA, 10 mL of 5 M sodium chloride, 0.1 mL of 100% Tween 20, and 949.9 mL of water were weighed and formulated to 1 L of solution. The solution was sterilized by filtration and stored at 4 °C.

### 2) KRAS assay buffer

20 mL of 1000 mM HEPES, 10 mL of 1000mM magnesium chloride, 30 mL of 5 M sodium chloride, 0.05 mL of 100% Tween 20, and 939.95mL of water were weighed and formulated to 1 L of solution. The solution was sterilized by filtration and stored at 4 °C.

### 3) KRAS/Bodipy GDP/Tb-SA mixture

9.5 µL of 95 µM KRAS^{G12D} protein and 440.5 µL of KRAS nucleotide exchange buffer were weighed and mixed. The mixture was incubated at room temperature for 1 hour, and then formulated to 1 L of solution together with 8.4 µL of 17.9 µM Tb-SA, 1.8 µL of 5mM Bodipy GDP and 9539.8 µL of KRAS assay buffer. After mixing, the solution was left to stand at room temperature for 6 hours, and stored at -80 °C.

### b. Assay reagents:

### 1) KRAS kinase solution

73.3 µL of KRASBodipy GDP/Tb-SA mixture and 2126.7 µL of KRAS assay buffer were weighed and formulated to 2200 µL of solution.

### 2) SOS/GTP mixture

1.59 µL of 166 µM SOS protein, 198 µL of 100 mM GTP and 2000.41 µL of KRAS assay buffer were weighed and formulated to 2200 µL of solution.

### 4. Assay process

1) The concentration of a stock solution of the control compound was 1 mM, and the concentration of a stock solution of compounds to be assayed was 10 mM. 9 µL of the control compound and compounds to be assayed were transfered to a 384-LDV plate;
2) The compounds on the LDV plate were serially diluted 3-fold with Bravo to 10 concentrations;
3) 9 nL of the compounds on the LDV plate were transfered to an assay plate using ECHO;
4) 3 µL of 3 nM Kras/0.5 nM TB-SA/30 nM BodipyGDP mixture and 3 µL of Ras buffer were sequentially added to each well of the assay plate using a Dragonfly automatic sampler, and the assay plate was centrifuged at 1000rpm/min for 1 minute;
5) The assay plate was incubated at room temperature for 1 hour;
6) 3 µL of 120 nM SOS/9 mM GTP mixture was added to each well of the assay plate using a Dragonfly automatic sampler, and the assay plate was centrifuged at 1000rpm/min for 1 minute;
7) The assay plate was incubated at room temperature for 1 hour;
8) The plate was read with Envision and data were recorded;
9) The data were analysed using Excel and Xlfit, and IC₅₀ of the compounds to be assayed were calculated.

### Assay method 2. Assay of p-ERK inhibition in AGS cells

### 1. Purpose

Compound that can effectively inhibit p-ERK in AGS cells was screened out by a HTRF method.

### 2. Assay process

1) AGS cells were inoculated in a transparent 96-well cell culture plate, and each well contained 80µL of cell suspension and 10000 cells. The cell plate was inoculated in a carbon dioxide incubator at 37 °C overnight;
2) After completion of the incubation, the cell supernatant was discarded. 80 µL of medium containing 0.02% serum was added to each well. The cell plate was incubated in a carbon dioxide incubator at 37 °C overnight;
3) 2 µL of the compound was weighed and added to 78 µL of the cell medium. After the mixture was mixed thoroughly, 20 µL of the solution of compound was weighed and added to the corresponding well of the cell plate. The cell plate was placed back to the carbon dioxide incubator and incubated for another 3 hours;
4) After completion of the incubation, the cell supernatant was discarded. 50 µL of 1X cell lysate was added to each well. The mixture was incubated with shaking at room temperature for 30 minutes;
5) Phospho-ERK1/2 Eu Cryptate antibody and Phospho-ERK1/2 d2 antibody were diluted 20-fold with detection buffer;
6) 16 µL of cell lysate supernatant was weighed and added into each well of a new 384 white microwell plate, and then 2 µL of the diluted solution of Phospho-ERK1/2 Eu Cryptate antibody and 2 µL of the diluted solution of Phospho-ERK1/2 d2 antibody dilution were added. The mixture was incubated at room temperature for at least 4 hours;
7) After completion of the incubation, HTRF was read with multi-label analyzer: excitation: 320nm, emission: 615nm, 665nm;
8). IC₅₀ of the compound to be assayed was calculated.

### Assay method 3. Assay of p-ERK inhibition in GP2D cells

### 1. Purpose

Compounds that can effectively inhibit p-ERK in GP2D cells were screened out by a HTRF method.

### 2. Assay process

1) GP2D cells were inoculated in a transparent 96-well cell culture plate, and each well contained 80µL of cell suspension and 8000 cells. The cell plate was inoculated in a carbon dioxide incubator at 37 °C overnight;
2) 2 µL of the compound was weighed and added to 78 µL of the cell medium. After the mixture was mixed thoroughly, 20 µL of the solution of compound was weighed and added to the corresponding well of the cell plate. The cell plate was placed back to the carbon dioxide incubator and incubated for another 1 hour;
3) After completion of the incubation, the cell supernatant was discarded. 50 µL of 1X cell lysate was added to each well. The mixture was incubated with shaking at room temperature for 30 minutes;
4) Phospho-ERK1/2 Eu Cryptate antibody and Phospho-ERK1/2 d2 antibody were diluted 20-fold with detection buffer;
5) 16 µL of cell lysate supernatant was weighed and added into each well of a new 384 white microwell plate, and then 2 µL of the diluted solution of Phospho-ERK1/2 Eu Cryptate antibody and 2 µL of the diluted solution of Phospho-ERK1/2 d2 antibody dilution were added. The mixture was incubated at room temperature for at least 4 hours;
6) After completion of the incubation, HTRF was read with multi-label analyzer: excitation: 320nm, emission: 615nm, 665nm;
7). IC₅₀ of the compounds to be assayed was calculated.

### Assay method 4. Assay of p-ERK inhibition in PANC0403 cells

### 1. Materials of the assay:

PANC0403 cells purchased from Nanjing Kebai; RPMI-1640 medium purchased from Biological Industries; fetal bovine serum purchased from Biosera; and Advanced Phospho-ERK1/2(THR202/TYR204) KIT purchased from Cisbio.

**Table 2. The composition of Advanced Phospho-ERK1/2(THR202/TYR204) KIT**

| Ingredient name | Storage temperature |
|---|---|
| Advanced PhosphoERK1/2 Eu Cryptate antibody | ≤-16°C |
| Advanced PhosphoERK1/2 d2 antibody | ≤-16°C |
| Blocking reagent (stock solution 100X) | ≤-16°C |
| Lysis buffer # 1 (stock solution 4X) | ≤-16°C |
| Detection buffer (ready-to-use) | ≤-16°C |

### 2. Method of the assay:

1) PANC0403 cells were inoculated in a transparent 96-well cell culture plate, and each well contained 80µL of cell suspension and 10000 PANC0403 cells. The cell plate was inoculated in a carbon dioxide incubator at 37 °C overnight;
2) The compound to be assayed was diluted to 2 mM as the first concentration with 100% DMSO, and then serially diluted 5-fold to the eighth concentration with a pipette, i.e., from 2 mM to 25.6 nM. 2 µL of the compound was weighed and added to 78 µL of a cell starvation medium. After the mixture was mixed thoroughly, 20 µL of the solution of compound was weighed and added to the corresponding well of the cell plate. The cell plate was placed back to the carbon dioxide incubator and incubated for another 3 hours; at this time, the concentration of the compound was 10 µM to 0.128 nM, and the concentration of DMSO was 0.5%;
3) After completion of the incubation, the cell supernatant was discarded. 50 µL of cell lysate was added to each well. The mixture was incubated with shaking at room temperature for 30 minutes;
4) Phospho-ERK1/2 Eu Cryptate antibody and Phospho-ERK1/2 d2 antibody were diluted 20-fold with Detection buffer;
5) 16 µL of cell lysate supernatant was weighed and added into each well of a new 384 white microwell plate, and then 2 µL of the diluted solution of Phospho-ERK1/2 Eu Cryptate antibody and 2 µL of the diluted solution of Phospho-ERK1/2 d2 antibody dilution were added. The mixture was incubated at room temperature overnight;
6) After completion of the incubation, HTRF was read with multi-label analyzer: excitation: 320nm, emission: 615nm, 665nm;

### 3. Data analysis:

The equation **(Sample-Min)/(Max-Min)*100%** was used to convert the raw data into inhibition rate, and the IC₅₀ value can be obtained by curve fitting with four parameters ("log(inhibitor) vs. response -- Variable slope" mode in GraphPad Prism).
**Max well:** The reading value of the positive control well was that of 1X lysate.
**Min well:** The reading value of the negative control well was that of the cell lysate in 0.5% DMSO cell well.

### Assay method 5. Anti-cell proliferation effect of compounds in tumor cell lines AsPC-1 and GP2D

### Purpose of study

In this assay, the effect of the compounds on inhibiting cell proliferation was studied by detecting the effect of the compounds on the cell activity in vitro in the tumor cell lines AsPC-1 and GP2D.

### Materials of the assay

**Table 3. Cell line details**

| Cell line | Tumor type | Growth characteristics | Culture method |
|---|---|---|---|
| AsPC-1 | Pancreatic cancer | Adherent | RPMI 1640 + 10% FBS |
| GP2D | Colon cancer | Adherent | DMEM + 10% FBS + 2 mM L-glutamine |

Ultra Low Cluster-96-well plate (Corning-7007)
Greiner CELLSTAR 96-well plate (# 655090)
Promega CellTiter-Glo 3D Luminescence Cell Viability Assay Kit (Promega-G9683)
2104-10 EnVision Plate Reader, PerkinElmer
RPMI 1640, DMEM, PBS (phosphate buffered saline), FBS (fetal bovine serum), Antibiotic-antimycotic, L-glutamine, DMSO (dimethylsulfoxide)

### Method and steps of the assay

### Cell culture

The tumor cell lines were cultured in a 37 °C, 5% CO₂ incubator according to the culture conditions indicated in the culture method. Cells were passaged regularly, and cells in logarithmic growth phase were used for plating.

### Cell plating

Cells were stained with trypan blue and viable cells were counted.

The concentration of the cells was adjusted to the appropriate concentration (AsPC-1, 7000 cells/well; GP2D, 8000 cells/well).

To each well of a ULA culture plate was added 135 µL of cell suspension. To the blank control well was added the same volume of culture medium without cells.

After plating, the ULA culture plate was immediately centrifuged at room temperature at 1000 rpm for 10 minutes. Note: After completion of the centrifugation, subsequent operations must be processed carefully to avoid unnecessary shocks.

The culture plate was incubated in a 37 °C, 5% CO₂, 100% relative humidity incubator overnight.

### Formulation of 10X compound working solution and treatment of cells with compound (day 1)

10X compound working solution (10X working solution in DMSO) was formulated, and then to the ULA culture plate was add 15 µL of 10X compound working solution, and to the vehicle control and blank control was added 15 µL of DMSO-cell culture medium mixture, respectively.

The 96-well cell plate was placed back to the incubator and incubated for 120 hours.

The sphere formation of cells was observed every day until the end of the assay.

### CellTiter-Glo luminescent cell viability detection (Day 5)

The following steps were performed according to the instructions of the Promega CellTiter-Glo 3D Luminescent Cell Viability Assay Kit (Promega # G9683).

150 µL (equal to the volume of cell culture medium in each well) of CellTiter-Glo 3D reagent was added to each well. The cell plate was wraped by aluminum foil to protect from light.

The culture plate was shaked on an orbital shaker for 5 minutes.

The mixture in wells was mixed thoroughly by pipetting up and down 10 times carefully with pipette, so as to make sure that the spheroids of cells were sufficiently detached before proceeding to the next step.

The solution in the ULA plate was then transferred to a black bottom culture plate (#655090) and left to stand at room temperature for 25 minutes to stabilize the luminescent signal.

The luminescent signal was detected on a 2104 EnVision plate reader.

### Data analysis

The following formula was used to calculate inhibition rate (IR) of the assay compound: IR (%) = (1 - (RLU of compound - RLU of blank control) / (RLU of vehicle control - RLU of blank control))* 100%. The inhibition rates of different concentrations of compounds were calculated in Excel, and then GraphPad Prism software was used to draw inhibition curves and calculate related parameters, including minimum inhibition rate, maximum inhibition rate, and IC₅₀.

### Assay method 6. Pharmacokinetic study of oral and intravenously injected assay compounds in CD-1 mouse

### Assay purpose

To assay the in vivo pharmacokinetics of oral and intravenously injected compounds in CD-1 mouse.

### Assay processs

The assay compound was mixed with 5% DMSO+95% (10%HP-β-CD) aqueous solution. The mixture was vortexed and sonicated to prepare a clear solution of 0.5 mg/mL (intravenous) or a clear solution of 3 mg/mL (oral). The clear solution was filtered by a microporous membrane for use. Male SD mice aged 7 to 10 weeks were selected, administered the candidate compound solutions intravenously at a dose of about 2 mg/kg, and administered the candidate compound solutions orally at a dose of about 30 mg/kg. Whole blood was collected for a certain period of time, and prepared to give plasma. Drug concentration was analyzed by LC-MS/MS method, and pharmacokinetic parameters were calculated by Phoenix WinNonlin software (Pharsight, USA).

### Assay method 7. Assay of plasma protein binding (PPB)

### Purpose of the assay

The protein binding rates of the compound in the plasma of CD-1 mouse, Sprague-Dawley rat, Beagle dog, cynomolgus monkey and human were determined by equilibrium dialysis method.

### Method of the assay

The plasmas of the above-mentioned five species were formulated to plasma samples with a compound concentration of 2 µM. The plasma samples were placed in a 96-well rapid equilibrium dialysis device, and dialyzed against phosphate buffered saline at 37±1 °C for 4 h. In this assay, warfarin was used as a control compound. The concentrations of analytes in plasma and dialysis buffer were determined by LC-MS/MS mothed.

### Assay method 8. Assay of p-ERK inhibition in AsPC-1 cells

### 1. Purpose

The inhibitory activity of the compounds on the p-ERK level in AsPC-1 cells was evaluated by HTRF method.

### 2. Materials of the assay:

ASPC-1 cells purchased from ATCC; RPMI-1640 medium purchased from GIbco; fetal bovine serum purchased from Hyclone; and Advanced Phospho-ERK1/2(THR202/TYR204) KIT purchased from Bioauxilium-

**Table 4. The composition of Advanced Phospho-ERK1/2(THR202/TYR204) KIT**

| Ingredient name | Storage temperature |
|---|---|
| Advanced PhosphoERK1/2 Eu Cryptate antibody | ≤-16°C |
| Advanced PhosphoERK1/2 d2 antibody | ≤-16°C |
| Blocking reagent (stock solution 100X) | ≤-16°C |
| Lysis buffer # 1 (stock solution 4X) | ≤-16°C |
| Detection buffer (ready-to-use) | ≤-16°C |

### 3. Method of the assay:

1) ASPC-1 cells were seeded in a 384-well cell culture plate with a white bottom, 8 µL of cell suspension per well, and each well contained 7500 cells. The cell plate was inoculated in a carbon dioxide incubator at 37 °C overnight;
2) The compound to be assayed was diluted to 3 mM as the first concentration with 100% DMSO, and then diluted to 3000, 1000, 300, 100, 30, 10, 3, 1, 0.3, and 0.1 µM using a pipette. 2 µL of the compound was weighted and added to 198 µL of a cell starvation medium. After the mixture was mixed thoroughly, 15 µL of the compound solution was weighted and added to 35 µL of cell starvation medium. The mixture was mixed thoroughly. The compound solution in the last step was then added to the corresponding cell plate wells at 4 µL per well. The cell plate was placed back in the carbon dioxide incubator and incubated for another 3 hours; at this time, the concentrations of the compound were 3000, 1000, 300, 100, 30, 10, 3, 1, 0.3, and 0.1 n M;
3) After completion of the incubation, 3 µL of 5X cell lysate was added to each well. The mixture was incubated with shaking at room temperature for 30 minutes;
4) Phospho-ERK1/2 Eu Cryptate antibody and Phospho-ERK1/2 d2 antibody were diluted 20 times with Detection buffer and mixed well at a ratio of 1:1. The mixture was added to the cell culture plate at 5 µL per well and incubated at room temperature for 2 h.
5) After completion of the incubation, HTRF was read with a multi-label analyzer: excitation: 320nm, emission: 615nm, 665nm.

### 4. Data analysis:

The equation **(Sample-Min)/(Max-Min)*100%** was used to convert the raw data into inhibition rate, and the IC₅₀ value can be obtained by curve fitting with four parameters ("log(inhibitor) vs. response -- Variable slope" mode in GraphPad Prism).
**Max well:** The reading value of the positive control well was that of 1X lysate.
**Min well:** The reading value of the negative control well was that of the cell lysate in 0.5% DMSO cell well.

### Related definitions

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, allergic reaction or other problems or complications, commensurate with a reasonable benefit/risk ratio.

The term "pharmaceutically acceptable salt" means a salt of compounds disclosed herein that is prepared by reacting the compound having a specific substituent disclosed herein with a relatively non-toxic acid or base. When compounds disclosed herein contain a relatively acidic functional group, a base addition salt can be obtained by bringing the compound into contact with a sufficient amount of base in a pure solution or a suitable inert solvent. The pharmaceutically acceptable base addition salt includes a salt of sodium, potassium, calcium, ammonium, organic amine or magnesium or similar salts. When compounds disclosed herein contain a relatively basic functional group, an acid addition salt can be obtained by bringing the compound into contact with a sufficient amount of acid in a pure solution or a suitable inert solvent. Certain specific compounds disclosed herein contain both basic and acidic functional groups and can be converted to any base or acid addition salt.

The pharmaceutically acceptable salt disclosed herein can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

Compounds disclosed herein may be present in a specific geometric or stereoisomeric form. The present disclosure contemplates all such compounds, including cis and trans isomers, (-)- and (+)-enantiomers, (*R*)- and (*S*)-enantiomers, diastereoisomer, (*D*)-isomer, (*L*)-isomer, and a racemic mixture and other mixtures, for example, a mixture enriched in enantiomer or diastereoisomer, all of which are encompassed within the scope disclosed herein. The substituent such as alkyl may have an additional asymmetric carbon atom. All these isomers and mixtures thereof are encompassed within the scope disclosed herein.

Compounds disclosed herein may contain an unnatural proportion of atomic isotopes at one or more of the atoms that make up the compounds. For example, a compound may be labeled with a radioisotope such as tritium (³H), iodine-125 (¹²⁵I) or C-14(¹⁴C). For another example, hydrogen can be replaced by heavy hydrogen to form a deuterated drug. The bond between deuterium and carbon is stronger than that between ordinary hydrogen and carbon. Compared with undeuterated drugs, deuterated drugs have advantages of reduced toxic side effects, increased drug stability, enhanced efficacy, and prolonged biological half-life of drugs. All changes in the isotopic composition of compounds disclosed herein, regardless of radioactivity, are included within the scope of the present disclosure.

The term "optional" or "optionally" means that the subsequent event or condition may occur but not requisite, that the term includes the instance in which the event or condition occurs and the instance in which the event or condition does not occur.

The term "substituted" means that one or more than one hydrogen atoms on a specific atom are substituted by a substituent, including deuterium and hydrogen variants, as long as the valence of the specific atom is normal and the substituted compound is stable. When the substituent is oxo (i.e., =O), it means two hydrogen atoms are substituted. Positions on an aromatic ring cannot be substituted by oxo. The term "optionally substituted" means an atom can be substituted by a substituent or not, unless otherwise specified, the species and number of the substituent may be arbitrary so long as being chemically achievable.

When any variable (such as R) occurs in the constitution or structure of the compound more than once, the definition of the variable at each occurrence is independent. Thus, for example, if a group is substituted by 0-2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When one of variables is a single bond, it means that the two groups linked by the single bond are connected directly. For example, when L in A-L-Z represents a single bond, the structure of A-L-Z is actually A-Z.

When an enumerated linking group does not indicate its linking direction, its linking direction is arbitrary. For example, when the linking group L in 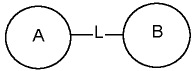 is -M-W-, the -M-W- can be linked to the ring A and the ring B in the same direction as the reading order from left to right to constitute 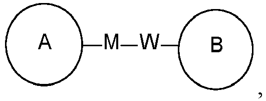 or can be linked to the ring A and the ring B in the reverse direction as the reading order from left to right to constitute 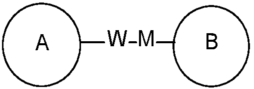 A combination of the linking groups, substituents and/or variants thereof is allowed only when such combination can result in a stable compound.

Unless otherwise specified, when a group has one or more connectable sites, any one or more sites of the group can be connected to other groups through chemical bonds. Where the connection position of the chemical bond is variable, and there is H atom(s) at a connectable site(s), when the connectable site(s) having H atom(s) is connected to the chemical bond, the number of H atom(s) at this site will correspondingly decrease as the number of the connected chemical bond increases, and the group will become a group of corresponding valence. The chemical bond between the site and other groups can be represented by a straight solid bond 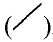 , a straight dashed bond 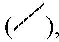 or a wavy line 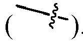 For example, the straight solid bond in -OCH₃ indicates that the group is connected to other groups through the oxygen atom in the group; the straight dashed bond in 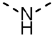 indicates that the group is connected to other groups through two ends of the nitrogen atom in the group; the wavy line in 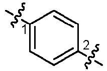 indicates that the group is connected to other groups through the 1- and 2-carbon atoms in the phenyl group; 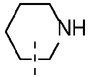 indicates that any connectable site on the piperidinyl group can be connected to other groups through one chemical bond, including at least four connection ways, 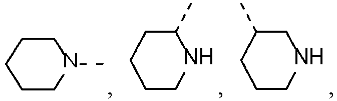 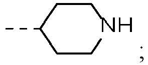 even if a H atom is drawn on -N-, 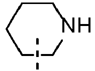 still includes the connection way of 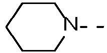 ; it's just that when one chemical bond is connected, the H at this site will be reduced by one, and the group will become the corresponding monovalent piperidinyl group.

Unless otherwise specified, a wedged solid bond 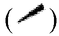 and a wedged dashed bond 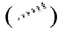 indicate the absolute configuration of a stereocenter; a straight solid bond 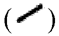 and a straight dashed bond 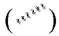 indicate the relative configuration of a stereocenter; a wavy line 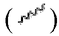 indicates a wedged solid bond 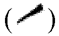 or a wedged dashed bond 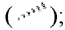 or a wavy line 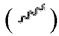 indicates a straight solid bond 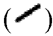 or a straight dashed bond

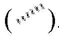

Unless otherwise specified, when a double bond structure exists in a compound, such as a carbon-carbon double bond, a carbon-nitrogen double bond, and a nitrogen-nitrogen double bond, and each atom on the double bond is connected to two different substituents (in a double bond involving a nitrogen atom, a lone pair of electrons on the nitrogen atom is regarded as a substituent connected thereto), if the atom on the double bond and its substituent in the compound are represented by 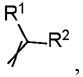 it represents the (Z) isomer, (E) isomer, or a mixture of the two isomers of the compound. Unless otherwise specified, the term "tautomer" or "tautomeric form" means that at room temperature, isomers with different functional groups are in dynamic equilibrium and can be rapidly converted to each other. If tautomerism is possible (such as in solution), chemical equilibrium of tautomerism can be achieved. For example, proton tautomers (also called prototropic tautomers) include interconversions through proton migration, such as keto-enol isomerization and imine-enamine isomerization. Valence isomers are tautomers that interconvert by reorganization of some of the bonding electrons. A specific example of keto-enol tautomerization is the interconversion between pentane-2,4-dione and 4-hydroxypent-3-en-2-one.

Unless otherwise specified, the term "halo" or "halogen", by itself or as part of another substituent, means a fluorine, chlorine, bromine, or iodine atom.

Unless otherwise specified, the term "C₁₋₃ alkyl" is used to represent a linear or branched saturated hydrocarbon group consisting of 1 to 3 carbon atoms. The C₁₋₃ alkyl includes C₁₋₂ alkyl, C₂₋₃ alkyl, etc. It may be monovalent (such as methyl), divalent (such as methylene) or multivalent (such as methenyl). Examples of the C₁₋₃ alkyl include, but are not limited to, methyl (Me), ethyl (Et), propyl (including n-propyl and isopropyl), and the like.

Unless otherwise specified, the term "C₁₋₃ alkoxy" means alkyl groups containing 1 to 3 carbon atoms and attached to the remainder of a molecule by an oxygen atom. The C₁₋₃ alkoxy group includes C₁₋₂, C₂₋₃, C₃, and C₂ alkoxy groups, and the like. Examples of C₁₋₃ alkoxy groups include, but are not limited to, methoxy, ethoxy, propoxy (including n-propoxy and isopropoxy), and the like.

Unless otherwise specified, "C₂₋₃ alkenyl" is used to represent a linear or branched hydrocarbon group consisting of 2 to 3 carbon atoms containing at least one carbon-carbon double bond, wherein the carbon-carbon double bond can be located at any position of the group. The C₂₋₃ alkenyl includes C₃ and C₂ alkenyl. The C₂₋₃ alkenyl may be monovalent, divalent or multivalent. Examples of the C₂₋₃ alkenyl include, but are not limited to, vinyl, propenyl, and the like.

Unless otherwise specified, "C₂₋₃ alkynyl" is used to represent a linear or branched hydrocarbon group consisting of 2 to 3 carbon atoms containing at least one carbon-carbon triple bond, wherein the carbon-carbon triple bond can be located at any position of the group. The C₂₋₃ alkynyl may be monovalent, divalent or multivalent. The C₂₋₃ alkynyl includes C₃ and C₂ alkynyl. Examples of the C₂₋₃ alkynyl include, but are not limited to, ethynyl, propynyl, and the like.

Unless otherwise specified, "C₂₋₄ alkynyl" is used to represent a linear or branched hydrocarbon group consisting of 2 to 4 carbon atoms containing at least one carbon-carbon triple bond, wherein the carbon-carbon triple bond can be located at any position of the group. The C₂₋₄ alkynyl group includes C₂₋₃, C₄, C₃ and C₂ alkynyl groups, etc. The C₂₋₄ alkynyl may be monovalent, divalent or multivalent. Examples of C₂₋₄ alkynyl groups include, but are not limited to, ethynyl, propynyl, butynyl, etc.

Unless otherwise specified, "C₃₋₅ cycloalkyl" refers to a saturated cyclic hydrocarbon group consisting of 3 to 5 carbon atoms, which is a monocyclic system. The C₃₋₅ cycloalkyl includes C₃₋₄ and C₄₋₅ cycloalkyl, etc., and may be monovalent, divalent, or multivalent. Examples of C₃₋₅ cycloalkyl include, but are not limited to, cyclopropyl, cyclobutyl, cyclopentyl, etc.

Unless otherwise specified, the terms "5-6 membered heteroaromatic ring" and "5-6 membered heteroaryl" may be used interchangeably herein. The term "5-6 membered heteroaryl" means a monocyclic group having a conjugated pi electron system and consisting of 5 to 6 ring atoms, in which 1, 2, 3 or 4 ring atoms are heteroatoms independently selected from O, S and N, and the remainder is carbon atoms. The nitrogen atom is optionally quaternized and the nitrogen and sulfur heteroatoms are optionally oxidized (i.e., NO and S(O)ₚ, p is 1 or 2). A 5-6 membered heteroaryl group can be attached to the remainder of a molecule through a heteroatom or a carbon atom. It may be monovalent, divalent or multivalent. The 5-6 membered heteroaryl group includes 5-membered and 6-membered heteroaryl groups. Examples of the 5-6 membered heteroaryl include, but are not limited to, pyrrolyl (including N-pyrrolyl, 2-pyrrolyl and 3-pyrrolyl, etc.), pyrazolyl (including 2-pyrazolyl and 3-pyrrolyl, etc.), imidazolyl (including *N*-imidazolyl, 2-imidazolyl, 4-imidazolyl and 5-imidazolyl, etc.), oxazolyl (including 2-oxazolyl, 4-oxazolyl and 5-oxazolyl, etc.), triazolyl (1*H*-1,2,3-triazolyl, 2*H*-1,2,3-triazolyl, 1*H*-1,2,4-triazolyl and 4*H*-1,2,4-triazolyl, etc.), tetrazolyl, isoxazolyl (3-isoxazolyl, 4-isoxazolyl and 5-isoxazolyl, etc.), thiazolyl (including 2-thiazolyl, 4-thiazolyl and 5-thiazolyl, etc.), furyl (including 2-furyl and 3-furyl, etc.), thienyl (including 2-thienyl, 3-thienyl, etc.), pyridyl (including 2-pyridyl, 3-pyridyl and 4-pyridyl, etc.), pyrazinyl or pyrimidinyl (including 2-pyrimidinyl, 4-pyrimidinyl, etc.).

Unless otherwise specified, the terms "5-10 membered heteroaromatic ring" and "5-10 membered heteroaryl" may be used interchangeably. The term "5-10 membered heteroaryl" means a cyclic group having a conjugated pi electron system and consisting of 5 to 10 ring atoms, in which 1, 2, 3 or 4 ring atoms are heteroatoms independently selected from O, S and N, and the remainder is carbon atoms. It may be a monocyclic, fused bicyclic or fused tricyclic ring system, wherein each ring is aromatic, and wherein the nitrogen atom is optionally quaternized and the nitrogen and sulfur heteroatoms are optionally oxidized (i.e., NO and S(O)ₚ, p is 1 or 2). A 5-10 membered heteroaryl can be attached to the remainder of a molecule through a heteroatom or a carbon atom. It may be monovalent, divalent or multivalent. The 5-10 membered heteroaryl group includes 5-8 membered, 5-7 membered, 5-6 membered, 5-membered and 6-membered heteroaryl groups. Examples of the 5-10 membered heteroaryl include, but are not limited to, pyrrolyl (including N-pyrrolyl, 2-pyrrolyl, 3-pyrrolyl, and the like), pyrazolyl (including 2-pyrazolyl and 3-pyrazolyl, and the like), imidazolyl (including N-imidazolyl, 2-imidazolyl, 4-imidazolyl, and 5-imidazolyl, and the like), oxazolyl (including 2-oxazolyl, 4-oxazolyl, and 5-oxazolyl, and the like), triazolyl (1H-1,2,3-triazolyl, 2H-1,2,3-triazolyl, 1H-1,2,4-triazolyl and 4H-1,2,4-triazolyl, and the like), tetrazolyl, isoxazolyl (3-isoxazolyl, 4-isoxazolyl and 5-isoxazolyl, and the like), thiazolyl (including 2-thiazolyl, 4-thiazolyl and 5-thiazolyl, and the like), furyl (including 2-furyl and 3-furyl, and the like), thienyl (including 2-thienyl and 3-thienyl, and the like), pyridyl (including 2-pyridyl, 3-pyridyl and 4-pyridyl, and the like), pyrazinyl or pyrimidinyl (including 2-pyrimidinyl and 4-pyrimidinyl, and the like), benzothiazolyl (including 5-benzothiazolyl, and the like), purinyl, benzimidazolyl (including 2-benzimidazolyl, and the like), benzoxazolyl, indolyl (including 5-indolyl, and the like), isoquinolyl (including 1-isoquinolyl, 5-isoquinolyl, and the like), quinoxalinyl (including 2-quinoxalinyl, 5-quinoxalinyl, and the like) or quinolyl (including 3-quinolyl, 6-quinolyl, and the like).

Unless otherwise specified, Cₙ₋ₙ₊ₘ or Cₙ-Cₙ₊ₘ includes any specific case of n to n+m carbons, for example, C₁₋₁₂ includes C₁, C₂, C₃, C₄, C₅, C₆, C₇, C₈, C₉, C₁₀, C₁₁ and C₁₂, also includes any range from n to n+m, for example, C₁₋₁₂ includes C₁₋₃, C₁₋₆, C₁₋₉, C₃₋₆, C₃₋₉, C₃₋₁₂, C₆₋₉, C₆₋₁₂ and C₉₋₁₂, etc.; similarly, n membered to n+m membered indicates that the number of atoms on a ring is n to n+m, for example, 3-12 membered ring includes 3-membered ring, 4-membered ring, 5-membered ring, 6-membered ring, 7-membered ring, 8-membered ring, 9-membered ring, 10-membered ring, 11-membered ring, and 12-membered ring, also includes any range from n to n+m, for example, 3-12 membered ring includes 3-6 membered ring, 3-9 membered ring, 5-6 membered ring, 5-7 membered ring, 6-7 membered ring, 6-8 membered ring, and 6-10 membered ring, and the like.

Compounds disclosed herein can be prepared by a variety of synthetic methods well known to those skilled in the art, including the following enumerated embodiment, the embodiment formed by the following enumerated embodiment in combination with other chemical synthesis methods, and equivalent replacement well known to those skilled in the art. Alternative embodiments include, but are not limited to the embodiment disclosed herein.

The structures of compounds disclosed herein can be confirmed by conventional methods well known to those skilled in the art. If the present disclosure relates to an absolute configuration of a compound, the absolute configuration can be confirmed by conventional techniques in the art, such as single crystal X-Ray diffraction (SXRD). In the single crystal X-Ray diffraction (SXRD), the diffraction intensity data of the cultivated single crystal is collected using a Bruker D8 venture diffractometer with a light source of CuKα radiation in a scanning mode of ϕ/ω scan; after collecting the relevant data, the crystal structure is further analyzed by the direct method (Shelxs97) to confirm the absolute configuration.

The solvent used in the present disclosure is commercially available. The present disclosure adopts the following abbreviations: PhNTf₂ represents N-phenylbis(trifluoromethanesulfonyl)imide; Pd₂(dba)₃ represents tris[dibenzylideneacetone]dipalladium; DavePhos represents 2-dicyclohexylphosphino-2'-(N,N-dimethylamino)-biphenyl; LIHMDS represents lithium bis(trimethylsilyl)amide; NaOH represents sodium hydroxide; NaHCO₃ represents sodium bicarbonate; Boc₂O represents di-tert-butyl dicarbonate; Et₃N represents triethylamine; Tf₂O represents trifluoroacetic anhydride; Xantphos represents 4,5-bis(diphenylphosphino)-9,9-dimethylxanthene; DMF represents N,N-dimethylformamide; DMAP represents 4-dimethylaminopyridine; TBDPSCI represents tert-butyldiphenylchlorosilane; DCM represents dichloromethane; EA represents ethyl acetate; Grubbs II catalyst represents 1,3-bis(2,4,6-trimethylphenyl)-2-(imidazolidinylidene)(dichlorophenylmethylene)(tricyclohe xylphosphine)ruthenium; TFA represents trifluoroacetic acid; THF represents tetrahydrofuran; NBS represents N-bromosuccinimide; PMB represents p-methoxybenzyl; Tf represents trifluoromethanesulfonyl; Boc represents tert-butyloxycarbonyl; TIPS represents triisopropylsilyl; and MOM represents methoxymethyl.

Compounds are named according to general naming principles in the art or by ChemDraw^{®} software, and commercially available compounds are named with their vendor directory names.

### BRIEF DESCRIPTION OF DRAWINGS

Fig. 1. Binding pattern diagram of compound A and KRAS^{G12D} protein;
Fig. 2. Binding pattern diagram of compound B and KRAS^{G12D} protein;
Fig. 3. Binding pattern diagram of compound C and KRAS^{G12D} protein;
Fig. 4. Binding pattern diagram of compound D and KRAS^{G12D} protein;
Fig. 5. Binding pattern diagram of compound E and KRAS^{G12D} protein;
Fig. 6. Binding pattern diagram of compound F and KRAS^{G12D} protein.

### Technical Effects

The compound of the present disclosure has a good inhibitory effect on KRAS^{G12D} mutant enzymes, can effectively inhibit p-ERK, has a good cell proliferation inhibitory activity on KRAS^{G12D} mutant cells, can effectively inhibit tumor growth in vivo, and has good drug resistance. The compound of the present disclosure has good pharmacokinetic properties.

### DETAILED DESCRIPTION

The present disclosure is described in detail below by means of examples. However, it is not intended that these examples have any disadvantageous limitations to the present disclosure. The present disclosure has been described in detail herein, and embodiments are also disclosed herein. It will be apparent to those skilled in the art that various changes and modifications may be made to the embodiments disclosed herein without departing from the spirit and scope disclosed herein.

### Calculation Example 1

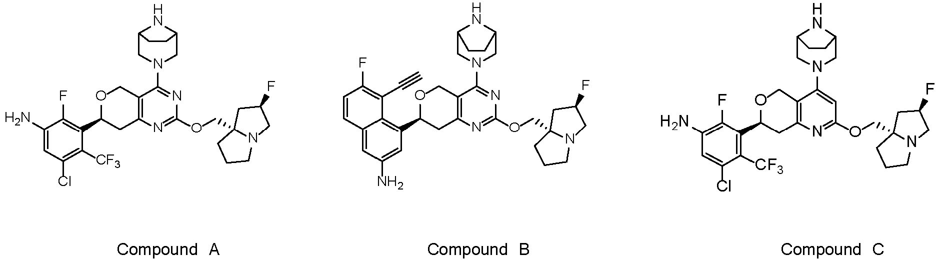

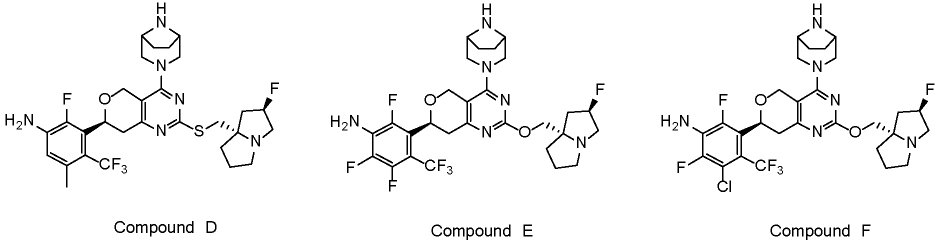

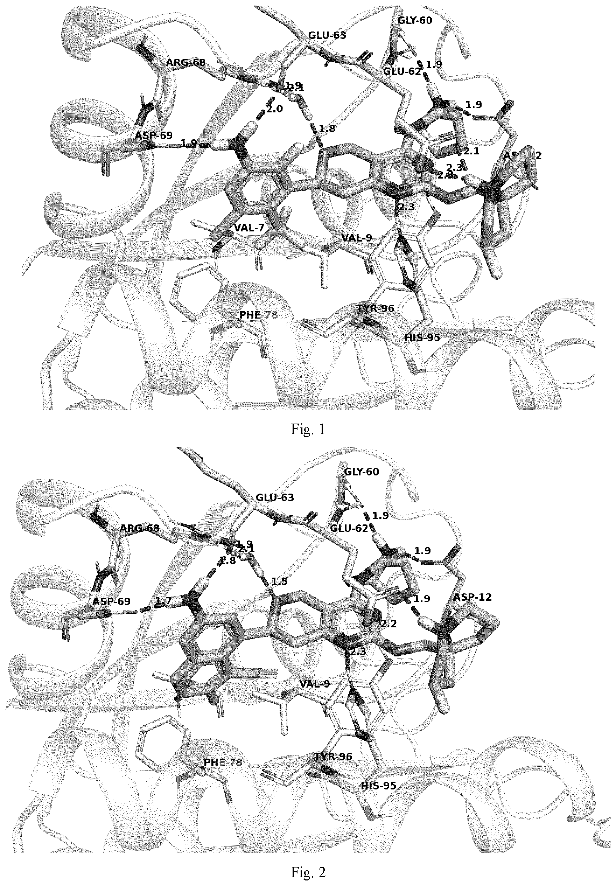

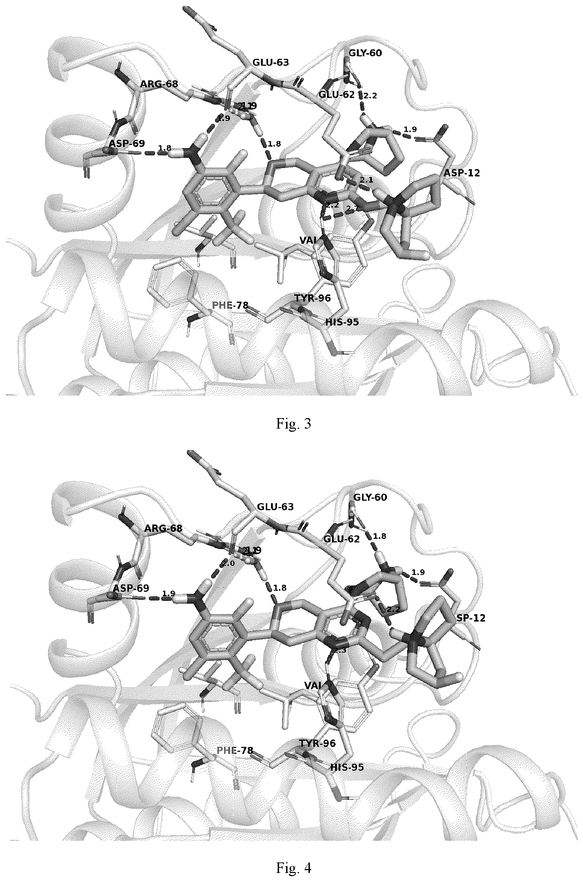

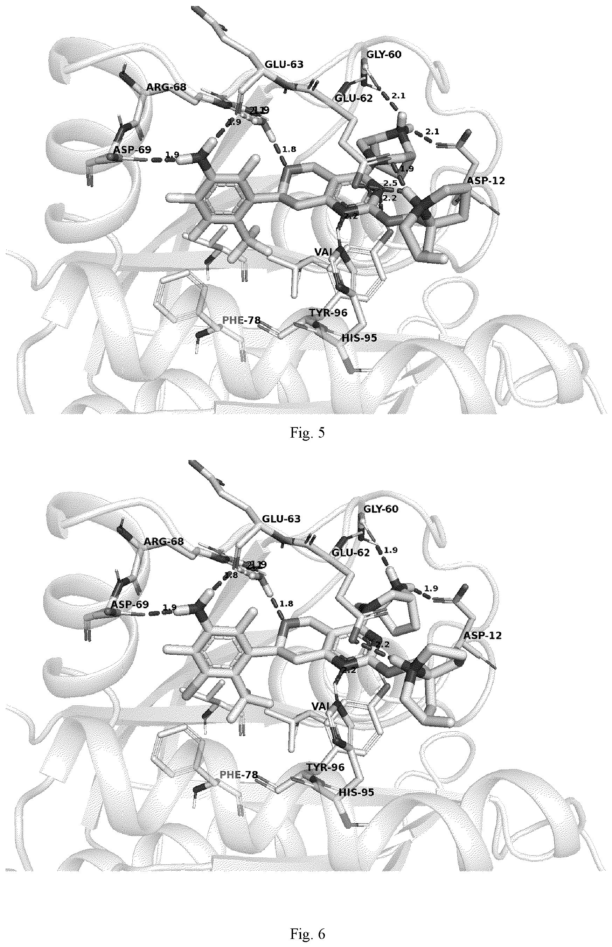

The present disclosure also provides the docking process of the compound or a pharmaceutically acceptable molecular thereof, which is performed by using Glide SP[1] and default options in Maestro (Schrödinger version 2017-2). The crystal structure PDB: 6UT0 of KRAS_G12C in the PDB database was selected. Cys12 was mimetically mutated to Asp12, which was used as a docking template after energy optimization. To prepare the protein, hydrogen atoms were added using the protein preparation wizard module of Maestro[2], and the OPLS3 force field was used. For the preparation of the ligand, the three-dimensional structure of the molecule was generated using LigPrep, and the energy was minimized[3]. The small molecule conformation was sampled using the confgen module. A cube docking grid with a side length of 25Å * 25Å * 25Å was generated with the ligand of 6UT0 as the center of mass. The reference compound was placed during the molecular docking process. The interaction types between protein receptors and ligands were analyzed, and then reasonable docking conformations were selected and saved based on the calculated docking scrores and binding modes, as shown in Figs. 1 to 6.
[1] Glide, Schrödinger, LLC, New York, NY, 2017.
[2] Maestro, Schrödinger, LLC, New York, NY, 2017.
[3] LigPrep, Schrödinger, LLC, New York, NY, 2017.

Conclusion: The compounds of the present disclosure have good binding to KRAS^{G12D}.

### Example 1

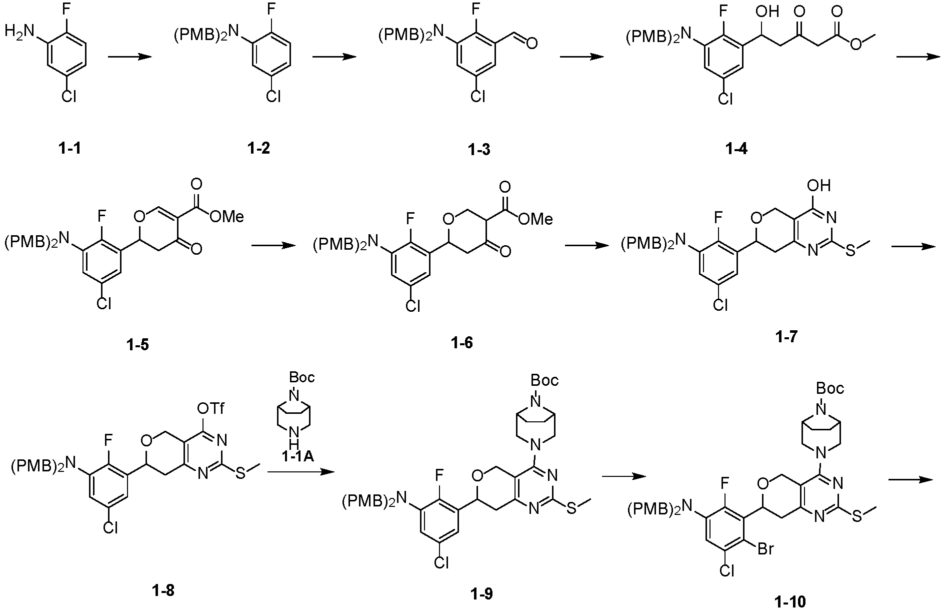

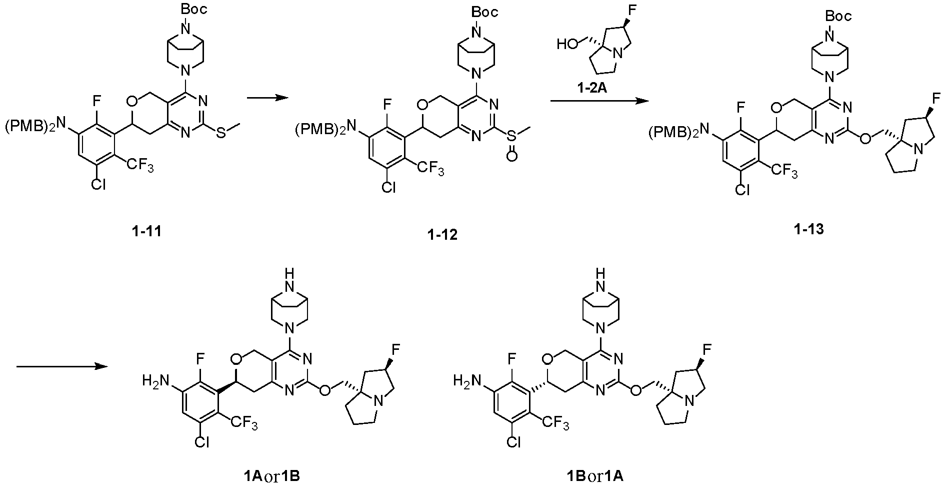

### Step 1: Synthesis of intermediate 1-2

To a pre-dried reaction flask were added compound **1-1** (20 g, 137.40 mmoL), N,N-dimethylformamide (200 mL), potassium iodide (22.81 g, 137.40 mmoL), and anhydrous potassium carbonate (47.47 g, 343.50 mmoL). P-methoxybenzyl chloride (44.11 g, 281.67 mmoL, 38.36 mL) was added under stirring, and then the mixture was heated to 65 °C and stirred for 4 hours. The reaction solution was cooled down to room temperature, and then filtered through a pad of Celite. The filter cake was rinsed with 200 mL of methyl tert-butyl ether, and then 200 mL of water was added to the filtrate for extraction. The aqueous phase was extracted with ethyl acetate (100 mL*2). The organic phases were combined, washed with saturated brine (200 mL*3), dried over anhydrous sodium sulfate, and filtered. The filtrate was concentrated to give a crude product. The crude product was slurried with 50 mL of petroleum ether for 48 Hours. The mixture was filtered. The filter cake was collected, and dried to give the product, compound **1-2,** which was directly used in the next step. ¹H NMR (400 MHz, DMSO-*d₆*) δ = 7.18 - 7.17 (m, 5H), 6.85 - 6.82 (m, 6H), 4.24 (s, 4H), 3.74 - 3.71 (m, 6H). MS m/z = 386.1 [M+H]⁺.

### Step 2: Synthesis of intermediate 1-3

2,2,6,6-tetramethylpiperidine (43.93 g, 311.00 mmol, 52.80 mL) was dissolved in tetrahydrofuran (300 mL), and then the mixture was cooled down to -5°C. n-Butyl lithium (2.5 M, 124.40 mL) was added. The mixture was stirred for 0.5 hours, and then cooled down to -60°C. A solution of compound **1-2** (30 g, 77.75 mmol) in tetrahydrofuran (30 mL) was added. The mixture was stirred for 0.5 hours, then N,N-dimethylformamide (113.66 g, 1.55 mol, 119.64 mL) was added. The mixture was stirred for another 0.5 hours. To the reaction solution was added 200 mL of saturated ammonium chloride. The mixture was extracted with ethyl acetate (200 mL). The layers were separated. The organic phase was washed with saturated brine (100 mL), dried over anhydrous sodium sulfate, and filtered. The filtrate was concentrated. The residue was purified by column (petroleum ether:ethyl acetate = 10:1) to give compound **1-3.**

### Step 3: Synthesis of intermediate 1-4

Sodium hydride (6.38 g, 159.47 mmol, 60% content) was dissolved in tetrahydrofuran (300 mL). The atmosphere was replaced with nitrogen twice, then the mixture was cooled down to 0°C. Methyl acetoacetate (18.52 g, 159.47 mmol, 17.15 mL) was added. The mixutre was stirred for 10 min, and then n-butyl lithium (2.5 M, 63.79 mL) was added. The mixture was stirred for another 10 min, and cooled down to -15°C. A solution of compound **1-3** (30 g, 72.49 mmol) in tetrahydrofuran (50 mL) was added. The mixture was stirred for another 30 min. To the reaction solution was added 100 mL of saturated ammonium chloride. The mixture was extracted with ethyl acetate (100 mL*2). The organic phases were combined, dried over anhydrous sodium sulfate, and filtered. The filtrate was concentrated. The residue was purified by column (petroleum ether:ethyl acetate = 10:1) to give compound **1-4.** MS m/z = 530.2 [M+H]⁺.

### Step 4: Synthesis of intermediate 1-5

Compound **1-4** (20 g, 37.74 mmoL) was dissolved in anhydrous dichloromethane (50 mL), and N,N-dimethylformamide dimethyl acetal (5.40 g, 45.28 mmoL, 6.02 mL) was added under nitrogen, and the mixture was reacted at 25°C for 16 hours. Boron trifluoride diethyl etherate (6.43 g, 45.28 mmoL, 5.59 mL) was added, and the mixture was reacted at 20°C for 1 hour. The reaction solution was added into 50 mL of saturated sodium bicarbonate solution. The mixture was extracted with dichloromethane (20 mL*2). The layers were separated. The organic phases were combined, washed with 30 mL of saturated brine, dried over anhydrous sodium sulfate, and filtered. The filtrate was concentrated. The crude product was purified by column chromatography (petroleum ether:ethyl acetate = 10:1-1:1) to give compound **1-5.** ¹H NMR (400 MHz, CDCl₃) δ = 8.45 (s, 1H), 7.14 (m, 4H), 7.00 - 6.69 (m, 6H), 5.80 (m, 1H), 4.27 - 4.10 (m, 5H), 3.79 (m, 1H), 3.94 - 3.66 (m, 8H), 2.98 - 2.73 (m, 1H). MS m/z =540.2 [M+H]⁺.

### Step 5: Synthesis of Intermediate 1-6

Compound 1-5 (12 g, 22.22 mmol) was added to anhydrous tetrahydrofuran (30 mL), and tri-sec-butyl lithium borohydride (11.83 g, 62.22 mmol, 13.60 mL) was added under nitrogen, and the mixture was reacted at -60 °C for 1 hour. The reaction solution was added to 40 mL of water. The mixture was extracted with ethyl acetate (20 mL*2). The organic phases were combined, and then washed with 20 mL of saturated brine, dried over anhydrous sodium sulfate, and concentrated. The crude product was purified by column chromatography (petroleum ether: ethyl acetate = 100:0 = 3:1) to give compound 1-6. MS m/z = 542.2 [M+H]⁺.

### Step 6: Synthesis of Intermediate 1-7

Compound **1-6** (5.5 g, 10.15 mmol) was added to ethanol (15 mL) and water (3 mL), and sodium bicarbonate (2.15 g, 20.30 mmol) and methylisothiourea sulfate (2.74 g, 30.44 mmol) were added. The mixture was reacted at 45-50°C for 16 hours. The reaction solution was extracted with water (20 mL) and ethyl acetate (20 mL*2). The combined organic phase was washed with saturated brine (20 mL*2), dried over anhydrous sodium sulfate and concentrated to give compound **1-7.** The crude product was directly used for the next step. MS m/z =582.2 [M+H]⁺.

### Step 7: Synthesis of Intermediate 1-8

Compound **1-7** (6 g, 8.04 mmol) was added to anhydrous dichloromethane (20 mL). N,N-diisopropylethylamine (3.12 g, 24.12 mmol, 4.20 mL) was added at 0°C. The mixture was cooled down to 0-10°C. Trifluoromethanesulfonic anhydride (4.08 g, 14.47 mmol, 2.39 mL) was added dropwise slowly. The mixture was reacted at 0°C for 0.5 hours. The reaction solution was added to 20 mL of saturated ammonium chloride. The mixture was extracted with anhydrous dichloromethane (10 mL*2). The organic phases were combined, washed with 20 mL of saturated brine, dried over anhydrous sodium sulfate, and filtered. The filtrate was concentrated. The crude product was purified by column chromatography (petroleum ether:ethyl acetate = 1:0-0:1) to give compound **1-8.** MS m/z = 714.1 [M+H]⁺.

### Step 8: Synthesis of intermediate 1-9 hydrochloride

Compound **1-8** (0.8 g, 1.12 mmoL), compound **1-1A** (475.62 mg, 2.24 mmoL), and DIPEA (434.33 mg, 3.36 mmoL, 585.35 µL) were added to N,N-dimethylformamide (5 mL). The mixture was reacted at 50°C for 1 hour. The reaction solution was added to saturated ammonium chloride (20 mL). The mixture was extracted with ethyl acetate (20 mL*2). The organic phases were combined, washed with 20 mL of saturated brine, dried over anhydrous sodium sulfate, and concentrated. The crude product was purified by column (petroleum ether:ethyl acetate = 3:1), and then separated by preparative HPLC (chromatographic column: Phenomenex Luna C18 250*50mm*10 µm; mobile phase: [water (0.05% hydrochloric acid)-acetonitrile]; (acetonitrile)%: 65%-95%, 10min) to give compound **1-9** hydrochloride. MS m/z =776.3 [M+H]⁺.

### Step 9: Synthesis of Intermediate 1-10

Compound **1-9** hydrochloride (1.2 g) was dissolved in N,N-dimethylformamide (5 mL). The mixture was cooled down to 0°C, and then N-bromosuccinimide (330.13 mg, 1.85 mmol) was added and the mixutre was stirred at 20°C for 1 hour. 30 mL of water was added to the reaction solution. The mixture was extracted with ethyl acetate (30 mL*2). The organic phases were combined, washed with saturated brine (20 mL*2), dried over anhydrous sodium sulfate, and filtered. The filtrate was concentrated. The residue was purified by column (petroleum ether:ethyl acetate = 3:1-1:1) to give compound **1-10.** MS m/z = 856.1 [M+H]⁺.

### Step 10: Synthesis of Intermediate 1-11

Compound **1-10** (0.3 g, 350.77 µmol), copper(I) oxide (133.61 mg, 701.55 µmol), and methyl fluorosulfonyl difluoroacetate (336.94 mg, 1.75 mmol, 223.14 µL) were dissolved in N,N-dimethylformamide (10 mL), and the mixture was stirred at 100 °C for 1 hour under nitrogen. The reaction solutions were combined and poured into 30 mL of water. The mixture was extracted with methyl tert-butyl ether (20 mL*2). The organic phases were combined and washed with saturated brine (20 mL*2), dried over anhydrous sodium sulfate, and filtered. The filtrate was concentrated. The crude product was purified by column (petroleum ether:ethyl acetate = 10:1-1:1) to give Compound **1-11.** MS m/z = 844.2 [M+H]⁺.

### Step 11: Synthesis of Intermediate 1-12

To a reaction flask was added compound **1-11** (0.35 g, 414.52 µmol). Anhydrous dichloromethane (5 mL) was added, and the mixture was stirred. Then m-chloroperbenzoic acid (126.24 mg, 621.78 µmol, 85% content) was added, and the mixture was reacted at 25°C for 1 hour. 5 mL of 5% sodium thiosulfate solution was added to the reaction solution. The mixture was extracted with dichloromethane (10 mL*2). The organic phases were combined, washed with saturated brine (5 mL*2), dried over anhydrous sodium sulfate, and filtered. The filtrate was concentrated. The crude product was puried by column (petroleum ether:ethyl acetate = 10:1-1:1) to give compound **1-12.** MS m/z = 860.2 [M+H]⁺.

### Step 12: Synthesis of Intermediate 1-13

Compound **1-2A** (256.28 mg, 1.61 mmol) was added to anhydrous tetrahydrofuran (5 mL), and sodium tert-butoxide (123.76 mg, 1.29 mmol) was added. The mixture was reacted at -15°C for 15 min. Compound **1-12** (0.277 g, 321.96 µmol) was added, and the mixture was reacted at -15°C for 1 hour. 5 mL of saturated ammonium chloride was added to the reaction solution, and the mixture was extracted with ethyl acetate (10 mL*3). The organic phases were combined, washed with saturated brine (20 mL*2), dried over anhydrous sodium sulfate, and filtered. The filtrate was concentrated. The crude product was puried by column (dichloromethane:methanol = 20:1-10:1) to give compound **1-13.** MS m/z = 955.3 [M+H]⁺.

### Step 13: Synthesis of compounds 1A and 1B

Compound **1-13** (0.450 g, 470.98 µmol) was added to trifluoroacetic acid (2.85 g, 24.96 mmol, 1.85 mL) and dichloromethane (9 mL). The mixture was reacted at -10-0°C for 1 hour. The reaction solution was poured into 10 mL of water. The lower dichloromethane phase was separated. The aqueous phase was adjusted to pH 8-10 with saturated sodium bicarbonate solution. The mixture was then extracted with ethyl acetate (5 mL*2). The organic phases were combined, washed with saturated brine (5 mL*2), dried over anhydrous sodium sulfate, and filtered. The filtrate was concentrated. The crude product was purified by HPLC (chromatographic column: Phenomenex C18 75*30mm*3µm; mobile phase: [water (0.05% hydrochloric acid)-acetonitrile]; (acetonitrile)%: 1%-35%, 8 min), and then separated by SFC (chromatographic column: DAICEL CHIRALCEL OD (250mm*30mm, 10µm); mobile phase: [supercritical CO₂-methanol (0.1% ammonia)]; methanol (0.1% ammonia) %: 36%-36%, 7min) to give compound **1A** and compound **1B.** Chiral SFC analysis (chromatographic column: DAICEL CHIRALCEL OD-3 (150mm*4.6mm, 3µm); mobile phase: [supercritical CO₂-methanol (0.1% diethylamine)]; (methanol)%: 40%-40%), compound 1A, Rt=3.151 minutes, ee value 99%; compound 1B, Rt=4.085 minutes, ee value 93%.

Compound **1A:** ¹H NMR (400 MHz, CD₃OD) δ = 6.93 (d, *J* = 8.0 Hz, 1H), 5.68 - 5.46 (m, 1H), 5.32 - 5.19 (m, 1H), 4.78 - 4.69 (m, 1H), 4.63 - 4.46 (m, 2H), 4.41 - 4.28 (m, 1H), 4.24 - 4.08 (m, 2H), 4.04 - 3.74 (m, 5H), 3.73 - 3.65 (m, 1H), 3.55 - 3.41 (m, 2H), 3.02 - 2.89 (m, 1H), 2.80 - 2.48 (m, 2H), 2.48 - 2.24 (m, 4H), 2.24 - 2.04 (m, 3H), 2.03 - 1.91 (m, 1H), 2.04 - 1.90 (m, 1H). MS m/z =615.4 [M+H]⁺.

Compound **1B:** ¹H NMR (400 MHz, CD₃OD) δ = 6.98 - 6.85 (m, 1H), 5.54 - 5.39 (m, 1H), 5.35 - 5.19 (m, 2H), 4.76 - 4.66 (m, 1H), 4.63 - 4.53 (m, 1H), 4.39 - 4.14 (m, 4H), 4.06 - 3.89 (m, 3H), 3.78 - 3.62 (m, 2H), 3.62 - 3.50 (m, 2H), 3.27 - 3.09 (m, 3H), 2.99 - 2.86 (m, 1H), 2.48 - 2.25 (m, 2H), 2.13 - 1.83 (m, 6H). MS m/z =615.4 [M+H]⁺.

### Example 2

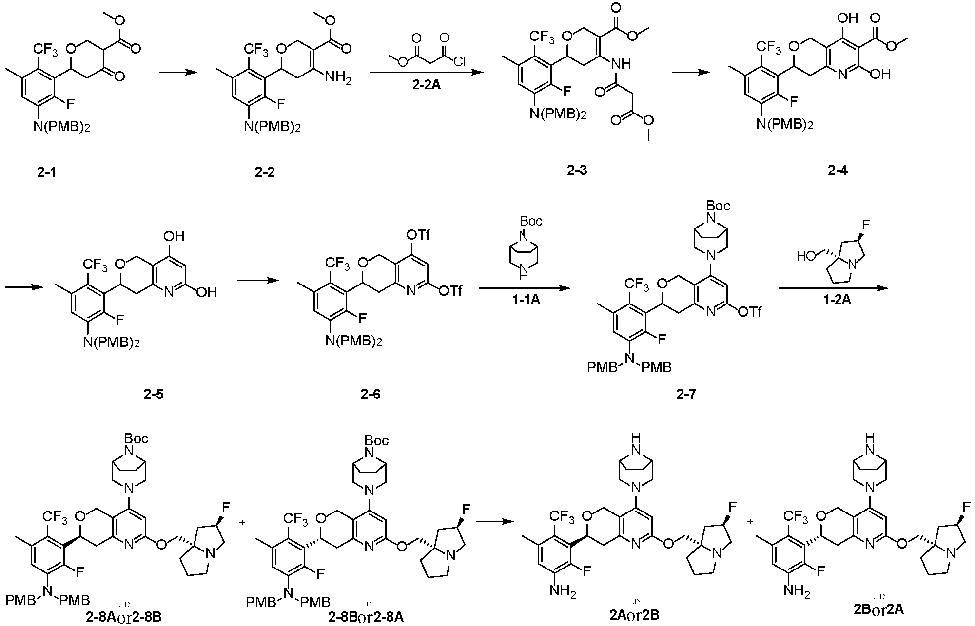

### Step 1: Synthesis of intermediate 2-2

Compound **2-1** (11.0 g, 18.66 mmol) was added to anhydrous methanol (150 mL), and then ammonium acetate (7.19 g, 93.29 mmol) was added. The resulting reaction solution was heated to 60 °C and stirred for 48 hours. The reaction solution was concentrated, and the residue was dissolved in 300 mL of ethyl acetate, and then 50 mL of water was added and the mixutre was stirred until clear. The water layer was separated, and the organic phase was washed with water (30 mL*2) and saturated brine (30 mL). The organic phase was concentrated to give a crude product, which was purified by column chromatography (petroleum ether:ethyl acetate = 30:1) to give compound **2-2.** ¹H NMR (400 MHz, CDCl₃) δ: 7.16 (d, *J*=8.4 Hz, 4H), 6.84 (d, *J*=8.4 Hz, 4H), 6.62 (d, *J*=8.4 Hz, 1H), 4.99 (br, *J*=10.5 Hz, 1H), 4.63 (d, *J*=13.6 Hz, 1H), 4.42 - 4.21 (m, SH), 3.80 (s, 6H), 3.71 (s, 3H), 3.07 (br, *J*=11.2, 16.2 Hz, 1H), 2.34-2.32 (m, 3H), 2.26-2.19 m, 1H); MS m/z=589.1 [M+H]⁺.

### Step 2: Synthesis of intermediate 2-3

Compound **2-2** (7.56 g, 12.84 mmol) and N,N-diisopropylethylamine (3.32 g, 25.69 mmol, 4.47 mL) were dissolved in tetrahydrofuran (150 mL). The mixture was cooled down to 0°C under nitrogen, and then compound **2-2A** (2.10 g, 15.41 mmol, 1.64 mL) was added dropwise. After the addition, the mixture was stirred at 15°C for 15 hours. The reaction solution was concentrated to give a crude product, which was purified by column chromatography (petroleum ether:ethyl acetate = 10:1) to give compound **2-3,** MS m/z = 689.1 [M+H]⁺.

### Step 3: Synthesis of intermediate 2-4

Compound **2-3** (2.94 g, 4.27 mmol) was dissolved in tetrahydrofuran (30 mL), and then sodium methoxide (461.27 mg, 8.54 mmol) and anhydrous methanol (0.5 mL) were added. The resulting reaction solution was stirred at 50°C for 4 hours under nitrogen. The reaction solution was cooled down to room temperature. The reaction solution was adjusted to pH of 4-5 with 0.5 M hydrochloric acid. The solvent was then removed by rotary evaporation. The residue was dissolved in 150 mL of ethyl acetate and then washed with 30 mL of water and 30 mL of saturated brine. The organic phase was concentrated to give a crude product, which was purified by column chromatography (petroleum ether:ethyl acetate = 10:1) to give compound **2-4.** MS m/z = 657.2 [M+H]⁺.

### Step 4: Synthesis of intermediate 2-5

Compound **2-4** (200 mg, 304.59 µmol) was dissolved in dimethyl sulfoxide (10 mL), and then lithium chloride (51.65 mg, 1.22 mmol, 24.95 µL) was added. The resulting reaction solution was heated to 120 °C and stirred for 4 hours. The mixture was diluted with 150 mL of ethyl acetate, and then washed with water (20 mL*3) and saturated brine (20 mL). The organic phase was concentrated to give a crude product, which was purified by column chromatography (methanol/dichloromethane = 1:10) to give compound **2-5,** MS m/z = 599.2 [M+H]⁺.

### Step 5: Synthesis of Intermediate 2-6

Compound **2-5** (400 mg, 668.24 µmol) and PhNTf₂ (477.46 mg, 1.34 mmol) were added to N,N-dimethylformamide (1.5 mL). N,N-diisopropylethylamine (345.46 mg, 2.67 mmol, 465.58 µL) was added. The reaction solution was stirred at 15°C for 3 hours. The reaction solution was concentrated to give a crude product, which was purified by column chromatography (petroleum ether:ethyl acetate = 10:1) to give compound **2-6,** MS m/z = 862.8 [M+H]⁺.

### Step 6: Synthesis of Intermediate 2-7

Compound **2-6** (580 mg, 672.30 µmol) and compound **1-1A** (142.72 mg, 672.30 µmol) were added to N,N-dimethylformamide (4 mL). N,N-diisopropylethylamine (173.78 mg, 1.34 mmol, 234.21 µL) was added. The resulting reaction solution was heated to 60 °C and stirred for 3 hours. The reaction solution was concentrated to give a crude product. The crude product was puried by column (petroleum ether:ethyl acetate = 10:1) to give Compound **2-7,** MS m/z = 925.4 [M+H]⁺.

### Step 7: Synthesis of intermediates 2-8A and 2-8B

Compound **2-7** (106mg, 114.60µmol) and compound **1-2A** (54.74mg, 343.81µmol) were added to dioxane (2mL). Pd₂(dba)₃ (20.99mg, 22.92µmol), DavePhos (18.04mg, 45.84µmol) and LIHMDS (1M, 343.81µL) were added. The atmosphere was replaced with nitrogen and the mixture was heated to 95°C for 15 hours. The reaction solution was concentrated. To the residue were added water (1 mL) and ethyl acetate (30 mL). The mixture was adjusted to a pH of 7-8 with 0.5M hydrochloric acid. The organic phase was separated. The aqueous phase was extracted twice with 10 mL of ethyl acetate. The organic phases were combined and concentrated to give a crude product. The crude product was separated by a preparative plate (ethyl acetate: methanol = 20:1). The sample separated from the above preparative plate was further purified by high performance liquid chromatography (chromatographic column: Welch Xtimate C18 100*40mm*3µm; mobile phase: [water (0.075% trifluoroacetic acid)-acetonitrile]; (acetonitrile)%: 43%-73%, 8min) and concentrated to give compound **2-8A,** MS m/z=934.9 [M+H]⁺ and compound **2-8B,** MS m/z=934.9 [M+H]⁺.

### Step 8: Synthesis of compound 2A hydrochloride and compound 2B hydrochloride

Trifluoroacetic acid (0.3 mL) was added to compound **2-8A** (43 mg, 46.04 µmol), and the mixture was stirred at 20 °C for 2 hours under nitrogen. The reaction solution was concentrated to give a crude product. The crude product was dissolved in acetonitrile and separated by high performance liquid chromatography (chromatographic column: Xtimate C18 150*40mm*5µm; mobile phase: [water (0.05% hydrochloric acid)-acetonitrile]; (acetonitrile)%: 5%-35%, 10min), to give compound **2A** hydrochloride, MS m/z=594.3 [M+H]⁺.

Trifluoroacetic acid (0.3 mL) was added to compound **2-8B** (52 mg, 55.67 µmol), and the resulting reaction solution was stirred at 20 °C for 2 hours under nitrogen. The reaction solution was concentrated to give a crude product. The crude product was purified by high performance liquid chromatography (chromatographic column: Xtimate C18 150*40mm*5µm; mobile phase: [water (0.05% hydrochloric acid)-acetonitrile]; (acetonitrile)%: 5%-35%, 10min), to give compound **2B** hydrochloride, LCMS m/z=594.4 [M+H]⁺.

### Example 3

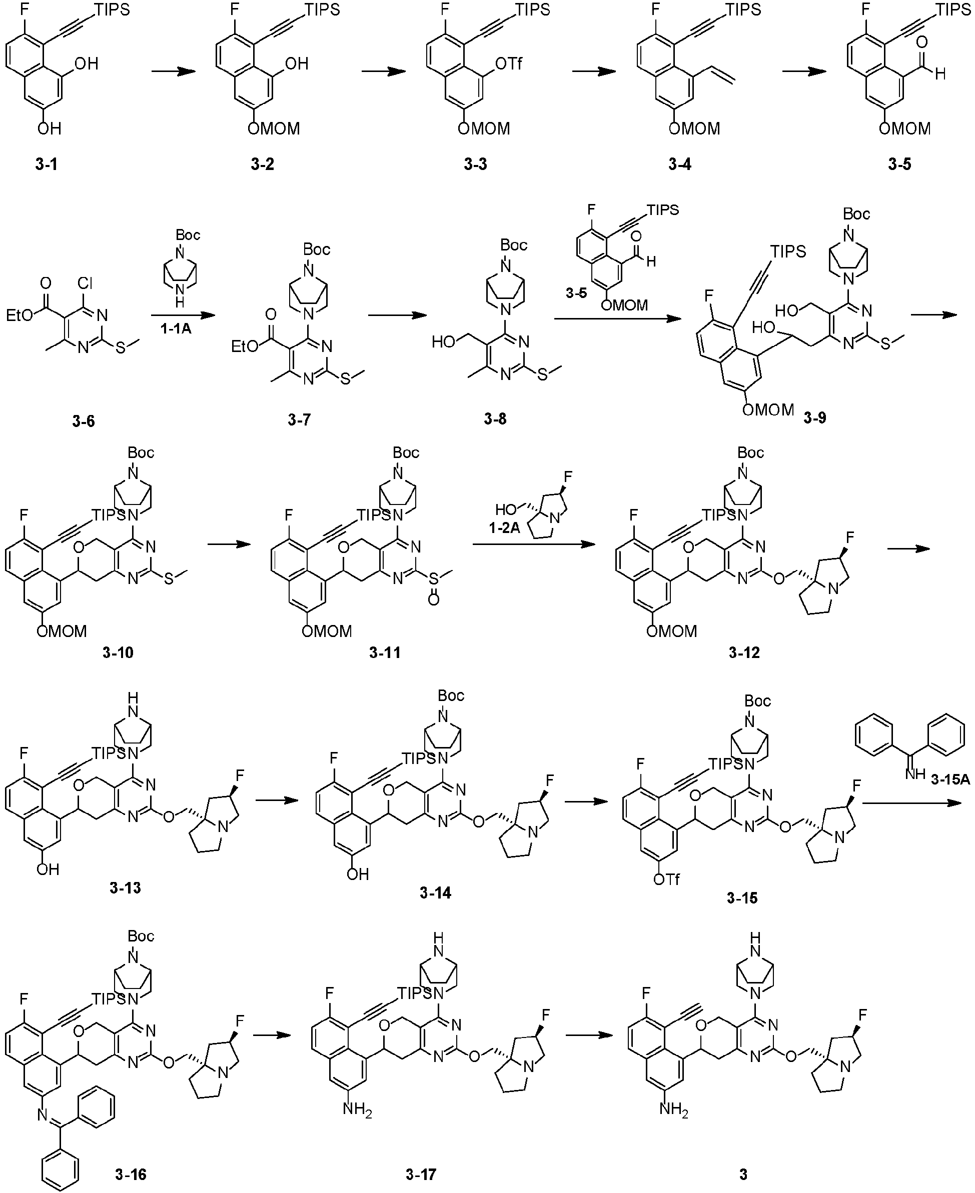

### Step 1: Synthesis of intermediate 3-2

Compound **3-1** (50 g, 139.46 mmol) was added to anhydrous dichloromethane (500 mL). N,N-diisopropylethylenediamine (54.07 g, 418.39 mmol, 72.87 mL) was added. The mixutre was cooled down to 0°C, and chloromethyl methyl ether (15.59 g, 193.64 mmol, 14.71 mL) was slowly added dropwise. The obtained mixture was slowly warmed to 18°C for 1 hour. The reaction solution was added to 500 mL of ice water. The mixture was extracted with DCM (100 mL*2). The organic phases were combined, and then washed respectively with 500 mL of saturated sodium carbonate, 500 mL of saturated ammonium chloride and 500 mL of semi-saturated brine, dried over anhydrous sodium sulfate, and filtered. The filtrate was concentrated. The crude product was purified by column chromatography (petroleum ether:ethyl acetate = 3:1) to give compound **3-2.** MS m/z =403.2 [M+H]⁺.

### Step 2: Synthesis of intermediate 3-3

Compound **3-2** (36 g, 89.42 mmol) and N,N-diisopropylethylenediamine (34.67 g, 268.27 mmol, 46.73 mL) were added to anhydrous dichloromethane (360 mL). The mixture was cooled down to -40°C, and trifluoromethanesulfonic anhydride (37.85 g, 134.14 mmol, 22.13 mL) was added dropwise. The mixture was reacted for 1 hour. The reaction solution was added to 300 mL of ice water. The mixture was separated and extracted. The organic phase was washed sequentially with 200 mL of saturated sodium bicarbonate solution, 200 mL of saturated ammonium chloride, and 200 mL of brine, dried over anhydrous sodium sulfate, and filtered. The filtrate was concentrated. The crude product was purified by column chromatography (petroleum ether:ethyl acetate = 10:1) to give compound **3-3.** ¹H NMR (400 MHz, CDCl₃) δ = 7.71 (dd, *J =* 5.2, 9.2 Hz, 1H), 7.43 (d, *J =* 2.4 Hz, 1H), 7.36 (d, *J =* 2.0 Hz, 1H), 7.33 (t, *J =* 8.8 Hz, 1H), 5.28 (s, 2H), 3.53 (s, 3H), 1.28 - 1.18 (m, 21H).

### Step 3: Synthesis of intermediate 3-4

Compound **3-3** (34 g, 63.59 mmol) was added to N,N-dimethylformamide (340 mL). Vinyl tributyltin (42.03 g, 132.55 mmol, 38.56 mL) and lithium chloride (10.78 g, 254.38 mmol, 5.21 mL) were added. The atmosphere was replaced 3 times with nitrogen. Triphenylphosphine palladium dichloride (4.46 g, 6.36 mmol) was added under nitrogen. The mixture was reacted at 30°C for 20 hours. 300 mL of 20% KF aqueous solution and 300 mL of methyl tert-butyl ether were added to the reaction solution. The mixutre was stirred for 20 minutes, and then filtered through a pad of Celite. The filter cake was rinsed with methyl tert-butyl ether (50 mL*4). The aqueous phase was removed, and the organic phase was washed with 500 mL of saturated brine, dried over anhydrous sodium sulfate, and filtered. The filtrate was concentrated under reduced pressure. The crude product was purified by column (petroleum ether:ethyl acetate = 5:1) to give compound **3-4.** MS m/z =413.3 [M+H]⁺.

### Step 4: Synthesis of intermediate 3-5

Compound **3-4** (20 g, 48.47 mmol) was added to anhydrous tetrahydrofuran (200 mL) and water (50 mL). The mixture was cooled down to 0°C. Sodium periodate (31.10 g, 145.42 mmol, 8.06 mL) and osmium tetroxide (1.5 g, 5.90 mmol, 306.12 µL) were added. The obtained mixture was slowly warmed to 18°C. The reaction mixture was reacted for 1 hour. The reaction mixture was added to 300 mL of 10% sodium thiosulfate solution. The mixture was extracted with ethyl acetate (100 mL*2), washed with 500 mL of saturated brine, dried over anhydrous sodium sulfate, and filtered. The filtrate was concentrated. The crude product was purified by column (petroleum ether:ethyl acetate = 5:1) to give compound **3-5.** MS m/z = 415.3 [M+H]⁺.

### Step 5: Synthesis of intermediate 3-7

To a solution of compound **3-6** (1 g, 4.05 mmol) and **1-1A** (1.03 g, 4.86 mmol) in dichloromethane was added N,N-diisopropylethylamine (1.05 g, 8.11 mmol). The resulting mixture was stirred at room temperature of 25°C for 1 hour. Water (20 mL) and dichloromethane (50 mL) were added to the reaction solution. The mixture was stirred for 5 minutes. The aqueous phase was removed, and the organic phase was concentrated under reduced pressure. The residue was purified by column chromatography (petroleum ether:ethyl acetate = 5:1) to give compound **3-7.** MS m/z = 423.1 [M+H]⁺.

### Step 6: Synthesis of Intermediate 3-8

To a solution of compound **3-7** (0.6 g, 1.42 mmol) in tetrahydrofuran (5 mL) was added lithium aluminum tetrahydride (0.1 g, 2.84 mmol) in batches at 0 °C (in an ice-water bath) under nitrogen. The resulting mixture was naturally warmed to 25°C and stirred for 2 hours. Water (0.1 g), 15% sodium hydroxide aqueous solution (0.1 g) and water (0.3 g) were added dropwise to the reaction solution in sequence. The mixture was stirred for 30 minutes, and then filtered. The filter cake was washed with tetrahydrofuran (10 mL). The filtrate was concentrated under reduced pressure to give compound **3-8.** MS m/z =381.1 [M+H]⁺.

### Step 7: Synthesis of Intermediate 3-9

To a solution of compound **3-8** (0.5 g, 1.33 mmol) in tetrahydrofuran (10 mL) was added lithium diisopropylamide (1.51 mL, 3.02 mmol, 2M) dropwise at -65 °C (in a dry ice-ethyl acetate bath) under nitrogen. After stirring for 30 minutes, a solution of compound **3-5** (0.5 g, 1.51 mmol) in tetrahydrofuran (5 mL) was added dropwise. The resulting mixture was naturally warmed to 25°C. 0.1M dilute hydrochloric acid aqueous solution (15 mL) and ethyl acetate (20 mL) were added to the reaction solution. The mixture was stirred for 10 minutes. The aqueous phase was removed, the organic phase was concentrated under reduced pressure, and the residue was purified by column chromatography (petroleum ether:ethyl acetate = 5:1) to give compound **3-9.** MS m/z =795.4 [M+H]⁺.

### Step 8: Synthesis of Intermediate 3-10

To a solution of compound **3-9** (0.51 g, 641.44 µmol) in tetrahydrofuran (10 mL) was added n-butyl lithium (2.5 M, 564.47 µL) at -65 °C (in a dry ice-ethyl acetate bath) under nitrogen. After stirring for 30 minutes, p-toluenesulfonyl chloride (183.43 mg, 962.16 µmol) in tetrahydrofuran (3 mL) was added dropwise. The resulting mixture was naturally warmed to 25 °C and stirred for 2 hours. The reaction was quenched by adding 50 ml of saturated aqueous ammonium chloride solution. The mixture was extracted with 50 ml of ethyl acetate. The aqueous phase was removed. The organic phase was concentrated under reduced pressure. The residue was purified by column chromatography (petroleum ether:ethyl acetate = 5:1) to give compound **3-10.** MS m/z = 777.4 [M+H]⁺.

### Step 9: Synthesis of Intermediate 3-11

To a solution of compound **3-10** (45 mg, 57.91 µmol) in dichloromethane (2 mL) was added meta-chloroperbenzoic acid (14.11 mg, 69.49 µmol, 85% content). The resulting mixture was stirred at room temperature of 25°C for 2 hours. 2 mL of saturated sodium bicarbonate aqueous solution, 2 mL of saturated sodium sulfite aqueous solution and 5 mL of dichloromethane were added to the reaction solution and the mixture was stirred for 5 minutes. The aqueous phase was removed, and the organic phase was concentrated under reduced pressure. The residue was purified by preparative TLC (petroleum ether:ethyl acetate = 5:1) to give compound **3-11.** MS m/z = 793.4 [M+H]⁺.

### Step 10: Synthesis of Intermediate 3-12

To a solution of compound **1-2A** (32.12 mg, 201.76 µmol) in tetrahydrofuran (2 mL) was added sodium tert-butoxide (19.39 mg, 201.76 µmol) at 0 °C (in an ice-water bath) under nitrogen. After stirring for 30 minutes, compound **3-11** (40 mg, 50.44 µmol) was added. The resulting mixture was naturally warmed to 25°C and stirred for 2 hours. The reaction solution was adjusted to pH of about 6 by adding 0.5M of dilute hydrochloric acid. 5 ml of ethyl acetate and 2 ml of saturated brine were added. The mixture was stirred for 5 minutes. The aqueous phase was removed, and the organic phase was concentrated under reduced pressure to give compound 3-12. MS m/z =888.5 [M+H]⁺.

### Step 11: Synthesis of Intermediate 3-13 hydrochloride

To a solution of compound **3-12** (45 mg, 50.67 µmol) in dichloromethane (1 mL) was added hydrochloric acid-ethyl acetate (4 M, 126.67 µL). The resulting mixture was stirred at room temperature for 2 hours, and the reaction solution was directly concentrated under reduced pressure to give compound 3-13 hydrochloride. MS m/z =744.4 [M+H]⁺.

### Step 12: Synthesis of Intermediate 3-14

To a solution of compound **3-13** hydrochloride (0.5 g, 612.06 µmol) in MeOH (10 mL) were added NaHCO₃ (2 M, 1.53 mL) and Boc₂O (267.16 mg, 1.22 mmol, 281.22 µL). The mixture was stirred at room temperature for 2 hours. The reaction solution was concentrated under reduced pressure to remove methanol. To the residue were added 10 mL of ethyl acetate and 5 mL of saturated brine, and the mixture was stirred for 5 minutes. The aqueous phase was removed, and the organic phase was concentrated under reduced pressure to give compound **3-14,** MS m/z =844.4 [M+H]⁺.

### Step 13: Synthesis of Intermediate 3-15

To a solution of compound **3-14** (1.2 g, 1.42 mmol) in DCM (5 mL) were added Et₃N (287.70 mg, 2.84 mmol, 395.74 µL) and Tf₂O (601.64 mg, 2.13mmol, 351.83 µL). The mixture was stirred at room temperature for 2 hours. 10 mL of water and 10 mL of dichloromethane were added to the reaction solution, and the mixture was stirred for 10 minutes. The aqueous phase was removed. The organic phase was concentrated under reduced pressure. The residue was purified by column chromatography (petroleum ether:ethyl acetate = 1:1) to give compound **3-15,** MS m/z = 976.2 [M+H]⁺.

### Step 14: Synthesis of Intermediate 3-16

Compound **3-15** (200 mg, 204.88 µmol), compound **3-15A** (74.26 mg, 409.76 µmol, 68.76 µL), cesium carbonate (200.26 mg, 614.64 µmol), and Xantphos (23.71 mg, 40.98 µmol) were added to anhydrous toluene (5 mL). The atmosphere was replaced three times with nitrogen. Tris(dibenzylideneacetone)dipalladium (18.76 mg, 20.49 µmol) was added. The mixture was reacted at 100°C for 3 hours under nitrogen. The reaction solution was cooled down to room temperature, filtered with a pad of Celite, and rinsed with 30 mL of ethyl acetate. The mother liquor was concentrated under reduced pressure to give compound **3-16.**

### Step 15: Synthesis of Intermediate 3-17 hydrochloride

Compound **3-16** (180 mg, 178.69 µmol) was added to 4M hydrochloric acid/methanol solution (5 mL). The mixture was reacted at room temperature for 1 hour. The reaction solution was directly concentrated to give crude compound **3-17 hydrochloride.** MS m/z =743.3 [M+H]⁺.

### Step 16: Synthesis of compound 3 hydrochloride

Compound **3-17** hydrochloride (180.00 mg), cesium fluoride (73.60 mg, 484.51 µmol), and anhydrous potassium carbonate (267.86 mg, 1.94 mmol) were added to N,N-dimethylformamide (10 mL). The mixture was reacted at 60°C for 16 hours. 10 mL of water was added to the reaction solution. The mixture was extracted with 10 mL*2 of ethyl acetate. The organic phases were combined, washed with saturated brine (10 mL*3), dried over anhydrous sodium sulfate, and filtered. The filtrate was concentrated. The product was purified by high performance liquid chromatography (chromatographic column: Phenomenex luna C18 80*40mm*3 µm; mobile phase: [water (0.05% hydrochloric acid)-acetonitrile]; (acetonitrile)%: 1%-20%, 7min) to give compound **3** hydrochloride. ¹H NMR (400 MHz, DMSO-*d₆*) δ = 7.75 - 7.63 (m, 1H), 7.42 - 7.24 (m, 2H), 6.91 - 6.81 (m, 1H), 6.57 - 6.41 (m, 1H), 5.58 - 5.45 (m, 2H), 5.36 - 5.14 (m, 1H), 5.06 - 4.90 (m, 1H), 4.84 - 4.58 (m, 2H), 3.97 - 3.78 (m, 3H), 3.66 - 3.38 (m, 2H), 3.10 - 2.89 (m, 4H), 2.85 - 2.64 (m, 1H), 2.15 - 1.89 (m, 6H), 1.87 - 1.56 (m, 9H). MS m/z =587.3 [M+H]⁺.

### Example 4

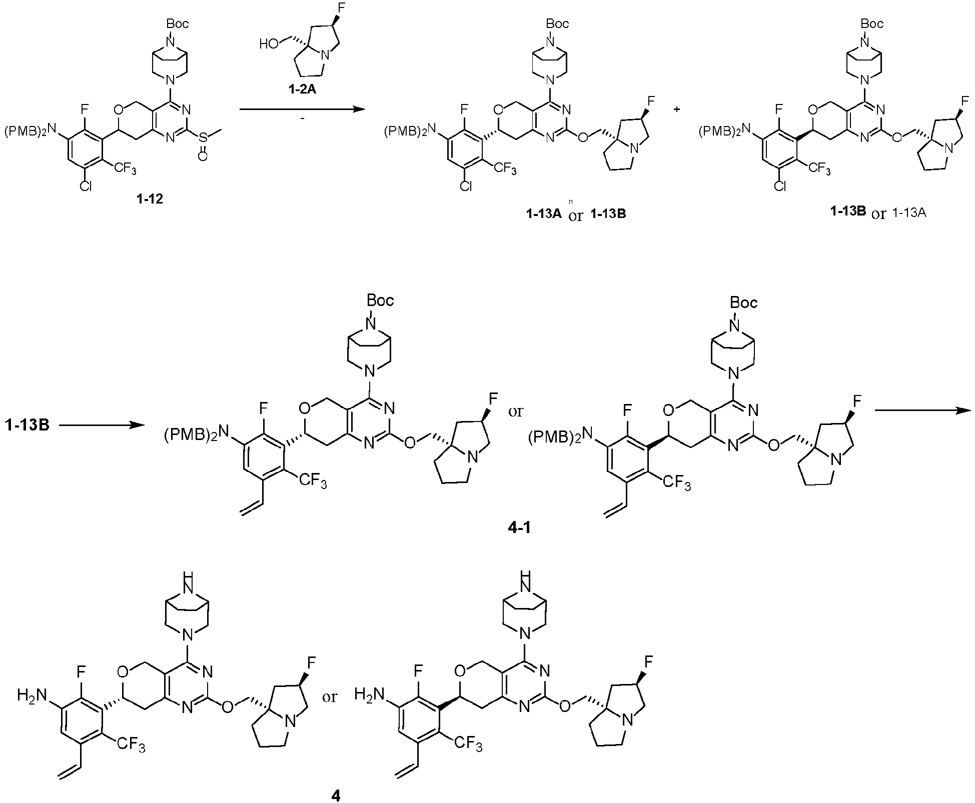

### Step 1: Synthesis of Intermediate 1-13B

Compound **1-12** (4.16 g, 26.15 mmol) was dissolved in anhydrous tetrahydrofuran (150 mL) under nitrogen. The mixture was cooled down to 20°C. Sodium tert-butoxide (2.85 g, 29.64 mmol) was added. The mixutre was stirred for 0.5 hours. A solution of compound **1-2A** (15 g, 17.43 mmol) in THF (15 mL) was added dropwise to the reaction solution. The mixture was stirred at this temperature for 0.5 hours. The reaction solution was poured into saturated aqueous ammonium chloride solution (200 mL). The mixture was extracted with ethyl acetate (100 mL x 3). The organic phases were combined and concentrated to give a crude product. The crude product was purified by high performance liquid chromatography (chromatographic column: Phenomenex luna C18 (250*70mm, 15 µm); mobile phase: [water (0.05% hydrochloric acid)-acetonitrile]; acetonitrile%: 40%-70%, 20min), and separated again (chromatographic column: Phenomenex luna c18 250mm*100mm*10µm; mobile phase: [water (0.025% trifluoroacetic acid)-acetonitrile]; acetonitrile%: 40%-70%, 20min) to give the product, which was purified by supercritical fluid chromatography (SFC) (chromatographic column: DAICEL CHIRALPAK IE (250mm*30mm, 10µm); mobile phase: A: supercritical CO₂, B: [acetonitrile/isopropanol = 1:1 (0.1% ammonia water)]; B%: 65%-65%, 45min) to give compound **1-13A** and **1-13B.** SFC analysis: (chromatographic column: DAICEL CHIRALPAK IE (50mm* 4.6mm, 3µm); mobile phase: A: supercritical CO2, B: [acetonitrile/isopropanol = 1:1 (0.1% diisopropylamine)]; B%: 50%-50%), compound **1-13A,** retention time = 2.143; compound **1-13B,** retention time = 4.935. MS m/z = 955.5 [M+H]⁺.

### Step 2: Synthesis of Intermediate 4-1

Compound **1-13B** (120 mg, 125.60 µmol), anhydrous potassium phosphate (53.32 mg, 251.19 µmol), and potassium ethylene trifluoroborate (25.24 mg, 188.39 µmol) were dissolved in 1,4-dioxane (1 mL) and water (0.2 mL). The atmosphere was replaced three times with nitrogen. Chloro[(n-butyldi(1-adamantyl)phosphine)-2-(2-aminobiphenyl)]palladium(II) (8.40 mg, 12.56 µmol) was added. The mixture was reacted at 80 °C for 3 hours. The reaction solution was poured into an aqueous solution (5 mL). The mixture was extracted with ethyl acetate (10 mL x 2), separated, dried over anhydrous sodium sulfate, and filtered. The filtrate was concentrated to give compound **4-1.** MS m/z =947.4 [M+H]⁺.

### Step 3: Synthesis of compound 4 hydrochloride

Compound **4-1** (100 mg, 105.59 µmol) was dissolved in anhydrous dichloromethane (2 mL). Trifluoroacetic acid (770.00 mg, 6.75 mmol) was added. The mixture was reacted at 20 °C for 3 hours. The reaction solution was concentrated to give a crude product. The product was purified by high performance liquid chromatography (chromatographic column: Phenomenex Luna 80*30mm*3µm; mobile phase: [water (0.05% hydrochloric acid)-acetonitrile]; acetonitrile%: 1%-30%, 8min) to give compound 4 hydrochloride. MS m/z =607.4 [M+H]⁺.

### Example 5

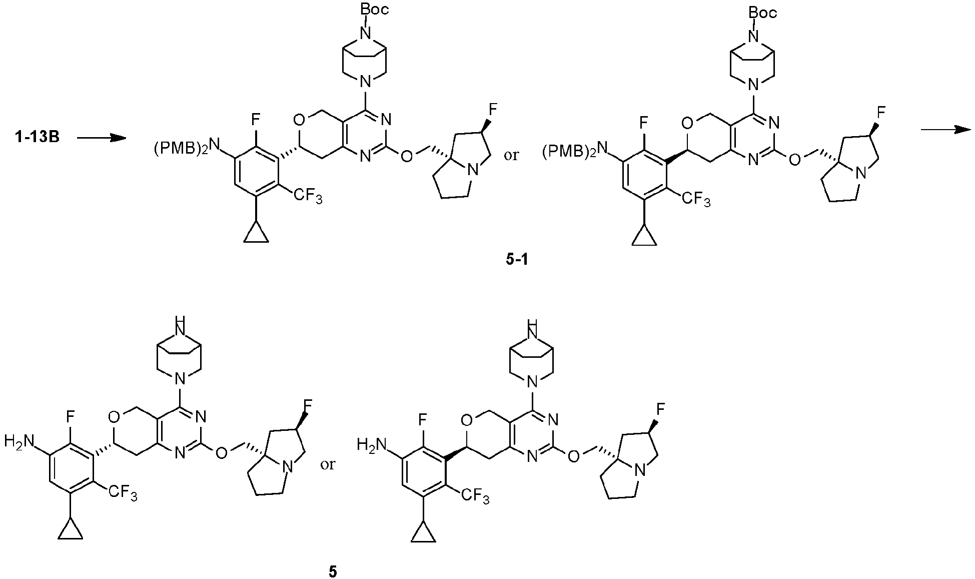

### Step 1: Synthesis of Intermediate 5-1

Compound **1-13B** (100 mg, 104.66 µmol), potassium phosphate (55.54 mg, 261.66 µmol), and cyclopropylboronic acid (89.90 mg, 1.05 mmol) were added to 1,4-dioxane (2.5 mL) and water (0.5 mL). The atmosphere was replaced three times with nitrogen. Chloro(2-dicyclohexylphosphine-2',6'-dimethoxy-1,1'-biphenyl)[2-(2'-amino-1,1'-biphenyl)] palladium (II) (7.54 mg, 10.47 µmol) was added. The mixture was reacted at 100°C for 3 hours under nitrogen. The reaction solution was cooled down to room temperature and concentrated under reduced pressure to give crude compound **5-1,** MS m/z =961.5 [M+H]⁺.

### Step 2: Synthesis of compound 5 hydrochloride

To compound **5-1** (150 mg, 156.08 µmol) was added trifluoroacetic acid (1.60 g, 14.05 mmol). The mixture was reacted in anhydrous dichloromethane (5 mL) at 0°C for 16 hours. The reaction solution was poured into 10 mL of water. The organic phase was separated. The aqueous phase was extracted with ethyl acetate (5 mL*2). The organic phases were combined, dried over anhydrous sodium sulfate, and filtered. The filtrate was concentrated. The residue was purified by high performance liquid chromatography (chromatographic column: Phenomenex Luna 80*30mm*3µm; mobile phase: [water (0.05% hydrochloric acid)-acetonitrile]; acetonitrile%: 1%-30%, 8min) to give compound 5 hydrochloride. MS m/z =621.3 [M+H]⁺.

### Example 6

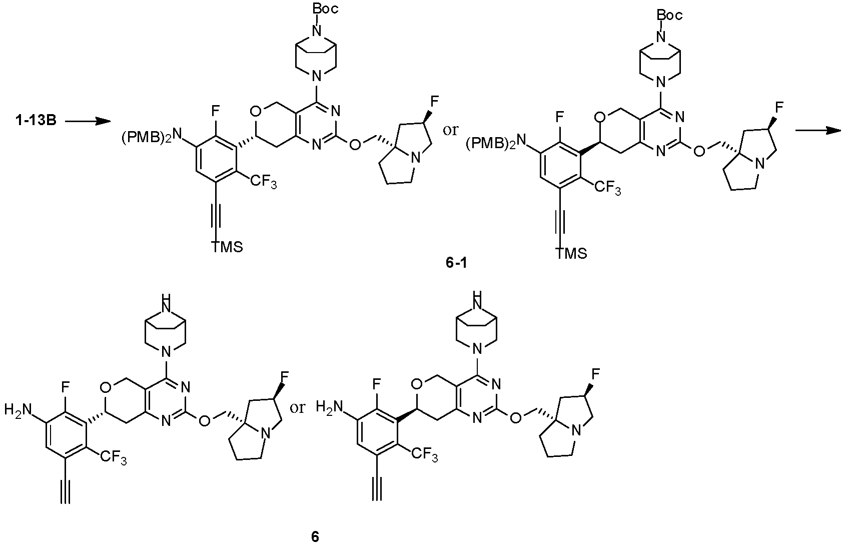

### Step 1: Synthesis of Intermediate 6-1

Compound **1-13B** (120 mg, 125.60 µmol), bis(acetonitrile)palladium(II)chloride (3.26 mg, 12.56 µmol), cesium carbonate (122.76 mg, 376.79 µmol), and 2-dicyclohexylphosphino-2',4',6'-triisopropylbiphenyl (11.97 mg, 25.12 µmol) were dissolved in acetonitrile (1.5 mL). The atmosphere was replaced three times with nitrogen. The mixture was stirred at room temperature for 0.5 hours. Trimethylsilylacetylene (49.34 mg, 502.38 µmol) was added to the reaction solution. The mixture was reacted at 80°C for 10 hours. The reaction solution was directly concentrated to give compound **6-1.** MS m/z = 1017.5 [M+H]⁺.

### Step 2: Synthesis of compound 6

Compound **6-1** (100 mg, 98.31 µmol) was dissolved in anhydrous dichloromethane (4 mL). Trifluoroacetic acid (1.54 g, 13.51 mmol) was added. The mixture was reacted at 20°C for 3 hours. The mixture was poured into a saturated sodium bicarbonate aqueous solution (5 mL). The layers were separated. The aqueous phase was extracted with dichloromethane (5 mL x 3), washed with saturated brine, dried over anhydrous sodium sulfate, and filtered. The filtrate was concentrated to give a crude product. The crude product was purified by preparative HPLC (chromatographic column: Waters Xbridge BEH C18 100*30mm*10µm; mobile phase: [water (10 mM NH₄HCO₃)-acetonitrile]; acetonitrile%: 30%-50%, 8min) to give compound **6.** MS m/z =605.4 [M+H]⁺.

### Example 7

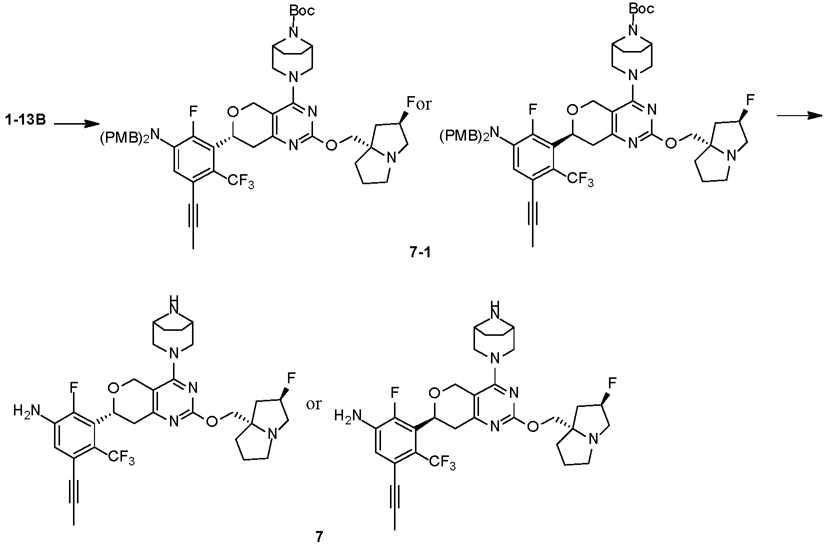

### Step 1: Synthesis of Intermediate 7-1

Compound **1-13B** (200 mg, 209.33 µmol) was dissolved in anhydrous toluene (2.5 mL). The mixture was bubbled by nitrogen for 10 min. Dichlorobis[di-tert-butyl-(4-dimethylaminophenyl)phosphine]palladium (II) (44.47 mg, 62.80 µmol) and 1-propynyltri-n-butyltin (275.56 mg, 837.30 µmol) were added under nitrogen. The mixture was reacted at 110 °C for 3 hours. The reaction solution was poured into 5 mL of water. The mixture was extracted with ethyl acetate (3 mL x 2). The organic phases were combined, washed with saturated brine (3 mL x 2), dried over anhydrous sodium sulfate, and filtered. The filtrate was concentrated to give compound **7-1,** MS m/z =959.3 [M+H]⁺.

### Step 2: Synthesis of compound 7 hydrochloride

Compound **7-1** (450 mg, 469.21 µmol) was dissolved in dichloromethane (2.5 mL). Trifluoroacetic acid (802.50 mg, 7.04 mmol) was added. The mixture was reacted at 25°C for 4 hours. The reaction solution was directly concentrated and purified by HPLC (chromatographic column: Phenomenex luna C18 250*50mm*10 µm; mobile phase: [water (0.05% hydrochloric acid)-acetonitrile]; acetonitrile %: 5%-35%, 10min) to give compound 7 hydrochloride, MS m/z =619.2 [M+H]⁺. ¹H NMR (400 MHz, CD₃OD) δ 7.00 - 6.92 (m, 1H), 5.74 - 5.50 (m, 1H), 5.31 - 5.17 (m, 1H), 5.03 - 4.92 (m, 2H), 4.79 - 4.72 (m, 2H), 4.31 - 4.16 (m, 2H), 4.09 - 3.92 (m, 4H), 3.90 - 3.83 (m, 1H), 3.64 - 3.54 (m, 1H), 3.54 - 3.36 (m, 3H), 3.11 - 3.01 (m, 1H), 2.84 - 2.61 (m, 2H), 2.57 - 2.47 (m, 1H), 2.44 - 2.33 (m, 1H), 2.36 (br d, *J=* 8.5 Hz, 6H), 2.06 - 2.02 (m, 3H).

### Example 8

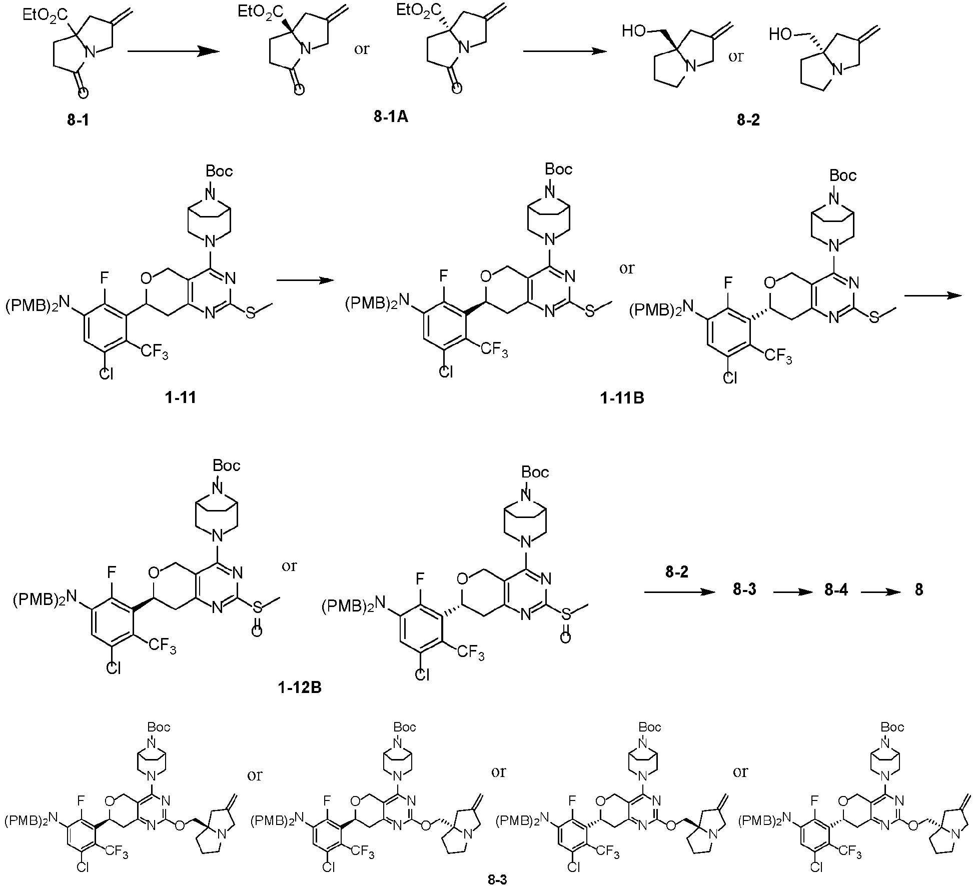

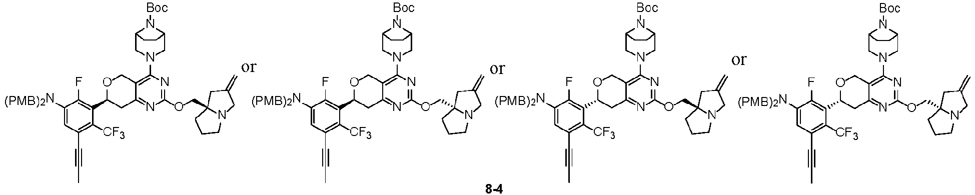

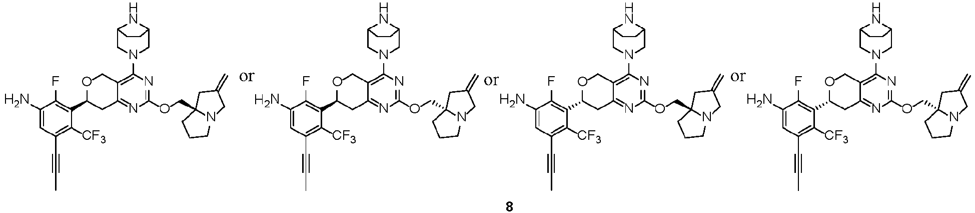

### Step 1: Synthesis of Intermediate 8-1A

Compound **8-1** was purified by preparative supercritical fluid chromatography (SFC) (chromatographic column: ChiralPak IH, 250*50mm, 10µm; mobile phase: A: supercritical CO₂, B: [0.1% ammonia - ethanol]; B%: 20%-20%, running time 3.7min, peak time: 1.266 min and 1.521 min). The sample with a peak time of 1.521min was collected to give compound **8-1A.** SFC analysis method (chromatographic column: Chiralpak IH-3, 100×4.6mm ID, 3µm; mobile phase: A (supercritical CO₂) and B (ethanol, containing 0.1% isopropylamine); gradient: B%=10~50%, 4 min; flow rate: 3.4 mL/min; wavelength: 220nm; pressure: 2000psi), compound **8-1A,** Rt=1.489 min, ee value 98.82%. ¹H NMR (400 MHz, CDCl₃) δ = 4.99 - 4.86 (m, 2H), 4.26 - 3.95 (m, 3H), 3.59 (m, 1H), 3.00 - 2.88 (m, 1H), 2.87 - 2.12 (m, 4H), 1.91 (s, 1H), 1.20 - 1.08 (m, 3H).

### Step 2: Synthesis of Intermediate 8-2

Lithium aluminum tetrahydride (1.55 g, 40.15 mmol) was dissolved in anhydrous tetrahydrofuran (30 mL). The mixture was cooled down to 0°C, and a solution of compound **8-1A** (2.8 g, 13.38 mmol) in anhydrous tetrahydrofuran (20 mL) was added under nitrogen. The mixture was reacted at 70°C for 1 hour. To the reaction solution were added 1.5 mL of water, 1.5 mL of 15% NaOH solution, and 4.5 mL of water at 0°C. The mixture was stirred for 20 minutes. The reaction solution was filtered. The filter cake was washed with 10 mL of tetrahydrofuran, and the filtrate was concentrated to give compound **8-2.** ¹H NMR (400 MHz, CDCl₃) δ = 4.99 - 4.86 (m, 2H), 4.26 - 3.95 (m, 3H), 3.59 (m, 1H), 3.00 - 2.88 (m, 1H), 2.74 - 2.27 (m, 4H), 1.91 (s, 1H), 1.20 - 1.08 (m, 3H).

### Step 3: Synthesis of Intermediate 1-11B

Compound **1-11** was purified by supercritical fluid chromatography (chromatographic column: (S,S)Whelk-01 100×4.6mm ID, 5.0µm; mobile phase: A: supercritical CO₂, B: [0.1% ethanolamine - isopropanol]; B%: 50%, flow rate: 2.5 mL/min, retention times of 2.705 min and 3.357 min). The sample with a retention time of 3.357 min was collected to give compound **1-11B,** preparative SFC (chiral column: REGIS (s,s) WHELK-O1 (250mm*50mm,10um); mobile phase: A: supercritical CO₂, B: [75% isopropanol -25 % acetonitrile]; B%: 50%-50%). Chiral SFC analysis (chromatographic column: (S,S)Whelk-O1 100×4.6mm ID, 5.0µm; mobile phase: A: supercritical CO₂, B: [75% isopropanol/25% acetonitrile/0.05% diethylamine]; B%: 50% - 50 %, flow rate: 2.5 mL/min), compound **1-11A,** retention time = 2.536 min, ee value 99 %; Compound **1-11B,** retention time =3.317 min, ee value 99 %, MS m/z =844.5 [M+H]⁺.

### Step 4: Synthesis of Intermediate 1-12B

Compound **1-11B** (1 g, 1.18 mmol) was added to 20 mL of dichloromethane. m-Chloroperbenzoic acid (240.44 mg, 1.18 mmol, 85% purity) was added. The mixture was reacted at room temperature for 1 hour. 5 mL of 5% sodium thiosulfate solution was added to the reaction mixture. The mixture was extracted with dichloromethane (10 mL x 2). The organic phases were combined. The organic phase was washed with saturated brine (5 mL x 2), dried over anhydrous sodium sulfate, and filtered. The filtrate was concentrated and purified by column chromatography (petroleum ether:ethyl acetate = 10:1-1:1) to give compound **1-12B,** MS m/z = 860.4 [M+H]⁺.

### Step 5: Synthesis of Intermediate 8-3

Compound **8-2** (103.29 mg, 674.14 µmol) was dissolved in anhydrous tetrahydrofuran (3 mL). Sodium tert-butoxide (64.78 mg, 674.14 µmol) was added. The mixture was cooled down to -15°C for 15 minutes. Compound **1-12B** (0.29 g, 337.07 µmol) was added. The mixture was reacted at -15°C to 25°C for another 1 hour. 5 mL of saturated ammonium chloride was added to the reaction solution. The mixture was extracted with ethyl acetate (5 mL x 2), washed with saturated saline (5 mL x 2), dried over anhydrous sodium sulfate and concentrated. The crude product was purified by column chromatography (petroleum ether:ethyl acetate = 0:1) to give compound **8-3,** MS m/z = 949.2 [M+H]⁺.

### Step 6: Synthesis of Intermediate 8-4

Compound **8-3** (0.24 g, 252.77 µmol) was dissolved in anhydrous toluene (2.5 mL). The reaction was bubbled by nitrogen for 10 minutes. Dichlorobis[di-tert-butyl-(4-dimethylaminophenyl)phosphine]palladium (53.69 mg, 75.83 µmol, 53.69 µL) and tributyl(1-propynyl)tin (332.76 mg, 1.01 mmol) were added under nitrogen. The mixture was reacted at 110°C for 2 hours. The reaction solution was poured into 5 mL of water. The mixture was extracted with ethyl acetate (3 mL x 2). The organic phases were combined, washed with saturated brine (3 mL x 2), dried over anhydrous sodium sulfate, and concentrated to give crude compound **8-4.** MS m/z =953.4 [M+H]⁺.

### Step 7: Synthesis of compound 8 hydrochloride

Compound **8-4** (100 mg, 104.92 µmol) was dissolved in trifluoroacetic acid (3 mL). The mixture was reacted at 25°C for 0.5 h. After the reaction was completed, the reaction solution was directly concentrated and purified by HPLC (chromatographic column: Xtimate C18 150*40mm*5µm; mobile phase: [water(0.05% HCl)-acetonitrile]; acetonitrile%: 5%-35%, 10 min) to give compound **8** hydrochloride, MS m/z =613.3 [M+H]⁺. ¹H NMR (400 MHz, CD₃OD) δ ppm 6.99 - 6.93 (m, 1 H), 5.36 (br d, *J*=6.75 Hz, 2 H), 5.24 (br dd, *J*=10.94, 4.31 Hz, 1 H), 5.01 - 4.92 (m, 2 H), 4.78 - 4.73 (m, 2 H), 4.36 (br d, *J*=14.13 Hz, 1 H), 4.29 - 4.18 (m, 2 H), 4.08 - 4.0 (m, 1 H), 3.95 (br d, *J*=14.38 Hz, 2 H), 3.85 - 3.77 (m, 1 H), 3.56 (br d, *J*=14.63 Hz, 1 H), 3.45 - 3.37 (m, 1 H), 3.30 - 3.23 (m, 1 H), 3.15 - 3.01 (m, 2 H), 2.85 (br d, *J*=15.63 Hz, 1 H), 2.45 (br dd, *J*=12.01, 6.50 Hz, 1 H), 2.31 - 2.03 (m, 11 H).

### Example 9

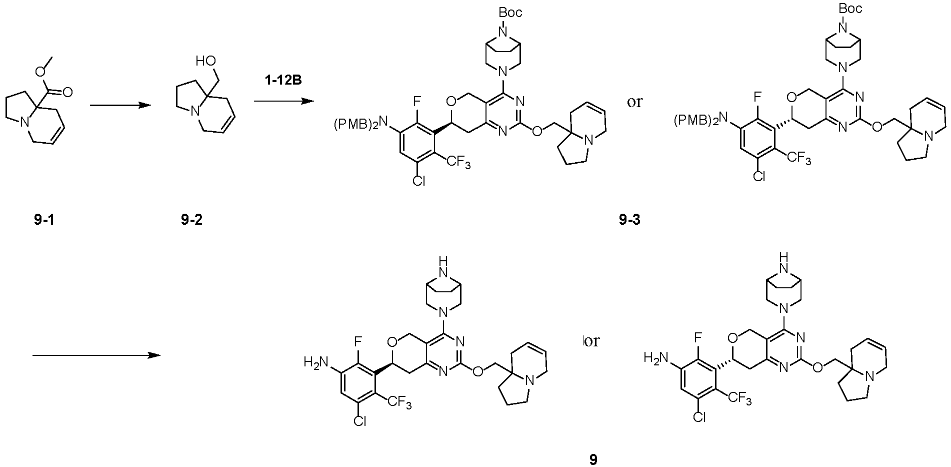

### Step 1: Synthesis of Intermediate 9-2

Compound **9-1** (1.2 g, 6.62 mmol) was weighed and added into THF (50 mL). The mixutre was cooled down to -20°C. LiAlH₄ (1 M, 6.62 mL) was added. The mixture was reacted at -20°C for 0.5 hours. The reaction was quenched with water. The reaction solution was directly concentrated. The residue was purified by column chromatography (dichloromethane:methanol = 10:1) to give compound **9-2,** MS m/z = 154.1 [M+H]⁺.

### Step 2: Synthesis of Intermediate 9-3

Compound **9-2** (73.43 mg, 479.26 µmol) was dissolved in anhydrous tetrahydrofuran (2 mL). Sodium tert-butoxide (46.06 mg, 479.26 µmol) was added. The mixture was cooled down to -15°C for 15 minutes. Compound **1-12B** (200 mg, 228.22 µmol) was added. The mixture was reacted at -15°C to 25°C for another 1 hour. 5 mL of saturated ammonium chloride was added to the reaction solution. The mixture was extracted with ethyl acetate (5 mL x 2), washed with saturated brine (5 mL x 2), dried over anhydrous sodium sulfate and concentrated. The residue was purified by column chromatography (petroleum ether:ethyl acetate = 0:1) to give compound **9-3.** MS m/z =949.4 [M+H]⁺.

### Step 3: Synthesis of compound 9 hydrochloride

Compound **9-3** (0.12 g, 126.39 µmol) was dissolved in anhydrous dichloromethane (1 mL). The mixture was cooled down to 0°C. Trifluoroacetic acid (302.62 mg, 2.65 mmol, 196.51 µL) was added, and the mixture was reacted at 25°C for 2 hours. The reaction solution was directly concentrated, and purified by HPLC (chromatographic column: Phenomenex Luna 80*30mm*3µm; mobile phase: [water (0.05% hydrochloric acid)-acetonitrile]; acetonitrile%: 5%-40%, running time 8min) to give compound **9** hydrochloride. MS m/z =609.1 [M+H]⁺, ¹H NMR (400 MHz, CD₃OD) δ = 6.90 (s, 1H), 6.13 - 5.78 (m, 2H), 5.31 - 5.21 (m, 1H), 4.98 - 4.89 (m, 3H), 4.28 - 4.14 (m, 2H), 4.07 - 3.68 (m, 6H), 3.54 - 3.33 (m, 4H), 3.11 - 2.99 (m, 1H), 2.61 - 2.49 (m, 2H), 2.37 - 1.94 (m, 8H).

### Example 10

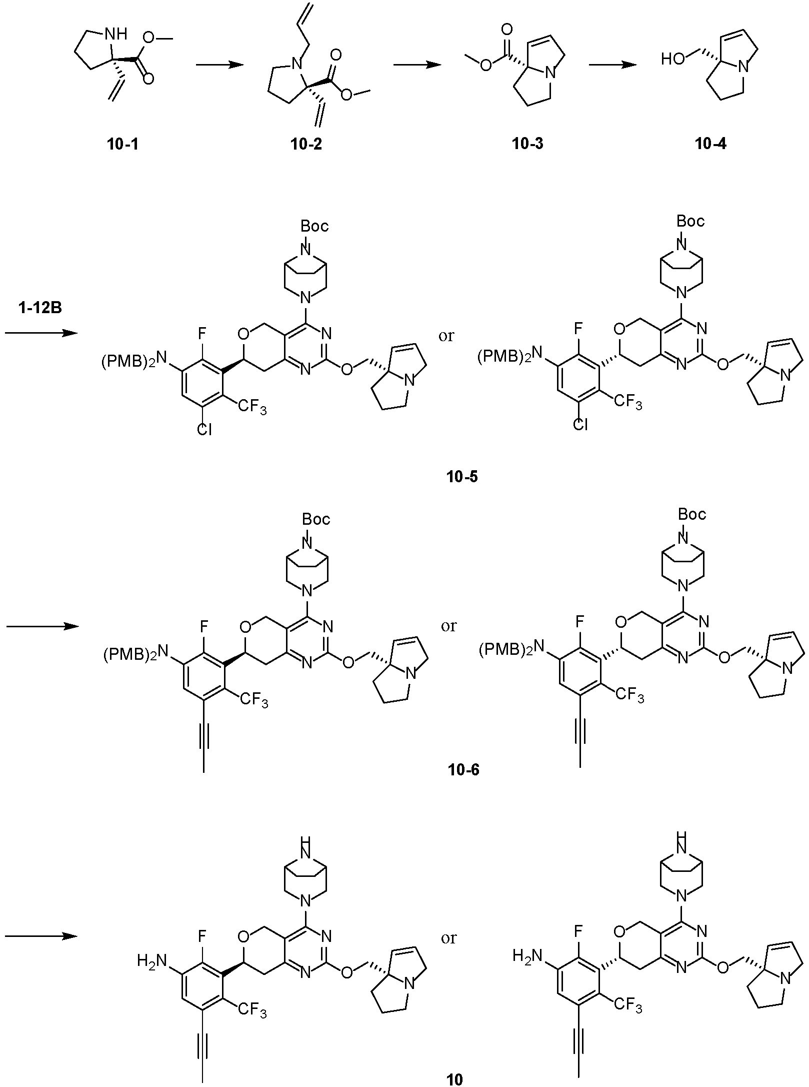

### Step 1: Synthesis of Intermediate 10-2

Compound **10-1** (1.0 g, 5.22 mmol) was dissolved in DMF (15 mL). K₂CO₃ (3.61 g, 26.09 mmol) was added. After stirring for 30 minutes, allyl bromide (757.46 mg, 6.26 mmol) was added. The mixture was reacted at room temperature for 5 hours. The reaction solution was diluted with 80 mL of ethyl acetate, and then filtered through a pad of Celite. The filtrate was concentrated under reduced pressure to remove the solvent to give a crude product. The crude product was purified by column chromatography (petroleum ether:ethyl acetate = 10:1) to give compound **10-2.** ¹H NMR (400 MHz, CDCl₃): δ = 6.07 - 5.92 (m, 1H), 5.88 - 5.73 (m, 1H), 5.27 - 5.18 (m, 1H), 5.16 - 5.08 (m, 2H), 5.04 - 4.96 (m, 1H), 3.72 - 3.61 (m, 3H), 3.30 - 3.18 (m, 1H), 3.10 - 2.93 (m, 2H), 2.75 - 2.63 (m, 1H), 2.34 - 2.17 (m, 1H), 1.91 - 1.72 (m, 3H).

### Step 2: Synthesis of Intermediate 10-3

Compound **10-2** (325 mg, 1.66 mmol) was dissolved in toluene (36 mL). Grubbs second-generation catalyst (70.65 mg, 83.22 µmol) was added. The mixture was heated to 120°C and stirred under nitrogen for 20 hours. The reaction solution was concentrated to give a crude product, which was purified by column chromatography (dichloromethane:methanol = 20:1) to give compound **10-3.** ¹H NMR (400 MHz, CDCl₃): δ = 5.94 - 5.77 (m, 2H), 4.12 - 4.00 (m, 1H), 3.83 - 3.72 (m, 3H), 3.49 - 3.41 (m, 1H), 3.39 - 3.30 (m, 1H), 2.74 - 2.59 (m, 1H), 2.43 - 2.27 (m, 1H), 1.93 - 1.82 (m, 3H).

### Step 3: Synthesis of Intermediate 10-4

Compound **10-3** (130 mg, 777.49 µmol) was dissolved in THF (3 mL). The mixture was cooled down to 0°C under nitrogen, and then lithium aluminum tetrahydride (1 M, 777.49 µL) was added dropwise. The reaction was stirred at 0°C for 0.5 hours. The reaction solution was quenched with 200 µL of water. 20 mL of ethyl acetate was added. The mixutre was stirred for 2 minutes. The mixture was dried over 2 g of anhydrous sodium sulfate, and filtered through celite. The filtrate was concentrated under reduced pressure to remove the solvent to give compound **10-4.** ¹H NMR (400 MHz, CDCl₃): δ = 5.74 - 5.63 (m, 1H), 5.59 - 5.47 (m, 1H), 3.92 - 3.70 (m, 1H), 3.44 - 3.23 (m, 3H), 3.14 - 2.99 (m, 1H), 2.62 - 2.47 (m, 1H), 1.76 - 1.51 (m, 5H).

### Step 4: Synthesis of Intermediate 10-5

Compound **10-4** (43.68 mg, 313.82 µmol) was dissolved in anhydrous tetrahydrofuran (2 mL). Sodium tert-butoxide (26.14 mg, 271.98 µmol) was added. The mixture was cooled down to -15°C for 15 minutes. Compound **1-12B** (180 mg, 209.22 µmol) was added. The mixture was reacted at -15°C to 25°C for 1 hour. To the reaction solution was added 5 mL of saturated ammonium chloride. The mixture was extracted with ethyl acetate (5 mL x 2), washed with saturated brine (5 mL x 2), dried over anhydrous sodium sulfate and concentrated. The crude product was purified by column chromatography (petroleum ether:ethyl acetate = 1:0-0:1) to give compound **10-5.** MS m/z = 935.2 [M+H]⁺.

### Step 5: Synthesis of Intermediate 10-6

Compound **10-5** (130.00 mg, 138.97 µmol) was dissolved in anhydrous toluene (1.5 mL). The mixture was bubbled by nitrogen for 10 minutes. Dichlorobis[di-tert-butyl-(4-dimethylaminophenyl)phosphine]palladium (29.52 mg, 41.69 µmol, 29.52 µL) and tributyl(1-propynyl)tin (182.95 mg, 555.89 µmol) were added under nitrogen. The mixture was reacted at 110°C for 2 hours. The reaction solution was poured into 5 mL of water. The mixture was extracted with ethyl acetate (3 mL x 2). The organic phases were combined and washed with saturated brine (3 mL x 2), dried over anhydrous sodium sulfate and concentrated. The crude product was purified by column chromatography (petroleum ether:ethyl acetate = 0:1) and then futher purified by high performance liquid chromatography (chromatographic column: Phenomenex Luna 80*30mm*3µm; mobile phase A: water (0.05% hydrochloric acid); mobile phase B: acetonitrile; running gradient: acetonitrile%: 35%-70%; running time: 8min) to give compound **10-6.** MS m/z =939.3 [M+H]⁺.

### Step 6: Synthesis of compound 10 hydrochloride

Compound **10-6** (30 mg, 31.95 µmol) was dissolved in anhydrous dichloromethane (0.5 mL). Trifluoroacetic acid (1.16 g, 10.16 mmol, 752.18 µL) was added at 0°C, and the mixture was reacted at 0-25°C for 3 h. The reaction solution was directly concentrated. The residue was purified by HPLC (chromatographic column: Phenomenex Luna 80*30mm*3µm; mobile phase A: water (0.05% hydrochloric acid); mobile phase B: acetonitrile; running gradient: acetonitrile%: 5%-35%; running time: 8 min) and freeze-dried to give compound 10 hydrochloride. MS m/z =599.1 [M+H]⁺.

### Example 11

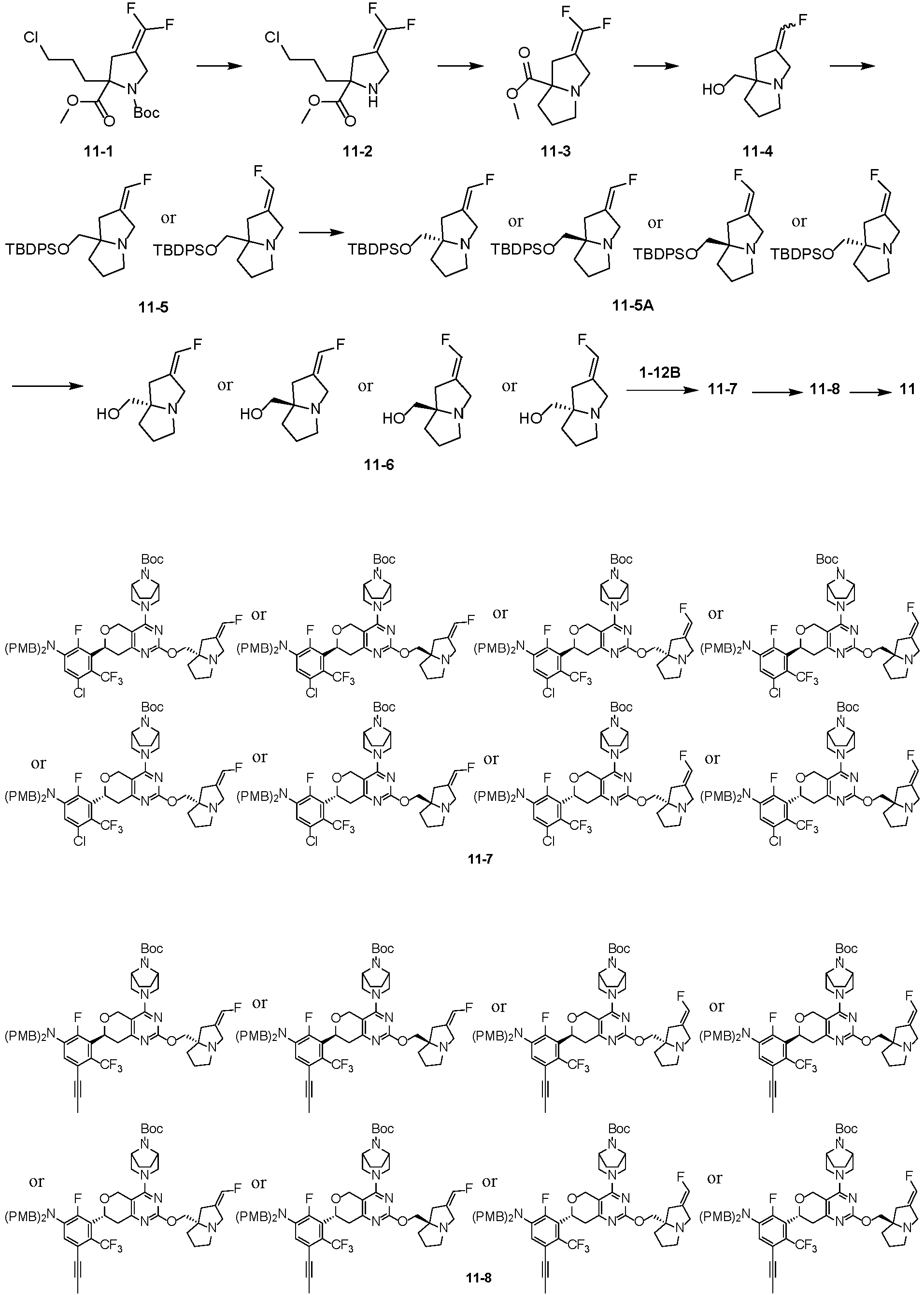

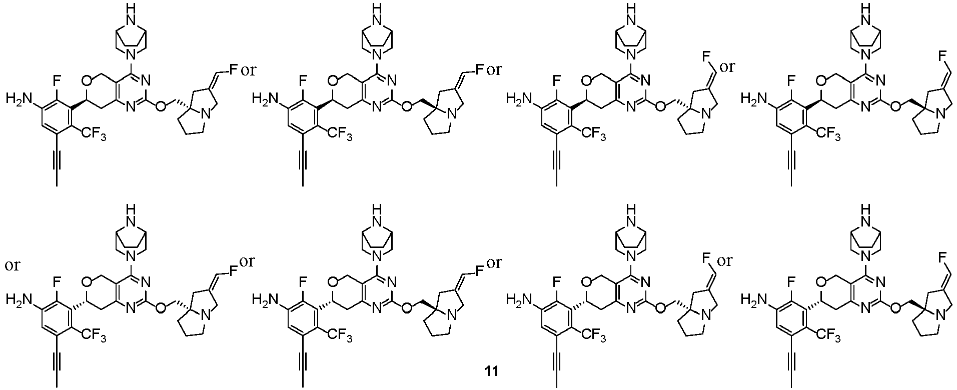

### Step 1: Synthesis of Intermediate 11-2

Compound **11-1** (20 g, 56.53 mmol) was dissolved in hydrochloric acid/ethyl acetate (4 M, 120 mL). The mixture was reacted at 25 °C for 2 hours. The reaction solution was directly concentrated to give the crude **11-2** hydrochloride. The crude product was directly used in the next step.

### Step 2: Synthesis of Intermediate 11-3

Crude **11-2** hydrochloride (20 g) was dissolved in DMF (65 mL). Potassium carbonate (14.2 g, 102 mmol) was added. The mixture was reacted at 25 °C for 12 hours. The reaction solution was diluted with 500 mL of ethyl acetate, washed sequentially with water (300 x 2) and 300 mL of saturated brine, dried over anhydrous sodium sulfate, and filtered. The filtrate was concentrated to give a crude product. The crude product was purified by column chromatography (petroleum ether:ethyl acetate = 2:1) to give compound **11-3.**

### Step 3: Synthesis of Intermediate 11-4

Compound **11-3** (7 g, 32.23 mmol) was dissolved in 2-methyltetrahydrofuran (75 mL). The atmosphere was replaced 3 times with nitrogen. A solution of red aluminum in toluene (37.2 g, 129 mmol, 35.8 mL, 70% purity) was slowly added at 10 °C under nitrogen. The mixture was reacted at 25 °C for 12 hours. The reaction solution was quenched by being added dropwise to 26.0 % aqueous sodium tartrate solution. The mixture was extracted with 2-methyltetrahydrofuran (200 mL). The aqueous phase was extracted with 2-methyltetrahydrofuran (50 mL x 3). The combined organic phase was washed with saturated brine, dried over anhydrous sodium sulfate, and filtered. The filtrate was concentrated to give compound **11-4.**

### Step 4: Synthesis of Intermediate 11-5 formate

Compound **11-4** (2.3 g, 13.4 mmol) was dissolved in DCM (30 mL). Imidazole (3.66 g, 53.7 mmol), DMAP (164 mg, 1.34 mmol) and TBDPSCl (7.38 g, 26. 9 mmol, 6.90 mL) were added. The mixture was reacted at 45 °C for 12 hours. Water (50 mL) was added to the reaction solution. The organic phase was separated, and the aqueous phase was extracted with dichloromethane (50 mL). The organic phases were combined, washed with 50 mL of saturated brine, dried over anhydrous sodium sulfate, and filtered. The filtrate was concentrated. Methyl tert-butyl ether (10 mL), n-heptane (21 mL) and HCl (2M, 21 mL) were added. The aqueous phase was separated, washed with a mixed solvent of methyl tert-butyl ether:n-heptane = 1:2 (20 mL*3), and adjusted to pH of 7 with aqueous sodium carbonate solution. The mixture was extracted sequentially with 270 mL of ethyl acetate and 40 mL of ethyl acetate. The organic phases were combined and washed with 20 mL of saturated brine, dried over anhydrous sodium sulfate, and filtered. The filtrate was concentrated to give a crude product. The crude product was purified by column chromatography (petroleum ether:ethyl acetate = 10:1) to give a crude intermediate **11-5** (which was the first point) with R_{f} = 0.6, which was further purified by high performance liquid chromatography: (chromatographic column: Phenomenex luna C18 250*80mm*10 µm; mobile phase: [water (0.225% formic acid)-acetonitrile]; acetonitrile %: 32%-62%, 12min) to give compound **11-5** formate. MS m/z = 410.3 [M+H]⁺. ¹H NMR (400 MHz, CDCl₃) δ ppm 7.62 - 7.53 (m, 4 H), 7.37 - 7.27 (m, 6 H), 6.47 - 6.17 (m, 1 H), 3.66 (br d, *J =* 14.92 Hz, 1 H), 3.38 - 3.30 (m, 2 H), 3.25 (br d, *J =* 14.79 Hz, 1 H), 3.03 - 2.94 (m, 1 H), 2.56 - 2.43 (m, 2 H), 2.12 (br d, *J =* 15.41 Hz, 1 H), 1.92 (ddd, *J =* 12.20, 7.92, 3.97 Hz, 1 H), 0.99 (s, 9 H) 1.82 - 1.51 (m, 3 H). ¹⁹F NMR (377 MHz, CDCl₃) δ ppm -131.66.

### Step 5: Synthesis of Intermediate 11-5A

Compound **11-5** (3.3 g, 7.57 mmol) was separated by preparative chiral SFC (chromatographic column: DAICEL CHIRALPAK IC (250mm*30mm, 10µm); mobile phase: A: supercritical CO₂, B: [0.1% NH₃ H₂O in methanol]; B%: 25%-25%, 4.5min, peak time: 2.117 min and 2.980 min). The sample with the peak time of 2.117min was collected to give compound **11-5A.** SFC analysis method of compound **11-5A** (chromatographic column: Chiralpak IC-3 50×4.6mm ID, 3µm; mobile phase: A (supercritical CO₂) and B (MeOH, containing 0.05 % diethylamine); gradient: B % = 5 ~ 10 %, flow rate: 3 mL / min), Rt= 2.014 min, ee value 98.5%. MS m/z = 410.3 [M+H]⁺.

### Step 6: Synthesis of Intermediate 11-6 hydrochloride

Compound **11-5A** (1.2 g, 2.93 mmol) was dissolved in 24 mL of 1,4-dioxane. Concentrated hydrochloric acid (12 M, 7.20 mL) was added. The mixture was reacted at 95 °C for 12 hours. The reaction solution was cooled down and diluted with 10 mL of water. The mixture was washed with 10 mL of ethyl acetate. The aqueous phase was freeze-dried to give compound **11-6** hydrochloride. 1H NMR (400 MHz, D2O) δ ppm 6.90 - 6.53 (m, 1 H), 4.22 (br d, *J*=15.04 Hz, 1 H), 3.97 (br d, *J*=15.04 Hz, 1 H), 3.79 - 3.69 (m, 1 H), 3.67 - 3.55 (m, 2 H), 3.24 - 3.14 (m, 1 H), 2.76 - 2.67 (m, 1 H), 2.64 - 2.54 (m, 1 H), 2.19 - 1.94 (m, 4 H). ¹⁹F NMR (376 MHz, CDCl₃) δ ppm -126.73.

### Step 7: Synthesis of Intermediate 11-7

Compound **1-12B** (300 mg, 349 µmol) was dissolved in anhydrous toluene (10.0 mL). Sdium tert-butoxide (134 mg, 1.39 mmol, 4 *eq*), 4A molecular sieve (500 mg) and compound **11-6** hydrochloride (109 mg, 523 µmol) were added. The mixture was reacted at 110 °C for 12 hours. The reaction solution was quenched by adding 30 mL of saturated aqueous ammonium chloride solution. The mixture was extracted with ethyl acetate (30 mL). The organic phase was washed with saturated brine (20 mL), dried over anhydrous sodium sulfate, and filtered. The filtrate was concentrated to give a crude product. The crude product was purified by column chromatography (petroleum ether:ethyl acetate = 1:1) to give compound **11-7.** ¹H NMR (400 MHz, CDCl₃) δ ppm 7.14 (d, *J =* 8.56 Hz, 4 H), 6.87 (d, *J =* 8.68 Hz, 5 H), 6.64 - 6.36 (m, 1 H), 5.28 - 5.18 (m, 1 H), 4.79 - 4.68 (m, 2 H), 4.44 - 4.24 (m, 6 H), 4.10 - 3.97 (m, 2 H), 3.94 - 3.74 (m, 8 H), 3.51 - 2.94 (m, 7 H), 2.77 - 2.55 (m, 2 H), 2.32 (br d, *J =* 15.16 Hz, 1 H), 2.22 - 2.11 (m, 1 H), 1.95 - 1.58 (m, 7 H), 1.51 (s, 9 H). ¹⁹F NMR (376 MHz, CDCl₃) δ ppm -119.86, -50.65.

### Step 8: Synthesis of Intermediate 11-8

Compound **11-7** (222 mg, 229 µmol) was dissolved in anhydrous toluene (10.0 mL). 1-Propynyltri-n-butyltin (755 mg, 2.29 mmol) and dichlorobis[di-tert-butyl-(4-dimethylaminophenyl)phosphine]palladium(II) (162 mg, 229 µmol, 162 µL) were added. The atmosphere was replaced 3 times with nitrogen. The mixture was reacted at 110 °C under nitrogen for 12 hours. The reaction solution was cooled down and concentrated to give a crude product, which was purified by column chromatography (petroleum ether:ethyl acetate = 1:1) to give compound **11-8.** MS m/z = 971.3 [M+H]⁺.

### Step 9: Synthesis of compound 11 formate

Compound **11-8** (149 mg, 129 µmol, 84% purity) was dissolved in dichloromethane (10 mL). Trifluoroacetic acid (770 mg, 6.75 mmol, 0.5 mL) was added. The mixture was reacted at 25°C for 12 hours. The reaction solution was diluted with 10 mL of water and 20 mL of ethyl acetate. The layers were separated. The organic phase was washed with 1 N of dilute hydrochloric acid (5 mL x 2). All aqueous phases were combined and adjusted to pH=8 with saturated NaHCO₃ aqueous solution. The solution was concentrated. To the resulting solid was added 20 mL of acetonitrile, and the mixture was stirred, and filtered. The filtrate was concentrated to give a crude product. The crude product was purified by high performance liquid chromatography (chromatographic column: Phenomenex luna C18 150*25mm* 10µm; mobile phase: [water (0.225% formic acid)-acetonitrile]; acetonitrile %: 9%-39%, 10min) and freeze-dried to give compound **11** formate. MS m/z = 631.3 [M+H]⁺. ¹H NMR (400 MHz, CD₃OD) δ ppm 6.93 (d, *J =* 8.44 Hz, 1 H), 6.90 - 6.65 (m, 1 H), 5.19 (br dd, *J =* 11.13, 3.79 Hz, 1 H), 4.77 - 4.69 (m, 2 H), 4.41 - 4.31 (m, 2 H), 4.26 (br d, *J =* 13.57 Hz, 1 H), 4.19 - 4.04 (m, 3 H), 3.86 - 3.73 (m, 2 H), 3.68 - 3.61 (m, 1 H), 3.52 - 3. 44 (m, 1 H), 3.37 (s, 3 H), 3.32 - 3.24 (m, 2 H), 3.06 - 2.81 (m, 3 H), 2.62 (br d, *J =* 16.14 Hz, 1 H), 2.31 - 2.05 (m, 6 H), 2.02 - 1.91 (m, 2 H).

### Example 12

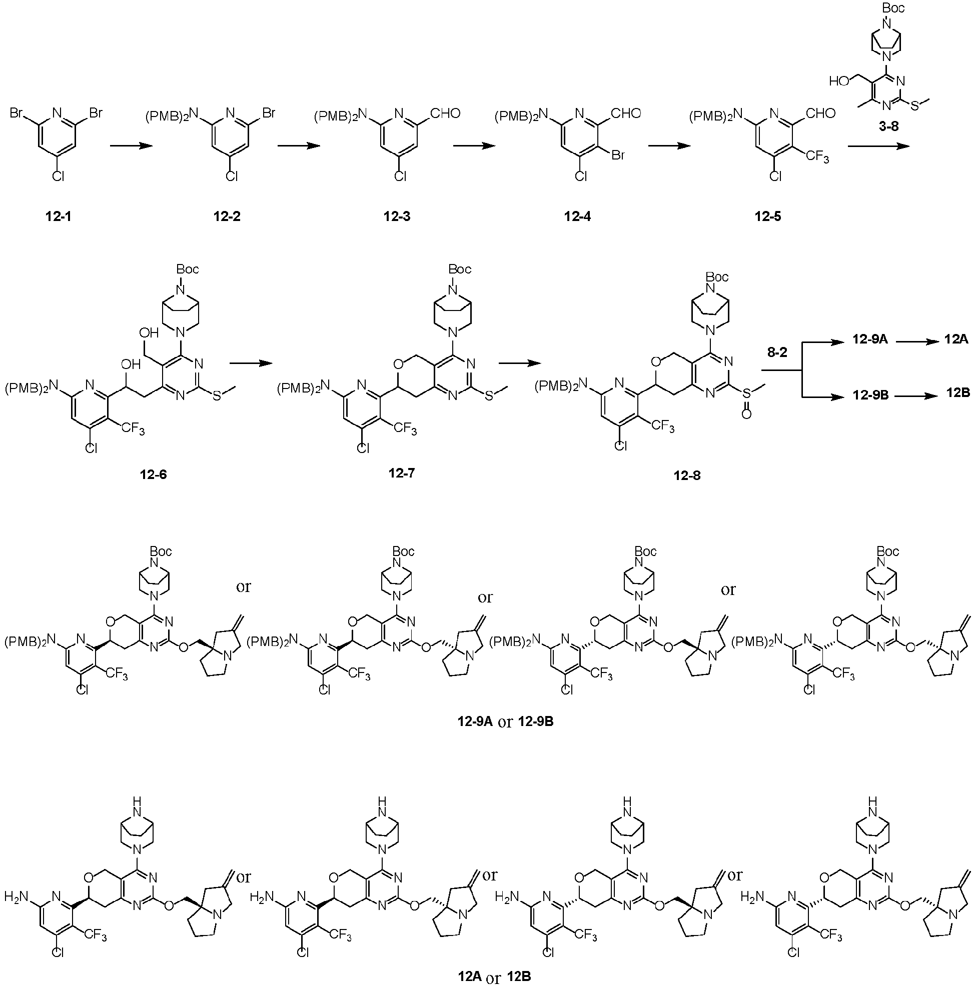

### Step 1: Synthesis of Intermediate 12-2

Compound **12-1** (20.0 g, 73.7 mmol) and bis-(4-methoxybenzyl)-amine (37.9 g, 147 mmol) were added to N-methylpyrrolidone (600 mL). The mixture was heated in an oil bath at 140 °C for 12 hours. The reaction solution was diluted with 250 mL of water, and washed with ethyl acetate (200 mL×3) and 200 mL of saturated brine. The organic phase was dried, and filtered. The filtrate was concentrated under reduced pressure to give a crude product. The crude product was purified by column (petroleum ether:ethyl acetate = 20:1) to give compound 12-2.

### Step 2: Synthesis of Intermediate 12-3

Compound **12-2** (22.0 g, 49.1 mmol) was dissolved in anhydrous tetrahydrofuran (220 mL). The mixture was cooled down to -78°C under nitrogen, and then n-butyl lithium (2.5 M, 21.6 mL) was added dropwise. After the addition was completed, the reaction was stirred for 1 hour, and then N,N-dimethylformamide (71.8 g, 982 mmol, 75.6 mL) was added dropwise. The mixture was stirred and reacted for 1 hour. The reaction was quenched by adding 100 mL of water. The mixture was extracted with ethyl acetate (100 mL×3). The organic phases were combined, and concentrated under reduced pressure to give a crude product. The crude product was purified by column (petroleum ether:ethyl acetate = 20:1) to give compound 12-3. MS m/z = 397.1 [M+H]⁺.

### Step 3: Synthesis of Intermediate 12-4

Compound **12-3** (15.0 g, 37.8 mmol) was dissolved in N,N-dimethylformamide (170 mL). N-bromosuccinimide (7.06 g, 39.6 mmol) was added. The mixuture was reacted at room temperature of 25°C under nitrogen for 1 hour. The reaction was quenched by adding 300 mL of water. The mixture was extracted with ethyl acetate (200 mL×3). The organic phases were combined, and concentrated under reduced pressure to give a crude product. The crude product was purified by column (petroleum ether:ethyl acetate = 10:1) to give compound 12-4. ¹H NMR (400 MHz, CDCl₃) δ ppm 10.10 (s, 1 H), 7.16 (d, *J =* 8.63 Hz, 4 H), 6.89 - 6.85 (m, 4 H), 6.76 (s, 1 H), 4.73 (s, 4 H), 3.81 (s, 6 H).

### Step 4: Synthesis of Intermediate 12-5

Compound **12-4** (14.2 g, 29.8 mmol) and methyl fluorosulfonyl difluoroacetate (28.6 g, 149 mmol, 18.9 mL) were added to N,N-dimethylformamide (160 mL). Cuprous iodide (5.68 g, 29.8 mmol) was added. The atmosphere was replaced with nitrogen and the mixture was stirred at 100°C for 2.5 hours. The reaction was quenched by adding 100 mL of water. The mixture was then extracted with ethyl acetate (300 mL×3). The organic phases were combined, and concentrated under reduced pressure to give a crude product. The crude product was purified by column chromatography (petroleum ether:ethyl acetate = 20:1) to give compound **12-5.** MS m/z = 465.0 [M+H]⁺.

### Step 5: Synthesis of Intermediate 12-6

Compound tetramethylpiperidine (1.45 g, 10.2 mmol) was dissolved in anhydrous tetrahydrofuran (100 mL). The mixture was cooled down to -40 °C under nitrogen, and then n-butyl lithium (2.5 M, 32.8 mL) was added dropwise. After the addition, the mixture was stirred for 0.5 hours. Compound **3-8** (7.80 g, 20.5 mmol) was dissolved in anhydrous tetrahydrofuran (50 mL). The mixture was added dropwise to the reaction flask at -60 °C. After the addition, the mixture was stirred for 0.3 hours. To the reaction was added compound **12-5** (10.4 g, 22.5 mmol) at -60 °C. After the addition, the mixture was stirred for 0.5 hours. The reaction was quenched by adding 300 mL of water. The mixture was then extracted with ethyl acetate (400 mLx 3). The organic phases were combined, and concentrated under reduced pressure to give a crude product. The crude product was purified by column chromatography (petroleum ether:ethyl acetate = 5:1) to give compound 12-6. MS m/z = 845.2 [M+H]⁺.

### Step 6: Synthesis of Intermediate 12-7

Compound **12-6** (6.1 g, 7.22 mmol) was dissolved in anhydrous toluene (60 mL). Cyanomethylene tri-n-butylphosphine (5.22 g, 21.6 mmol) was added under nitrogen. After the addition, the reaction was stirred at 100 °C for 12 hours. The reaction solution was concentrated under reduced pressure to give a crude product. The crude product was purified by column chromatography (petroleum ether:ethyl acetate = 5:1) to give compound **12-7.** MS m/z = 827.2 [M+H]⁺. ¹H NMR (400 MHz, CDCl3) δ ppm 7.09 (br d, *J =* 8.50 Hz, 4 H), 6.84 (br d, *J =* 8.50 Hz, 4 H), 6.53 (s, 1 H), 5.20 (br dd, *J =* 10.01, 3.00 Hz, 1 H), 4.81 - 4.54 (m, 7 H), 4.37 - 4.25 (m, 2 H), 3.80 (s, 7 H), 3.67 - 3.55 (m, 1 H), 3.50 - 3.37 (m, 2 H), 3.14 - 2.92 (m, 2 H), 2.49 (s, 3 H), 1.94 (br s, 3 H), 1.50 (s, 9 H).

### Step 7: Synthesis of Intermediate 12-8

Compound **12-7** (600 mg, 725 µmol) was dissolved in anhydrous dichloromethane (6 mL). m-Chloroperbenzoic acid (176 mg, 870 µmol, 85% purity) was added under nitrogen. After the addition, the mixture was stirred at 25 °C for 1 hour. The reaction was quenched by adding water (10 mL). The mixture was extracted with ethyl acetate (10 mL × 3). The organic phases were combined, and concentrated under reduced pressure to give a crude product. The crude product was purified by column chromatography (petroleum ether:ethyl acetate = 2:1) to give compound **12-8.**

### Step 8: Synthesis of Intermediates 12-9A and 12-9B

Compound **12-8** (600 mg, 698 µmol) was dissolved in anhydrous toluene (5 mL). Sodium tert-butoxide (201 mg, 2.09 mmol) and compound **8-2** (128 mg, 837 µmol) were added under nitrogen. After the addition, the mixture was stirred at 80 °C for 12 hours. The reaction was quenched by adding water (10 mL). The mixture was then extracted with ethyl acetate (10 mL×3). The organic phases were combined, and concentrated under reduced pressure to give a crude product. The crude product was purified by column chromatography (petroleum ether: ethyl acetate = 2:1) to give compound **12-9.** Compound **12-9** was subjected to preparative SFC chiral separation (chiral column: DAICEL CHIRALCEL OD (250 mm*30 mm, 10 µm); mobile phase: [supercritical CO₂-methanol (0.1 % ammonia)]; methanol (0.1% ammonia) %: 40% - 40 %) to give compounds **12-9A** and **12-9B.** SFC analysis: (chiral column: DAICEL CHIRALCEL OD - 3 (50mm* 4.6mm, 3µm); mobile phase: [supercritical CO₂-methanol (0.05% diethylamine)]; methanol %: 5%-40%), compound **12-9A,** Rt = 2.091min, ee value 99 %; MS m/z = 932.4 [M+H]⁺; compound **12-9B,** Rt =2.33 Imin, MS m/z = 932.3 [M+H]⁺.

### Step 9: Synthesis of compound 12A trifluoroacetate and compound 12B trifluoroacetate

Compound **12-9A** (40.0 mg, 42.9 µmol) was dissolved in trifluoroacetic acid (1.54 g, 13.4 mmol, 1 mL). The mixture was reacted at 50°C for 9 hours. The reaction solution was concentrated under reduced pressure, and the crude product was purified by high performance liquid chromatography (column: Phenomenex luna C18 150*25mm* 10µm; mobile phase: [water (0.075 % trifluoroacetic acid)-acetonitrile]; acetonitrile %: 8%-38%, 8min) to give compound **12A** trifluoroacetate. MS m/z = 592.2 [M+H]⁺. ¹H NMR (400 MHz, CD₃OD) δ ppm 6.73 (s, 1 H), 5.34 (br d, *J =* 8.00 Hz, 2 H), 5.27 (dd, *J =* 10.13, 3.25 Hz, 1 H), 4.98 (br s, 1 H), 4.74 (d, *J =* 13.76 Hz, 1 H), 4.69 - 4.59 (m, 2 H), 4.46 (br d, *J =* 14.26 Hz, 1 H), 4.37 (br d, *J =* 14.26 Hz, 1 H), 4.19 (br d, *J =* 13.13 Hz, 2 H), 3.99 - 3.92 (m, 2 H), 3.84 - 3.76 (m, 1 H), 3.73 (br d, *J =* 13.38 Hz, 1 H), 3.57 (dd, *J* = 18.26, 10.01 Hz, 1 H), 3.42 (br d, *J=* 14.01 Hz, 1 H), 3.26 (dt, *J=* 11.48, 7.08 Hz, 1 H), 3.07 (br d, *J=* 16.01 Hz, 1 H), 2.93 (dd, *J* = 18.32, 3.19 Hz, 1 H), 2.82 (br d, *J =* 16.13 Hz, 1 H), 2.39 (dd, *J =* 11.88, 6.50 Hz, 1 H), 2.31 - 2.10 (m, 6H), 2.04 - 1.95(m, 1 H).

Compound **12-9B** (40.0 mg, 42.9 µmol) was dissolved in trifluoroacetic acid (1.54 g, 13.4 mmol, 1 mL). The mixture was reacted at 50 °C for 9 hours. The reaction solution was concentrated under reduced pressure, and the crude product was purified by high performance liquid chromatography (column: Phenomenex luna C18 150*25mm* 10µm; mobile phase: [water (0.075 % trifluoroacetic acid)-acetonitrile]; acetonitrile %: 8%-38%, 8min) to give compound **12B** trifluoroacetate. MS m/z = 592.3 [M+H]⁺. ¹H NMR (400 MHz, CD₃OD) δ ppm 6.71 (s, 1 H), 5.32 (br d, *J =* 8.50 Hz, 2 H), 5.25 (dd, *J =* 10.19, 3.19 Hz, 1 H), 4.96 (s, 1 H), 4.73 (d, *J =* 13.76 Hz, 1 H), 4.63 (s, 2 H), 4.46 (br d, *J =* 14.13 Hz, 1 H), 4.36 (br d, *J* = 14.38 Hz, 1 H), 4.17 (br d, *J =* 13.01 Hz, 2 H), 4.00 - 3.89 (m, 2 H), 3.83 - 3.69 (m, 2 H), 3.55 (dd, *J =* 18.20, 10.07 Hz, 1 H), 3.41 (br d, *J* = 14.13 Hz, 1 H), 3.24 (dt, *J =* 11.54, 7.11 Hz, 1 H), 3.05 (br d, *J =* 16.01 Hz, 1 H), 2.91 (dd, *J =* 18.20, 3.31 Hz, 1 H), 2.80 (br d, *J* = 16.26 Hz, 1 H), 2.43 - 2.33 (m, 1 H), 2.28 - 2.08 (m, 6 H), 2.03 - 1.93 (m, 1 H).

### Example 13

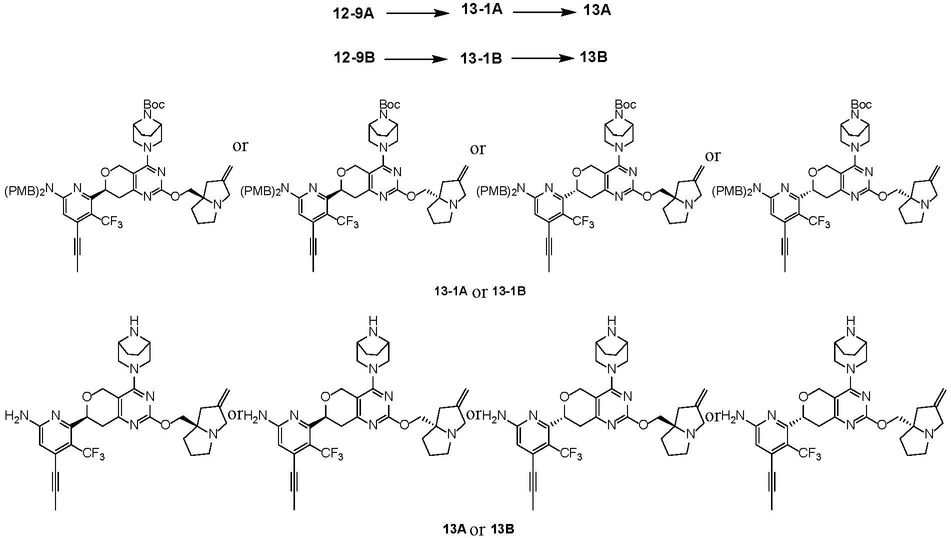

### Step 1: Synthesis of Intermediates 13-1A and 13-1B

Compound **12-9A** (250 mg, 268 µmol) was dissolved in anhydrous toluene (1 mL). 1-Propynyltri-n-butyltin (264 mg, 804 µmol) and dichlorobis[di-tert-butyl-(4-dimethylaminophenyl)phosphine]palladium (II) (37.9 mg, 53.6 µmol) were added. The atmosphere was replaced 3 times with nitrogen. The mixture was reacted at 100 °C under nitrogen for 12 hours. The reaction solution was cooled down and concentrated to give a crude product. The crude product was separated by a thin layer preparative plate (dichloromethane:methanol = 15:1) to give compound **13-1A.**

Referring to step 1, compound **13-1B** was obtained using **12-9B** as starting material.

### Step 2: Synthesis of compound 13A trifluoroacetate and compound 13B trifluoroacetate

Compound **13-1A** (120 mg, 128 µmol) was dissolved in trifluoroacetic acid (1.54 g, 13.4 mmol, 1 mL). The mixture was reacted at 50 °C for 9 hours. The reaction solution was concentrated under reduced pressure. The crude product was purified by high performance liquid chromatography (column: Phenomenex luna C18 150*25mm* 10µm; mobile phase: [water (0.075 % trifluoroacetic acid)-acetonitrile]; acetonitrile %: 10%-40%, 9min) to give compound **13A** trifluoroacetate. MS m/z = 596.3 [M+H]⁺. ¹H NMR (400 MHz, CD₃OD) δ ppm 6.80 (s, 1 H), 5.43 - 5.26 (m, 3 H), 5.00 (br d, *J =* 13.88 Hz, 2 H), 4.81 (d, *J =* 13.63 Hz, 1 H), 4.60 (d, *J =* 2.50 Hz, 2 H), 4.45 - 4.30 (m, 2 H), 4.24 - 4.13 (m, 2 H), 3.99 - 3.84 (m, 2 H), 3.83 - 3.64 (m, 2 H), 3.40 - 3.34 (m, 2 H), 3.28 - 3.21 (m, 1 H), 3.06 (br d, *J =* 16.26 Hz, 1 H), 2.95 (dd, *J* = 18.07, 3.56 Hz, 1 H), 2.81 (br d, *J* = 16.01 Hz, 1 H), 2.38 (br dd, *J* = 11.63, 6.38 Hz, 1 H), 2.29 - 2.12 (m, 8H), 1.99 (br t, *J =* 9.44 Hz, 1 H).

Compound **13-1B** (120 mg, 128 µmol) was dissolved in trifluoroacetic acid (1.54 g, 13.4 mmol, 1 mL). The mixture was reacted at 50 °C for 9 hours. The reaction solution was concentrated under reduced pressure. The crude product was purified by high performance liquid chromatography (column: Phenomenex luna C18 150*25mm* 10µm; mobile phase: [water (0.075 % trifluoroacetic acid)-acetonitrile]; acetonitrile %: 8%-38%, 8min) to give compound **13B** trifluoroacetate. MS m/z = 596.2 [M+H]⁺. ¹H NMR (400 MHz, CD₃OD) δ ppm 6.80 (s, 1 H), 5.31 (br d, *J =* 8.88 Hz, 3 H), 4.99 (br d, *J =* 13.76 Hz, 2 H), 4.80 (d, *J =* 13.88 Hz, 1 H), 4.58 (d, *J =* 2.38 Hz, 2 H), 4.43 - 4.31 (m, 2 H), 4.16 (br d, *J =* 16.01 Hz, 2 H), 3.95 - 3.84 (m, 2 H), 3.79 - 3.67 (m, 2 H), 3.37 (br d, *J =* 13.63 Hz, 2 H), 3.28 - 3.22 (m, 1 H), 3.07 - 2.91 (m, 2 H), 2.80 (br d, *J =* 15.88 Hz, 1 H), 2.40 - 2.33 (m, 1 H), 2.23 - 2.10 (m, 8 H), 2.02 - 1.93 (m, 1 H).

### Example 14

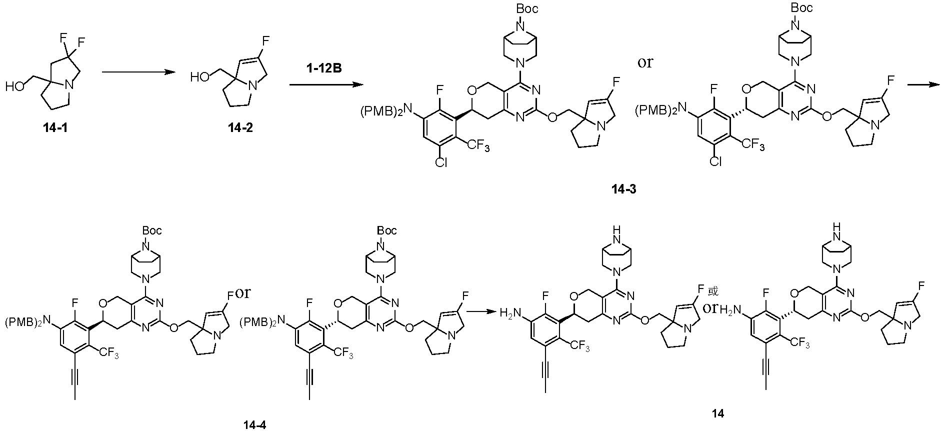

### Step 1: Synthesis of Intermediate 14-2

Compound **14-1** was dissolved in DMF (2.00 mL). To the reaction solution was added potassium tert-butoxide (299 mg, 2.67 mmol). The reaction solution was heated to 80 °C for 12 hours. Water (10 mL) was added to the reaction solution. The mixture was extracted with dichloromethane:methanol = 5:1 (10 mL*3). The organic phases were combined, washed with saturated brine (20 mL), dried over anhydrous sodium sulfate, and concentrated under reduced pressure. The crude product was purified by column (dichloromethane:methanol = 5:1) to give compound **14-2.** ¹H NMR **(400** MHz, CDCl₃) δ ppm 5.03 (s, 1 H) 3.89 (ddd, *J =* 15.04, 5.07, 2.14 Hz, 1 H) 3.38 - 3.44 (m, 1 H) 3.26 - 3.36 (m, 2 H) 3.13 - 3.22 (m, 1 H) 2.59 - 2.66 (m, 1 H) 1.61 - 1.90 (m, 5 H); ¹⁹F NMR (376 MHz, CDCl₃) δ ppm -128.29 (br s, 1 F).

### Step 2: Synthesis of Intermediate 14-3

Compound **1-12B** (120 mg, 139 µmol), compound **14-2** (43.9 mg, 279 µmol) and sodium tert-butoxide (40.2 mg, 418 µmol) were added to toluene (2.00 mL). The reaction solution was heated to 100 °C for 5 hours. The reaction solution was concentrated under reduced pressure. The crude product was purified by column (petroleum ether:ethyl acetate = 1:1) to give compound **14-3.** MS m/z = 953.1 [M+H]⁺.

### Step 3: Synthesis of Intermediate 14-4

Compound **14-3** (75.0 mg, 78.7 µmol) and 1-propynyltri-n-butyltin (104 mg, 315 µmol) were added to toluene (2.00 mL). The atmosphere was replaced three times with nitrogen. Dichlorobis[di-tert-butyl-(4-dimethylaminophenyl)phosphine]palladium(II) (16.7 mg, 23.6 µmol) was added to the reaction solution. The atmosphere was replaced with nitrogen for another 3 times. The mixture was heated to 110 °C for 3 hours. The reaction solution was filtered using a pad of Celite, and the filtrate was concentrated under reduced pressure to give a crude product. The crude product was purified by column (petroleum ether:ethyl acetate = 1:1) to give compound **14-4,** MS m/z = 957.4 [M+H]⁺.

### Step 4: Synthesis of compound 14

Compound **14-4** (73.0 mg, 76.3 µmol) was dissolved in dichloromethane (1.00 mL). TFA (0.10 mL) was added to the reaction solution. The mixture was reacted at 25 °C for 3 hours. The reaction solution was adjusted to pH of 7 with a saturated sodium bicarbonate solution. The mixture was concentrated under reduced pressure to give a crude product. The crude product was purified by high performance liquid chromatography (column: Waters Xbridge 150*25mm* 5µm; mobile phase: [water (0.05% ammonia water)-acetonitrile]; acetonitrile %: 53%-83%, 9min) to give compound **14,** MS m/z = 617.4 [M+H]⁺. ¹H NMR (400 MHz, CDCl₃) δ ppm 6.91 - 6.82 (m, 1 H), 5.49 - 5.38 (m, 1 H), 5.21 - 5.10 (m, 1 H), 4.75 - 4.69 (m, 2H), 4.18 - 3.92 (m, 6 H), 3.65 (br d, *J* = 1.96 Hz, 2 H), 3.44 (br s, 1 H), 3.37 - 3.25 (m, 3 H), 3.10 - 3.04 (m, 1 H), 3.00 - 2.91 (m, 1 H), 2.67 - 2.58 (m, 1 H), 2.19 - 2.08 (m, 1 H), 2.04 (s, 2 H), 2.03 - 1.97 (m, 1 H), 1.90 - 1.83 (m, 3 H), 1.78 - 1.72 (m, 3 H), 1.26 (br s, 1H), 0.88 - 0.80 (m, 2 H).

### Example 15

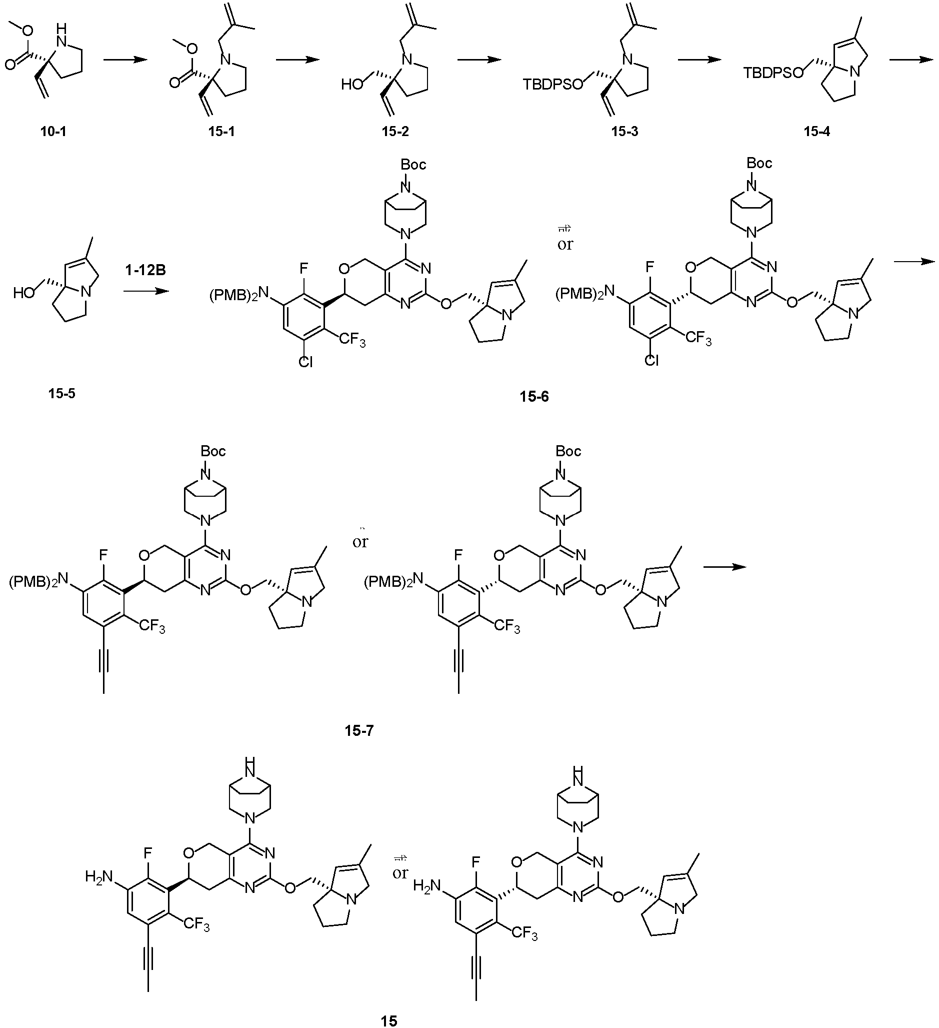

### Step 1: Synthesis of Intermediate 15-1

Compound **10-1** (3.00 g, 15.6 mmol) was dissolved in DMF (21 mL). Potassium carbonate (8.65 g, 62.6 mmol) and compound 3-bromo-2-methylpropene (2.54 g, 18.7 mmol) were added. The mixture was reacted at room temperature for 12 hours. 100 mL of water was poured into the reaction solution, and the mixture was extracted three times with ethyl acetate (10 mL). The organic phase was washed with saturated brine (100 mL), dried over anhydrous sodium sulfate, and filtered. The filtrate was concentrated under reduced pressure to give a crude product. The crude product was purified by column chromatography (petroleum ether:ethyl acetate = 20:1) to give compound **15-1.**

### Step 2: Synthesis of Intermediate 15-2

Compound **15-1** (2.5 g, 11.9 mmol) was dissolved in anhydrous THF (20.0 mL). Lithium aluminum hydride (680 mg, 17.9 mmol) was added at 0 °C. The mixture was then reacted at room temperature for 1 hour. To the reaction solution were added 1 mL of water, 1 mL of 15% NaOH aqueous solution, and 3 mL of water. The mixture was dried over anhydrous sodium sulfate, and filtered. The filtrate was concentrated under reduced pressure to give compound **15-2,** MS m/z = 182.1 [M+H]⁺.

### Step 3: Synthesis of Intermediate 15-3

Compound **15-2** (1.7 g, 9.38 mmol) was dissolved in anhydrous DCM (20.0 mL). Imidazole (2.55 g, 37.5 mmol) and 4-dimethylaminopyridine (114 mg, 937 µmol) were added, and then tert-butyldiphenylsilyl chloride (5.16 g, 18.7 mmol) was added at 0 °C. The mixture was reacted at room temperature for 12 hours. 50 mL of water was added to the reaction solution, and the mixturte was extracted three times with DCM (100 mL). The organic phase was washed with saturated brine (100 mL), dried over anhydrous sodium sulfate, and filtered. The filtrate was concentrated under reduced pressure to give a crude product. The crude product was purified by column chromatography (petroleum ether:ethyl acetate = 15:1) to give compound **15-3,** MS m/z = 420.4 [M+H]⁺.

### Step 4: Synthesis of Intermediate 15-4

Compound **15-3** (2 g, 4.77 mmol) was dissolved in anhydrous toluene (200 mL). Grubbs catalyst (597 mg, 953 µmol) was added. The mixture was reacted at 110 °C for 12 hours. The reaction solution was concentrated under reduced pressure to give a crude product. The crude product was purified by thin plate chromatography (dichloromethane:methanol = 20:1) to give compound **15-4,** MS m/z = 392.2 [M+H]⁺.

### Step 5: Synthesis of Intermediate 15-5 hydrochloride

Compound **15-4** (200 mg, 510 µmol) was dissolved in anhydrous dioxane (1.0 mL). HCI (12 M, 212 µL) was added. The mixture was reacted at 95 °C for 12 hours. 10 mL of water was added to the reaction solution. After extraction, the aqueous phase was lyophilized to give a crude product which could be directly used in the next step. The compound **15-5** hydrochloride was obtained. MS m/z = 154.1 [M+H]⁺.

### Step 6: Synthesis of Intermediate 15-6

Compound **1-12B** (300 mg, 348 µmol) was dissolved in anhydrous toluene (2 mL). Sodium tert-butoxide (100 mg, 1.05 mmol) and compound **15-5** hydrochloride (64.1 mg) were added. The mixture was reacted at room temperature for 12 hours. 30 mL of saturated aqueous ammonium chloride solution was poured into the reaction solution, and the mixture was extracted with ethyl acetate (30 mL). The organic phase was washed with saturated brine (20 mL), dried over anhydrous sodium sulfate, and filtered. The filtrate was concentrated under reduced pressure to give a crude product. The crude product was purified by column chromatography (dichloromethane:methanol = 10:1) to give compound **15-6,** MS m/z = 949.5 [M+H]⁺.

### Step 7: Synthesis of Intermediate 15-7

Compound **15-6** (80 mg, 84.2 µmol) was dissolved in anhydrous toluene (2 mL). 1-Propynyltri-n-butyltin (83.1 mg, 252 µmol) and dichlorobis[di-tert-butyl-(4-dimethylaminophenyl)phosphine]palladium (II) (11. mg, 16.8 µmol) were added. The atmosphere was replaced three times with nitrogen. The mixture was reacted at 110 °C for 2 hours under nitrogen. The reaction solution was cooled down and concentrated under reduced pressure to give a crude product. The crude product was purified by thin plate chromatography (dichloromethane:methanol = 10:1) to give compound 15-7, MS m/z = 953.6 [M+H]⁺.

### Step 8: Synthesis of compound 15 trifluoroacetate

Compound **15-7** (40 mg, 41.9 µmol) was dissolved in dichloromethane (1.0 mL). Tifluoroacetic acid (4.79 mg, 41.9 µmol) was added. The mixture was reacted at 25°C for 1 hour. The reaction solution was concentrated under reduced pressure to give a crude product. The crude product was purified by high performance liquid chromatography (column: Phenomenex luna C18 150*25mm* 10µm; mobile phase: [water (0.075 % trifluoroacetic acid)-acetonitrile]; acetonitrile %: 15%-45%, 9min) and freeze-dried to give compound **15** trifluoroacetate. MS m/z = 613.5 [M+H]⁺. ¹H NMR (CD₃OD, 400 MHz) δ 6.92 (d, *J =* 8.5 Hz, 1H), 5.48 (br s, 1H), 5.20-5.10 (m, 1H), 4.95-4.90 (m, 3H), 4.80-4.70 (m, 1H), 4.64 (d, *J =* 12.3 Hz, 1H), 4.55-4.51 (m, 2H), 4.33 (br d, *J =* 14.5 Hz, 1H), 4.15 (br dd, *J =* 2.1, 16.1 Hz, 2H), 3.92 (br d, *J =* 15.4 Hz, 1H), 3.82 (br d, *J =* 13.1 Hz, 1H), 3.81-3.70 (m, 1H), 3.71-3.61 (m, 1H), 2.92 (br dd, *J =* 4.1, 18.4 Hz, 1H), 2.30-2.11 (m, 8H), 2.01-1.91 (m, 4H), 1.85 (s, 3H).

### Example 16

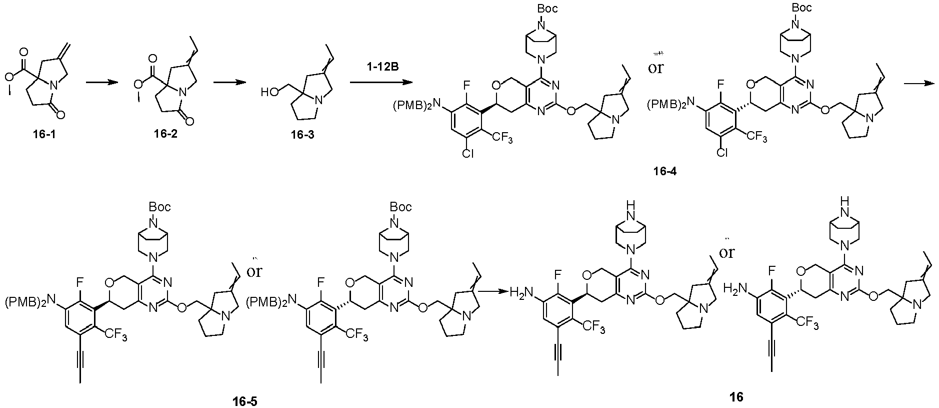

### Step 1: Synthesis of Intermediate 16-2

Compound **16-1** (500 mg, 2.56 mmol) was dissolved in anhydrous toluene (1 mL). Grubbs catalyst (217 mg, 256 µmol) and compound propylene/dichloromethane solution (0.5 M, 5.12 mL) were added. The mixture was reacted at 50 °C for 2 hours. The reaction solution was concentrated under reduced pressure to give a crude product. The crude product was purified by column chromatography (petroleum ether:ethyl acetate = 10:1) to give compound **16-2,** MS m/z = 210.0 [M+H]⁺.

### Step 2: Synthesis of Intermediate 16-3

Compound **16-2** (210 mg, 1.00 mmol) was dissolved in anhydrous THF (1.0 mL). Lithium aluminum hydride (76.1 mg, 2.01 mmol) was added at 0 °C. The mixture was reacted at 70 °C for 12 hours. To the reaction solution were added 0.7 mL of water, 0.7 mL of 15% NaOH aqueous solution, and 2 mL of water. The mixture was dried over anhydrous sodium sulfate, and filtered. The filtrate was concentrated under reduced pressure to give a crude product, which was directly used in the next step. Compound **16-3** was obtained.

### Step 3: Synthesis of Intermediate 16-4

Compound **1-12B** (300 mg, 348 µmol) was dissolved in anhydrous THF (1 mL). Sodium tert-butoxide (100 mg, 1.05 mmol) and compound **16-3** (58.3 mg, 348 µmol) were added. The mixture was reacted at room temperature for 12 hours. The reaction solution was quenched by adding 30 mL of saturated aqueous ammonium chloride solution. The mixture was extracted with ethyl acetate (30 mL). The organic phase was washed with saturated brine (20 mL), dried over anhydrous sodium sulfate, and filtered. The filtrate was concentrated to give a crude product. The crude product was purified by column chromatography (dichloromethane:methanol = 10:1) to give compound **16-4,** MS m/z = 963.5 [M+H]⁺.

### Step 4: Synthesis of Intermediate 16-5

Compound **16-4** (90.0 mg, 93.4 µmol) was dissolved in anhydrous toluene (1 mL). 1-Propynyltri-n-butyltin (122 mg, 373 µmol) and dichlorobis [di-tert-butyl-(4-dimethylaminophenyl)phosphine]palladium (II) (19.8 mg, 28.0 µmol) were added. The atmosphere was replaced three times with nitrogen. The mixture was reacted at 110 °C under nitrogen for 12 hours. The reaction solution was cooled down and concentrated under reduced pressure to give a crude product. The crude product was purified by thin plate chromatography (dichloromethane:methanol = 12:1) to give compound **16-5,** MS m/z = 967.5 [M+H]⁺.

### Step 5: Synthesis of compound 16 trifluoroacetate

Compound **16-5** (70.00 mg, 72.3 µmol) was dissolved in dichloromethane (1.0 mL). Trifluoroacetic acid (539 mg, 4.73 mmol) was added. The mixture was reacted at 25°C for 12 hours. The reaction solution was concentrated under reduced pressure to give a crude product. The crude product was purified by high performance liquid chromatography (column: Phenomenex luna C18 150*25mm* 10µm; mobile phase: [water (0.075 % trifluoroacetic acid)-acetonitrile]; acetonitrile %: 18%-48%, 9min) and freeze-dried to give compound **16** trifluoroacetate, MS m/z = 6 27.3 [M+H]⁺.

### Example 17

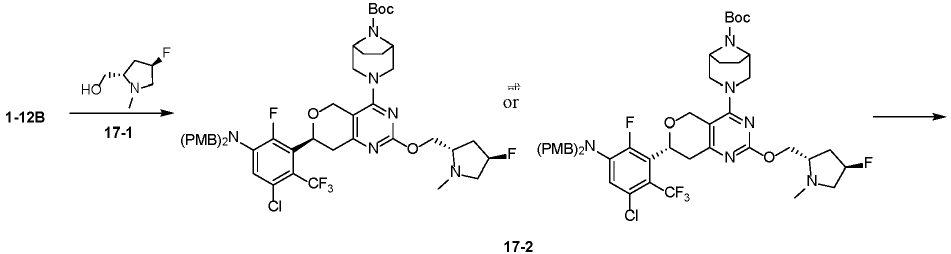

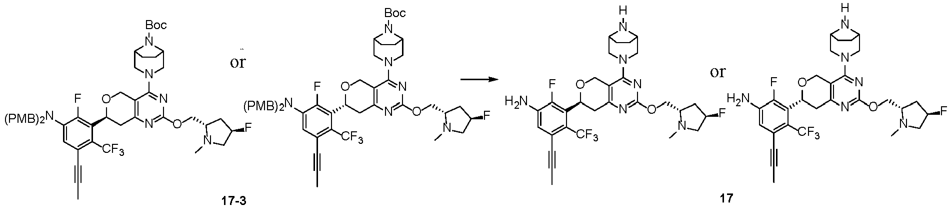

### Step 1: Synthesis of Intermediate 17-2

Compound **1-12B** (870 mg, 1.01 mmol) was dissolved in anhydrous toluene (10.0 mL). Sodium tert-butoxide (777 mg, 8.09 mmol) and compound **17-1** (404 mg, 3.03 mmol) were added. The mixture was reacted at 100 °C for 12 hours. To the reaction solution was added 30.0 mL of saturated aqueous ammonium chloride solution. The mixture was extracted with ethyl acetate (50.0 mL). The organic phase was washed with saturated brine (30.0 mL), dried over anhydrous sodium sulfate, and filtered. The filtrate was concentrated under reduced pressure to give a crude product. The crude product was purified by column chromatography (petroleum ether:ethyl acetate = 2:1) to give compound **17-2,** MS m/z = 929.4 [M+H]⁺.

### Step 2: Synthesis of Intermediate 17-3 formate

Compound **17-2** (445 mg, 479 µmol) was dissolved in anhydrous toluene (4.00 mL). 1-Propynyltri-n-butyltin (630 mg, 1.92 mmol) and dichlorobis[di-tert-butyl-(4-dimethylaminophenyl)phosphine]palladium(II) (102 mg, 144 µmol) were added. The atmosphere was replaced three times with nitrogen. The mixture was reacted at 110 °C for 3 hours under nitrogen. The reaction solution was cooled down and concentrated under reduced pressure to give a crude product. The crude product was purified by high performance liquid chromatography (column: Phenomenex luna C18 150*25mm* 10µm; mobile phase: [water(0.225% formic acid)-acetonitrile]; acetonitrile %: 40%-70%, 10min), and freeze-dried to give compound **17-3** formate, MS m/z = 933.5 [M+H]⁺.

### Step 3: Synthesis of compound 17

The compound **17-3** formate (210 mg) was dissolved in dichloromethane (2.00 mL). Trifluoroacetic acid (3.00 mL) was added. The mixture was reacted at 25°C for 4 hours. The reaction solution was quenched by adding 6.00 mL of aqueous ammonia. The mixture was concentrated to give a crude product. The crude product was purified by high performance liquid chromatography (column: Waters Xbridge 150*25mm*5µm; mobile phase: [water (0.05% aqueous ammonia)-acetonitrile]; acetonitrile %: 28%-58%) and freeze-dried to give compound **17.** MS m/z = 593.3 [M+H]⁺.

### Example 18

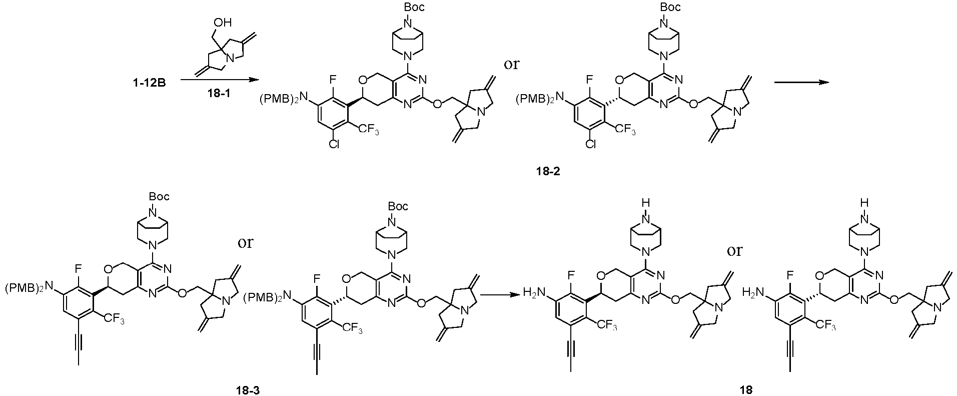

### Step 1: Synthesis of Intermediate 18-2

Compound **18-1** (192.05 mg, 1.16 mmol) was dissolved in THF (10 mL). Sodium tert-butoxide (111.70 mg, 1.16 mmol) was added. The mixture was stirred at 0°C for 1 hour. Compound **1-12B** (0.5 g, 581.16 µmol) was added. The mixture was reacted at 0°C for 1 hour. Water (10 mL) was added to the reaction solution. The mixture was extracted with ethyl acetate (20 mL * 3). The organic phases were combined, washed with saturated brine (20 mL), and dried over anhydrous sodium sulfate. The filtrate was concentrated under reduced pressure. The residue was purified by column chromatography (dichloromethane:methanol = 20:1) to give compound **18-2,** MS m/z = 961.7 [M+H]⁺.

### Step 2: Synthesis of Intermediate 18-3

Compound **18-2** (0.526 g, 547.07 µmol) was dissolved in toluene (20 mL). Compound 1-propynyl tri-n-butyltin (900.24 mg, 2.74 mmol) and dichlorobis[di-tert-butyl-(4-dimethylaminophenyl)phosphine]palladium (II) (116.21 mg, 164.12 µmol) were added. The atmosphere was replaced with nitrogen for 5 times. The mixture was reacted at 120°C for 5 hours. Water (10 mL) was added to the reaction system. The mixutre was filtered by a pad of Celite. The filter cake was washed with ethyl acetate (20 mL). The filtrate was extracted with ethyl acetate (20 mL* 3). The organic phases were combined, washed with saturated brine (20 mL), and dried over anhydrous sodium sulfate. The filtrate was concentrated under reduced pressure. The residue was purified by column (dichloromethane:methanol = 10:1) to give compound **18-3,** MS m/z = 965.7 [M+H]⁺.

### Step 3: Synthesis of compound 18 hydrochloride

Compound **18-3** (0.5 g, 518.09 µmol) was dissolved in DCM (10 mL). Trifluoroacetic acid (4.13 g, 36.25 mmol) was added. The mixture was reacted at 25°C for 1 hour. The reaction solution was concentrated under reduced pressure. The crude product was purified by HPLC (column: Xtimate C18 150*40mm*5µm; mobile phase: [water (0.05% hydrochloric acid)-acetonitrile]; (acetonitrile)%: 5%-35%, 10min) to give compound **18** hydrochloride, MS m/z = 625.3 [M+H]⁺.

### Example 19

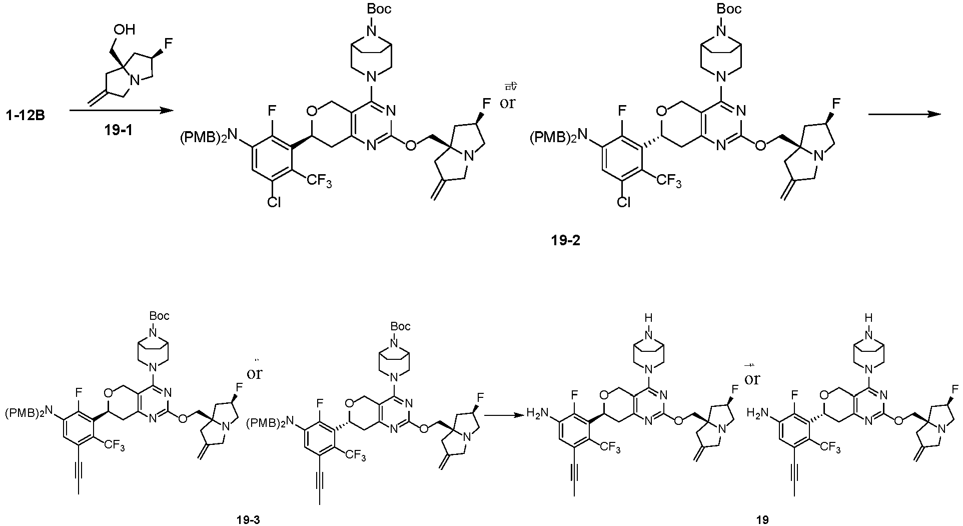

### Step 1: Synthesis of Intermediate 19-2

Compound **19-1** (199.00 mg, 1.16 mmol) was dissolved in THF (10 mL). Sodium tert-butoxide (111.70 mg, 1.16 mmol) was added. The reaction system was stirred at 0°C for 1 hour. Compound **1-12B** (0.5 g, 581.16 µmol) was added. The mixture was reacted at 0°C for 1 hour. Water (10 mL) was added to the reaction solution. The mixture was extracted wtih ethyl acetate (20 mL* 3). The organic phases were combined, washed with saturated brine (20 mL), and dried over anhydrous sodium sulfate. The filtrate was concentrated under reduced pressure. The residue was purified by column chromatography (dichloromethane:methanol = 20:1) to give compound **19-2,** MS m/z = 967.6 [M+H]⁺.

### Step 2: Synthesis of Intermediate 19-3

Compound **19-2** (0.442 g, 456.87 µmol) was dissolved in toluene (10 mL). Compound 1-propynyl tri-n-butyltin (601.44 mg, 1.83 mmol) and dichlorobis[di-tert-butyl-(4-dimethylaminophenyl)phosphine]palladium (II) (97.05 mg, 137.06 µmol) were added. The atmosphere was replaced with nitrogen for 5 times. The mixture was reacted at 120°C for 5 hours. Water (10 mL) was added to the reaction system, and the mixture was filtered through a pad of Celite. The filter cake was washed with ethyl acetate (20 mL), and the filtrate was extracted with ethyl acetate (20 mL* 3). The organic phases were combined, washed with saturated brine (20 mL), and dried over anhydrous sodium sulfate. The filtrate was concentrated under reduced pressure. The residue was purified by column chromatography (dichloromethane:methanol = 10:1) to give compound **19-3,** MS m/z = 971.8 [M+H]⁺.

### Step 3: Synthesis of compound 19 hydrochloride

Compound **19-3** (0.442 g, 455.17 µmol) was dissolved in DCM (5 mL). Trifluoroacetic acid (15.58 g, 136.67 mmol) was added. The mixture was reacted at 25°C for 1 hour. The reaction solution was concentrated under reduced pressure, and the crude product was purified by HPLC (column: Xtimate C18 150*40mm*5µm; mobile phase: [water (0.05% hydrochloric acid)-acetonitrile]; (acetonitrile)%: 5%-35%, 10min) to give compound **19** hydrochloride, MS m/z = 631.3 [M+H]⁺.

### Example 20

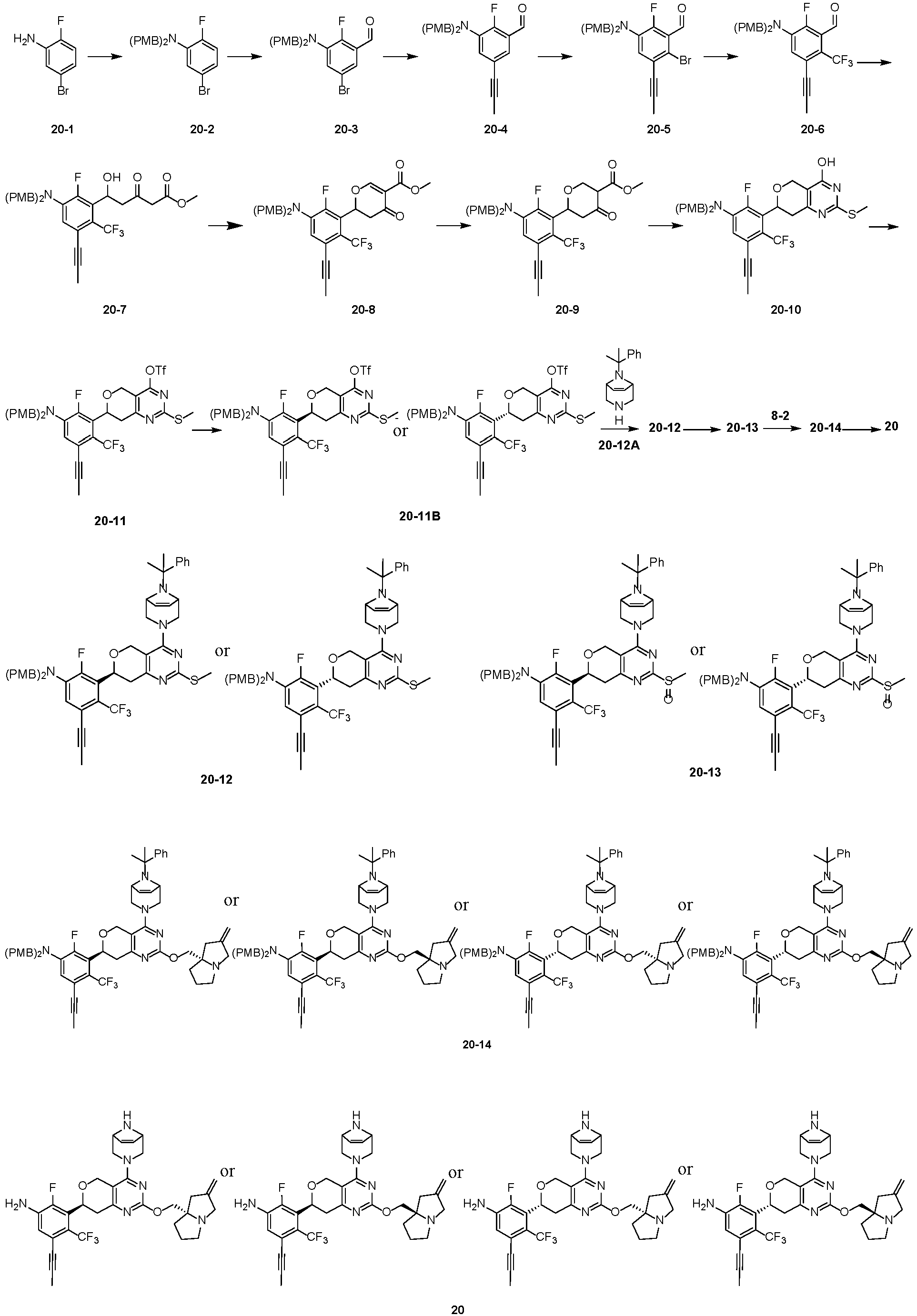

### Step 1: Synthesis of compound 20-2

Compound **20-1** (480 g, 2.53 mol) was weighed. DMF (2500 mL), 4-methoxybenzyl chloride (5.18 mol, 702.79 mL), potassium carbonate (872.82 g, 6.32 mol), and potassium iodide (419.35 g, 2.53 mol) were added. The mixture was reacted at 65 °C for 2 hours. Water (1000 mL) was added. The mixture was extracted with ethyl acetate (1000 mL * 3). The organic phase was concentrated under reduced pressure to give compound **20-2,** MS m/z = 430.0 [M+H]⁺.

### Step 2: Synthesis of compound 20-3

Compound 2,2,6,6-tetramethylpiperidine (220.59 g, 1.56 mol, 265.13 mL) was weighed. THF (3000 mL) was added, and n-butyl lithium (2.5 M, 499.73 mL) was added at -5 °C. The mixture was stirred for 0.5 hours. The mixture was cooled down to -60 °C. To the mixture was added **20-2** (280 g, 624.67 mmol). The mixture was stirred for 0.5 hours. Finally, DMF (228.28 g, 3.12 mol, 240.30 mL) was added. The mixture was reacted for another 0.5 hours. The reaction solution was poured into water (1000 mL). The mixture was adjusted to pH of 7 by adding 1N of hydrochloric acid. The mixture was extracted with ethyl acetate (1000 mL * 3). The filtrate was concentrated under reduced pressure, and purified by column chromatography (petroleum ether:ethyl acetate = 10:1) to give compound **20-3.**

### Step 3: Synthesis of Compound 20-4

**20-3** (370 g, 807.30 mmol) was weighed. Toluene (1500 mL), dichlorobis[di-tert-butyl-(4-dimethylaminophenyl)phosphine]palladium (2.86 g, 4.04 mmol, 2.86 mL) and tributyl(1-propynyl)tin (265.69 g, 807.30 mmol) were added. The mixture was reacted at 120°C for 2 hours under nitrogen. The mixture was concentrated under reduced pressure, and purified by column chromatography (petroleum ether:ethyl acetate = 5:1) to give compound **20-4.** MS m/z = 418.1 [M+H]⁺.

### Step 4: Synthesis of Compound 20-5

**20-4** (450 g, 970.13 mmol) was weighed. DMF (100 mL) and N-bromosuccinimide (189.93 g, 1.07 mol) were added. The mixture was reacted at 25 °C for 2 hours. Additional N-bromosuccinimide (17.27 g, 97.01 mmol) was added. The mixture was reacted for another 3 hours. The reaction solution was concentrated under reduced pressure and purified by column chromatography (petroleum ether:ethyl acetate = 5:1) to give compound **20-5.** MS m/z = 496.0 [M+H]⁺.

### Step 5: Synthesis of Compound 20-6

**20-5** (55 g, 110.81 mmol) was weighed. DMF (300 mL), methyl fluorosulfonyl difluoroacetate (42.57 g, 221.61 mmol, 28.19 mL), and cuprous iodide (42.21 g, 221.61 mmol) were added. The mixture was reacted at 110 °C under nitrogen for 2 hours. The reaction was quenched by adding 500 mL of water. The mixture was extracted with ethyl acetate (600 mL*3). The organic phases were combined. The combined organic phase was washed sequentially with water (800 mL*2) and saturated brine (800 mL), dried with anhydrous sodium sulfate, and filtered. The filtrate was concentrated. The residue was purified by column chromatography (petroleum ether:ethyl acetate = 10:1) to give compound **20-6.** MS m/z = 485.9 [M+H]⁺.

### Step 6: Synthesis of Compound 20-7

To a solution of sodium hydride (6.34 g, 158.61 mmol, 60% purity) in tetrahydrofuran (350 mL) was added dropwise methyl acetoacetate (18.42 g, 158.61 mmol, 17.10 mL) at 0°C. The mixture was reacted for 15 minutes. A solution of **20-6** (35 g, 72.10 mmol) in tetrahydrofuran (350 mL) was added. The mixture was reacted for 0.5 hours. The reaction was quenched by adding 200 mL of saturated ammonium chloride solution. The mixture was extracted with ethyl acetate (300 mL*2). The organic phases were combined, washed with saturated brine (400 mL), dried over anhydrous sodium sulfate, and filtered. The filtrate was concentrated under reduced pressure. The residue was purified by column chromatography (petroleum ether:ethyl acetate = 10:1-1:1) to give compound **20-7.** MS m/z = 624.2 [M+Na]⁺.

### Step 7: Synthesis of Compound 20-8

**20-7** (38 g, 63.17 mmol) was weighed. Dichloromethane (300 mL) and N,N-dimethylformamide dimethyl acetal (9.03 g, 75.80 mmol) were added. The mixture was reacted at 25°C for 16 hours. The reaction solution was cooled down to 0 °C. Boron trifluoride diethyl etherate (10.76 g, 75.80 mmol, 9.32 mL) was added. The mixutre was reacted at 0 °C for another 1 hour. To the reaction system was added 200 mL of saturated sodium bicarbonate solution. The organic phase was separated. The aqueous phase was extracted with 200 mL of dichloromethane. The extracted organic phases were combined. The combined organic phase was washed with 250 mL of saturated brine, dried with anhydrous sodium sulfate, and filtered. The filtrate was concentrated under reduced pressure. The residue was purified by column chromatography (petroleum ether:ethyl acetate = 10:1-1:1) to give compound **20-8.** MS m/z = 612.1 [M+H]⁺.

### Step 8: Synthesis of Compound 20-9

**20-8** (30 g, 49.05 mmol) was weighed. Tetrahydrofuran (300 mL) was added. To the mixture was added tri-sec-butyl lithium borohydride (1 M, 53.96 mL) at -60 °C. The mixture was reacted at -60°C for 1 hour. The reaction was quenched by adding 200 mL of water. The mixture was extracted with ethyl acetate (300 mL* 2). The organic phases were combined, washed with 300 mL of saturated brine, dried over anhydrous sodium sulfate, and filtered. The filtrate was concentrated under reduced pressure. The residue was purified by column chromatography (petroleum ether:ethyl acetate = 10:1-5:1) to give compound **20-9.** MS m/z = 614.1 [M+H]⁺.

### Step 9: Synthesis of compound 20-10

**20-9** (20 g, 32.59 mmol) was weighed. Ethanol (200 mL), 2-methyl-2-thioisourea sulfate (27.22 g, 97.78 mmol), and sodium carbonate (6.91 g, 65.19 mmol) were added. The mixture was reacted at 50°C for 13 hours. The reaction solution was concentrated to dryness. 40 mL of water was added. The mixture was extracted with ethyl acetate (50 mL*2). The organic phases were combined. The combined organic phase was washed with 60 mL of saturated brine, dried with anhydrous sodium sulfate, and filtered. The filtrate was concentrated under reduced pressure to give compound **20-10.** MS m/z = 654.3 [M+H]⁺.

### Step 10: Synthesis of Compound 20-11B

**20-10** (21 g, 32.13 mmol) was weighed. DMF (2 00 mL), N,N-diisopropylethylamine (12.46 g, 96.38 mmol, 16.79 mL), and N-phenylbis(trifluoromethanesulfonyl)imide (13.77 g, 38.55 mmol) were added. The mixture was reacted at 25°C for 1 hour. To the system was added 300 mL of water. The mixture was extracted with ethyl acetate (300 mL*3). The organic phase was washed sequentially with water (400 mL*2) and saturated brine (400 mL), dried with anhydrous sodium sulfate, and filtered. The filtrate was concentrated under reduced pressure and purified by column chromatography (petroleum ether:ethyl acetate = 10:1) to give compound **20-11,** which was separated by SFC (chromatographic column: DAICEL CHIRALPAK IG (250mm*50mm, 10µm); mobile phase: [supercritical CO₂-ethanol (0.1% ammonia water)]; ethanol%: 25%-25%) to give compound **20-11A** and compound **20-11B.** Chiral SFC analysis (chromatographic column: Chiral Pak IG-3 (100mm*4.6mm, 3µm); mobile phase: [supercritical CO₂-ethanol (0.05% diethylamine)]; (ethanol (0.05% diethylamine))%: 5%-40%), compound **20-11A,** Rt = 2.574 minutes, ee value 99%; compound **20-11B,** Rt = 3.055 minutes, ee value 99%.

### Step 11: Synthesis of Compound 20-12

Compound **20-11B** (2 g, 2.55 mmol) and **20-12A** (871.78 mg, 3.82 mmol) were weighed and dissolved in DMF (10 mL). N,N-diisopropylethylamine (986.88 mg, 7.64 mmol) was added. The mixture was reacted at 100°C for 1 hour. The reaction solution was concentrated under reduced pressure and purified by column chromatography (petroleum ether:ethyl acetate = 5:1) to give compound **20-12,** MS m/z = 864.0 [M+H]⁺.

### Step 12: Synthesis of Compound 20-13

Compound **20-12** (1.8 g, 2.08 mmol) was weighed and dissolved in THF (20 mL). m-Chloroperbenzoic acid (422.96 mg, 2.08 mmol, 85% purity) was added. The mixture was stirred at 25°C for 0.5 hours. The reaction solution was concentrated under reduced pressure to give compound **20-13.** MS m/z= 880.0 [M+H]⁺.

### Step 13: Synthesis of Compound 20-14

Compound **8-2** (626.81 mg, 4.09 mmol) was weighed and dissolved in THF (5 mL). Sodium tert-butoxide (393.15 mg, 4.09 mmol) was added at 0°C. One hour later, **20-13** (1.8 g, 2.05 mmol) was added. The mixture was reacted for another 1 hour. The reaction was quenched by adding 10 mL of water. The mixture was adjusted to pH of about 6 with 1N dilute hydrochloric acid. The mixture was extracted with ethyl acetate (100 mL*2). The organic phases were combined, dried with anhydrous sodium sulfate, and filtered. The filtrate was concentrated under reduced pressure, and purified by column chromatography (dichloromethane:methanol = 10:1) to give compound **20-14,** MS m/z = 969.1 [M+H]⁺.

### Step 14: Synthesis of Compound 20 hydrochloride

Compound **20-14** (1.4 g, 1.44 mmol) was weighed and dissolved in trifluoroacetic acid (10 mL). The mixture was reacted at 25°C for 1 hour. The reaction solution was concentrated under reduced pressure, and the crude product was purified by high performance liquid chromatography (column: Xtimate C18 150*40mm*5µm; mobile phase: [water (0.05% hydrochloric acid)-acetonitrile]; (acetonitrile)%: 5%-35%, 10min) to give compound **20** hydrochloride, MS m/z = 610.6 [M+H]⁺.

### Example 21

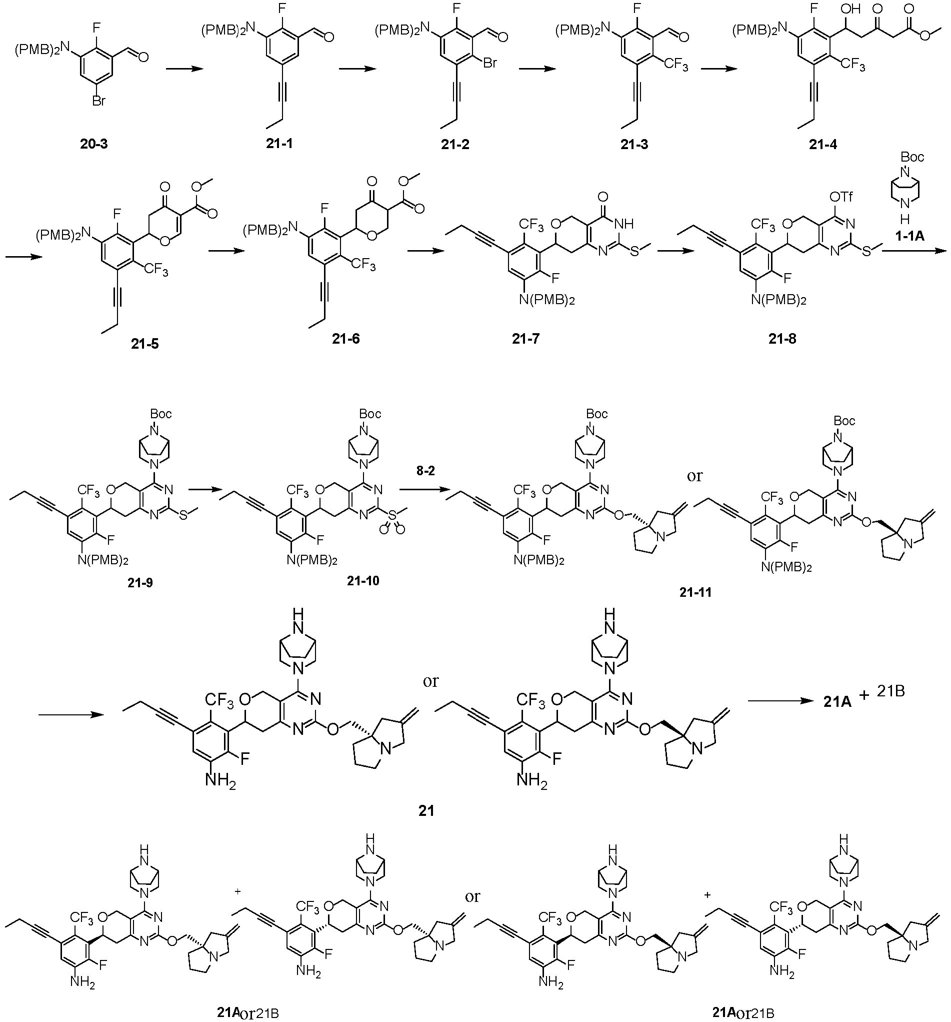

### Step 1: Synthesis of Intermediate 21-1

Compound **20-3** (19 g, 41.46 mmol) and pentynoic acid (4.88 g, 49.75 mmol) were added to dimethyl sulfoxide (200 mL). Dichlorobistriphenylphosphine palladium (290.98 mg, 414.56 µmol), 1,4-bis(diphenylphosphino)butane (353.59 mg, 829.12 µmol) and 1.8-diazabicyclo[5.4.0]undecane-7-ene (18.93 g, 124.37 mmol, 18.75 mL) were added. The atmosphere was replaced with nitrogen. The resulting mixture was heated to 110-115°C, and reacted with stirring for 18 hours. The reaction solution was diluted with 500 mL of ethyl acetate, and washed with water (50 mL×3) and saturated brine (100 mL). The organic phase was concentrated under reduced pressure to give a crude product, which was purified by column chromatography (petroleum ether:ethyl acetate = 5:1) to give compound **21-1.** ¹H NMR (400 MHz, CDCl₃) δ: 10.34 (s, 1H), 7.42 (dd, *J*=2.0, 5.6 Hz, 1H), 7.16 (d, *J*=8.4 Hz, 4H), 7.11-7.06 (m, 1H), 6.87 - 6.82 (m, 4H), 4.24 (s, 4H), 3.82 - 3.78 (m, 6H), 2.37 (q, *J*=7.6 Hz, 2H), 1.21 (t, *J*=7.6 Hz, 3H).

### Step 2: Synthesis of Intermediate 21-2

Compound **21-1** (4.0 g, 9.27 mmol) was dissolved in DMF (30 mL). N-bromosuccinimide (2.14 g, 12.05 mmol) was added. The resulting reaction solution was stirred at 50°C for 3 hours under nitrogen. The reaction solution was diluted with 150 mL of ethyl acetate, and then washed with water (20 mL×3) and saturated brine (20 mL). The organic phase was concentrated under reduced pressure to give a crude product. The crude product was purified by column chromatography (petroleum ether:ethyl acetate = 5:1) to give compound 21-2, MS m/z = 510.1 [M+H]⁺.

### Step 3: Synthesis of Intermediate 21-3

Compound **21-2** (4.6 g, 9.01 mmol) and compound methyl fluorosulfonyl difluoroacetate (6.93 g, 36.05 mmol, 4.59 mL) were added to N,N-dimethylformamide (30 mL). Cuprous iodide (3.43 g, 18.03 mmol) was added. The resulting reaction solution was heated to 105°C under nitrogen and stirred for 3 hours. The reaction solution was concentrated under reduced pressure to give a crude product. The crude product was purified by column (petroleum ether:ethyl acetate = 5:1) to give compound **21-3,** MS m/z = 500.0 [M+H]⁺.

### Step 4: Synthesis of Intermediate 21-4

Sodium hydride (856.78 mg, 21.42 mmol, 60% purity) was suspended in anhydrous tetrahydrofuran (30 mL). The resulting mixture was cooled down to 0 °C. Methyl acetoacetate (2.09 g, 17.99 mmol, 1.94 mL) was then added dropwise. After the addition, the mixture was stirred for 30 minutes, and then n-butyl lithium (2.5 M, 7.88 mL) was added dropwise. After the addition, the mixture was stirred for another 30 minutes. The ice bath was removed. The mixture was cooled down to -78 °C. Finally, to the mixture was added dropwise a solution of compound **21-3** (4.28 g, 8.57 mmol) in 15 mL of tetrahydrofuran. After the addition, the mixture was stirred for 0.5 hours. The reaction was quenched with 1M hydrochloric acid. The mixture was adjusted to a pH of 5 and the organic phase was separated. The aqueous phase was extracted with 30 mL of ethyl acetate for three times. The organic phases were combined and concentrated under reduced pressure to give a crude product. The crude product was purified by column chromatography (petroleum ether:ethyl acetate = 2:1) to give compound **21-4.** MS m/z = 638.2 [M+Na]⁺.

### Step 5: Synthesis of Intermediate 21-5

Compound **21-4** (5.0 g, 6.01 mmol) was dissolved in dichloromethane (40 mL), and then N,N-dimethylformamide dimethyl acetal (2.15 g, 18.03 mmol, 2.40 mL) was added. The resulting reaction solution was stirred for 18 hours under nitrogen. The reaction solution was cooled down to 0°C, and then boron trifluoride diethyl etherate (853.03 mg, 6.01 mmol) was added dropwise. After the addition was completed, the reaction solution was stirred for 1 hour. The reaction was quenched with 20 mL of saturated sodium bicarbonate. The mixture was then extracted with 30 mL of dichloromethane for 3 times. The organic phases were combined, and concentrated under reduced pressure to give a crude product. The crude product was purified by column (petroleum ether:ethyl acetate = 2:1) to give compound **21-5.** MS m/z = 626.0 [M+H]⁺.

### Step 6: Synthesis of Intermediate 21-6

Compound **21-5** (2.6 g, 4.16 mmol) was dissolved in anhydrous tetrahydrofuran (20 mL). The mixture was cooled down to -78°C under nitrogen, and then tri-sec-butyl lithium borohydride (1 M, 4.57 mL) was added dropwise. After the addition was completed, the reaction solution was stirred at -78°C for 0.5 hours. The reaction was quenched by adding 15 mL of water. The mixture was adjusted to a pH of 5-6 with 1M hydrochloric acid, and the organic phase was separated. The aqueous layer was extracted with 30 mL × 3. The organic phases were combined, and concentrated under reduced pressure to give a crude product. The crude product was purified by column (petroleum ether:ethyl acetate = 5:1) to give compound **21-6,** MS m/z = 650.0 [M+Na]⁺.

### Step 7: Synthesis of Intermediate 21-7

Compound **21-6** (0.6 g, 955.99 µmol) and 2-methylthiourea sulfate (539.83 mg, 2.87 mmol) were added to ethanol (15 mL), and then sodium carbonate (202.65 mg, 1.91 mmol) was added. The resulting reaction solution was heated to 65°C under nitrogen and stirred for 18 hours. The reaction solution was concentrated under reduced pressure. To the residue were added 10 mL of water and 80 mL of ethyl acetate. The mixture was adjusted to a pH of 6 with 2M hydrochloric acid under stirring. The aqueous layer was separated, and the organic phase was washed with 10 mL of saturated brine, dried over anhydrous sodium sulfate, and concentrated under reduced pressure to give compound **21-7,** MS m/z= 668.3 [M+H]⁺.

### Step 8: Synthesis of Intermediate 21-8

Compound **21-7** (640 mg, 958.50 µmol) and compound N-phenylbis(trifluoromethanesulfonyl)imide (513.63 mg, 1.44 mmol) were added to N,N-dimethylformamide (5 mL), and then N,N-diisopropylethylamine (247.76 mg, 1.92 mmol) was added. The atmosphere was replaced with nitrogen. The reaction solution was heated to 35°C and stirred for 1.5 hours. The reaction solution was diluted with 50 mL of ethyl acetate, and then washed with water (10 mL × 3). The organic phase was concentrated under reduced pressure to give a crude product, which was purified by column chromatography (petroleum ether:ethyl acetate = 5:1) to give compound **21-8.** MS m/z = 800.4 [M+H]⁺.

### Step 9: Synthesis of Intermediate 21-9

Compound **21-8** (340 mg, 425.12 µmol) and compound **1-1A** (180.50 mg, 850.24 µmol) were added to N,N-dimethylformamide (3 mL), and then N,N-diisopropylethylamine (164.83 mg, 1.28 mmol) was added. The resulting reaction solution was heated to 105°C under nitrogen and stirred for 1 hour. The reaction solution was concentrated under reduced pressure to give a crude product. The crude product was purified by column chromatography (petroleum ether:ethyl acetate = 2:1) to give compound **21-9.** MS m/z = 862.4 [M+H]⁺.

### Step 10: Synthesis of Intermediate 21-10

Compound **21-9** (223 mg, 258.71 µmol) was dissolved in dichloromethane (3 mL), and then m-chloroperbenzoic acid (52.52 mg, 258.71 µmol, 85% purity) was added. The resulting reaction solution was stirred at 25°C for 1 hour under nitrogen. The reaction solution was concentrated under reduced pressure to give a crude product, which was purified by column chromatography (petroleum ether:ethyl acetate = 1:1) to give compound **21-10.** MS m/z = 894.4 [M+H]⁺.

### Step 11: Synthesis of Intermediate 21-11

Compound **8-2** (76.44 mg, 498.89 µmol) and sodium tert-butoxide (47.95 mg, 498.89 µmol) were added to tetrahydrofuran (2 mL). The mixture was stirred at 25°C for 0.5 hours. A solution of compound **21-10** (223 mg, 249.45 µmol) in 2 mL of tetrahydrofuran was added dropwise to the reaction solution. The mixture was stirred at 25 °C for 0.5 hours. The reaction solution was diluted with 30 mL of ethyl acetate, and then washed with 5 mL of saturated brine. The organic phase was dried over anhydrous sodium sulfate, and concentrated under reduced pressure to give compound **21-11.** MS m/z= 967.8 [M+H]⁺.

### Step 12: Synthesis of Compound 21

Compound **21-11** (238 mg, 246.10 µmol) was added to trifluoroacetic acid (2 mL). The reaction was stirred at 25°C for 1 hour. The reaction solution was concentrated under reduced pressure to give a crude product, which was purified by HPLC (column: Xtimate C18 150*40mm*5µm; mobile phase: [water (0.05% hydrochloric acid)-acetonitrile]; (acetonitrile)%: 5%-35%, 10min) to give compound **21** hydrochloride, MS m/z= 627.4 [M+H]⁺.

### Step 13: Synthesis of Compounds 21A and 21B

The compound **21** hydrochloride was separated by SFC (chromatographic column: DAICEL CHIRALPAK IC (250mm*30mm, 10 µm); mobile phase: [supercritical CO₂-methanol (0.1% ammonia water)]; methanol %: 60%-60%) to give compound **21A** and compound **21B,** which were subjected to chiral SFC analysis (chromatographic column: Chiral Pak IC (100mm*4.6mm, 3µm); mobile phase: [supercritical CO₂-ethanol (0.05 _{%} diethylamine)]; (methanol (0.05% diethylamine))%: 60%-60%), compound **21A,** Rt = 1.696 minutes, ee value 99%, MS m/z = 627.4 [M+H]⁺; ¹H NMR (400MHz, CDCl₃) δ: 6.89 (d, *J*=8.4 Hz, 1H), 5.21 (d, *J*=12.0 Hz, 2H), 5.11 (d, *J*=7.6 Hz, 1H), 4.80 - 4.50 (m, 4H), 4.44 - 4.27 (m, 2H), 4.23 - 3.82 (m, 5H), 3.77 - 3.48 (m, 3H), 3.35-3.20 (m, 1H), 3.11 - 2.86 (m, 3H), 2.63 - 2.51 (m, 1H), 2.38 (q, *J*=7.2 Hz, 3H), 2.30 - 2.06 (m, 5H), 2.04 - 1.75 (m, 2H), 1.20 (t, *J*=7.6 Hz, 3H). Compound **21B,** Rt = 5.728 min, ee value 99%, MS m/z = 627.3 [M+H]⁺, ¹H NMR (400MHz, CDCl₃) δ: 6.86 (d, *J*=8.4 Hz, 1H), 5.19 - 5.08 (m, 1H), 4.95 (br s, 2H), 4.80 - 4.64 (m, 3H), 4.15 - 4.03 (m, 4H), 4.01-3.93 (m, 1H), 3.82 - 3.67 (m, 3H), 3.59 - 3.43 (m, 2H), 3.36 - 3.14 (m, 4H), 3.00-2.90 (m, 1H), 2.80 (d, *J*=15.0 Hz, 1H), 2.72 - 2.62 (m, 1H), 2.45-2.32 (m, 3H), 2.22 - 2.11 (m, 1H), 2.09 - 1.84 (m, 5H), 1.82 - 1.67 (m, 2H), 1.21 (t, *J*=7.2 Hz, 3H).

### Example 22

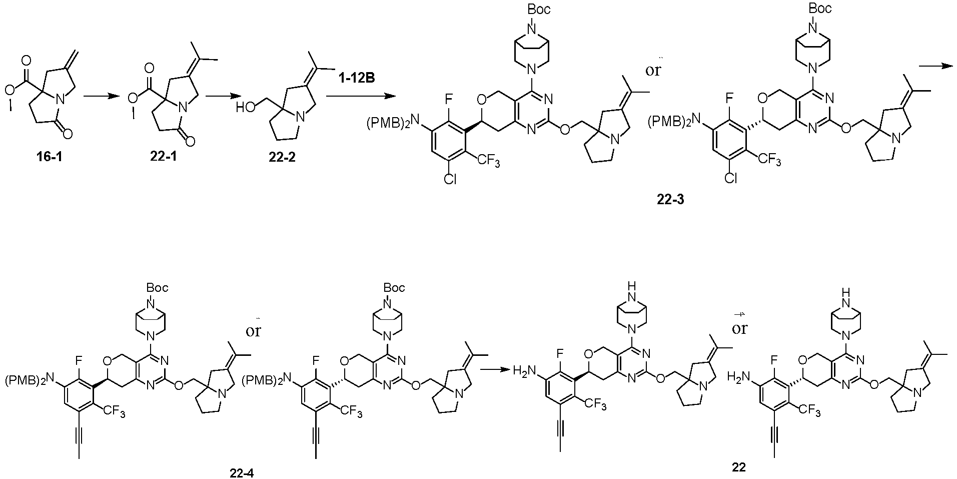

### Step 1: Synthesis of Intermediate 22-1 formate

Compound **16-1** (1.28 g, 6.56 mmol) was dissolved in toluene (10.0 mL). Isobutylene/tetrahydrofuran solution (2.40 M, 2.73 mL) and Hoveyda-Grubbs catalyst (411 mg, 656 µmol) were added. The mixture was reacted at 120 °C for 12 hours. The reaction solution was filtered using a pad of Celite, and the filtrate was concentrated under reduced pressure to give a crude product. The crude product was purified by high performance liquid chromatography (column: Phenomenex Luna C18 150*25mm*10µm; mobile phase: [water (0.225 % formic acid)-acetonitrile]; acetonitrile: 25%-55%) to give compound **22-1.** MS m/z = 224.1 [M+1]⁺.

### Step 2: Synthesis of Intermediate 22-2

Compound **22-1** (200 mg) was dissolved in THF (2.00 mL). The mixture was cooled down to 0°C, and red aluminum/toluene solution (1.03 g, 3.58 mmol, 70.0% purity) was slowly added dropwise at 0 °C. After the addition was completed, the reaction solution was reacted at 25°C for 12 hours. Methanol (3.00 ml) was added to the reaction solution, and the mixture was concentrated under reduced pressure to give a crude product. The crude product was purified by column chromatography (dichloromethane:methanol = 10:1) to give compound **22-2.** MS m/z = 182.1 [M+1]⁺.

### Step 3: Synthesis of Intermediate 22-3 formate

Compound **1-12B** (100 mg, 116 µmol) was dissolved in toluene (1.00 mL), and compound **22-2** (105 mg, 581 µmol), 4A molecular sieve (100 mg) and sodium tert-butoxide (33.5 mg, 349 µmol) were added to the solution. The reaction solution was reacted at 110 °C for 12 hours. The reaction solution was cooled down and filtered using a pad of Celite. The filtrate was concentrated under reduced pressure to give a crude product. The crude product was purified by high performance liquid chromatography (column: Phenomenex luna C18 150*25mm* 10µm; mobile phase: [water (0.225 % formic acid)-acetonitrile]; acetonitrile: 50%-80%) to give compound **22-3** formate. MS m/z = 977.3 [M+1]⁺.

### Step 4: Synthesis of Intermediate 22-4

The compound **22-3** formate (45.0 mg, 46.0 µmol) was dissolved in toluene (1.00 mL), and 1-propynyltri-n-butyltin (60.6 mg, 184 µmol) and dichlorobis[di-tert-butyl-(4-dimethylaminophenyl)phosphine]palladium (II) (9.78 mg, 13.8 µmol) were added to the reaction solution. The reaction solution was reacted at 110 °C for 3 hours. The reaction solution was cooled down and filtered with a pad of Celite. The filtrate was concentrated under reduced pressure to give a crude product. The crude product was purified by high performance liquid chromatography (column: Phenomenex Luna C18 150 *25mm*10µm; mobile phase: [water (0.075 % trifluoroacetic acid)-acetonitrile]; acetonitrile: 52%-82%) to give compound **22-4** trifluoroacetate. MS m/z = 981.4 [M+1]⁺.

### Step 5: Synthesis of compound 22 formate

The compound **22-4** trifluoroacetate (35.0 mg, 35.7 µmol) was dissolved in dichloromethane (1.00 mL), and trifluoroacetic acid (0.20 mL) was added to the reaction solution. The reaction solution was reacted at 25 °C for 1 hour, and then directly concentrated under reduced pressure to give a crude product. The crude product was purified sequentially by HPLC (column: Phenomenex luna C18 150*25mm* 10µm; mobile phase: [water (0.075 % trifluoroacetic acid)-acetonitrile]; acetonitrile: 20%-50% over 9 min); (column: Waters Xbridge 150*25mm* 5µm; mobile phase: [water (0.1 % ammonia)-acetonitrile]; acetonitrile: 55 % -85 % over 10 min); (column: Phenomenex luna C18 150*25mm* 10µm; mobile phase: [water (0.225 % formic acid)-acetonitrile]; acetonitrile: 15%-45% over 9 min) to give compound **22** formate. MS m/z = 641.3 [M+1]⁺.

### Example 23

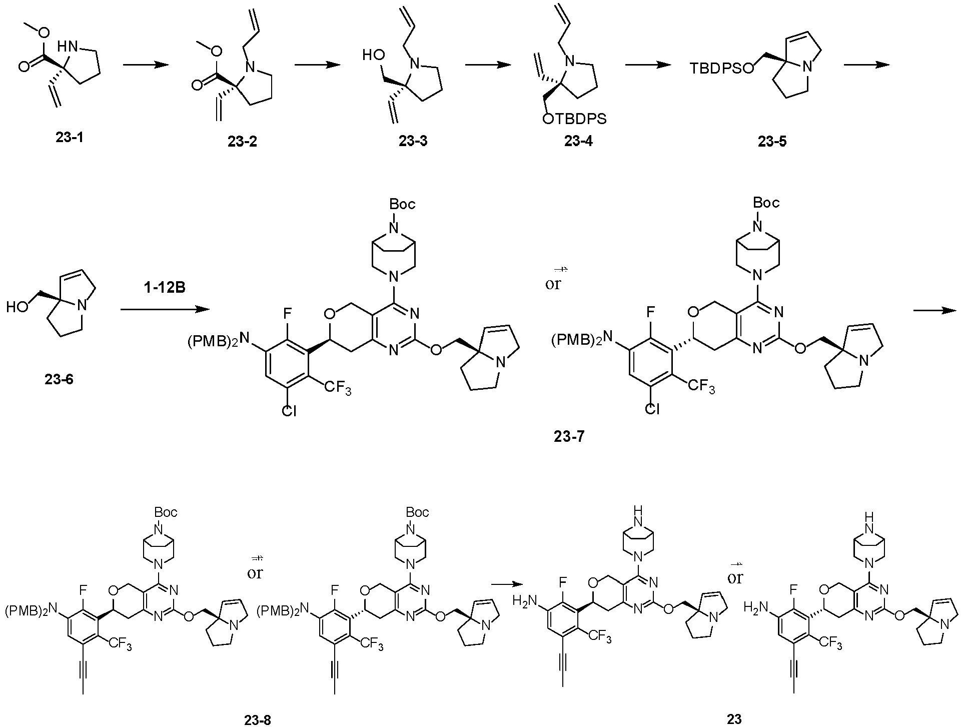

### Step 1: Synthesis of Intermediate 23-2

Compound **23-1** (5.5 g) was dissolved in DMF (50 mL), and potassium carbonate (15.8 g, 114 mmol) and compound allyl bromide (4.17 g, 34.4 mmol) were added. The mixture was reacted at room temperature for 12 hours. 100 mL of water was poured into the reaction solution. The mixture was extracted with ethyl acetate (200 mL) for three times. The organic phase was washed with saturated brine (100 mL), dried over anhydrous sodium sulfate, and filtered. The filtrate was concentrated under reduced pressure to give a crude product. The crude product was purified by column chromatography (petroleum ether:ethyl acetate = 20:1) to give compound **23-2.**

### Step 2: Synthesis of Intermediate 23-3

Compound **23-2** (5.5 g, 28.1 mmol) was dissolved in anhydrous THF (37.0 mL), and lithium aluminum hydride (2.5 M, 13.5 mL) was added at 0 °C. The mixture was reacted at room temperature for 1 hour. To the reaction solution were added 1.2 mL of water, 1.2 mL of 15% NaOH aqueous solution, and finally 3.6 mL of water. The organic phase was dried over anhydrous sodium sulfate, filtered and concentrated under reduced pressure to give a crude product which was directly used in the next step. Compound **23-3** was obtained.

### Step 3: Synthesis of Intermediate 23-4

Compound **23-3** (2.5 g) was dissolved in anhydrous DCM (20.0 mL). Imidazole (4.07 g, 59.7 mmol) and 4-dimethylaminopyridine (182 mg, 1.49 mmol) were added, and then tert-butyldiphenylsilyl chloride (8.22 g, 29.9 mmol) was added at 0 °C. The mixture was reacted at room temperature for 12 hours. The reaction was quenched by adding 100 mL of water to the reaction solution, and the mixture was extracted with DCM (100 mL) for three times. The organic phase was washed with saturated brine (100 mL), dried over anhydrous sodium sulfate, and filtered. The filtrate was concentrated under reduced pressure to give a crude product. The crude product was purified by column chromatography (petroleum ether:ethyl acetate = 10:1) to give compound **23-4.** MS m/z = 406.2 [M+H]⁺.

### Step 4: Synthesis of Intermediate 23-5

Compound **23-4** (1.5 g, 3.70 mmol) was dissolved in anhydrous toluene (30 mL) and Grubbs catalyst (313.93 mg, 369.78 µmol) was added. The mixture was reacted at 120 °C for 12 hours. The reaction solution was concentrated under reduced pressure to give a crude product. The crude product was purified by thin plate chromatography (dichloromethane:methanol = 10:1) to give compound **23-5.** MS m/z = 378.2 [M+H]⁺.

### Step 5: Synthesis of Intermediate 23-6 hydrochloride

Compound **23-5** (200 mg, 529 µmol) was dissolved in anhydrous dioxane (6.0 mL) and hydrochloric acid (12 M, 44.1 µL) was added. The mixture was reacted at 95 °C for 12 hours. To the reaction solution was added 10 ml of water. The mixture was extracted. The aqueous phase was freeze-dried to give a crude product that can be directly used in the next step. Compound 23-6 hydrochloride was obtained.

### Step 6: Synthesis of Intermediate 23-7

The compound **23-6** hydrochloride (207 mg) was dissolved in anhydrous toluene (3 mL), and sodium tert-butoxide (250 mg, 2.60 mmol) and compound **1-12B** (320 mg, 371 µmol) were added. The mixture was reacted at room temperature for 12 hours. 30 mL of saturated aqueous ammonium chloride solution was poured into the reaction solution. The mixture was extracted with ethyl acetate (30 mL). The organic phase was washed with saturated brine (20 mL), dried over anhydrous sodium sulfate, and filtered. The filtrate was concentrated under reduced pressure to give a crude product. The crude product was purified by column chromatography (dichloromethane:methanol = 10:1) to give compound **23-7.** MS m/z = 935.3 [M+H]⁺.

### Step 7: Synthesis of Intermediate 23-8

Compound **23-7** (130 mg, 138 µmol) was dissolved in anhydrous toluene (1 mL), and 1-propynyltri-n-butyltin (182 mg, 555 µmol) and dichlorobis[di-tert-butyl-(4-dimethylaminophenyl)phosphine]palladium(II) (9.84 mg, 13.9 µmol) were added. The atmosphere was replaced three times with nitrogen. The mixture was reacted at 130 °C for 4 hours under nitrogen. The reaction solution was cooled down and concentrated under reduced pressure to give a crude product. The crude product was purified by thin plate chromatography (dichloromethane:methanol = 10:1) to give compound **23-8.** MS m/z = 939.4 [M+H]⁺.

### Step 8: Synthesis of compound 23 trifluoroacetate

Compound **23-8** (100 mg, 106 µmol) was dissolved in dichloromethane (1.0 mL), and trifluoroacetic acid (182 mg, 1.60 mmol) was added. The mixture was reacted at 25°C for 0.5 hours. The reaction solution was concentrated under reduced pressure to give a crude product. The crude product was purified by high performance liquid chromatography (column: Phenomenex luna C18 150*25mm*10µm; mobile phase: [water (0.075 % trifluoroacetic acid)-acetonitrile]; acetonitrile: 10%-40% over 9 min) and freeze-dried to give compound **23** trifluoroacetate. MS m/z = 599.3 [M+H]⁺. ¹H NMR (400 MHz, CD₃OD) 6.92 (d, *J=* 8.4 Hz, 1H), 6.00 (d, J = 6.4 Hz, 1H), 5.87 (td, *J=* 2.4, 6.4 Hz, 1H), 5.18 (dd, *J* = 4.4, 11.2 Hz, 1H), 4.88 - 4.85 (m, 1H), 4.76 - 4.70 (m, 2H), 4.61 - 4.52 (m, 2H), 4.36 - 4.28 (m, 1H), 4.20 - 4.11 (m, 2H), 4.04 (br d, *J =* 16.0 Hz, 1H), 3.85 - 3.74 (m, 2H), 3.66 (br d, *J =* 13.6 Hz, 1H), 3.35 (br d, *J =* 6.4 Hz, 2H), 3.29 - 3.24 (m, 1H), 2.92 (br dd, *J =* 3.6, 18.0 Hz, 1H), 2.31 - 2.09 (m, 7H), 2.04 - 1.95 (m, 4H).

### Example 24

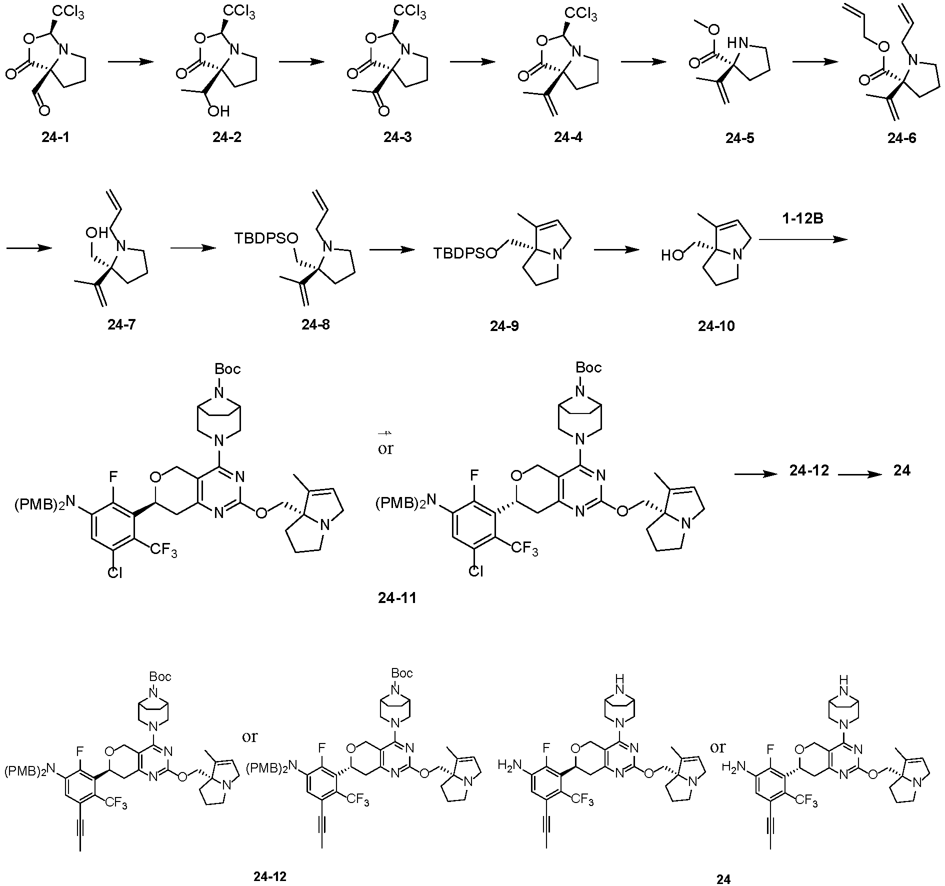

### Step 1: Synthesis of Intermediate 24-2

Compound **24-1** (54 g, 198 mmol) was dissolved in anhydrous THF (450 mL). The mixture was cooled down to -78 °C, and methylmagnesium bromide (3 M, 79.2 mL) was added dropwise. The mixture was reacted at -78 °C for 0.5 hours, and then reacted at room temperature for 12 hours. The reaction solution was quenched by adding 200 mL of hydrochloric acid (1M). The mixture was extracted with ethyl acetate (200 mL) for three times. The organic phase was washed with saturated brine (100 mL), dried over anhydrous sodium sulfate, and filtered. The filtrate was concentrated under reduced pressure to give a crude product. The crude product was purified by column chromatography (petroleum ether:ethyl acetate = 5:1) to give compound **24-2.** MS m/z = 288.0 [M+1]⁺.

### Step 2: Synthesis of Intermediate 24-3

Compound **24-2** (38 g, 131 mmol) was dissolved in anhydrous DCM (380 mL). Dess-Martin oxidant (111 g, 263 mmol, 81.6 mL) was added. The mixture was reacted at room temperature for 4 hours. 200 mL of water was poured into the reaction solution. The mixture was extracted with ethyl acetate (200 mL) for 3 times. The organic phase was washed with saturated brine (100 mL), dried over anhydrous sodium sulfate, and filtered. The filtrate was concentrated under reduced pressure to give a crude product. The crude product was purified by column chromatography (petroleum ether:ethyl acetate = 5:1) to give compound **24-3.** MS m/z = 285.9 [M+1]⁺.

### Step 3: Synthesis of Intermediate 24-4

Methyltriphenylphosphine bromide (8.42 g, 23.5 mmol) was dissolved in anhydrous tetrahydrofuran (45 mL). The mixture was cooled down to -78 °C. n-Butyllithium (2.5 M, 9.42 mL) was added dropwise. The mixture was reacted at 0 °C for 2 hours. Then the mixture was cooled down to -78 °C. **24-3** (4.5 g, 15.7 mmol) dissolved in THF (20 mL) was added dropwise. The mixture was reacted at room temperature for 10 hours. 100 mL of HCl (1M) aqueous solution was poured into the reaction solution and the mixture was extracted with EA (100 mL) for three times. The organic phase was washed with saturated brine (100 mL), dried over anhydrous sodium sulfate, and filtered. The filtrate was concentrated under reduced pressure to give a crude product. The crude product was purified by column chromatography (petroleum ether:ethyl acetate = 10:1) to give compound **24-4.**

### Step 4: Synthesis of Intermediate 24-5 hydrochloride

Compound **24-4** (5.5 g, 19.3 mmol) was dissolved in a methanol solution of hydrochloric acid (4 M, 61.1 mL). The mixture was reacted at room temperature for 12 hours. The reaction solution was concentrated under reduced pressure to give compound **24-5** hydrochloride. MS m/z = 170.1 [M+1]⁺.

### Step 5: Synthesis of Intermediate 24-6

The compound **24-5** hydrochloride (4.5 g) was dissolved in DMF (40 mL), and potassium carbonate (14.7 g, 106 mmol) and allyl bromide (3.86 g, 31.9 mmol) were added. The mixture was reacted at room temperature for 1.5 hours. 100 mL of water was poured into the reaction solution and the mixture was extracted with ethyl acetate (200 mL) for three times. The organic phase was washed with saturated brine (100 mL), dried over anhydrous sodium sulfate, and filtered. The filtrate was concentrated under reduced pressure to give a crude product. The crude product was purified by column chromatography (petroleum ether:ethyl acetate = 10:1) to give compound **24-6.**

### Step 6: Synthesis of Intermediate 24-7

Compound **24-6** (2.5 g, 11.9 mmol) was dissolved in anhydrous tetrahydrofuran (20.0 mL), and lithium aluminum hydride (2.5 M, 6.32 mL) was added at 0°C. The mixture was reacted at room temperature for 1 hour. To the reaction solution were added 0.6 mL of water, 0.6 mL of 15% sodium hydroxide aqueous solution, and finally 1.8 mL of water. The mixture was dried over anhydrous sodium sulfate, filtered and concentrated under reduced pressure to give a crude compound **24-7.** MS m/z = 182.1 [M+H]⁺.

### Step 7: Synthesis of Intermediate 24-8

Compound **24-7** (2.3 g) was dissolved in anhydrous dichloromethane (23.0 mL). Imidazole (3.46 g, 50.7 mmol), 4-dimethylaminopyridine (155 mg, 1.27 mmol), and then tert-butyldiphenylsilyl chloride (6.97 g, 25.3 mmol) were added at 0 °C. The mixture was reacted at room temperature for 12 hours. 50 mL of water was poured into the reaction solution. The mixture was extracted with ethyl acetate (100 mL) for three times. The organic phase was washed with saturated brine (10 mL), dried over anhydrous sodium sulfate, and filtered. The filtrate was concentrated under reduced pressure to give a crude product. The crude product was purified by column chromatography (petroleum ether:ethyl acetate = 10:1) to give compound **24-8.** MS m/z = 420.3 [M+H]⁺.

### Step 8: Synthesis of Intermediate 24-9

Compound **24-8** (2.5 g, 5.96 mmol) was dissolved in anhydrous toluene (50 mL) and Grubbs catalyst (1.01 g, 1.19 mmol) was added. The mixture was reacted at 120 °C for 12 hours. The reaction solution was concentrated under reduced pressure to give a crude product. The crude product was purified by column chromatography (dichloromethane:methanol = 20:1) to give compound **24-9.** MS m/z = 392.2 [M+H]⁺.

### Step 9: Synthesis of Intermediate 24-10

Compound **24-9** (1.00 g, 2.55 mmol) was dissolved in anhydrous dioxane (6.0 mL) and hydrochloric acid (12 M, 212 µL) was added. The mixture was reacted at 95 °C for 12 h. 10 mL of water was added to the reaction solution. The mixture was extracted with ethyl acetate (20 mL * 3). The aqueous phase was freeze-dried to give a crude compound **24-10.**

### Step 10: Synthesis of Intermediate 24-11

Compound **24-10** (92.6 mg) was dissolved in anhydrous toluene (3 mL). Sodium tert-butoxide (101 mg, 1.06 mmol) and compound **1-12B** (130 mg, 151 µmol) were added. The mixture was reacted at 95°C for 12 hours. The reaction solution was concentrated under reduced pressure to give a crude product. The crude product was purified by column chromatography (dichloromethane:methanol = 10:1) to give compound **24-11.**

### Step 11: Synthesis of Intermediate 24-12

Compound **24-11** (89 mg, 93.7 µmol) was dissolved in anhydrous toluene (1 mL), and 1-propynyltri-n-butyltin (123 mg, 374 µmol) and dichlorobis[di-tert-butyl-(4-dimethylaminophenyl)phosphine]palladium (II) (6.64 mg, 9.37 µmol) were added. The atmosphere was replaced three times with nitrogen. The mixture was reacted at 130 °C under nitrogen for 0.5 hours. The reaction solution was cooled down and concentrated under reduced pressure to give a crude product. The crude product was purified by thin plate chromatography (dichloromethane:methanol = 10:1) to give compound **24-12.**

### Step 12: Synthesis of compound 24 trifluoroacetate

Compound **24-12** (90 mg, 94.4 µmol) was dissolved in dichloromethane (1.0 mL), and trifluoroacetic acid (161 mg, 1.42 mmol) was added. The mixture was reacted at 25°C for 1.5 hours. The reaction solution was concentrated under reduced pressure to give a crude product. The crude product was purified by high performance liquid chromatography (column: Phenomenex luna C18 150*25mm*10µm; mobile phase: [water (0.075 % trifluoroacetic acid)-acetonitrile]; acetonitrile: 20%-50% over 7 min) and freeze-dried to give compound **24** trifluoroacetate. MS m/z = 613.2 [M+H]⁺. ¹H NMR (400 MHz, METHANOL-d4) 6.92 (d, *J=* 8.2 Hz, 1H), 5.61 (d, *J=* 1.4 Hz, 1H), 5.18 (br dd, *J=* 4.4, 11.6 Hz, 1H), 4.91 - 4.89 (m, 2H), 4.86 - 4.82 (m, 1H), 4.80 - 4.70 (m, 2H), 4.51 - 4.41 (m, 2H), 4.29 (br d, *J* = 14.0 Hz, 1H), 4.19 - 4.10 (m, 2H), 3.91 (br d, *J =* 15.2 Hz, 1H), 3.85 - 3.74 (m, 2H), 3.63 (br d, *J* = 14.4 Hz, 1H), 3.27 (br d, *J* = 5.2 Hz, 2H), 2.95 (br d, *J* = 1.2 Hz, 1H), 2.28 - 2.08 (m, 7H), 2.02 (s, 3H), 1.83 (d, *J*=1.6 Hz, 3H).

### Example 25

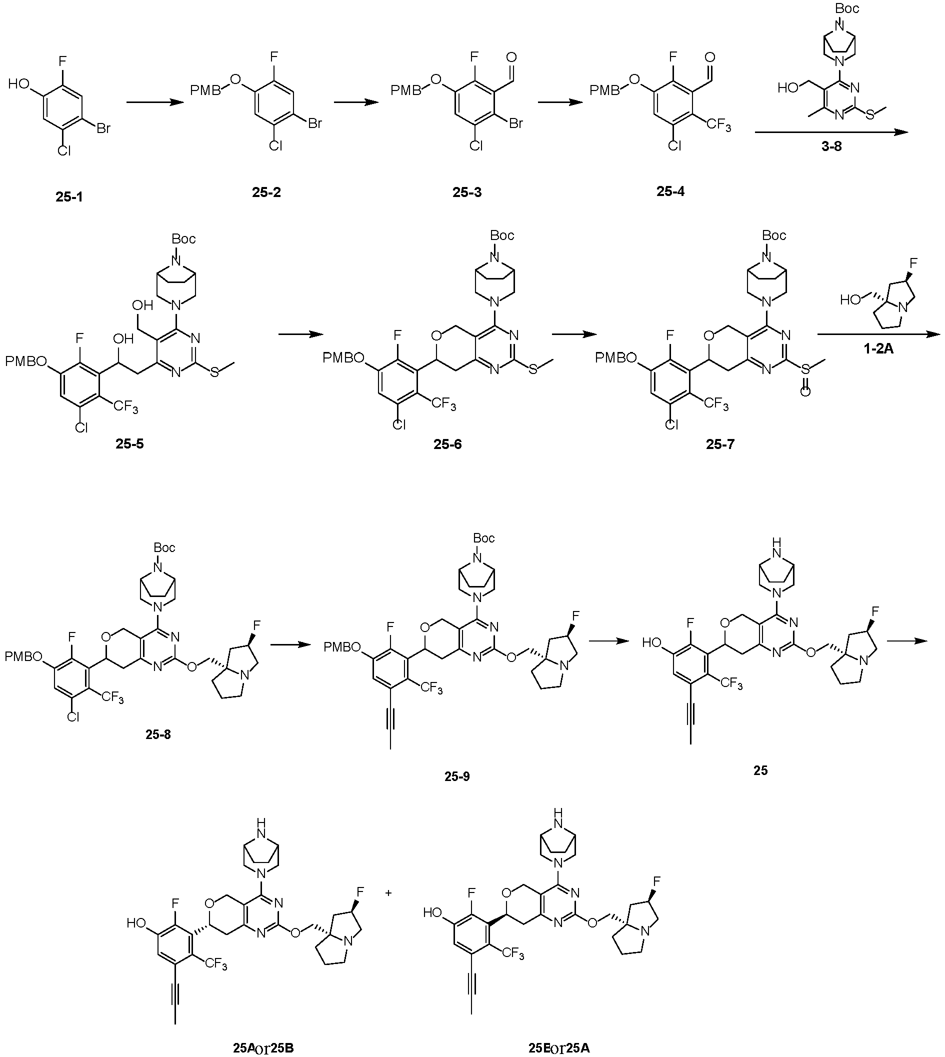

### Step 1: Synthesis of Intermediate 25-2

Compound **25-1** (9.80 g, 43.5 mmol) was dissolved in anhydrous DMF (98 mL) and then p-methoxybenzyl chloride (8.17 g, 52.2 mmol), potassium iodide (7.22 g, 43.5 mmol) and potassium carbonate (15.0 g, 109 mmol) were added. The mixture was reacted at 50 °C for 12 hours. 50.0 mL of water was poured into the reaction solution and the mixture was extracted with ethyl acetate (150 mL). The organic phase was washed with saturated brine (100 mL), dried over anhydrous sodium sulfate, and filtered. The filtrate was concentrated under reduced pressure to give a crude product. The crude product was purified by column chromatography (petroleum ether:ethyl acetate = 10:1) to give compound **25-2.** MS m/z = 345.6 [M+H]⁺.

### Step 2: Synthesis of Intermediate 25-3

2,2,6,6-tetramethylpiperidine (21.3 g, 150 mmol) was dissolved in tetrahydrofuran (130 mL) and the atmosphere was replaced three times with nitrogen. The mixutre was cooled down to -5 °C, and n-butyl lithium (2.50 M, 60.2 mL) was added dropwise and stirred for 0.5 hours. Then the mixutre was cooled down to -60 °C, and compound **25-2** (13.0 g, 37.6 mmol) was added. The mixutre was stirred for 0.5 hours. Then DMF (55.0 g, 752 mmol) was added at -60 °C and the mixture was stirred for 0.5 hours. The reaction was quenched by adding 200 mL of saturated ammonium chloride solution at 0 °C. The mixture was extracted with ethyl acetate (300 mL). The organic phase was washed with saturated brine (300 mL), dried over anhydrous sodium sulfate, and filtered. The filtrate was concentrated under reduced pressure to give a crude product. The crude product was purified by column chromatography (petroleum ether:ethyl acetate = 50:1) to give compound **25-3.** MS m/z = 373.28 [M+H]⁺.

### Step 3: Synthesis of Intermediate 25-4

Compound **25-3** (4.9 g, 13.12 mmol), cuprous iodide (5.00 g, 26.23 mmol), and methyl 2,2-difluoro-2-fluorosulfonylacetate (9.57 g, 49.84 mmol) were dissolved in DMF (50 mL). The atmosphere was replaced 3 times with nitrogen. The mixture was reacted at 110 °C for 2.5 hours. The reaction solution was cooled down, and filtered through a pad of Celite. Water (100 mL) was added to the organic phase. The mixture was extracted once with ethyl acetate (100 mL). The organic phase was washed with saturated brine (100 mL) for 6 times, dried over anhydrous sodium sulfate, and filtered. The filtrate was concentrated under reduced pressure to give a crude product. The crude product was purified by column chromatography (petroleum ether:ethyl acetate = 20:1) to give compound **25-4.** ¹H NMR (CDCl₃, 400 MHz) 10.29 (q, *J* = 4.25 Hz, 1 H), 7.35 (d, *J* = 8.63 Hz, 2 H), 7.21 (d, *J* = 7.25 Hz, 1 H), 7.00 - 6.90 (m, 2 H), 5.14 (s, 2 H), 3.84 (s, 3 H).

### Step 4: Synthesis of Intermediate 25-5

2,2,6,6-tetramethylpiperidine (3.68 g, 26.02 mmol, 4.42 mL) was dissolved in THF (30 mL). The atmosphere was replaced 3 times with nitrogen. The mixture was cooled down to -40 °C, and n-butyl lithium (2.5 M, 10.06 mL) was slowly added dropwise. The mixture was reacted at -40 °C for 0.5 hours. The reaction system was cooled down to -60 °C. To the above reaction solution was slowly added dropwise a solution of **3-8** (3.3 g, 8.67 mmol) in THF (10 mL). The mixture was warmed to -40 °C and reacted for 15 minutes. Then to the above reaction solution was added compound **25-4** (3.77 g, 10.41 mmol) in batches, and the mixture was reacted at room temperature for 2 hours. The reaction solution was quenched by adding saturated aqueous ammonium chloride solution (50 mL) and the mixture was extracted twice with ethyl acetate (50 mL). The organic phase was washed twice with saturated brine (100 mL), dried over anhydrous sodium sulfate, and filtered. The filtrate was concentrated under reduced pressure to give a crude product. A mixed solution of petroleum ether and ethyl acetate (20 mL) (petroleum ether:ethyl acetate = 5:1) was added to the crude product and the mixture was stirred for 12 hours. The solid was collected by filtration to give compound **25-5.**

### Step 5: Synthesis of Intermediate 25-6

Compound **25-5** (2.7 g, 3.63 mmol) and tributyl cyanomethylene phosphate (1.75 g, 7.27 mmol) were dissolved in toluene (27 mL). The atmosphere was replaced with nitrogen. The mixture was reacted at 110 °C for 12 hours. 50 mL of aqueous ammonium chloride solution was poured into the reaction solution, and the mixture was extracted twice with ethyl acetate (50 mL). The organic phase was washed twice with saturated brine (100 mL), dried over anhydrous sodium sulfate, and filtered. The filtrate was concentrated under reduced pressure to give a crude product. The crude product was purified by column chromatography (petroleum ether:ethyl acetate = 10:1) to give compound **25-6.** MS m/z = 725.3 [M+H]⁺.

### Step 6: Synthesis of Intermediate 25-7

Compound **25-6** (1.5 g, 2.07 mmol) was dissolved in anhydrous DCM (15.0 mL), and m-chloroperbenzoic acid (461.92 mg, 2.28 mmol, 85% purity) was added in batches at 0 °C. The mixture was reacted at room temperature overnight. The reaction solution was directly blended and purified by column chromatography (petroleum ether: ethyl acetate = 1: 1) to give compound **25-7.** MS m/z = 741.3 [M+H]⁺.

### Step 7: Synthesis of Intermediate 25-8

Compound **25-7** (1.9 g, 2.56 mmol) was dissolved in anhydrous toluene (19 mL), and compound **1-2A** (816.20 mg, 5.13 mmol), sodium tert-butoxide (985.42 mg, 10.25 mmol) and 4A molecular sieve (1 g) were added. The atmosphere was replaced with nitrogen. The mixture was reacted at 100 °C for 12 hours. The reaction solution was concentrated under reduced pressure to give a crude product. The crude product was purified by column chromatography (petroleum ether:ethyl acetate = 1:1) to give compound **25-8.** MS m/z = 836.5 [M+H]⁺.

### Step 8: Synthesis of Intermediate 25-9

Compound **25-8** (1.50 g, 1.79 mmol) was dissolved in anhydrous toluene (15 mL), and 1-propynyltri-n-butyltin (2.36 g, 7.17 mmol) and dichlorobis[di-tert-butyl-(4 -dimethylaminophenyl)phosphine]palladium (II) (381.01 mg, 538.09 µmol) were added. The atmosphere was replaced three times with nitrogen. The mixture was reacted at 110 °C under nitrogen for 12 hours. The reaction solution was cooled down and concentrated under reduced pressure to give a crude product. The crude product was purified by column chromatography (petroleum ether:ethyl acetate = 1:1) to give compound **25-9.** MS m/z = 840.3 [M+H]⁺.

### Step 9: Synthesis of compound 25

Compound **25-9** (1.27 g, 1.51 mmol) was dissolved in dichloromethane (13 mL), and trifluoroacetic acid (3.99 g, 35.00 mmol) was added. The mixture was reacted at 20°C for 3 hours. The reaction solution was adjusted to pH of 7 with saturated sodium bicarbonate aqueous solution. The mixture was concentrated under reduced pressure to give a crude product. The crude product was purified by high performance liquid chromatography (column: Kromasil Eternity XT 250*80mm*10µm; mobile phase: [water (0.1% ammonia water)-acetonitrile]; acetonitrile: 10%-40% over 20 min) and freeze-dried to give compound **25.** MS m/z = 620.4 [M+H]⁺.

### Step 10: Synthesis of compounds 25A and 25B

Compound **25** was separated by SFC (column: DAICEL CHIRALPAK IC (250 mm*30 mm, 10 µ m); mobile phase: [supercritical CO₂-ethanol (0.1 % ammonia)]; Ethanol (0.1% ammonia water) %: 50% -50%) to give compound **25A** and compound **25B.**

Compound **25A,** chiral SFC analysis (chromatographic column: Chiral Pak IC-3 (100mm*4.6mm,3µm); mobile phase: [supercritical CO₂-ethanol (0.05% diethylamine)]; (ethanol (0.05%diethylamine))%: 40%- 40%), Rt = 0.695 min, ee value 99%, MS m/z = 620.2 [M+H]⁺; ¹H NMR (CDCl₃, 400 MHz) δ 7.07 (d, *J=* 8.13 Hz, 1 H), 5.30 - 5.04 (m, 2 H), 4.78 - 4.59 (m, 2 H), 4.13 - 3.89 (m, 3 H), 3.56 (br s, 2 H), 3.45 - 2.77 (m, 12 H), 2.29 - 2.05 (m, 3 H), 2.01 - 1.57 (m, 8 H).

Compound **25B,** chiral SFC analysis (chromatographic column: Chiral Pak IC-3 (100mm*4.6mm, 3µm); mobile phase: [supercritical CO₂-ethanol (0.05% diethylamine)]; (ethanol (0.05% diethylamine))%: 40%- 40%), Rt = 1.229 minutes, ee value 99%, MS m/z = 620.2 [M+H]⁺. ¹H NMR (DMSO-d6, 400 MHz) 9.44 - 8.91 (m, 2 H), 7.12 (br d, *J=* 8.25 Hz, 1 H), 5.68 - 5.45 (m, 1 H), 5.12 (br dd, *J=* 10.88, 3.75 Hz, 1 H), 4.95 - 4.63 (m, 2 H), 4.45 (s, 2 H), 4.31 - 4.01 (m, 3 H), 3.95 - 3.51 (m, 6 H), 3.39 - 3.09 (m, 3 H), 2.99 - 2.81 (m, 1 H), 2.54 (br d, *J =* 4.38 Hz, 1 H), 2.44 (br s, 1 H), 2.35 - 2.24 (m, 1 H), 2.23 - 2.06 (m, 4 H), 1.99 - 1.87 (m, 2 H), 1.87 - 1.72 (m, 1 H).

### Example 26

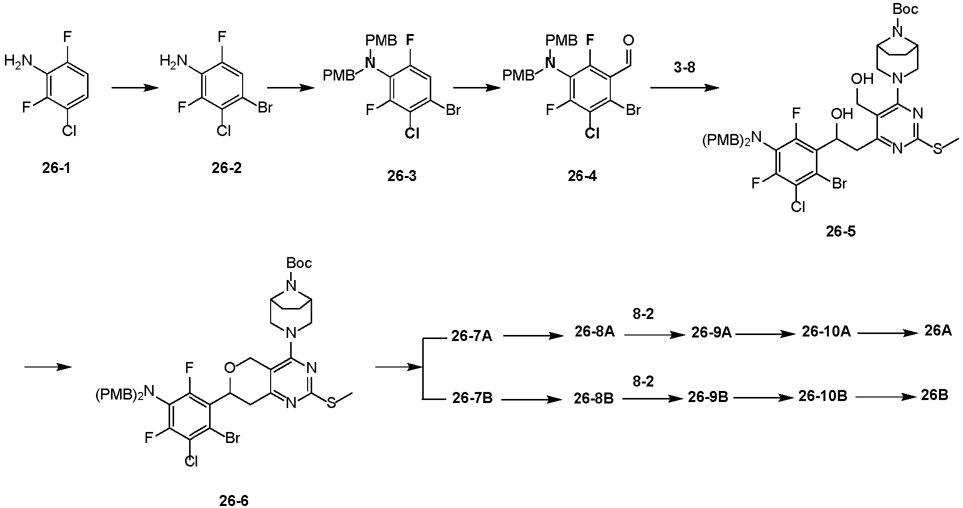

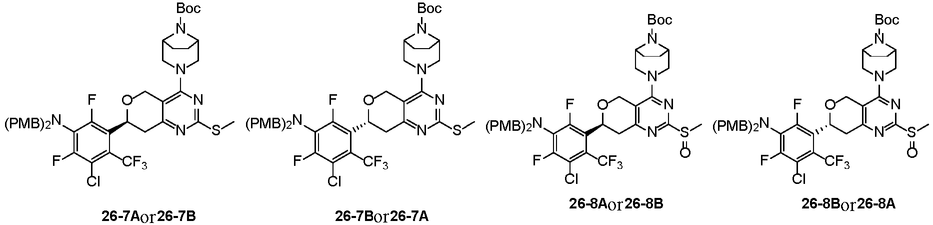

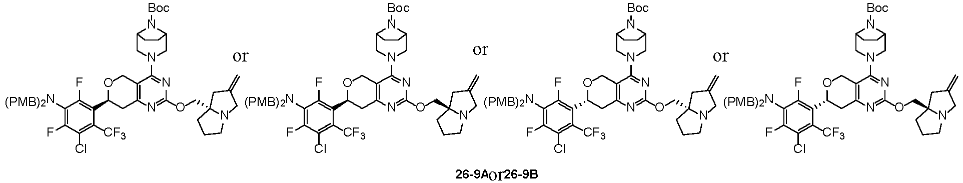

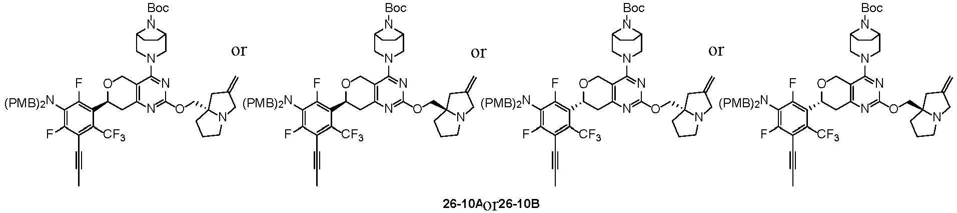

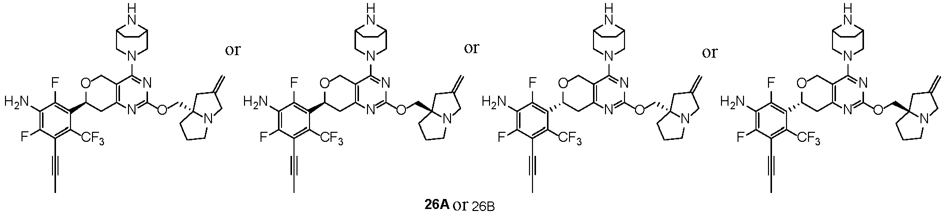

### Step 1: Synthesis of Intermediate 26-2

Compound **26-1** (5.00 g, 30.6 mmol) was dissolved in anhydrous dichloromethane (50.0 mL) and NBS (6.53 g, 36.7 mmol) was added. The mixture was reacted at room temperature for 1 hour. 50.0 mL of water was added to the reaction solution. The mixture was extracted with dichloromethane (150 mL). The organic phase was washed with saturated brine (150 mL), dried over anhydrous sodium sulfate, and filtered. The filtrate was concentrated under reduced pressure to give a crude product. The crude product was purified by column chromatography (petroleum ether:ethyl acetate = 20:1) to give compound **26-2.** ¹H NMR (400 MHz, CDCl₃) δ ppm 7.14 (dd, *J =* 9.88, 2.25 Hz, 1 H) 3.60 - 4.02 (m, 2 H).

### Step 2: Synthesis of Intermediate 26-3

Compound **26-2** (5.60 g, 23.1 mmol) and p-methoxybenzyl chloride (8.68 g, 55.4 mmol) were dissolved in anhydrous DMF (56.0 mL), and potassium iodide (3.83 g, 23.1 mmol) and potassium carbonate (7.98 g, 57.7 mmol) were added. The mixture was reacted at 80 °C for 2 hours. 100 mL of water was poured into the reaction solution and extracted with ethyl acetate (150 mL). The organic phase was washed with saturated brine (100 mL), dried over anhydrous sodium sulfate, and filtered. The filtrate was concentrated under reduced pressure to give a crude product. The crude product was purified by column chromatography (petroleum ether:ethyl acetate = 20:1) to give compound **26-3.** ¹H NMR (400 MHz, CDCl₃) δ ppm 7.20 (d, *J =* 8.50 Hz, 4 H) 7.09 (dd, *J* =10.38, 2.13 Hz, 1 H) 6.83 (d, *J =* 8.63 Hz, 4 H) 4.16 (s, 4 H) 3.80 (s, 6 H).

### Step 3: Synthesis of Intermediate 26-4

2,2,6,6-tetramethylpiperidine (10.3 g, 73.2 mmol, 12.4 mL) was dissolved in tetrahydrofuran (90.0 mL). The atmosphere was replaced three times with nitrogen. The mixture was cooled down to -5 °C and n-butyl lithium (2.50 M, 29.3 mL) was added dropwise. The mixture was stirred for 0.5 hours. The mixture was cooled down to -60 °C and compound **26-4** (9.20 g, 18.3 mmol) was added. The mixture was stirred for 1 hour. Then DMF (26.8 g, 366 mmol) was added at -60 °C. The mixture was stirred for another 0.5 hours. 100 mL of saturated ammonium chloride solution was added at 0 °C. The mixture was extracted with ethyl acetate (300 mL). The organic phase was washed with saturated brine (300 mL), dried over anhydrous sodium sulfate, and filtered. The filtrate was concentrated under reduced pressure to give a crude product. The crude product was purified by column chromatography (petroleum ether:ethyl acetate = 30:1) to give compound **26-4.** MS m/z = 510.8 [M+H]⁺.

### Step 4: Synthesis of Intermediate 26-5

2,2,6,6-tetramethylpiperidine (3.56 g, 25.2 mmol, 4.28 mL) was dissolved in tetrahydrofuran (50.0 mL). The atmosphere was replaced three times with nitrogen. The mixture was then cooled down to -40 °C and n-butyl lithium (2.5 M, 9.76 mL) was added dropwise. The mixture was stirred for 0.5 hours. The mixture was then cooled down to -60 °C and compound **3-8** (3.20 g, 8.41 mmol) was added. The mixture was stirred for 15 minutes. Then compound **26-4** (5.37 g, 10.1 mmol) was added at -60 °C. The mixture was stirred at room temperature for another 0.5 hours. 100 mL of saturated ammonium chloride solution was added at 25 °C. The mixture was extracted with ethyl acetate (300 mL). The organic phase was washed with saturated brine (300 mL), dried over anhydrous sodium sulfate, and filtered. The filtrate was concentrated under reduced pressure to give a crude product. The crude product was purified by high performance liquid chromatography (column: Phenomenex luna C18 (250*70mm, 10 µm); mobile phase: [water (0.075 % trifluoroacetic acid)-acetonitrile]; acetonitrile: 80%-100% over 20 min) and freeze-dried to give compound **26-5.**

### Step 5: Synthesis of Intermediate 26-6

Compound **26-5** (2.20 g, 2.47 mmol) and tributyl cyanomethylene phosphate (1.19 g, 4.94 mmol) were dissolved in anhydrous toluene (20.0 mL). The mixture was reacted at 110 °C for 12 hours under nitrogen. The reaction solution was concentrated under reduced pressure to give a crude product. The crude product was purified by column chromatography (petroleum ether:ethyl acetate = 5:1) to give compound **26-6.**

### Step 6: Synthesis of Intermediates 26-7A and 26-7B

Compound **26-6** (1.10 g, 1.26 mmol) and methyl fluorosulfonyl difluoroacetate (919.60 mg, 4.79 mmol) were dissolved in anhydrous DMF (10.0 mL), and then cuprous iodide (480 mg, 2.52 mmol) was added. The mixture was reacted at 110 °C for 2.5 hours. 50.0 mL of water was added at 25 °C, and the mixture was extracted with ethyl acetate (150 mL). The organic phase was washed with saturated brine (150 mL), dried over anhydrous sodium sulfate, and filtered. The filtrate was concentrated under reduced pressure to give a crude product. The crude product was purified by column chromatography (petroleum ether:ethyl acetate = 5:1) to give compound **26-7,** which was then separated by preparative SFC (Chiral column: DAICEL CHIRALCEL OD (250mm*30mm, 10µm); mobile phase: supercritical CO₂-ethanol (0.1% ammonia water); ethanol %: 40% - 40%) to give compound **26-7A** and compound **26-7B.** Chiral SFC analysis (chromatographic column: (S,S)Whelk-O1 50*4.6mm ID, 3.5µm; mobile phase: [supercritical CO₂-ethanol (0.05% diethylamine)]; (ethanol (0.05% diethylamine))%: 50%-50%), compound **26-7A,** Rt = 1.791 minutes, ee value 99%, MS m/z = 862.3 [M+H]⁺. ¹H NMR (400 MHz, CD₃OD) δ ppm 7.15 (d, *J =* 8.50 Hz, 4 H), 6.85 (d, *J =* 8.63 Hz, 4 H), 5.21 - 5.09 (m, 1 H), 4.70 (s, 2 H), 4.21 (s, 6 H), 3.81 (s, 6 H), 3.57 - 2.97 (m, 5 H), 2.54 (s, 3 H), 1.96 (br s, 3 H), 1.66 (br d, *J =* 5.88 Hz, 1 H), 1.50 (s, 10 H). Compound **26-7B,** Rt = 2.166 min, ee value 99%, MS m/z = 862.3 [M+H]⁺.

### Step 7: Synthesis of Intermediates 26-8A and 26-8B

Compound **26-7A** (170 mg, 197 µmol) was dissolved in anhydrous dichloromethane (2.00 mL), and m-chloroperbenzoic acid (44.0 mg, 217 µmol, 85% purity) was added. The mixture was reacted at room temperature for 2 hours. The mixture was extracted with dichloromethane (60.0 mL). The organic phase was washed with saturated brine (60.0 mL), dried over anhydrous sodium sulfate, and filtered. The filtrate was concentrated under reduced pressure to give a crude product. The crude product was purified by column chromatography (petroleum ether:ethyl acetate = 2:1) to give compound **26-8A.** MS m/z = 878.2 [M+H]⁺.

Referring to step 7, compound **26-8B** was obtained using **26-7B** as the starting material. MS m/z = 878.2 [M+H]⁺

### Step 8: Synthesis of Intermediates 26-9A and 26-9B formate

Compound **26-8A** (150 mg, 171 µmol) was dissolved in anhydrous toluene (2.00 mL). Sodium tert-butoxide (131 mg, 1.37 mmol), compound **8-2** (52.3 mg, 342 µmol) and molecular sieves (70.0 mg) were added. The mixture was reacted at 100 °C for 12 hours. 60.0 mL of saturated aqueous ammonium chloride solution was poured into the reaction solution. The mixture was extracted with ethyl acetate (50.0 mL). The organic phase was washed with saturated brine (60.0 mL), dried over anhydrous sodium sulfate, and filtered. The filtrate was concentrated under reduced pressure to give a crude product. The crude product was purified by high performance liquid chromatography (column: Phenomenex luna C18 150*25mm*10µm; mobile phase: [water (0.225 % formic acid)-acetonitrile]; acetonitrile: 40%-70%) and freeze-dried to give compound **26-9A** formate. MS m/z = 967.3 [M+H]⁺.

Referring to step 8, compound **26-9B** formate was obtained using **26-8B** as the starting material. MS m/z = 967.3 [M+H]⁺.

### Step 9: Synthesis of Intermediates 26-10A and 26-10B

The compound **26-9A** formate (20.0 mg) was dissolved in anhydrous toluene (1.00 mL), and 1-propynyltri-n-butyltin (27.2 mg, 82.7 µmol) and dichlorobis[di-tert-butyl-(4-dimethylaminophenyl)phosphine]palladium (II) (4.39 mg, 6.20 µmol) were added. The atmosphere was replaced three times with nitrogen. The mixture was reacted at 110 °C for 12 hours under nitrogen. The mixture was concentrated under reduced pressure to remove the tolune. 2.00 mL of ethyl acetate was added. The residue was purified by preparative thin layer chromatography to give compound **26-10A.** MS m/z = 971.3 [M+H]⁺.

Referring to step 9, compound **26-10B** was obtained using the **26-9B** formate as the starting material. MS m/z = 971.3 [M+H]⁺.

### Step 10: Synthesis of compounds 26A and 26B trifluoroacetate

Compound **26-10A** (40.0 mg, 41.2 µmol) was dissolved in dichloromethane (1.00 mL), and trifluoroacetic acid (307 mg, 2.69 mmol) was added. The mixture was reacted at 25°C for 12 hours. 3.00 mL of saturated sodium carbonate solution was added to the reaction solution. The mixture was concentrated under reduced pressure to give a crude product. The crude product was purified by high performance liquid chromatography (column: Phenomenex Luna C18 150*25mm*10µm; mobile phase: [water (0.075 % trifluoroacetic acid)-acetonitrile]; acetonitrile: 15%-45%) and freeze-dried to give **26A** trifluoroacetate. MS m/z = 631.3 [M+H]⁺. ¹H NMR (400 MHz, CD₃OD) δ ppm 5.31 (br d, *J =* 7.88 Hz, 3 H), 4.84 - 4.83 (m, 2 H), 4.77 - 4.71 (m, 1 H), 4.58 (br s, 2 H), 4.43 - 4.31 (m, 2 H), 4.23 - 4.13 (m, 2 H), 3.98 - 3.64 (m, 4 H), 3.36 (br s, 1 H), 3.25 (br d, *J* = 2.88 Hz, 1 H), 3.09 - 2.91 (m, 2 H), 2.79 (br d, *J =* 16.01 Hz, 1 H), 2.44 - 2.34 (m, 1 H), 2.32 - 2.20 (m, 4 H), 2.18 - 2.05 (m, 5 H), 2.02 - 1.90 (m, 1 H).

Compound **26-10B** (46.0 mg, 47.4 µmol) was dissolved in dichloromethane (1.00 mL), and trifluoroacetic acid (307 mg, 2.69 mmol) was added. The mixture was reacted at 25°C for 12 hours. The reaction solution was concentrated under reduced pressure to give a crude product. The crude product was purified by high performance liquid chromatography (column: Phenomenex Luna C18 150*25mm*10µm; mobile phase: [water (0.075 % trifluoroacetic acid)-acetonitrile]; acetonitrile: 15%-45%) and freeze-dried to give **26B** trifluoroacetate. MS m/z = 631.3 [M+H]⁺. ¹H NMR (400 MHz, CD₃OD) δ ppm 5.34 - 5.16 (m, 3 H), 4.88 (br s, 2 H), 4.77 - 4.71 (m, 1 H), 4.56 (s, 2 H), 4.34 (br d, *J =* 14.26 Hz, 2 H), 4.20 - 4.10 (m, 2 H), 3.92 (br d, *J =* 14.63 Hz, 1 H), 3.86 - 3.63 (m, 3 H), 3.27 - 3.21 (m, 1 H), 3.08 - 2.89 (m, 3 H), 2.79 (br d, *J =* 15.76 Hz, 1 H), 2.36 (br dd, *J =* 11.88, 6.38 Hz, 1 H), 2.30 - 2.10 (m, 7 H), 2.09 (s, 3 H).

### Biological test data

### Assay example 1. Assay of p-ERK inhibition in GP2D cells

### 1. Purpose

Compound that can effectively inhibit p-ERK in KRAS^{G12D} mutant GP2D cells was screened out by a HTRF method.

### 2. Assay process

1) GP2D cells were inoculated in a transparent 96-well cell culture plate, and each well contained 80µL of cell suspension and 8000 cells. The cell plate was inoculated in a carbon dioxide incubator at 37 °C overnight;
2) 2 µL of the compound was weighed and added to 78 µL of the cell medium. After the mixture was mixed thoroughly, 20 µL of the solution of compound was weighed and added to the corresponding well of the cell plate. The cell plate was placed back to the carbon dioxide incubator and incubated for another 1 hour;
3) After completion of the incubation, the cell supernatant was discarded. 50 µL of 1X cell lysate was added to each well. The mixture was incubated with shaking at room temperature for 30 minutes;
4) Phospho-ERK1/2 Eu Cryptate antibody and Phospho-ERK1/2 d2 antibody were diluted 20-fold with detection buffer;
5) 16 µL of cell lysate supernatant was weighed and added into each well of a new 384 white microwell plate, and then 2 µL of the diluted solution of Phospho-ERK1/2 Eu Cryptate antibody and 2 µL of the diluted solution of Phospho-ERK1/2 d2 antibody dilution were added. The mixture was incubated at room temperature for at least 4 hours;
6) After completion of the incubation, HTRF was read with multi-label analyzer: excitation: 320nm, emission: 615nm, 665nm;
7). IC₅₀ of the compound to be assayed was calculated.

### 3. Assay result

The result is shown in Table 5.

**Table 5 IC₅₀ value of the compound on inhibiting p-ERK in GP2D cells**

| **Compound No.** | **GP2D p-ERK IC₅₀ (nM)** |
|---|---|
| Compound 1B | 0.76 |

Assay conclusion: The compound of the present disclosure has significant inhibitory effect on p-ERK in KRAS^{G12D} mutant GP2D cells.

### Assay example 2. Assay of p-ERK inhibition in AGS cells

### 1. Purpose

Compound that can effectively inhibit p-ERK in KRAS^{G12D} mutant AGS cells was screened out by a HTRF method.

### 2. Assay process

1) AGS cells were inoculated in a transparent 96-well cell culture plate, and each well contained 80µL of cell suspension and 10000 cells. The cell plate was inoculated in a carbon dioxide incubator at 37 °C overnight;
2) After completion of the incubation, the cell supernatant was discarded. 80 µL of medium containing 0.02% serum was added to each well. The cell plate was incubated in a carbon dioxide incubator at 37 °C overnight;
3) 2 µL of the compound was weighed and added to 78 µL of the cell medium. After the mixture was mixed thoroughly, 20 µL of the solution of compound was weighed and added to the corresponding well of the cell plate. The cell plate was placed back to the carbon dioxide incubator and incubated for another 3 hours;
4) After completion of the incubation, the cell supernatant was discarded. 50 µL of 1X cell lysate was added to each well. The mixture was incubated with shaking at room temperature for 30 minutes;
5) Phospho-ERK1/2 Eu Cryptate antibody and Phospho-ERK1/2 d2 antibody were diluted 20-fold with detection buffer;
6) 16 µL of cell lysate supernatant was weighed and added into each well of a new 384 white microwell plate, and then 2 µL of the diluted solution of Phospho-ERK1/2 Eu Cryptate antibody and 2 µL of the diluted solution of Phospho-ERK1/2 d2 antibody dilution were added. The mixture was incubated at room temperature for at least 4 hours;
7) After completion of the incubation, HTRF was read with multi-label analyzer: excitation: 320nm, emission: 615nm, 665nm;
8). IC₅₀ of the compound to be assayed was calculated.

### 3 Assay result

The result is shown in Table 6.

**Table 6 IC₅₀ value of the compound on inhibiting p-ERK in AGS cells**

| **Compound No.** | **AGS p-ERK IC₅₀ (nM)** |
|---|---|
| Compound **3** hydrochloride | 64.6 |

Assay conclusion: The compound of the present disclosure has significant inhibitory effect on p-ERK in KRAS^{G12D} mutant AGS cells.

### Assay example 3. Assay of p-ERK inhibition in AsPC-1 cells

### 1. Purpose

Compound that can effectively inhibit p-ERK in KRAS^{G12D} mutant AsPC-1 cells was screened out by a HTRF method.

### 2. Assay materials:

ASPC-1 cells purchased from Procell; RPMI-1640 culture medium purchased from VivaCell; fetal bovine serum purchased from Biosera; and Advanced Phospho-ERK1/2(THR202/TYR204) KIT purchased from Cisbio.

**Table 7. The composition of Advanced Phospho-ERK1/2(THR202/TYR204) KIT**

| Ingredient Name | Storage temperature |
|---|---|
| Advanced PhosphoERK1/2 Eu Cryptate Antibody | ≤-16°C |
| Advanced PhosphoERK1/2 d2 Antibody | ≤-16°C |
| Blocking reagent (stock solution 100X) | ≤-16°C |
| Lysis buffer # 1 (stock solution 4X) | ≤-16°C |
| Detection buffer (ready-to-use) | ≤-16°C |

### 3. Method of the assay:

6) ASPC-1 cells were inoculated in a transparent 96-well cell culture plate, and each well contained 80µL of cell suspension and 8000 cells. The cell plate was inoculated in a carbon dioxide incubator at 37 °C overnight;
7) After completion of the incubation, the cell supernatant was discarded. 80 µL of RPMI-1640 blank culture medium was added to each well for starvation overnight;
8) The compound to be assayed was diluted to 2 mM as the first concentration with 100% DMSO, and then serially diluted 5-fold to the eighth concentration with a pipette, i.e., from 2 mM to 25.6 nM. 2 µL of the compound was weighed and added to 78 µL of a cell starvation medium. After the mixture was mixed thoroughly, 20 µL of the solution of compound was weighed and added to the corresponding well of the cell plate. The cell plate was placed back to the carbon dioxide incubator and incubated for another 1 hour; at this time, the concentration of the compound was 10 µM to 0.128 nM, and the concentration of DMSO was 0.5%;
9) After completion of the incubation, the cell supernatant was discarded. 50 µL of cell lysate was added to each well. The mixture was incubated with shaking at room temperature for 30 minutes;
10) Phospho-ERK1/2 Eu Cryptate antibody and Phospho-ERK1/2 d2 antibody were diluted 20-fold with Detection buffer;
11) 16 µL of cell lysate supernatant was weighed and added into each well of a new 384 white microwell plate, and then 2 µL of the diluted solution of Phospho-ERK1/2 Eu Cryptate antibody and 2 µL of the diluted solution of Phospho-ERK1/2 d2 antibody dilution were added. The mixture was incubated at room temperature for 4 hours;
12) After completion of the incubation, HTRF was read with multi-label analyzer: excitation: 320nm, emission: 615nm, 665nm.

### 4. Data analysis:

The equation **(Sample-Min)/(Max-Min)*100%** was used to convert the raw data into inhibition rate, and the IC₅₀ value can be obtained by curve fitting with four parameters ("log(inhibitor) vs. response -- Variable slope" mode in GraphPad Prism). Table 1 provides the inhibitory effects of the compounds of the present disclosure on p-ERK.
**Max well:** The reading value of the positive control well was that of 1X lysate.
**Min well:** The reading value of the negative control well was that of the cell lysate in 0.5% DMSO cell well.

### 5 Assay results

The results are shown in Table 8.

**Table 8 IC₅₀ values of the compounds on inhibiting p-ERK in AsPC-1 cells**

| **Compound No.** | **AsPC-1 p-ERK IC₅₀ (nM)** |
|---|---|
| Compound **1B** | 12.3 |
| Compound **6** | 21.9 |
| Compound **7** hydrochloride | 3.2 |
| Compound **8** hydrochloride | 0.6 |
| Compound **9** hydrochloride | 35.9 |
| Compound **10** hydrochloride | 1.1 |
| Compound **11** formate | 0.9 |

Assay conclusion: The compounds of the present disclosure have a significant inhibitory effect in KRAS^{G12D} mutant AsPC-1 cells.

### Assay example 4. Assay of p-ERK inhibition in AsPC-1 cells

### 1. Purpose

Compound that can effectively inhibit p-ERK in KRAS^{G12D} mutant AsPC-1 cells was screened out by an in-cell western method.

### 2. Assay materials:

ASPC-1 cells purchased from ATCC; RPMI-1640 medium purchased from ATCC; fetal bovine serum purchased from Ausgenex; Phospho-p44/42 MAPK (Erk1/2) (Thr202/Tyr204) (D13.14.4E) XP^{®} Rabbit mAb purchased from CST; GAPDH (D4C6R) Mouse mAb purchased from CST; IRDye 680RD Goat anti-Mouse IgG (H+L) purchased from LI-COR, and IRDye 800CW Goat anti-Rabbit IgG (H+L) purchased from LI-COR.

### 3. Method of the assay:

13) ASPC-1 cells were inoculated in a black bottom and transparent 384-well cell culture plate, and each well contained 40µL of cell suspension and 6000 cells. The cell plate was inoculated in a carbon dioxide incubator at 37 °C overnight;
14) The compound to be assayed was diluted to 10 mM as the first concentration with 100% DMSO, and then serially diluted 3-fold to the nineth concentration with a pipette, i.e., from 10 mM to 1.52 µM. 40 nL of the compound was transferred to the cell plate by the ECHO instrument, and centrifuged at low speed. The cell plate was placed back to the carbon dioxide incubator and incubated for another 1 hour; at this time, the concentration of the compound was 10 µM to 1.52 nM, and the concentration of DMSO was 0.1%;
15) After completion of the incubation, 40 µL of 8% paraformaldehyde fixative was added to each well and allowed to stand at room temperature for 20 minutes;
16) The well was washed twice with PBS buffer; 40 µL of precooled methanol solution was added to each well; the well was washed twice with PBS buffer; 20 µL of blocking solution was added to each well and allowed to stand at room temperature for 60 minutes;
17) The blocking solution was removed and 20 µL of a primary antibody mixture (Phospho-p44/42 MAPK (Erk1/2) (Thr202/Tyr204) (D13.14.4E) XP^{®} Rabbit mAb diluted 1000 times, GAPDH (D4C6R) Mouse mAb diluted 2000 times) was added to each well, and incubated at 4°C overnight;
18) The primary antibody mixture was removed and the cells were washed with PBS buffer for three times; 20 µL of a secondary antibody mixture (IRDye 800CW Goat anti-Rabbit IgG (H+L) diluted 2000 times, IRDye 680RD Goat anti Mouse IgG (H+L) diluted 2000 times) was added to each well and incubated at room temperature in the dark for 60 minutes;
19) The primary antibody mixture was removed, and the cells were washed with PBS buffer for three times; the plate was centrifuged upside down at 1000 rpm for 1 min, and then scanned using an Odyssey CLx instrument.

### 4. Data analysis:

The relative expression level was calculated using the equation **(total fluorescence value of the 800 channel/total fluorescence value of the 700 channel)**, and the raw data was converted into inhibition rate using the equation **(Max-Sample)/(Max-Min)*100%**. The IC₅₀ value was obtained by four-parameter curve fitting (log(inhibitor) vs. responseVariable slope mode in the plug-in XLfit in Excel).
**Max well:** The reading value of the positive control well was the highest concentration of the positive drug.
**Min well:** The reading value of the negative control well was that in 0.1% DMSO cell well.

### 5 Assay results

The results are shown in Table 9.

**Table 9 IC₅₀ values of the compounds on inhibiting p-ERK in AsPC-1 cells**

| **Compound No.** | **AsPC-1 p-ERK IC₅₀ (nM)** |
|---|---|
| Compound 12A trifluoroacetate | 15.0 |
| Compound 13A trifluoroacetate | 4.2 |
| Compound 15 trifluoroacetate | 5.6 |
| Compound 18 hydrochloride | 10.1 |
| Compound 19 hydrochloride | 2.5 |
| Compound 20 hydrochloride | 1.3 |

Assay conclusion: The compounds of the present disclosure have significant inhibitory effect on p-ERK in KRAS^{G12D} mutant AsPC-1 cells.

### Assay example 5. Anti-cell proliferation effect of compounds in tumor cell line AsPC-1

### Purpose of the study

In this assay, the effect of the compounds on inhibiting cell proliferation was studied by detecting the effect of the compounds on the cell activity in vitro in the KRAS^{G12D} mutant tumor cell line AsPC-1.

### Assay materials

Cell line: AsPC-1; Tumor type: pancreatic cancer; Growth characteristics: adherent growth; Culture method: RPMI 1640 + 10% FBS
Ultra Low Cluster-96-well plate (Corning-7007)
Greiner CELLSTAR 96-well plate (# 655090)
Promega CellTiter-Glo 3D Luminescence Cell Viability Assay Kit (Promega-G9683)
2104-10 EnVision Plate Reader, PerkinElmer
RPMI 1640, DMEM, PBS (phosphate buffered saline), FBS (fetal bovine serum), Antibiotic-antimycotic, L-glutamine, DMSO (dimethylsulfoxide)

### Method and steps of the assay

### Cell culture

The tumor cell line was cultured in a 37 °C, 5% CO₂ incubator according to the culture conditions indicated in the culture method. Cells were passaged regularly, and cells in logarithmic growth phase were used for plating.

### Cell plating

Cells were stained with trypan blue and viable cells were counted.

The cell concentration was adjusted to an appropriate concentration.

### Cell line: AsPC-1; Density (per well): 7000 cells.

To each well of a ULA culture plate was added 135 µL of cell suspension. To the blank control well was added the same volume of culture medium without cells.

After plating, the ULA culture plate was immediately centrifuged at room temperature at 1000 rpm for 10 minutes. Note: After completion of the centrifugation, subsequent operations must be processed carefully to avoid unnecessary shocks.

The culture plate was incubated in a 37 °C, 5% CO₂ incubator at 100% relative humidity overnight.

### Formulation of 10X compound working solution and treatment of cells with compound (day 1)

10X compound working solution (10X working solution in DMSO) was formulated. To the ULA culture plate was add 15 µL of the 10X compound working solution, and to the vehicle control and blank control was added 15 µL of a DMSO-cell culture medium mixture, respectively.

The 96-well cell plate was placed back to the incubator and incubated for 120 hours.

The sphere formation of cells was observed every day until the end of the assay.

### CellTiter-Glo luminescent cell viability detection (Day 5)

The following steps were performed according to the instructions of the Promega CellTiter-Glo 3D Luminescent Cell Viability Assay Kit (Promega # G9683).

150 µL (equal to the volume of cell culture medium in each well) of CellTiter-Glo 3D reagent was added to each well. The cell plate was wraped by aluminum foil to protect from light.

The culture plate was shaked on an orbital shaker for 5 minutes.

The mixture in wells was mixed thoroughly by pipetting up and down 10 times carefully with pipette, so as to make sure that the spheroids of cells were sufficiently detached before proceeding to the next step.

The solution in the ULA plate was then transferred to a black bottom culture plate (#655090) and left to stand at room temperature for 25 minutes to stabilize the luminescent signal.

The luminescent signal was detected on a 2104 EnVision plate reader.

### Data analysis

The following formula was used to calculate inhibition rate (IR) of the assay compound: IR (%) = (1 - (RLU of compound - RLU of blank control) / (RLU of vehicle control - RLU of blank control))* 100%. The inhibition rates of different concentrations of compounds were calculated in Excel, and then GraphPad Prism software was used to draw inhibition curves and calculate related parameters, including minimum inhibition rate, maximum inhibition rate, and IC₅₀.

### Assay results

The results are shown in Table 10.

**Table 10 IC₅₀ value of the compounds on inhibiting KRAS^{G12D} mutant AsPC-1 cells**

| **Compound No.** | KRAS^{G12D} **AsPC-1 IC₅₀ (nM)** |
|---|---|
| Compound **1B** | 74.6 |
| Compound **6** | 94.6 |
| Compound **7** hydrochloride | 37.7 |

Assay conclusion: The compounds of the present disclosure show excellent anti-proliferation effect on KRAS^{G12D} mutant AsPC-1 cells.

### Assay example 6. Anti-proliferation effects of compounds in tumor cell line AsPC-1

### 1. Purpose

Anti-cell proliferation effect of the compounds in the KRAS^{G12D} mutant tumor cell line AsPC-1 was verified by a 3D-CTG method.

### 2. Assay materials:

ASPC-1 cells purchased from ATCC; RPMI-1640 culture medium purchased from ATCC; fetal bovine serum purchased from Augenex; CellTiter-Glo^{®} 3D assay kit (3D-CTG) purchased from Promega; and CellCarrier-96 Spheroid ULA/CS purchased from PE.

### 3. Method of the assay:

20) ASPC-1 cells were inoculated in a transparent 96-well cell culture plate, and each well contained 195 µL of cell suspension and 2000 cells;
21) The compound to be assayed was diluted to 10 mM as the first concentration with 100% DMSO, and then serially diluted 5-fold to the eighth concentration with a pipette, i.e., from 10 mM to 0.13 µM. 2 µL of the serially diluted compound was weighed and added to 48 µL of cell culture medium for secondary dilution. After the mixture was mixed thoroughly, 5 µL of the secondarily diluted compound was weighed and added to the corresponding 195 µL well of the cell plate. The cell plate was placed back to the carbon dioxide incubator and incubated for 7 days; at this time, the concentration of the compound was 10 µM to 0.128 nM, and the concentration of DMSO was 0.1%;
22) After completion of the incubation, 100 µL of cell supernatant was discarded. 60 µL of 3D-CTG was added to each well. The mixture was incubated with shaking with 200 rpm at room temperature for 20 minutes; the cell plate was inoculated in an incubator at room temperature for 1h;
23) 100 µL of supernatant in the plate was transferred to a 96-well black-bottom transparent plate and the luminescence was read in BMG.

### 4. Data analysis:

The equation **Inhibition%=(Ave_H-Sample)/(Ave_H-Ave_L)** was used to convert the raw data into inhibition rate, and the IC₅₀ value can be obtained by curve fitting with four parameters ("log(inhibitor) vs. response -- Variable slope" mode in GraphPad Prism).
**H well:** the reading value of DMSO wells
**L well:** the reading value of Medium

### 5 Assay results

The results are shown in Table 11.

**Table 11 IC₅₀ values of compounds on inhibiting KRAS^{G12D} mutant AsPC-1 cells**

| **Compound No.** | **KRAS^{G12D} AsPC-1 IC₅₀ (nM)** |
|---|---|
| Compound **8** hydrochloride | 9.0 |
| Compound **16** trifluoroacetate | 18.6 |
| Compound **20** hydrochloride | 9.6 |

Assay conclusion: The compounds of the present disclosure show excellent anti-proliferation effect on KRAS^{G12D} mutant AsPC-1 cells.

### Assay example 7. Pharmacokinetic study of oral and intravenously injected assay compound in CD-1 mouse

### Assay purpose

To assay the in vivo pharmacokinetics of oral and intravenously injected compound in CD-1 mouse.

### Assay processs

The assay compound was mixed with 5% DMSO+95% (10%HP-β-CD) aqueous solution. The mixture was vortexed and sonicated to prepare a clear solution of 0.5 mg/mL (intravenous) or a clear solution of 3 mg/mL (oral). The clear solution was filtered by a microporous membrane for use. Male CD-1 mice aged 7 to 10 weeks were selected, administered the candidate compound solutions intravenously, and administered the candidate compound solutions orally. Whole blood was collected for a certain period of time, and prepared to obtain plasma. Drug concentration was analyzed by LC-MS/MS method, and pharmacokinetic parameters were calculated by Phoenix WinNonlin software (Pharsight, USA).

### Assay results

**Table 12. Intravenous (IV) PK data**

| Assay sample | Compound **8** hydrochloride |
|---|---|
| Dosage (mg/kg) | 2.22 |
| C₀ (nM) | 1704 |
| T_{1/2} (h) | 12.6 |
| V_{d} (L/kg) | 52.9 |
| Cl (mL/Kg/min) | 76 |
| AUC₀₋ₗₐₛₜ (nM.h) | 598 |

**Table 13. Oral (PO) PK data**

| Assay sample | Compound **8** hydrochloride |
|---|---|
| Dosage (mg/kg) | 101 |
| Cₘₐₓ (nM) | 4410 |
| Tₘₐₓ | 1 |
| T_{1/2} (h) | 7.26 |
| AUC₀₋ₗₐₛₜ (nM.h) | 9977 |
| F | 36.7% |

### Assay results

The compound of the present disclosure has long half-life, high exposure, good oral bioavailability, and good pharmacokinetic properties in mice.

### Assay example 8. Assay on pharmacodynamics in vivo

### Assay method:

A Balb/c nude mouse model of subcutaneously xenograft tumor of human colon cancer GP2D cells was established. 0.2 mL (2×10⁶) of GP2D cells (Matrigel was added, and the volume ratio was 1:1) were inoculated subcutaneously on the right back of each mouse. When the average tumor volume reached 140 mm³, the mice were divided into groups with 6 mice in each group and administered. On the day of the assay, the animals were administered the corresponding drugs according to the corresponding groups. The first group G1 was set as a negative control group, which was given 5%DMSO+95%(10%HP-β-CD) alone by gavage administration. The second group G2 to the fourth group G4 were given compound **8** hydrochloride, and the dosage and protocol of administration were shown in Table 14.

**Table 14 Pharmacodynamic study of the assay compound on transplanted tumor of human diffuse large B lymphoma TMD8 in mice**

| **Grou p** | **Number of animals** | **Assay compound** | **Dosage (mg/kg)** | **Administ ration volume (mL/kg)** | **Route and frequency of administration** |
|---|---|---|---|---|---|
| G1 | 6 | Negative control | (N/A) | (N/A) | PO, BID *28 |
| G2 | 6 | Compound **8** hydrochloride | 50 | 10 | PO,QD*28 |
| G3 | 6 | Compound **8** hydrochloride | 100 | 10 | PO,QD*28 |
| G4 | 6 | Compound **8** hydrochloride | 200 | 10 | PO,QD*28 |

| | | | | | |
|---|---|---|---|---|---|
| Note: PO means oral administration, QD means once daily, and BID means once daily. | | | | | |

During the assay, the body weight and the tumor size of animals were measured twice a week. Meanwhile, clinical symptoms of animals were observed and recorded every day. Each administration was referenced to the most recent body weight of animals.

The length (a) and width (b) of a tumor were measured with a digital vernier caliper. The calculation formula of tumor volume (TV) was: TV=a×b²/2.

### Assay results:

Compound **8** hydrochloride has a significant inhibitory effect on the xenograft tumor of human colon cancer GP2D in mice. After 28 days of administration, the second group G2 (50 mg / kg, PO, QD) has a tumor volume inhibition rate TGI (%) of 47.0% on the 28th day; the third group G3 (100 mg /kg, PO, QD) and the fourth group G4 (200 mg /kg, PO, QD) respectively have a tumor volume inhibition rate TGI (%) of 75.9% and 94.7% on day 28; and detailed results are shown in Table 15.

**Table 15 Effects of the assay compound on animal tumor size in xenograft tumor model of human colon cancer GP2D in mice**

| Group | Number of animals | Assay compound | Frequency of administration | Dose mg/kg | Tumor volume inhibition rate TGI (%) |
|---|---|---|---|---|---|
| G1 | 6 | Negative control | PO, BID *28 | NA | N/A |
| G2 | 6 | Compound **8** hydrochloride | PO,QD*28 | 50 | 47.0 |
| G3 | 6 | Compound **8** hydrochloride | PO,QD*28 | 100 | 75.9 |
| G4 | 6 | Compound **8** hydrochloride | PO,QD*28 | 200 | 94.7 |

| | | | | | |
|---|---|---|---|---|---|
| Note: N/A means no detection. | | | | | |

**Assay conclusion:** In terms of in vivo efficacy, the compound of the present invention exhibits a good tumor inhibition effect in the GP2D cell line, with obvious dose dependence.

## Claims

1. A compound represented by formula (VI) or a pharmaceutically acceptable salt thereof, 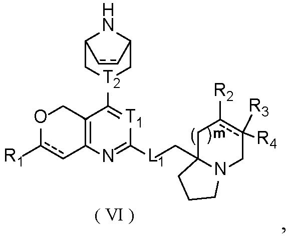 wherein 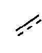
is selected from a single bond and a double bond;
T₁ is selected from CRₐ and N;
T₂ is selected from CH and N;
L₁ is selected from O and S;
R₁ is selected from phenyl, naphthyl, 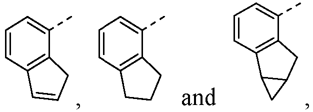 wherein the phenyl, naphthyl, 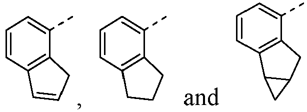 are each independently optionally substituted with 1, 2, 3, 4 or 5 R_{b};
R₂ is H;
R₃ is selected from H, F, CN, CH₃ and OCH₃, wherein the CH₃ and OCH₃ are optionally substituted with 1, 2 or 3 halogens;
alternatively, R₂ and R₃ together with the atoms to which they are attached form a phenyl group or a 5-6 membered heteroaryl group, wherein the phenyl group or the 5-6 membered heteroaryl group is optionally substituted with 1, 2 or 3 halogens;
R₄ is absent or is selected from H, CH₃ and -CH=CH₂, wherein the CH₃ and -CH=CH₂ are optionally substituted with 1, 2 or 3 halogens;
alternatively, R₃ and R₄ together with the atom to which they are attached form 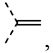 wherein the 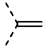 is optionally substituted with 1 or 2 R_{c};
alternatively, R₃ and R₄ together with the atom to which they are attached form a cyclopropyl group, which is optionally substituted with 1, 2 or 3 halogens;
Rₐ is selected from Hand CN;
each R_{b} is independently selected from H, F, Cl, Br, I, OH, NH₂, CN, C₁₋₃ alkyl, C₁₋₃ alkoxy, C₂₋₄ alkynyl, C₂₋₄ alkynyl-cyclopropyl, C₂₋₃ alkenyl, -C(=O)C₁₋₃ alkyl and C₃₋₅ cycloalkyl, wherein the C₁₋₃ alkyl, C₁₋₃ alkoxy, C₂₋₄ alkynyl, C₂₋₄ alkynyl-cyclopropyl, C₂₋₃ alkenyl, -C(=O)C₁₋₃ alkyl and C₃₋₅ cycloalkyl are optionally substituted with 1, 2, 3, 4 or 5 halogens;
each R_{c} is independently selected from F, Cl, Br, I, CN, CH₃ and OCH₃;
m is selected from 0 and 1.

2. A compound represented by formula (P-1) or a pharmaceutically acceptable salt thereof, 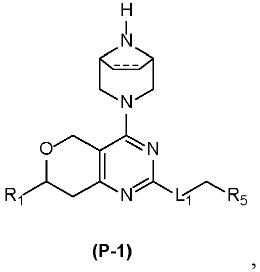 wherein 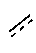
is selected from a single bond and a double bond;
L₁ is selected from O and S;
R₁ is selected from phenyl, naphthyl, 5-10 membered heteroaryl, 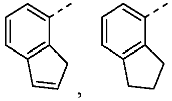 and 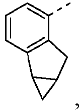 wherein the phenyl, naphthyl, 5-10 membered heteroaryl, 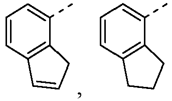 and 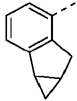 are each independently optionally substituted with 1, 2, 3, 4 or 5 R_{b}, and R₅ is selected from 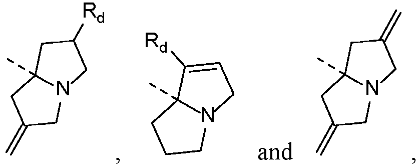 wherein the 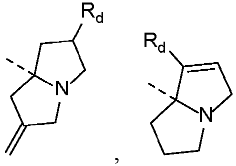 and 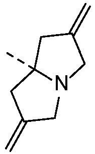 are each independently optionally substituted with 1, 2, 3, 4 or 5 R_{d};
alternatively, R₁ is 5-10 membered heteroaryl, wherein the 5-10 membered heteroaryl is optionally substituted with 1, 2, 3, 4 or 5 R_{b}, and R₅ is 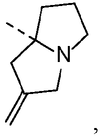 wherein the 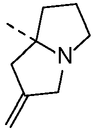 is optionally substituted with 1, 2, 3, 4 or 5 R_{d};
each R_{b} is independently selected from H, F, Cl, Br, I, OH, NH₂, CN, C₁₋₃ alkyl, C₁₋₃ alkoxy, C₂₋₄ alkynyl, C₂₋₄ alkynyl-cyclopropyl, C₂₋₃ alkenyl, -C(=O)C₁₋₃ alkyl and C₃₋₅ cycloalkyl, wherein the C₁₋₃ alkyl, C₁₋₃ alkoxy, C₂₋₄ alkynyl, C₂₋₄ alkynyl-cyclopropyl, C₂₋₃ alkenyl, -C(=O)C₁₋₃ alkyl and C₃₋₅ cycloalkyl are optionally substituted with 1, 2, 3, 4 or 5 R;
each R_{d} is independently selected from F, Cl, Br, I, CN, CH₃ and OCH₃;
each R is independently selected from halogen and D.

3. A compound represented by formula (P-2) or a pharmaceutically acceptable salt thereof: 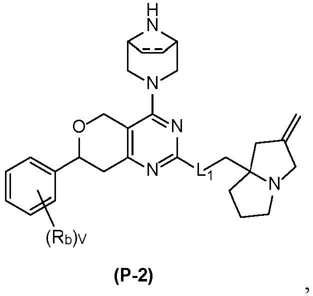 wherein 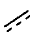
is selected from a single bond and a double bond;
L₁ is selected from O and S;
each R_{b} is independently selected from F, Cl, Br, I, OH, NH₂, CN, C₁₋₃ alkyl, C₁₋₃ alkoxy, C₂₋₄ alkynyl, C₂₋₄ alkynyl-cyclopropyl, C₂₋₃ alkenyl, -C(=O)C₁₋₃ alkyl and C₃₋₅ cycloalkyl, wherein the C₁₋₃ alkyl, C₁₋₃ alkoxy, C₂₋₄ alkynyl, C₂₋₄ alkynyl-cyclopropyl, C₂₋₃ alkenyl, -C(=O)C₁₋₃ alkyl and C₃₋₅ cycloalkyl are optionally substituted with 1, 2, 3, 4 or 5 R;
each R is independently selected from halogen and D;
v is selected from 1, 2, 3, 4 and 5;
provided that at least one R_{b} is substituted with 1, 2, 3, 4 or 5 D.

4. The compound according to claim 1 or a pharmaceutically acceptable salt thereof, wherein each R_{b} is independently selected from H, F, Cl, Br, I, OH, NH₂, CN, CH₃, CH₂CH₃, OCH₃, OCH₂CH₃, -CH=CH₂, -Ca≡CH, -C≡CCH₃, -C≡CCH₂CH₃, 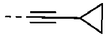 and cyclopropyl, wherein the CH₃, CH₂CH₃, OCH₃, OCH₂CH₃, -CH=CH₂, -C≡CH, -C≡CCH₃, -C≡CCH₂CH₃, 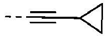 and cyclopropyl are optionally substituted with 1, 2, 3, 4 or 5 halogens; alternatively, each R_{b} is independently selected from H, F, Cl, Br, I, OH, NH₂, CN, CH₃, CH₂F, CF₂H, CF₃, CH₂CH₃, CF₂CF₃, -CH=CH₂, -C≡CH, -C≡CF, -C≡CBr, -C≡CCH₃, -C≡CCF₃, -C≡CCH₂CH₃, 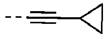 and cyclopropyl.

5. The compound according to claim 2 or 3 or a pharmaceutically acceptable salt thereof, wherein each R_{b} is independently selected from H, F, Cl, Br, I, OH, NH₂, CN, CH₃, CH₂CH₃, OCH₃, OCH₂CH₃, -CH=CH₂, -C≡CH, -C≡CCH₃, -C≡CCH₂CH₃ , 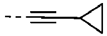 and cyclopropyl, wherein the CH₃, CH₂CH₃, OCH₃, OCH₂CH₃, -CH=CH₂, -C≡CH, -C≡CCH₃, -C≡CCH₃CH₃, 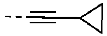 and cyclopropyl are optionally substituted with 1, 2, 3, 4 or 5 R; alternatively, each R_{b} is independently selected from H, F, Cl, Br, I, OH, NH₂, CN, CH₃, CD₃, CH₂F, CF₂H, CF₃, CH₂CH₃, CF₂CF₃, -CH=CH₂, -C≡CH, -C≡CF, -C≡CBr, -C≡CCH₃ 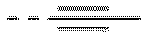 CD₃, -C≡CCF₃, -C≡CCH₂CH₃, 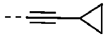 and cyclopropyl.

6. The compound according to claim 1 or a pharmaceutically acceptable salt thereof, wherein

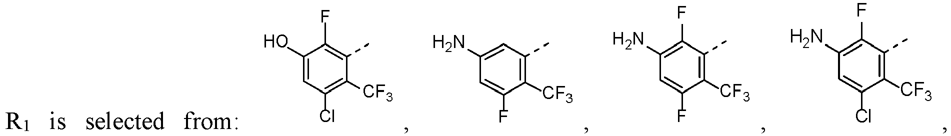

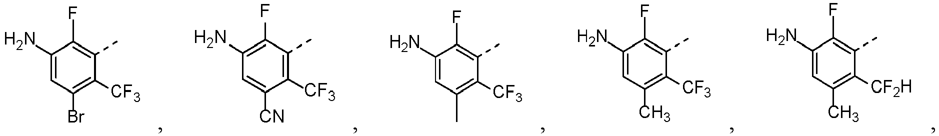

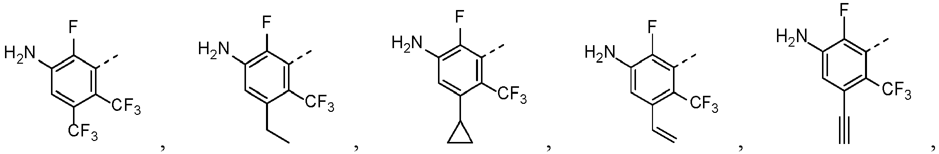

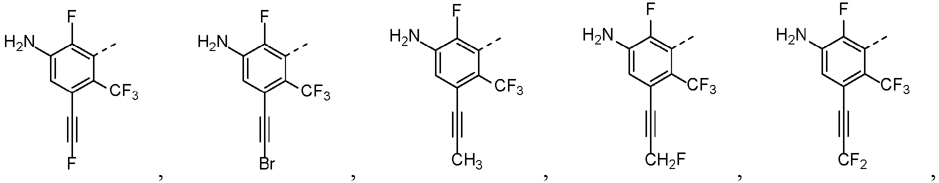

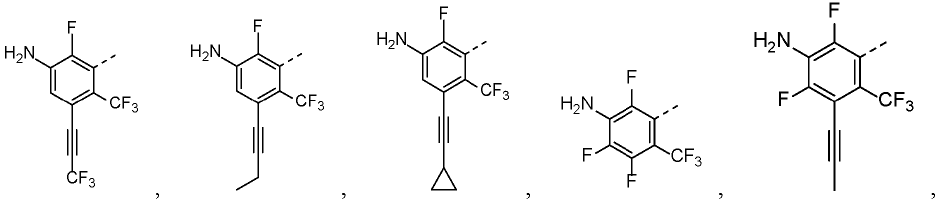

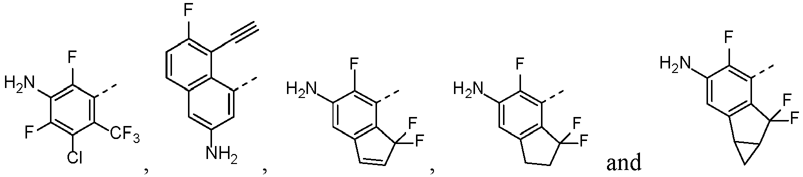

7. The compound according to claim 2 or a pharmaceutically acceptable salt thereof, wherein
R₁ is selected from 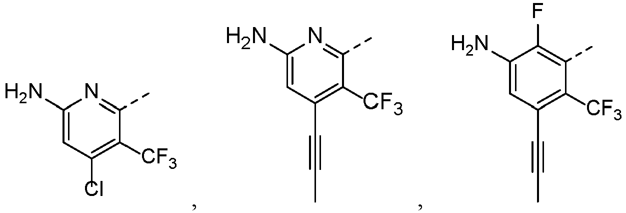 and

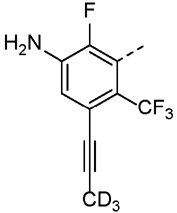

8. The compound according to claim 1 or a pharmaceutically acceptable salt thereof, wherein R₃ is selected from H, F, CN, CH₃ and OCH₃.

9. The compound according to claim 1 or a pharmaceutically acceptable salt thereof, wherein R₂ and R₃ together with the atoms to which they are attached form a phenyl group, a furyl group and a pyridyl group, wherein the phenyl group, the furyl group and the pyridyl group are optionally substituted with one F.

10. The compound according to claim 1 or a pharmaceutically acceptable salt thereof, wherein R₄ is absent or is selected from H, CH₂F and -CH=CH₂.

11. The compound according to claim 1 or a pharmaceutically acceptable salt thereof, wherein R₃ and R₄ together with the atom to which they are attached form

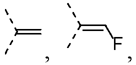

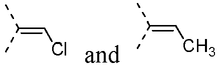

12. The compound according to claim 1 or a pharmaceutically acceptable salt thereof, wherein R₃ and R₄ together with the atom to which they are attached form

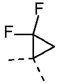

13. The compound according to claim 1 or a pharmaceutically acceptable salt thereof, wherein the structural moiety 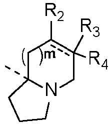 is selected from

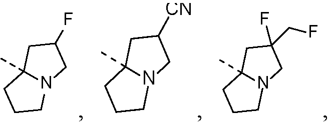

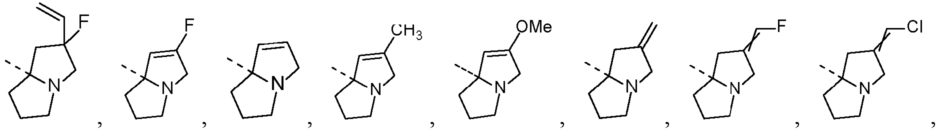

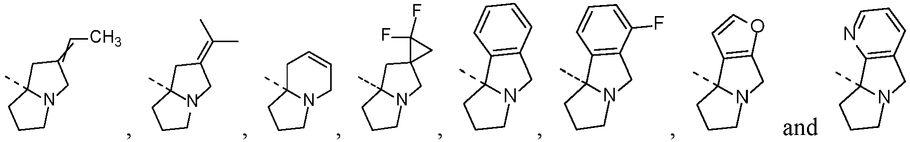

14. The compound according to claim 1 or a pharmaceutically acceptable salt thereof, wherein the structural moiety 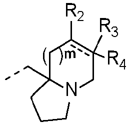 is selected from

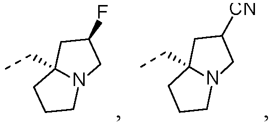

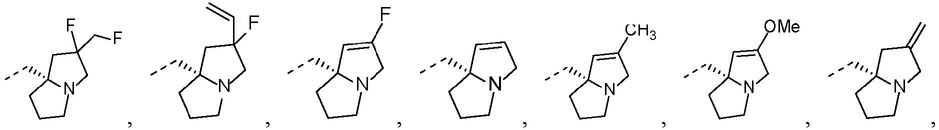

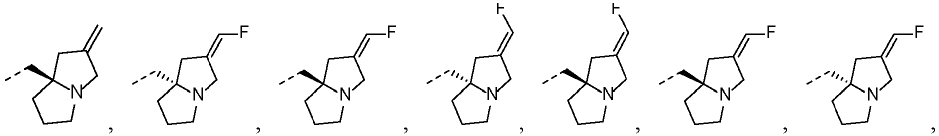

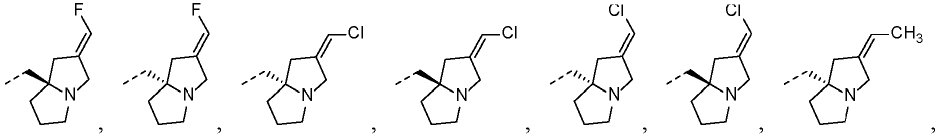

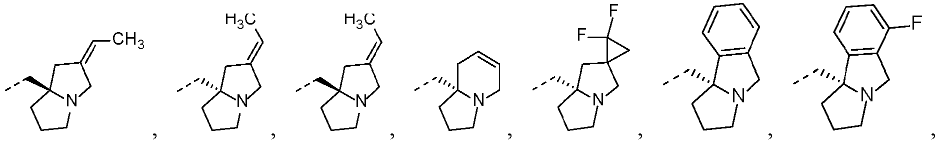

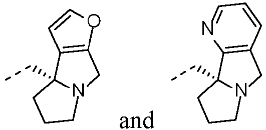

15. The compound according to claim 1, 2 or 3 or a pharmaceutically acceptable salt thereof, wherein the structural moiety

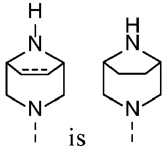

16. The compound according to claim 1 or a pharmaceutically acceptable salt thereof, 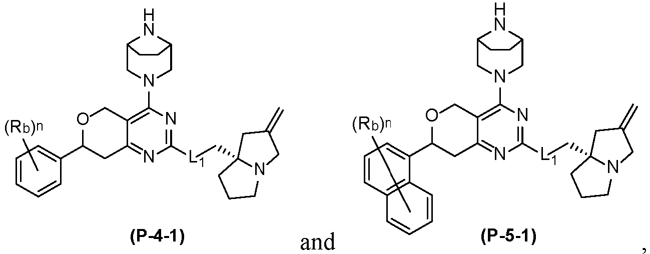 wherein
each of R_{b} and L₁ is as defined in claim 1;
n is selected from 0, 1, 2, 3, 4 and 5.

17. A compound or a pharmaceutically acceptable salt thereof, wherein the compound is selected from

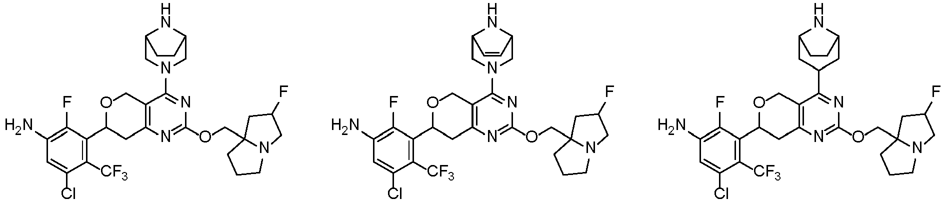

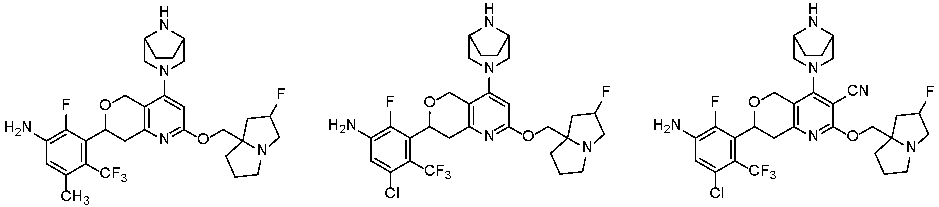

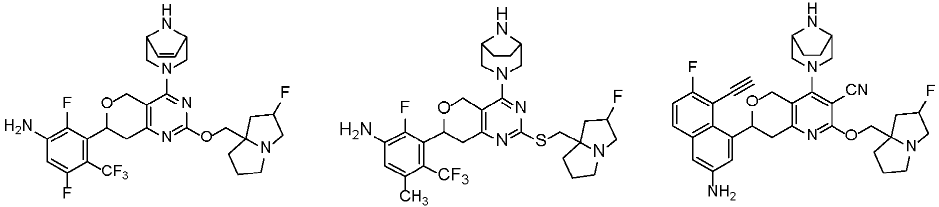

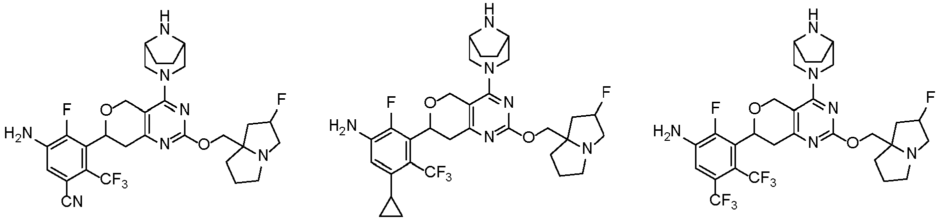

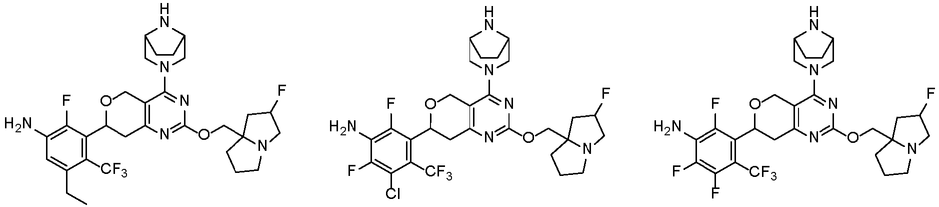

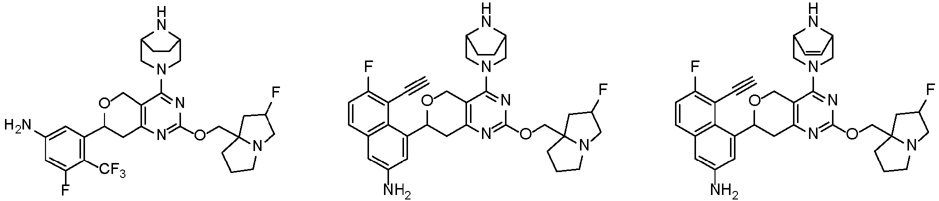

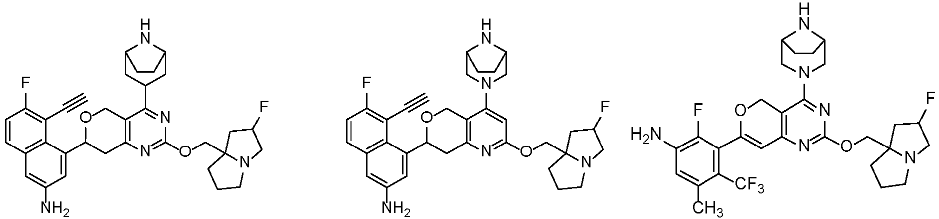

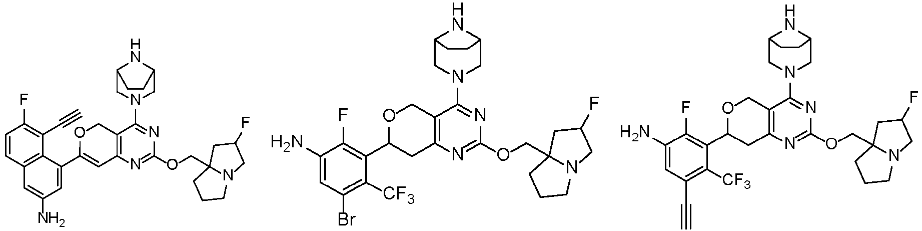

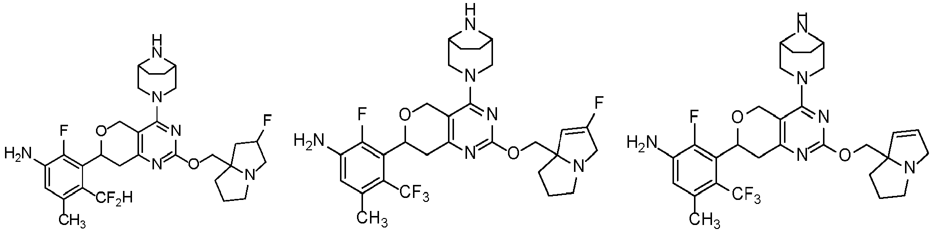

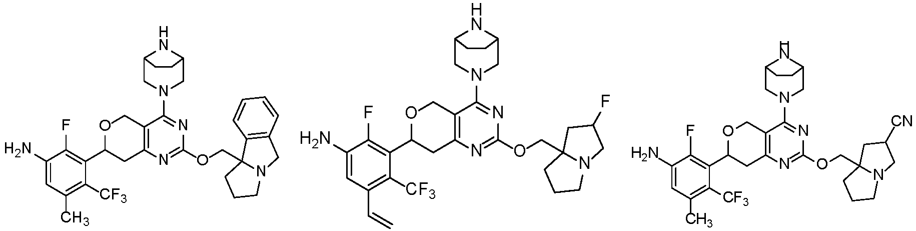

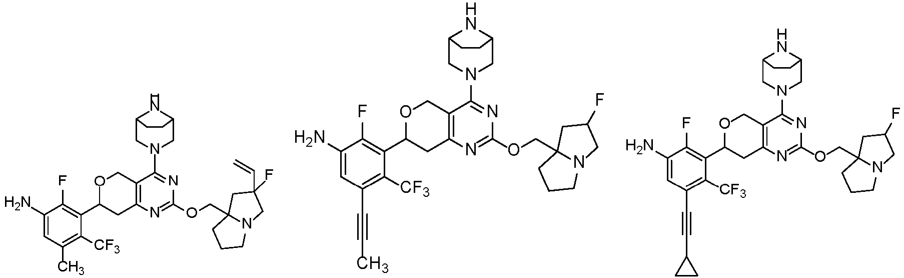

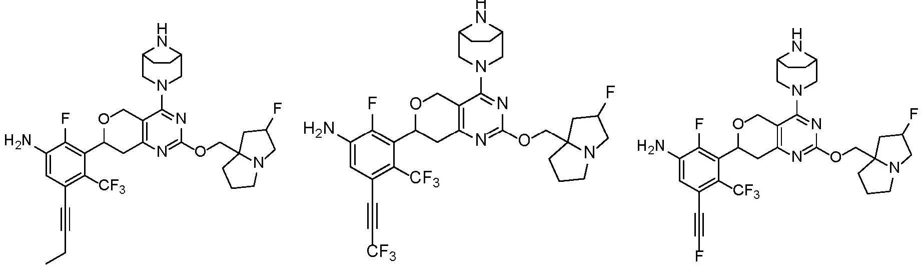

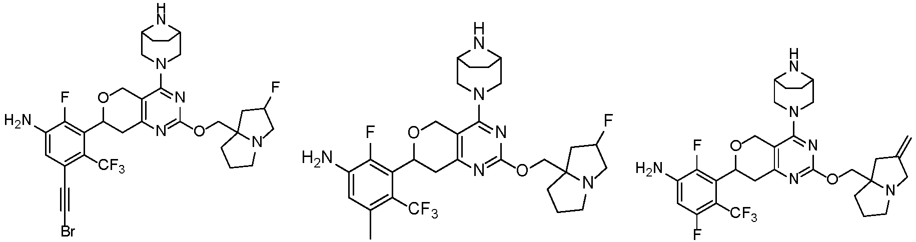

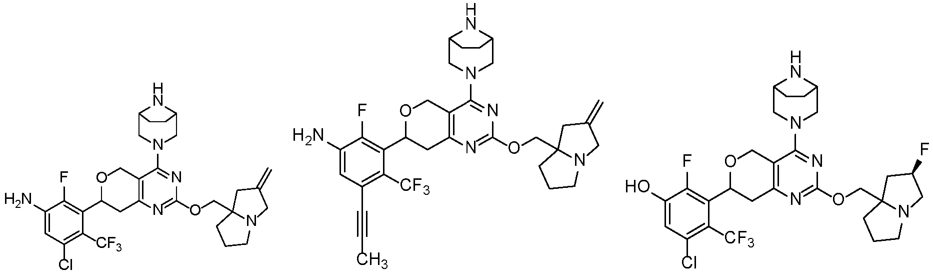

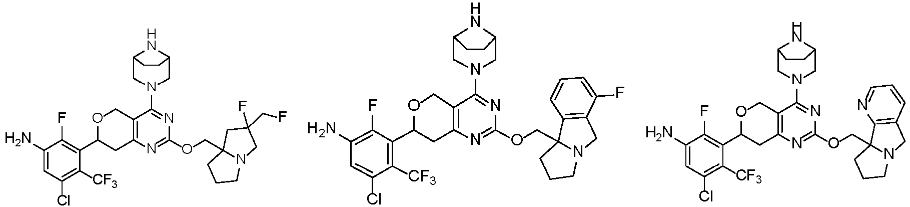

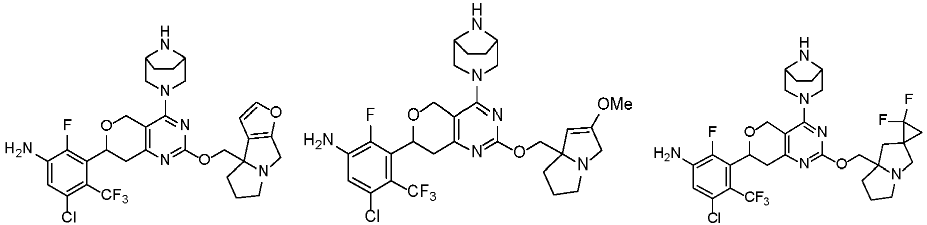

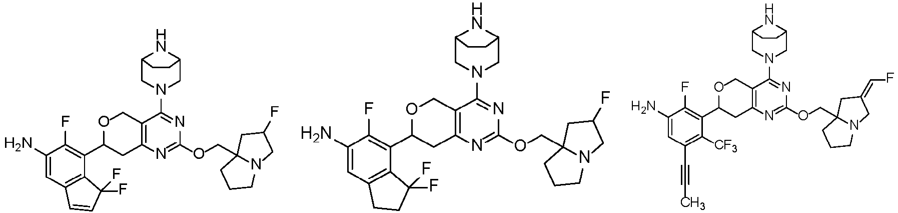

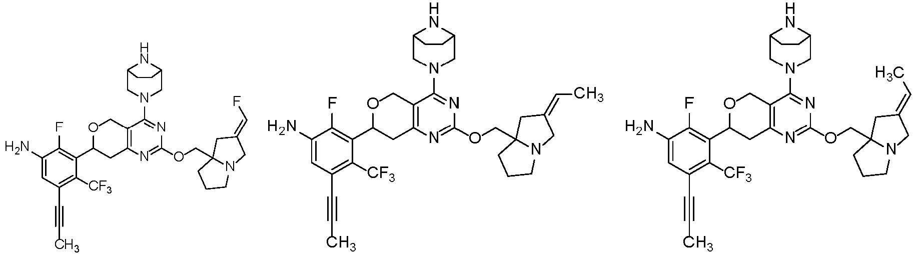

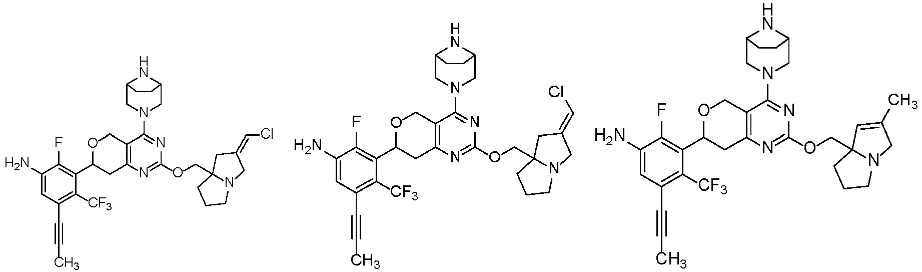

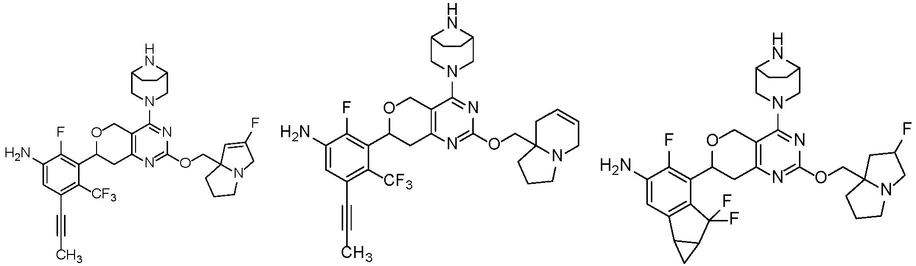

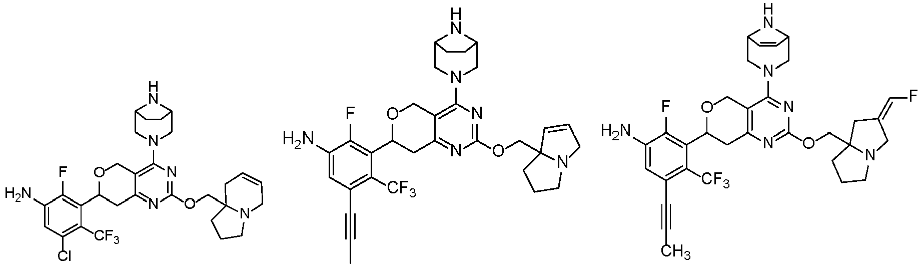

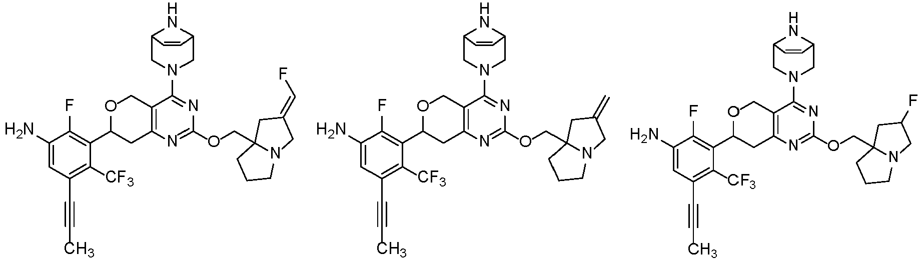

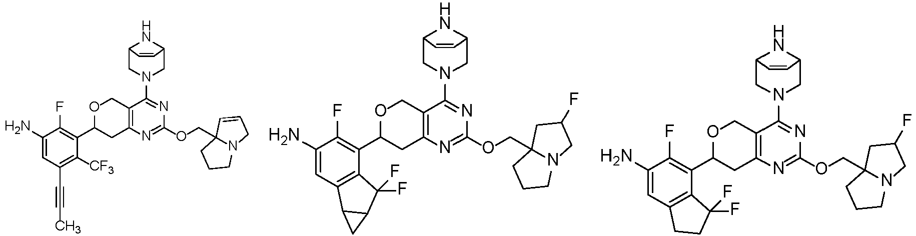

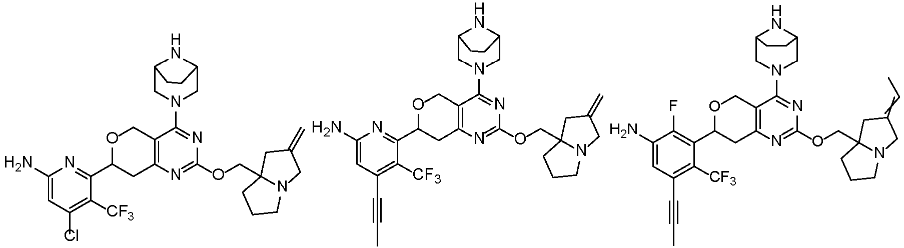

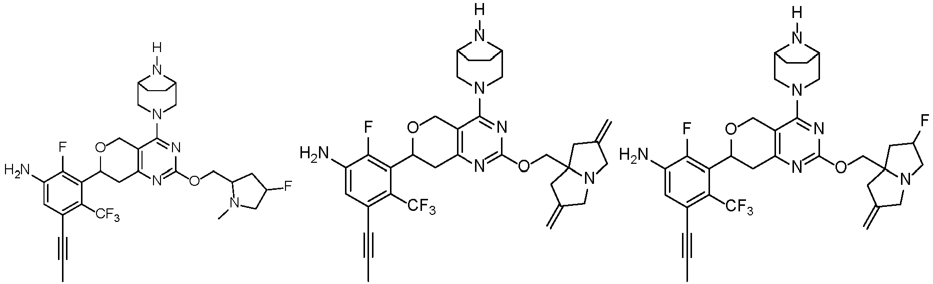

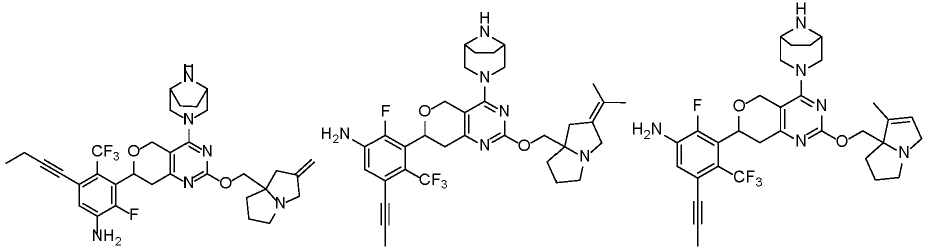

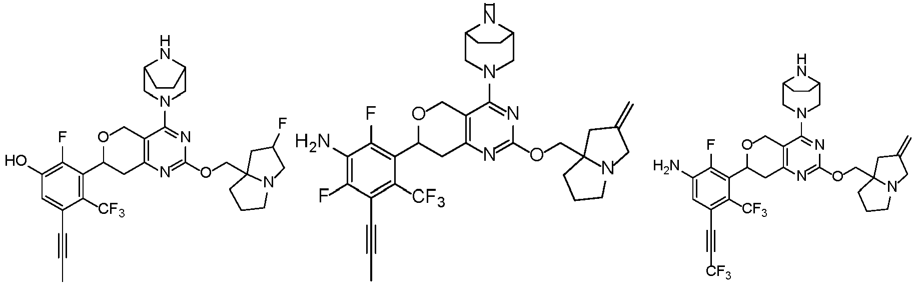

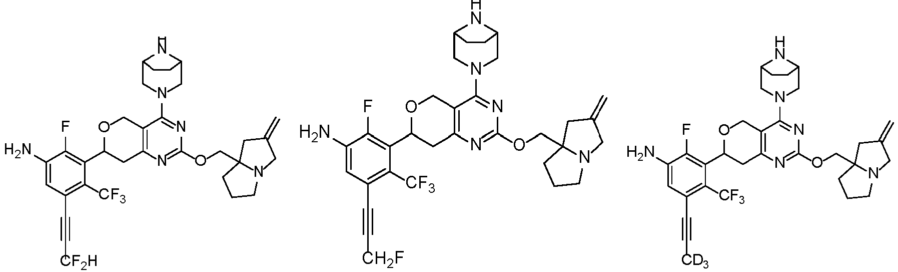

18. The compound according to claim 17 or a pharmaceutically acceptable salt thereof, wherein the compound is selected from:

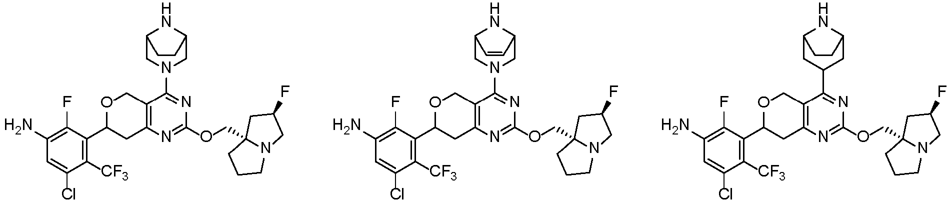

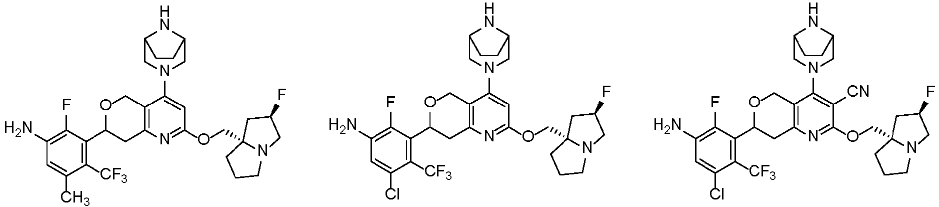

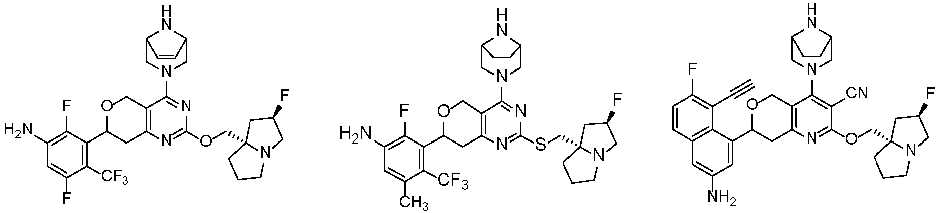

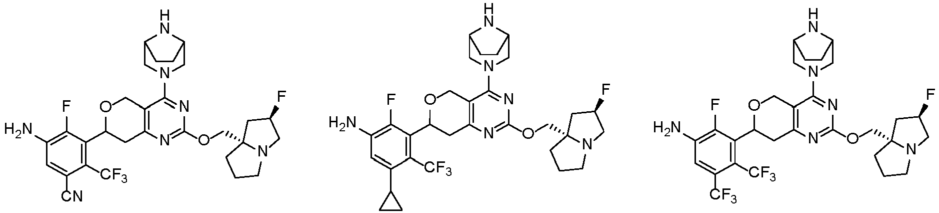

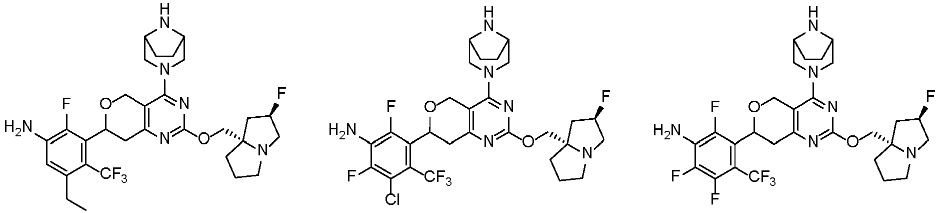

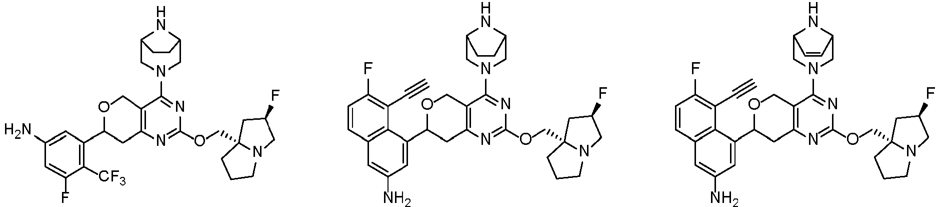

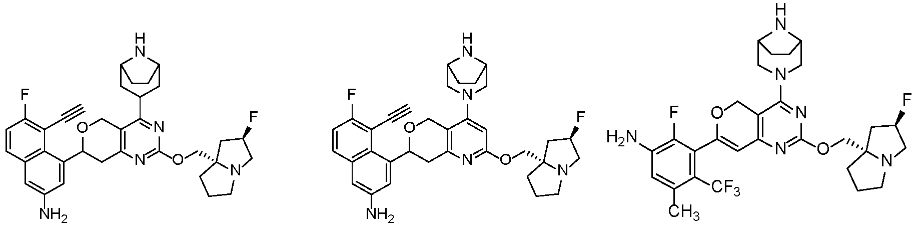

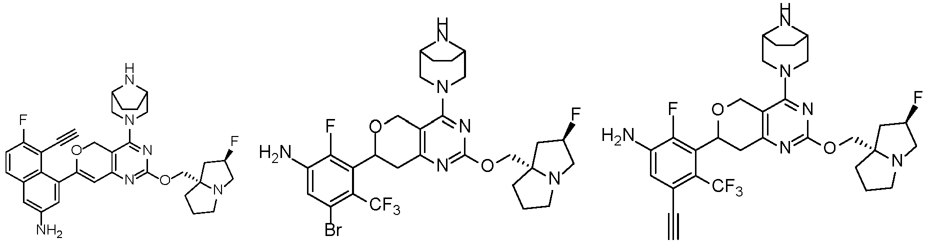

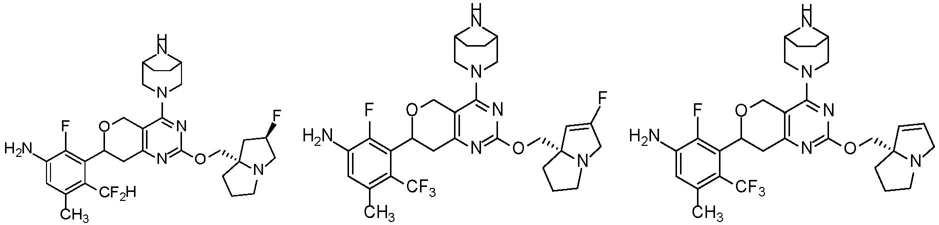

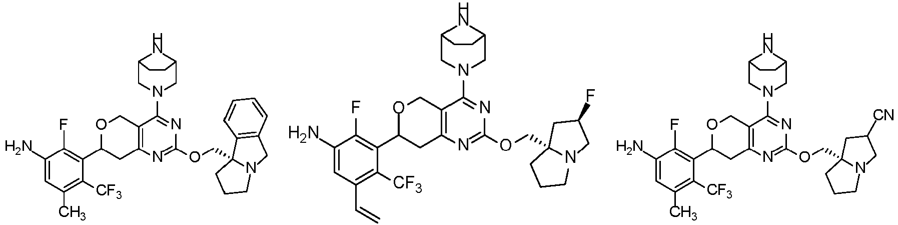

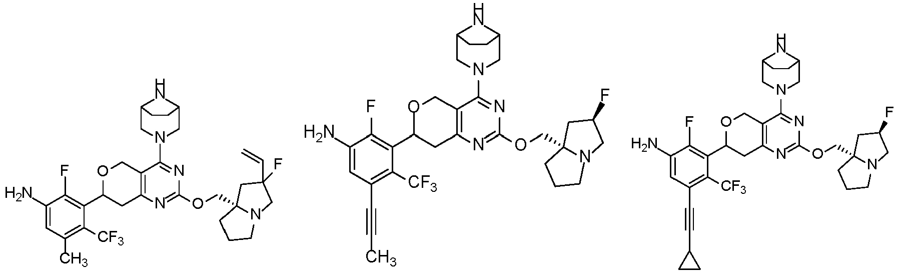

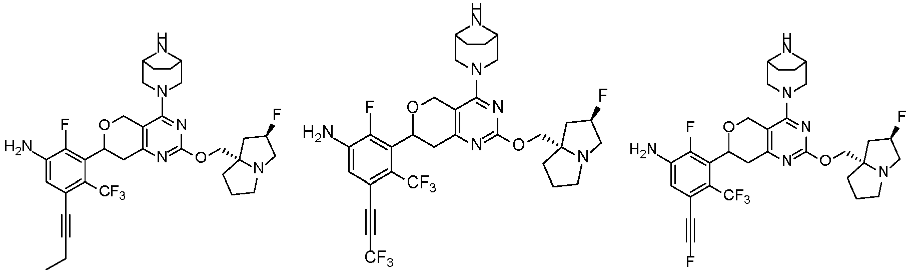

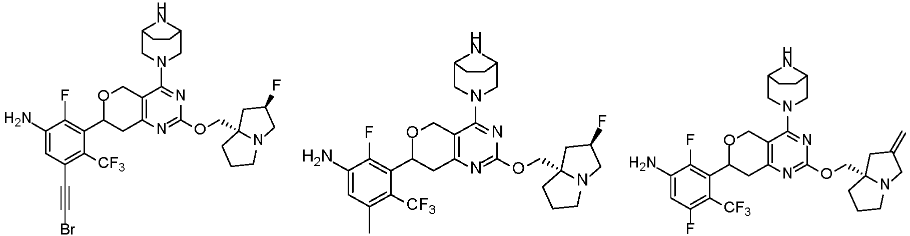

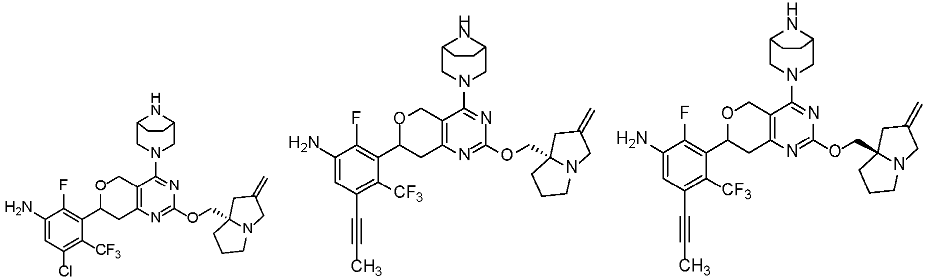

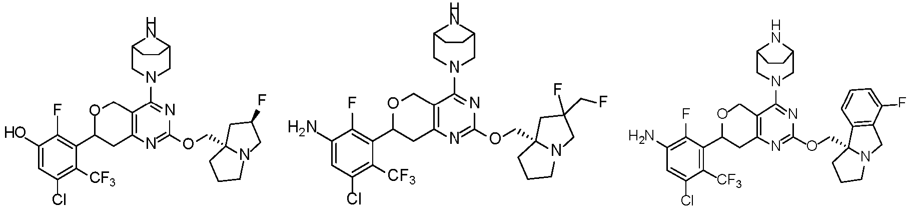

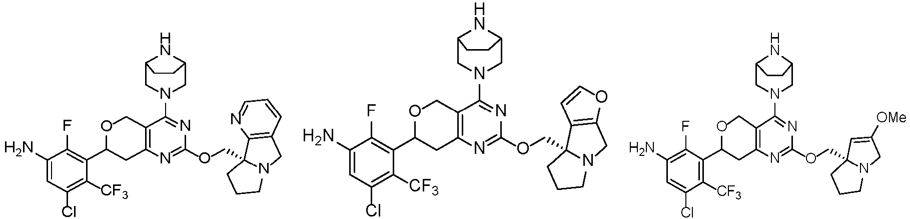

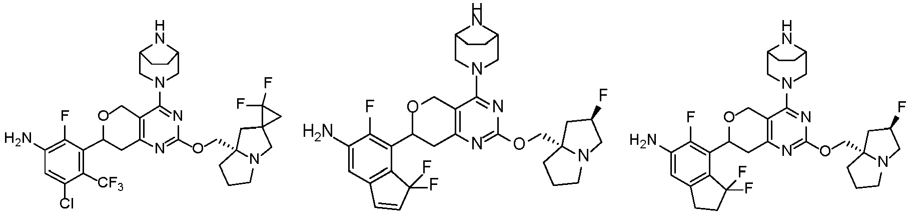

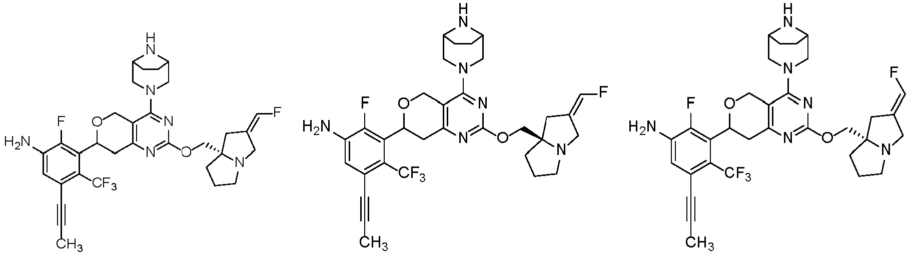

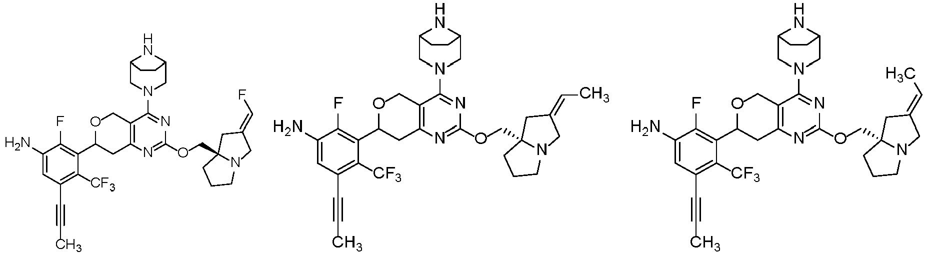

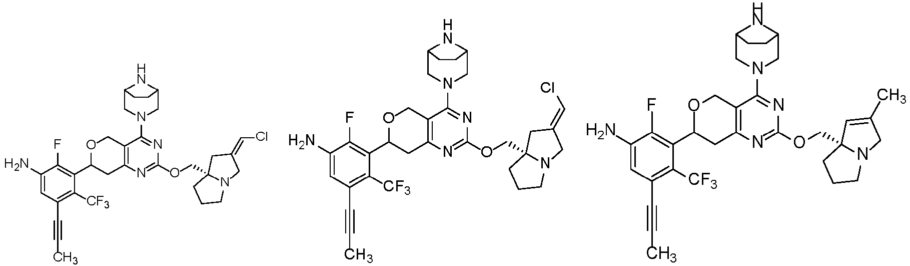

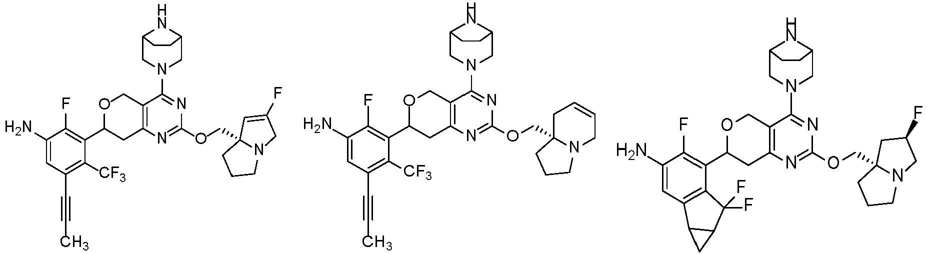

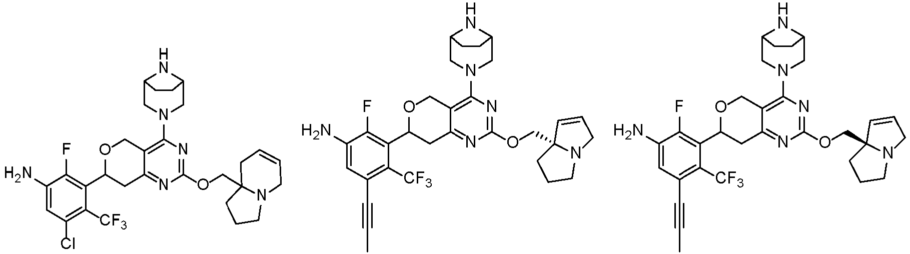

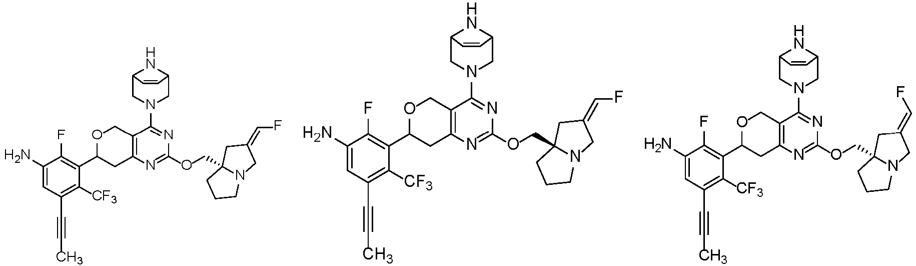

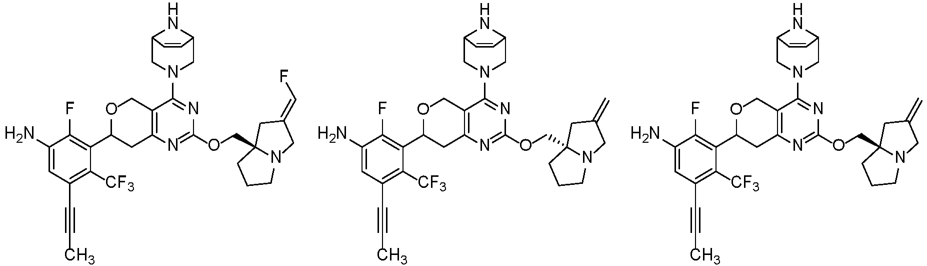

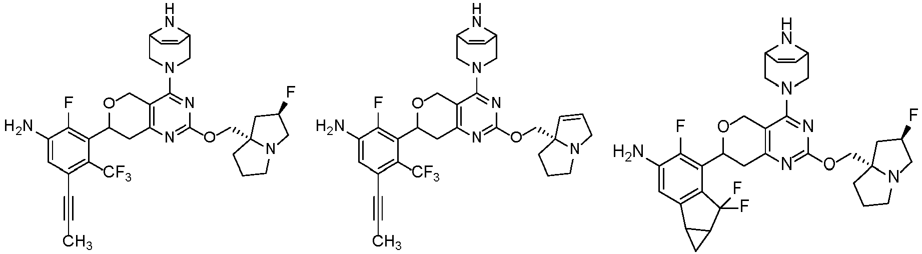

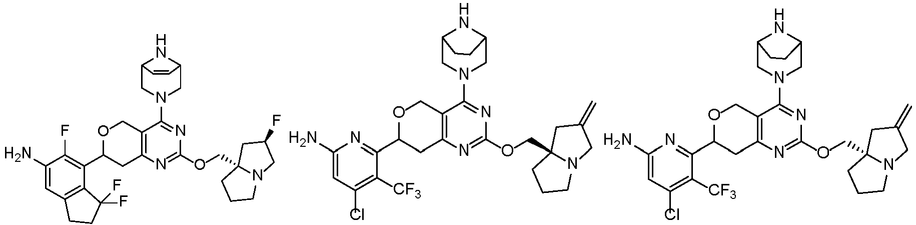

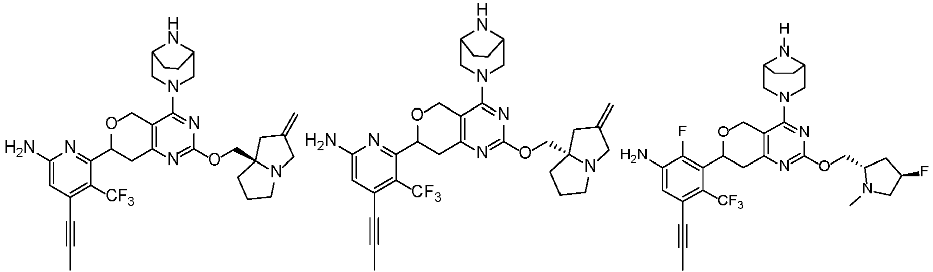

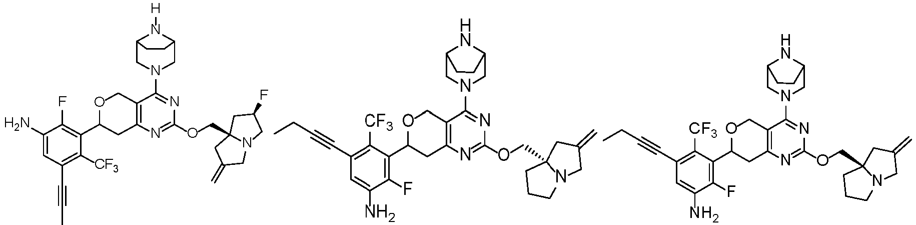

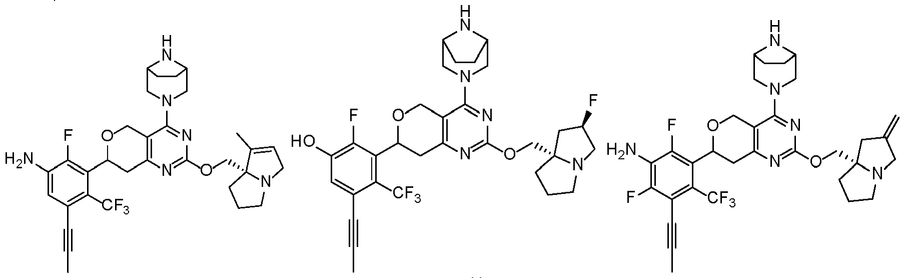

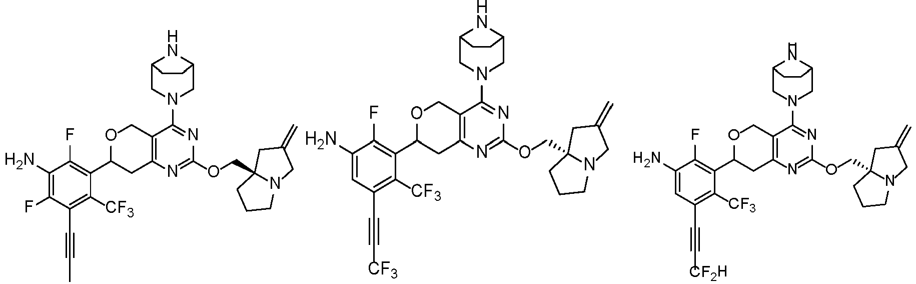

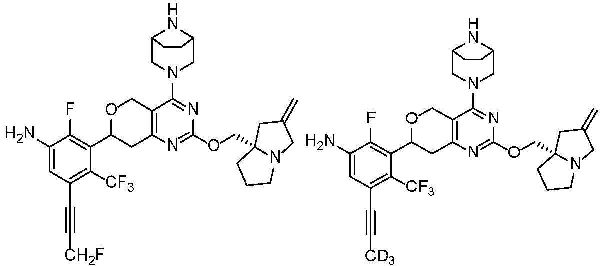

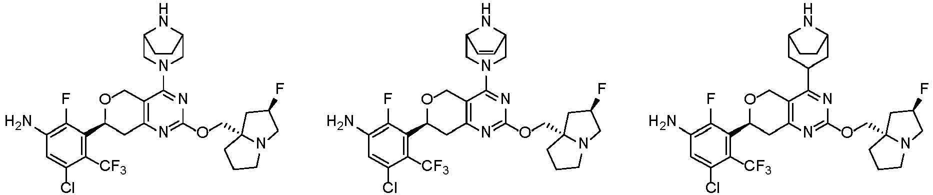

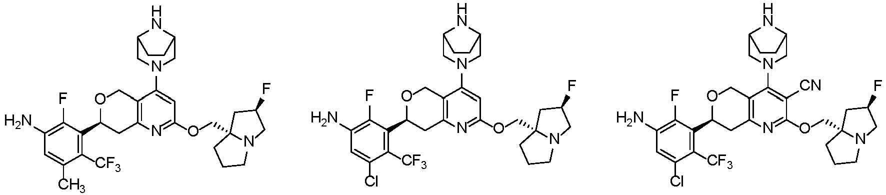

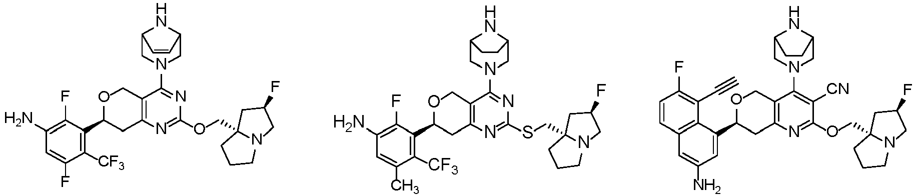

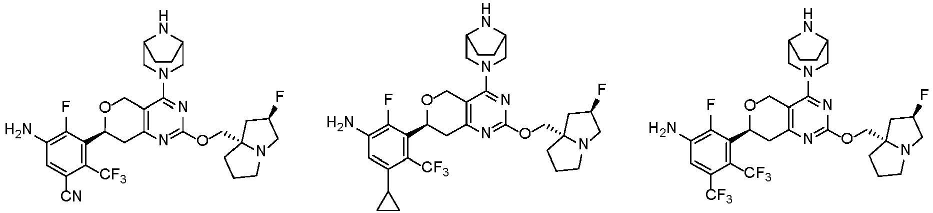

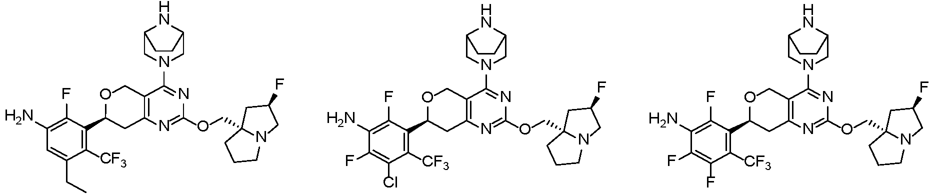

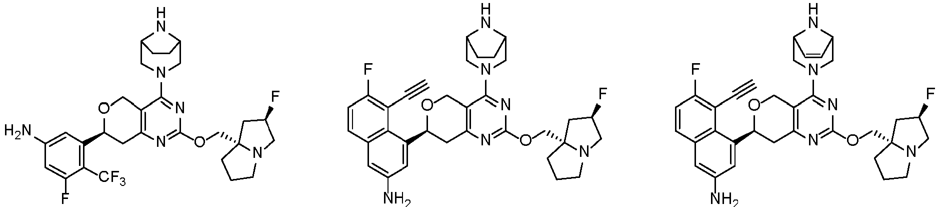

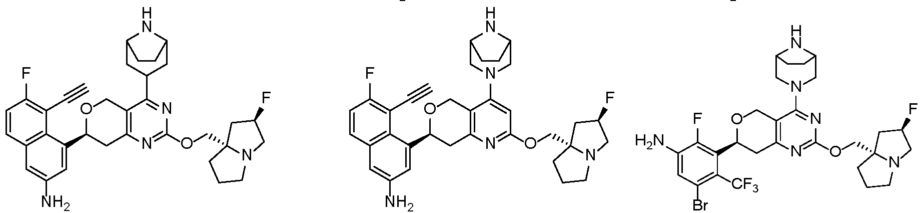

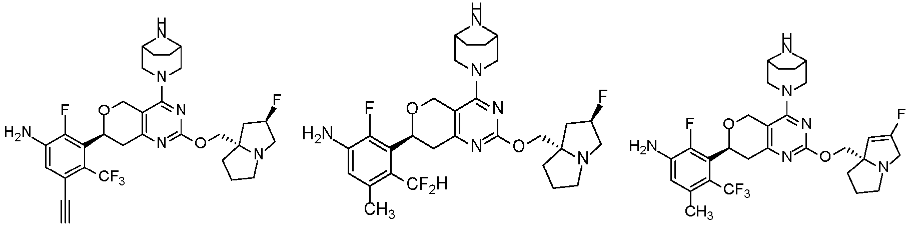

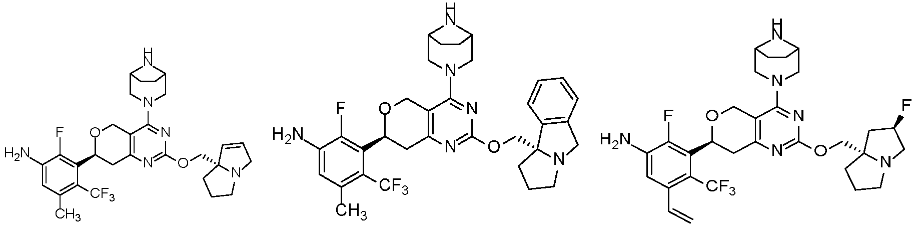

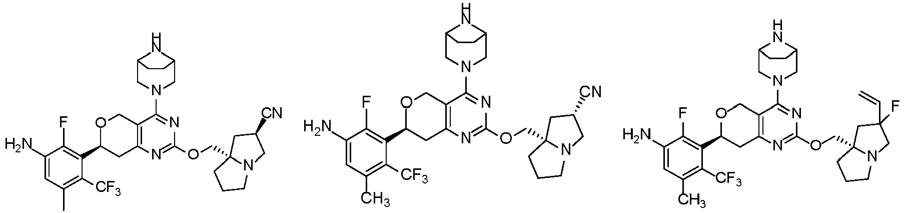

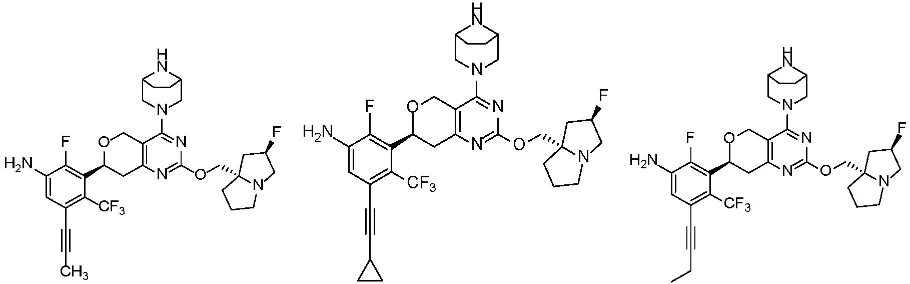

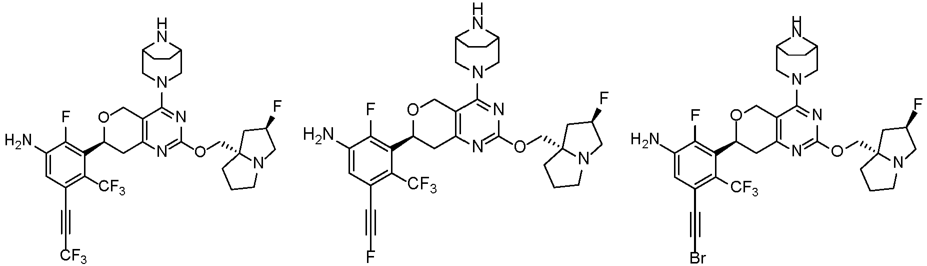

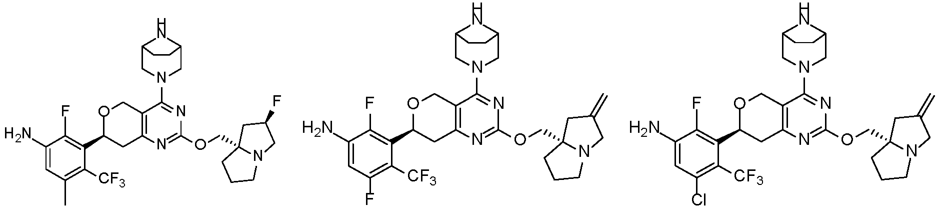

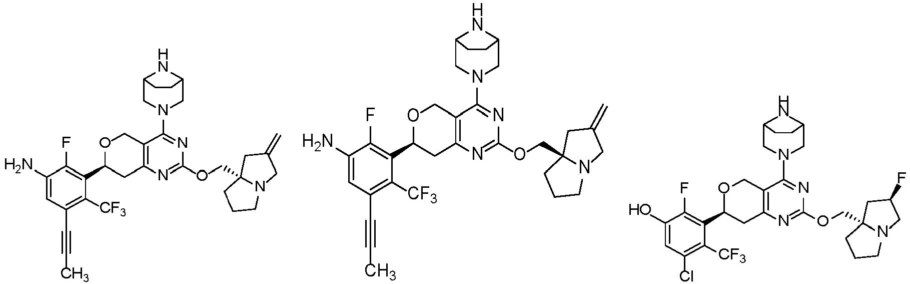

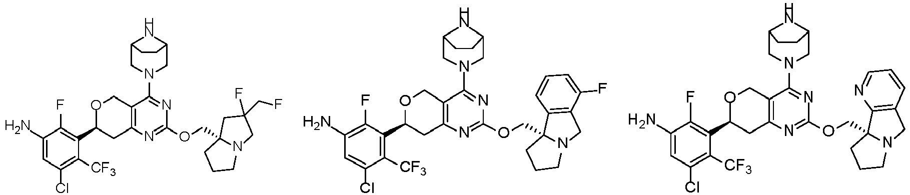

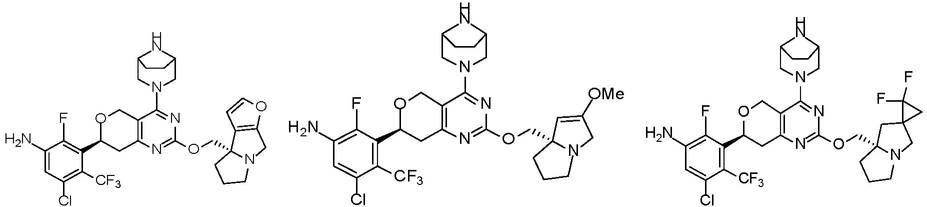

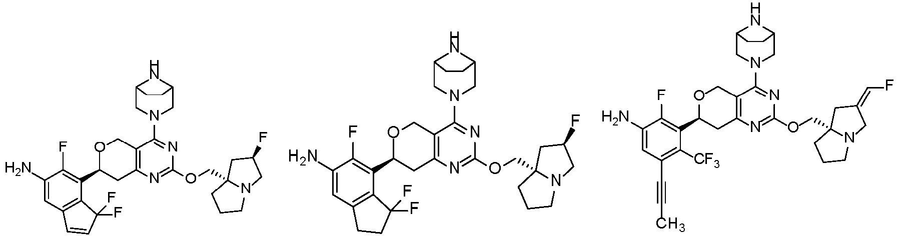

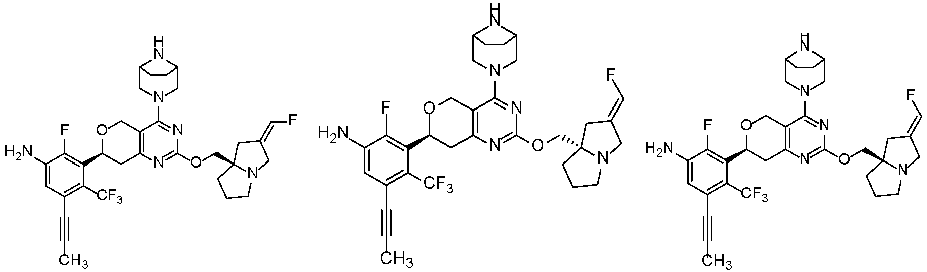

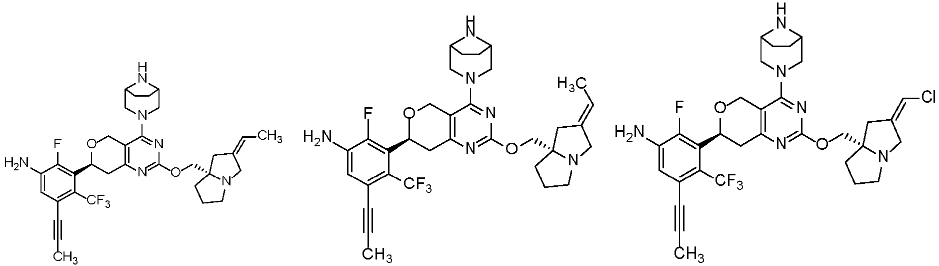

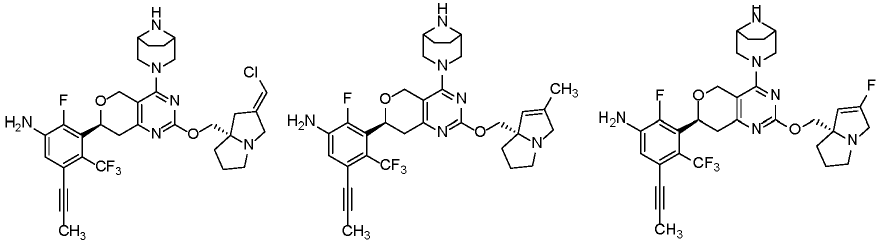

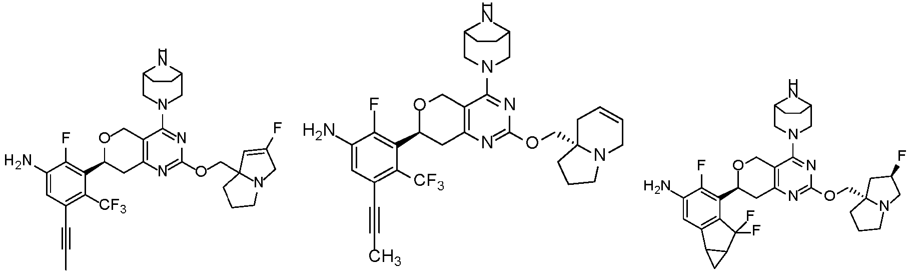

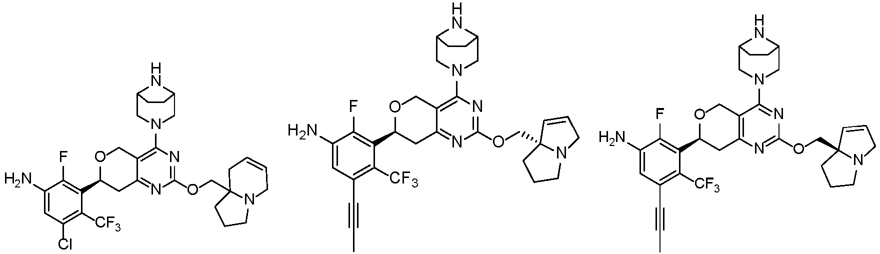

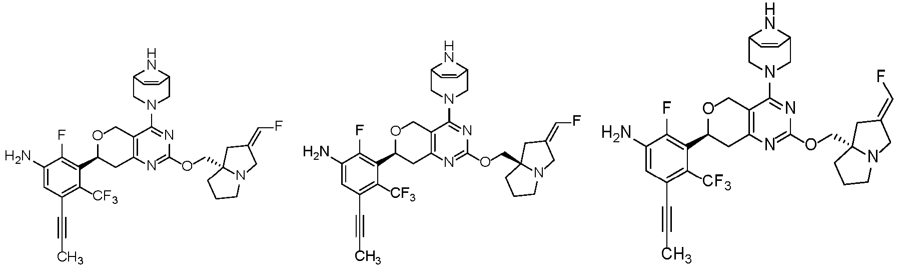

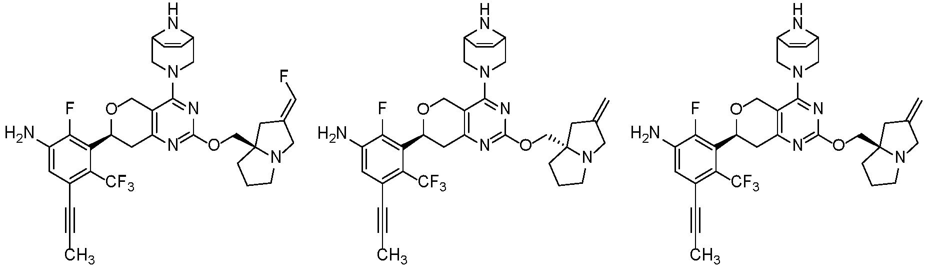

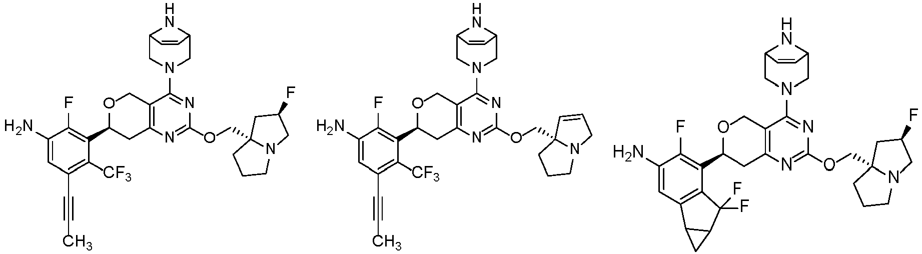

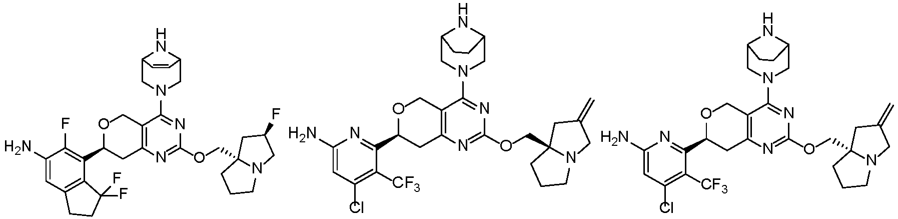

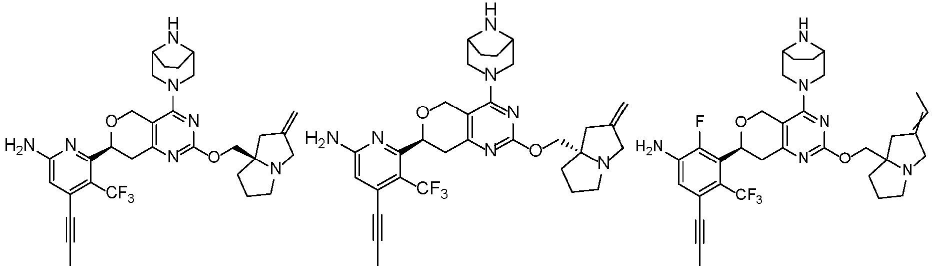

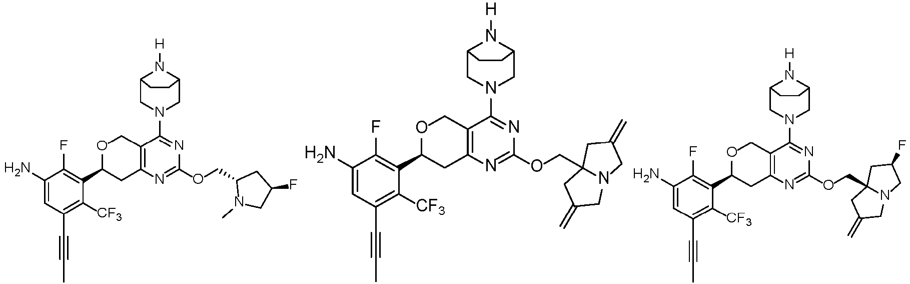

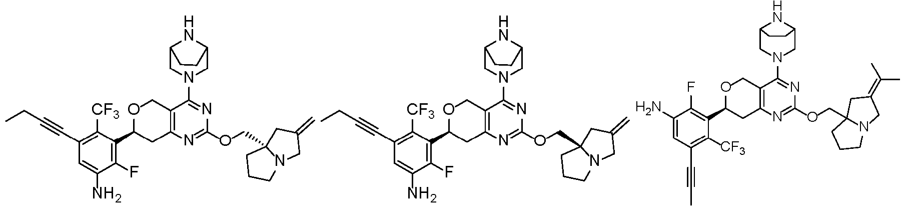

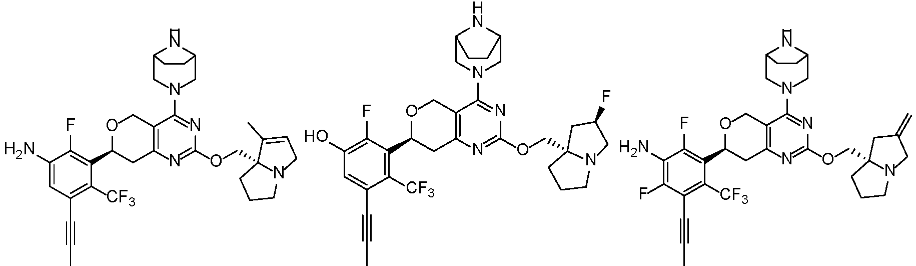

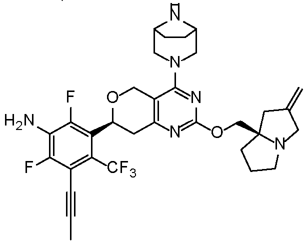

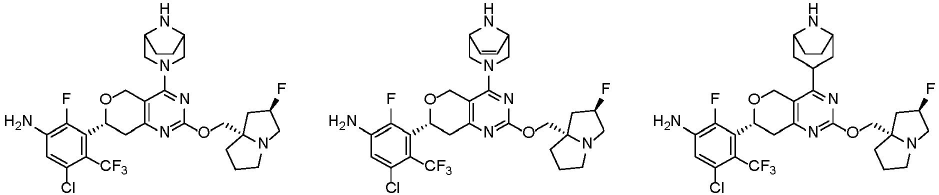

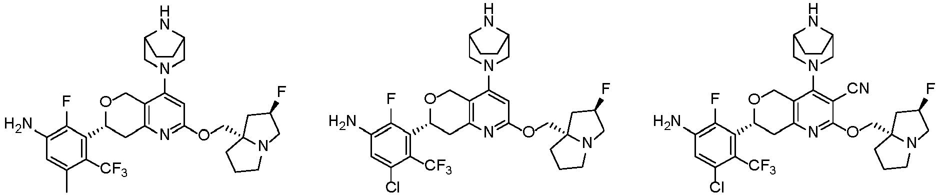

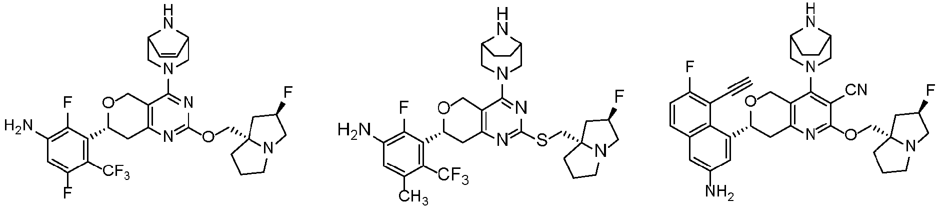

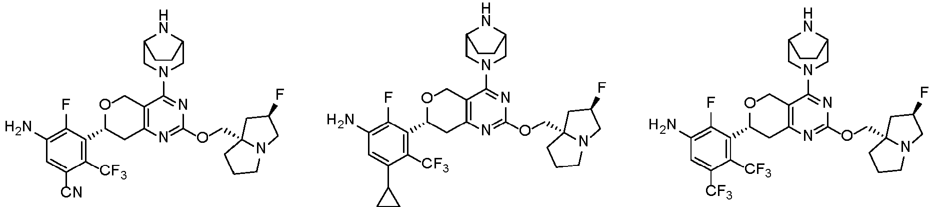

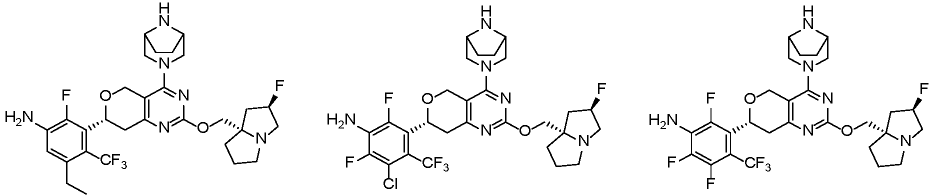

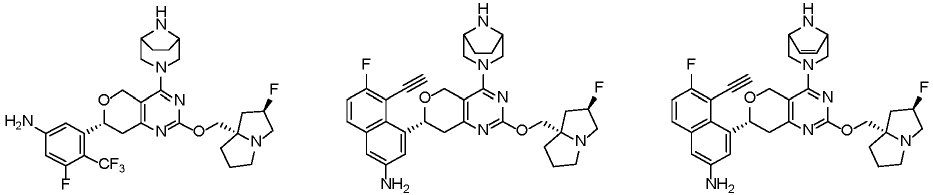

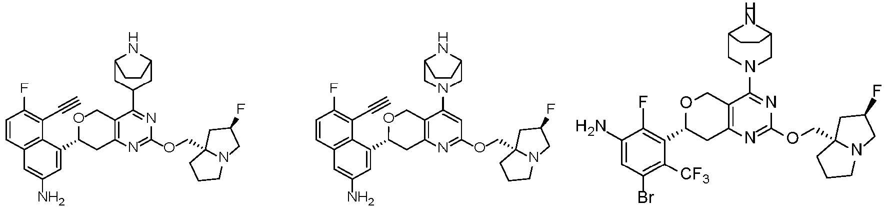

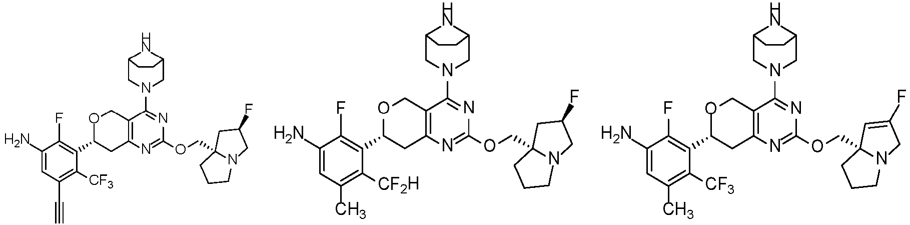

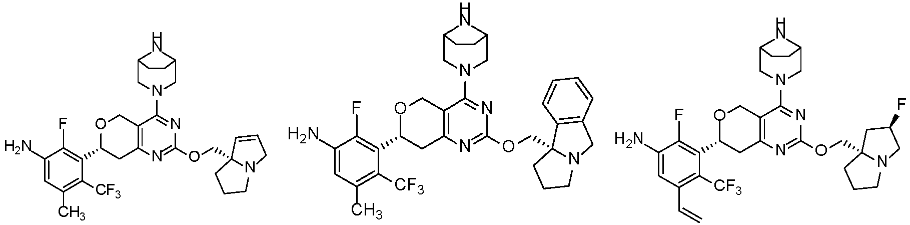

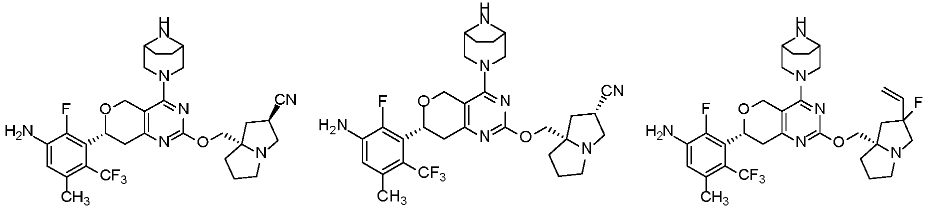

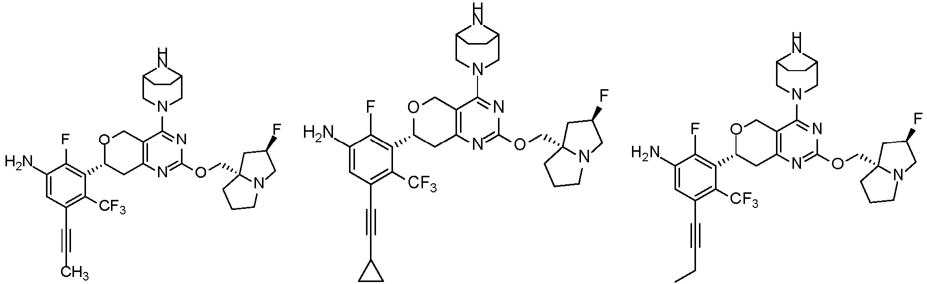

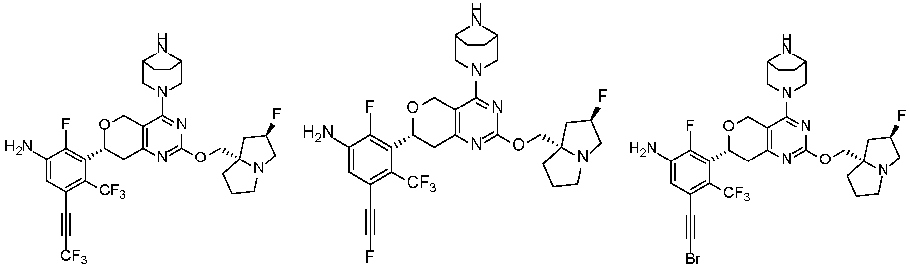

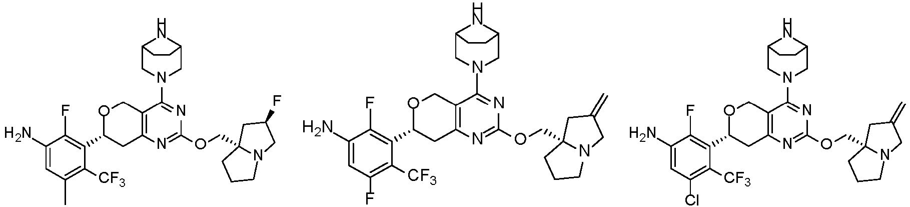

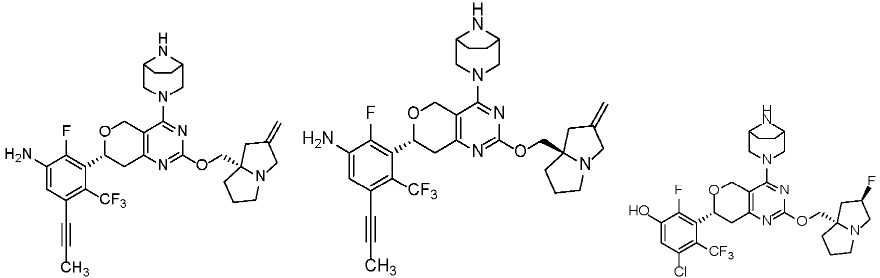

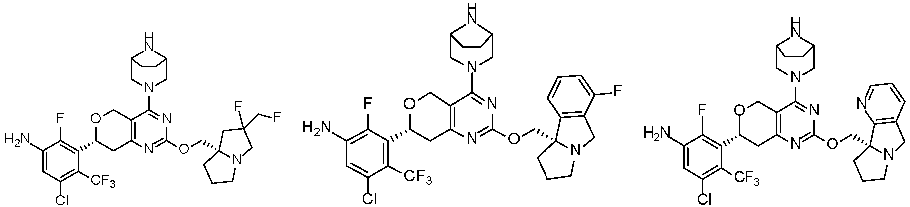

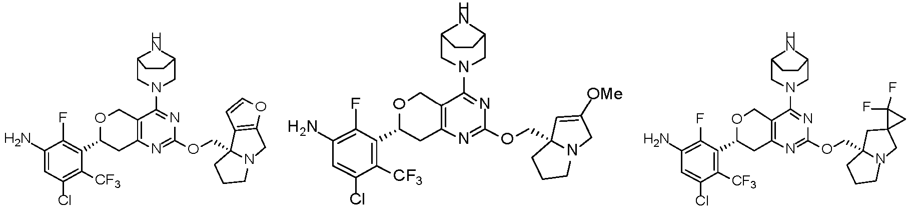

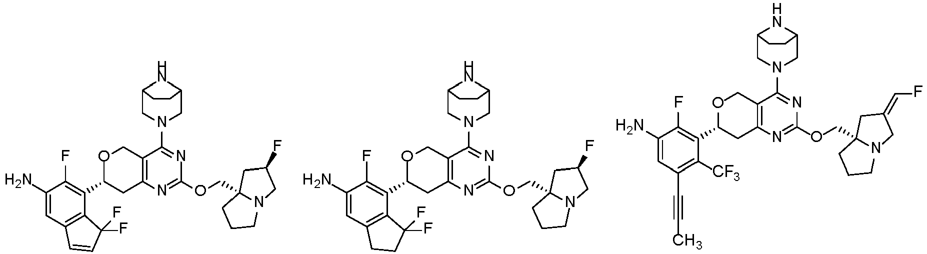

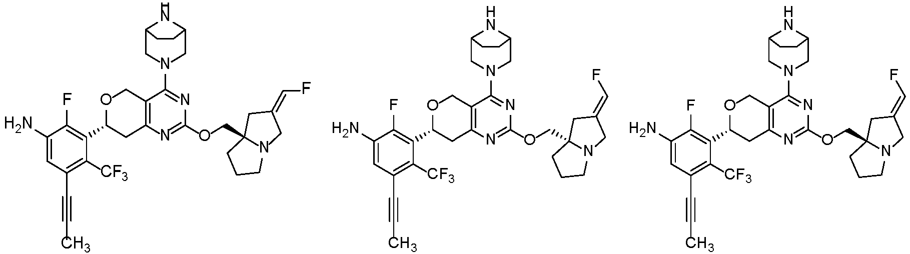

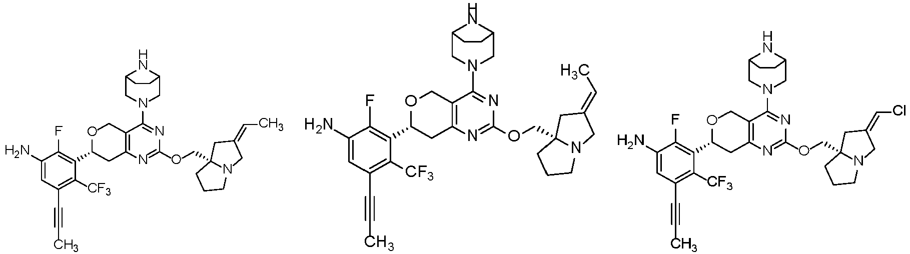

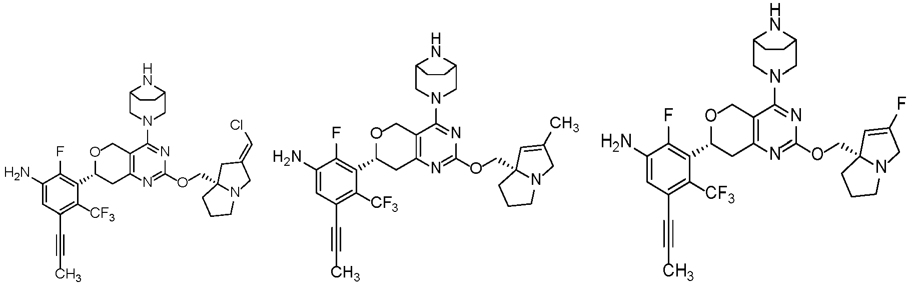

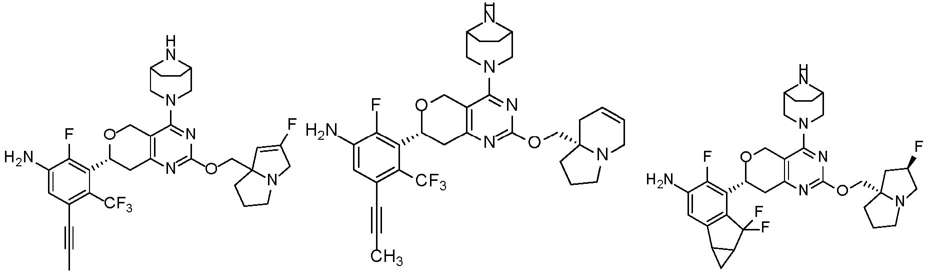

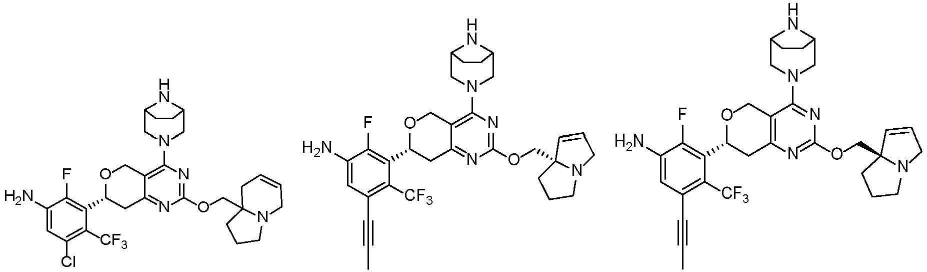

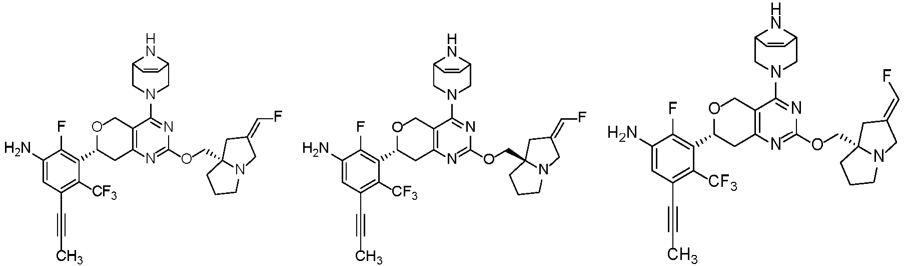

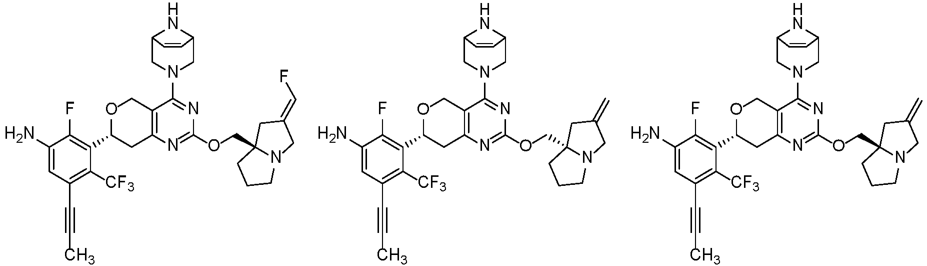

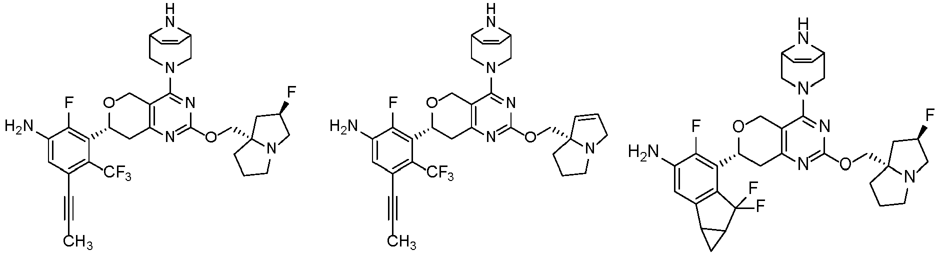

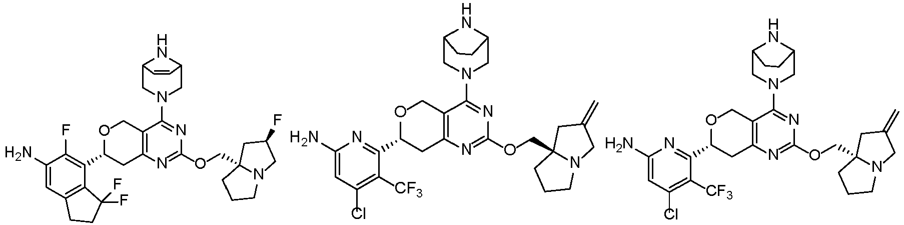

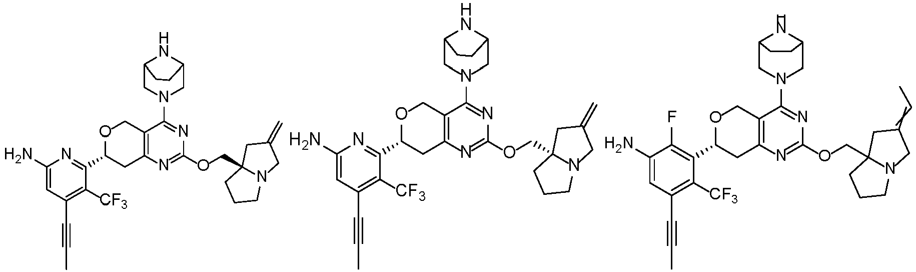

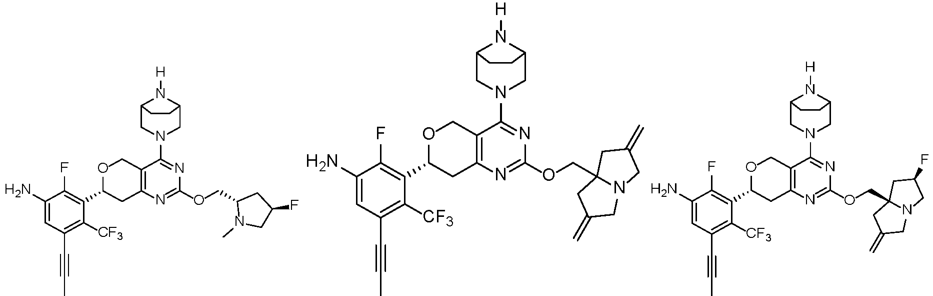

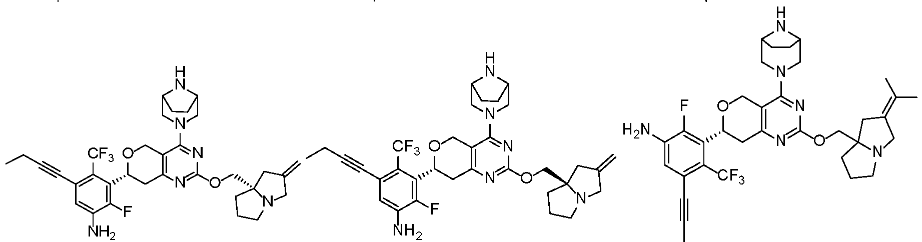

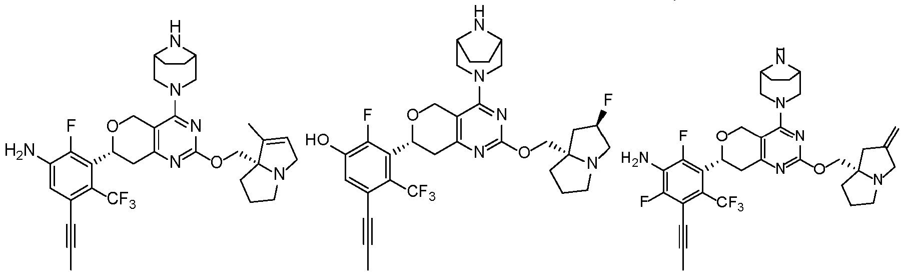

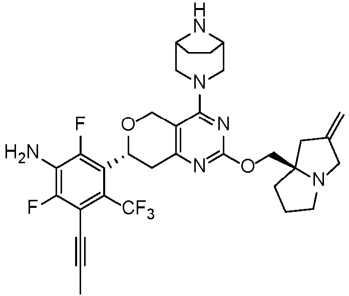

19. Use of the compound according to any one of claims 1-18 or a pharmaceutically acceptable salt thereof in the manufacture of a medicament for treating a disease associated with KRAS^{G12D} mutation.

20. Use of the compound according to any one of claims 1-19 or a pharmaceutically acceptable salt thereof in the manufacture of a medicament for treating a disease associated with a tumor.
